# EUROPÄISCHE PATENTANMELDUNG

(11) **EP 1 696 032 A1**
(43) Veröffentlichungstag der Anmeldung: **30.08.2006**
(21) Anmeldenummer: 05090042.2
(22) Anmeldetag: 23.02.2005
(51) Int. Cl.: C12N 15/54, C12N 9/10, C12P 19/04, C12N 15/80

(54) **Verfahren und Mittel zur Herstellung von Hyaluronan in Pilzen**

(71) Anmelder: Bayer CropScience GmbH, 65929 Frankfurt/Main (DE)
(72) Erfinder: Quanz, Martin, 10997 Berlin (DE)
(74) Vertreter: Kossmann, Jochen

(57) **Zusammenfassung**

Die vorliegende Erfindung betrifft Pilzzellen und Pilze, die Hyaluronan synthetisieren, sowie Verfahren zur Herstellung solcher Pilze, als auch Verfahren zur Herstellung von Hyaluronan mit Hilfe dieser Pilzzellen oder Pilze. Weiterhin betrifft die vorliegende Erfindung die Verwendung von Pilzen zur Herstellung von Hyaluronan und Lebens- oder Futtermitteln, die Hyaluronan enthalten.

SEQUENCE LISTING

<110>  Bayer CropScience GmbH
       Bayer CropScience GmbH

<120>  Verfahren und Mittel zur Herstellung von Hyaluronan

<130>  BCS 05-5001-EP

<160>  65

<170>  PatentIn version 3.1

<210>  1
<211>  1707
<212>  DNA
<213>  Paramecium bursaria Chlorella Virus 1

<220>
<221>  CDS
<222>  (1)..(1707)
<223>

<300>
<308>  PB42580
<309>  1995-12-24
<313>  (50903)..(52609)

<400>  1
atg ggt aaa aat ata atc ata atg gtt tcg tgg tac acc atc ata act      48
Met Gly Lys Asn Ile Ile Ile Met Val Ser Trp Tyr Thr Ile Ile Thr
1               5                   10                  15

tca aat cta atc gcg gtt gga gga gcc tct cta atc ttg gct ccg gca      96
Ser Asn Leu Ile Ala Val Gly Gly Ala Ser Leu Ile Leu Ala Pro Ala
            20                  25                  30

att act ggg tat gtt cta cat tgg aat att gct ctc tcg aca atc tgg      144
Ile Thr Gly Tyr Val Leu His Trp Asn Ile Ala Leu Ser Thr Ile Trp
        35                  40                  45

gga gta tca gct tat ggt att ttc gtt ttt ggg ttt ttc ctt gca caa 192
Gly Val Ser Ala Tyr Gly Ile Phe Val Phe Gly Phe Phe Leu Ala Gln
    50                  55                  60

gtt tta ttt tca gaa ctg aac agg aaa cgt ctt cgc aag tgg att tct 240
Val Leu Phe Ser Glu Leu Asn Arg Lys Arg Leu Arg Lys Trp Ile Ser
65                  70                  75                  80

ctc aga cct aag ggt tgg aat gat gtt cgt ttg gct gtg atc att gct 288
Leu Arg Pro Lys Gly Trp Asn Asp Val Arg Leu Ala Val Ile Ile Ala
                85                  90                  95

gga tat cgc gag gat cct tat atg ttc cag aag tgc ctc gag tct gta 336
Gly Tyr Arg Glu Asp Pro Tyr Met Phe Gln Lys Cys Leu Glu Ser Val
            100                 105                 110

cgt gac tct gat tat ggc aac gtt gcc cgt ctg att tgt gtg att gac 384
Arg Asp Ser Asp Tyr Gly Asn Val Ala Arg Leu Ile Cys Val Ile Asp
        115                 120                 125

ggt gat gag gac gat gat atg agg atg gct gcc gtt tac aag gcg atc 432
Gly Asp Glu Asp Asp Asp Met Arg Met Ala Ala Val Tyr Lys Ala Ile
    130                 135                 140

tac aat gat aat atc aag aag ccc gag ttt gtt ctg tgt gag tca gac 480
Tyr Asn Asp Asn Ile Lys Lys Pro Glu Phe Val Leu Cys Glu Ser Asp
145                 150                 155                 160

gac aag gaa ggt gaa cgc atc gac tct gat ttc tct cgc gac att tgt 528
Asp Lys Glu Gly Glu Arg Ile Asp Ser Asp Phe Ser Arg Asp Ile Cys
                165                 170                 175

gtc ctc cag cct cat cgt gga aaa cgg gag tgt ctt tat act ggg ttt 576
Val Leu Gln Pro His Arg Gly Lys Arg Glu Cys Leu Tyr Thr Gly Phe
            180                 185                 190

caa ctt gca aag atg gac ccc agt gtc aat gct gtc gtt ctg att gac 624
Gln Leu Ala Lys Met Asp Pro Ser Val Asn Ala Val Val Leu Ile Asp
        195                 200                 205

agc gat acc gtt ctc gag aag gat gct att ctg gaa gtt gta tac cca 672
Ser Asp Thr Val Leu Glu Lys Asp Ala Ile Leu Glu Val Val Tyr Pro
    210                 215                 220

ctt gca tgc gat ccc gag atc caa gcc gtt gca ggt gag tgt aag att 720
Leu Ala Cys Asp Pro Glu Ile Gln Ala Val Ala Gly Glu Cys Lys Ile
225                 230                 235                 240

tgg aac aca gac act ctt ttg agt ctt ctc gtc gct tgg cgg tac tat 768
Trp Asn Thr Asp Thr Leu Leu Ser Leu Leu Val Ala Trp Arg Tyr Tyr
                245                 250                 255

tct gcg ttt tgt gtg gag agg agt gcc cag tct ttt ttc agg act gtt 816
Ser Ala Phe Cys Val Glu Arg Ser Ala Gln Ser Phe Phe Arg Thr Val
            260                 265                 270

cag tgc gtt ggg ggg cca ctg ggt gcc tac aag att gat atc att aag 864
Gln Cys Val Gly Gly Pro Leu Gly Ala Tyr Lys Ile Asp Ile Ile Lys
        275                 280                 285

gag att aag gac ccc tgg att tcc cag cgc ttt ctt ggt cag aag tgt 912
Glu Ile Lys Asp Pro Trp Ile Ser Gln Arg Phe Leu Gly Gln Lys Cys
    290                 295                 300

act tac ggt gac gac cgc cgg cta acc aac gag atc ttg atg cgt ggt 960
Thr Tyr Gly Asp Asp Arg Arg Leu Thr Asn Glu Ile Leu Met Arg Gly
305                 310                 315                 320

aaa aag gtt gtg ttc act cca ttt gct gtt ggt tgg tct gac agt ccg     1008
Lys Lys Val Val Phe Thr Pro Phe Ala Val Gly Trp Ser Asp Ser Pro
                325                 330                 335

acc aat gtg ttt cgg tac atc gtt cag cag acc cgc tgg agt aag tcg     1056
Thr Asn Val Phe Arg Tyr Ile Val Gln Gln Thr Arg Trp Ser Lys Ser
            340                 345                 350

tgg tgc cgc gaa att tgg tac acc ctc ttc gcc gcg tgg aag cac ggt     1104
Trp Cys Arg Glu Ile Trp Tyr Thr Leu Phe Ala Ala Trp Lys His Gly
        355                 360                 365

ttg tct gga att tgg ctg gcc ttt gaa tgt ttg tat caa att aca tac     1152
Leu Ser Gly Ile Trp Leu Ala Phe Glu Cys Leu Tyr Gln Ile Thr Tyr
    370                 375                 380

ttc ttc ctc gtg att tac ctc ttt tct cgc cta gcc gtt gag gcc gac     1200
Phe Phe Leu Val Ile Tyr Leu Phe Ser Arg Leu Ala Val Glu Ala Asp
385                 390                 395                 400

cct cgc gcc cag aca gcc acg gtg att gtg agc acc acg gtt gca ttg     1248
Pro Arg Ala Gln Thr Ala Thr Val Ile Val Ser Thr Thr Val Ala Leu
                405                 410                 415

att aag tgt ggg tat ttt tca ttc cga gcc aag gat att cgg gcg ttt     1296
Ile Lys Cys Gly Tyr Phe Ser Phe Arg Ala Lys Asp Ile Arg Ala Phe
            420                 425                 430

tac ttt gtg ctt tat aca ttt gtt tac ttt ttc tgt atg att ccg gcc     1344
Tyr Phe Val Leu Tyr Thr Phe Val Tyr Phe Phe Cys Met Ile Pro Ala
        435                 440                 445

agg att act gca atg atg acg ctt tgg gac att ggc tgg ggt act cgc     1392
Arg Ile Thr Ala Met Met Thr Leu Trp Asp Ile Gly Trp Gly Thr Arg
    450                 455                 460

ggt gga aac gag aag cct tcc gtt ggc acc cgg gtc gct ctg tgg gca     1440
Gly Gly Asn Glu Lys Pro Ser Val Gly Thr Arg Val Ala Leu Trp Ala
465                 470                 475                 480

aag caa tat ctc att gca tat atg tgg tgg gcc gcg gtt gtt ggc gct     1488
Lys Gln Tyr Leu Ile Ala Tyr Met Trp Trp Ala Ala Val Val Gly Ala
                485                 490                 495

gga gtt tac agc atc gtc cat aac tgg atg ttc gat tgg aat tct ctt     1536
Gly Val Tyr Ser Ile Val His Asn Trp Met Phe Asp Trp Asn Ser Leu
            500                 505                 510

tct tat cgt ttt gct ttg gtt ggt att tgt tct tac att gtt ttt att     1584
Ser Tyr Arg Phe Ala Leu Val Gly Ile Cys Ser Tyr Ile Val Phe Ile
        515                 520                 525

gtt att gtg ctg gtg gtt tat ttc acc ggc aaa att acg act tgg aat     1632
Val Ile Val Leu Val Val Tyr Phe Thr Gly Lys Ile Thr Thr Trp Asn
    530                 535                 540

ttc acg aag ctt cag aag gag cta atc gag gat cgc gtt ctg tac gat     1680
Phe Thr Lys Leu Gln Lys Glu Leu Ile Glu Asp Arg Val Leu Tyr Asp
545                 550                 555                 560

gca act acc aat gct cag tct gtg tga                                 1707
Ala Thr Thr Asn Ala Gln Ser Val
                565

<210>  2

<211>  568

<212>  PRT

<213>  Paramecium bursaria Chlorella Virus 1

<400>  2

Met Gly Lys Asn Ile Ile Ile Met Val Ser Trp Tyr Thr Ile Ile Thr
1               5                   10                  15

Ser Asn Leu Ile Ala Val Gly Gly Ala Ser Leu Ile Leu Ala Pro Ala
            20                  25                  30

Ile Thr Gly Tyr Val Leu His Trp Asn Ile Ala Leu Ser Thr Ile Trp
        35                  40                  45

Gly Val Ser Ala Tyr Gly Ile Phe Val Phe Gly Phe Phe Leu Ala Gln
    50                  55                  60

Val Leu Phe Ser Glu Leu Asn Arg Lys Arg Leu Arg Lys Trp Ile Ser
65                  70                  75                  80

Leu Arg Pro Lys Gly Trp Asn Asp Val Arg Leu Ala Val Ile Ile Ala
                85                  90                  95

Gly Tyr Arg Glu Asp Pro Tyr Met Phe Gln Lys Cys Leu Glu Ser Val
            100                 105                 110

Arg Asp Ser Asp Tyr Gly Asn Val Ala Arg Leu Ile Cys Val Ile Asp
        115                 120                 125

Gly Asp Glu Asp Asp Asp Met Arg Met Ala Ala Val Tyr Lys Ala Ile
    130                 135                 140

Tyr Asn Asp Asn Ile Lys Lys Pro Glu Phe Val Leu Cys Glu Ser Asp
145                 150                 155                 160

Asp Lys Glu Gly Glu Arg Ile Asp Ser Asp Phe Ser Arg Asp Ile Cys
                165                 170                 175

Val Leu Gln Pro His Arg Gly Lys Arg Glu Cys Leu Tyr Thr Gly Phe
            180                 185                 190

Gln Leu Ala Lys Met Asp Pro Ser Val Asn Ala Val Val Leu Ile Asp
        195                 200                 205

Ser Asp Thr Val Leu Glu Lys Asp Ala Ile Leu Glu Val Val Tyr Pro
    210                 215                 220

Leu Ala Cys Asp Pro Glu Ile Gln Ala Val Ala Gly Glu Cys Lys Ile
225                 230                 235                 240

Trp Asn Thr Asp Thr Leu Leu Ser Leu Leu Val Ala Trp Arg Tyr Tyr
                245                 250                 255

Ser Ala Phe Cys Val Glu Arg Ser Ala Gln Ser Phe Phe Arg Thr Val
            260                 265                 270

Gln Cys Val Gly Gly Pro Leu Gly Ala Tyr Lys Ile Asp Ile Ile Lys
        275                 280                 285

Glu Ile Lys Asp Pro Trp Ile Ser Gln Arg Phe Leu Gly Gln Lys Cys
    290                 295                 300

Thr Tyr Gly Asp Asp Arg Arg Leu Thr Asn Glu Ile Leu Met Arg Gly
305                 310                 315                 320

Lys Lys Val Val Phe Thr Pro Phe Ala Val Gly Trp Ser Asp Ser Pro
                325                 330                 335

Thr Asn Val Phe Arg Tyr Ile Val Gln Gln Thr Arg Trp Ser Lys Ser
            340                 345                 350

Trp Cys Arg Glu Ile Trp Tyr Thr Leu Phe Ala Ala Trp Lys His Gly
        355                 360                 365

Leu Ser Gly Ile Trp Leu Ala Phe Glu Cys Leu Tyr Gln Ile Thr Tyr
    370                 375                 380

Phe Phe Leu Val Ile Tyr Leu Phe Ser Arg Leu Ala Val Glu Ala Asp
385                 390                 395                 400

Pro Arg Ala Gln Thr Ala Thr Val Ile Val Ser Thr Thr Val Ala Leu
                405                 410                 415

Ile Lys Cys Gly Tyr Phe Ser Phe Arg Ala Lys Asp Ile Arg Ala Phe
            420                 425                 430

Tyr Phe Val Leu Tyr Thr Phe Val Tyr Phe Phe Cys Met Ile Pro Ala
        435                 440                 445

Arg Ile Thr Ala Met Met Thr Leu Trp Asp Ile Gly Trp Gly Thr Arg
    450                 455                 460

Gly Gly Asn Glu Lys Pro Ser Val Gly Thr Arg Val Ala Leu Trp Ala
465                 470                 475                 480

Lys Gln Tyr Leu Ile Ala Tyr Met Trp Trp Ala Ala Val Val Gly Ala
                485                 490                 495

Gly Val Tyr Ser Ile Val His Asn Trp Met Phe Asp Trp Asn Ser Leu
            500                 505                 510

Ser Tyr Arg Phe Ala Leu Val Gly Ile Cys Ser Tyr Ile Val Phe Ile
        515                 520                 525

Val Ile Val Leu Val Val Tyr Phe Thr Gly Lys Ile Thr Thr Trp Asn
    530                 535                 540

Phe Thr Lys Leu Gln Lys Glu Leu Ile Glu Asp Arg Val Leu Tyr Asp
545                 550                 555                 560

Ala Thr Thr Asn Ala Gln Ser Val
                565

<210>  3

<211>  1707

<212>  DNA

<213>  Artificial sequence

<220>

<223>  Synthetic nucleotide sequence coding for a Paramecium bursaria Ch
       lorella Virus 1 Hyaluronansynthase protein

<220>

<221>  CDS

<222>  (1)..(1707)

<223>

<400>  3
atg ggt aag aac att atc att atg gtg tcc tgg tac aca att att aca       48
Met Gly Lys Asn Ile Ile Ile Met Val Ser Trp Tyr Thr Ile Ile Thr
1               5                   10                  15

agt aat ctc atc gca gtt ggt ggt gca tct ctt att ctc gct cca gct       96
Ser Asn Leu Ile Ala Val Gly Gly Ala Ser Leu Ile Leu Ala Pro Ala
            20                  25                  30

atc act gga tat gtt ctt cac tgg aac atc gcc ctc tca act att tgg      144
Ile Thr Gly Tyr Val Leu His Trp Asn Ile Ala Leu Ser Thr Ile Trp
        35                  40                  45

gga gtt tcc gca tat ggt att ttt gtt ttc ggg ttc ttt ttg gct cag      192
Gly Val Ser Ala Tyr Gly Ile Phe Val Phe Gly Phe Phe Leu Ala Gln
    50                  55                  60

gtt ctg ttc tca gag ctc aat cgt aag aga ctc agg aag tgg att agc      240
Val Leu Phe Ser Glu Leu Asn Arg Lys Arg Leu Arg Lys Trp Ile Ser
65                  70                  75                  80

ctt aga cca aag ggg tgg aat gac gtt cgt ctc gct gtc att atc gct      288
Leu Arg Pro Lys Gly Trp Asn Asp Val Arg Leu Ala Val Ile Ile Ala
                85                  90                  95

ggc tac cgt gaa gat cct tac atg ttt caa aag tgc ttg gaa tca gtt 336
Gly Tyr Arg Glu Asp Pro Tyr Met Phe Gln Lys Cys Leu Glu Ser Val
            100                 105                 110

agg gat agt gat tat ggc aac gtc gct aga ctg atc tgt gtg att gat 384
Arg Asp Ser Asp Tyr Gly Asn Val Ala Arg Leu Ile Cys Val Ile Asp
        115                 120                 125

gga gat gag gac gac gat atg agg atg gca gct gtt tat aag gct atc 432
Gly Asp Glu Asp Asp Asp Met Arg Met Ala Ala Val Tyr Lys Ala Ile
    130                 135                 140

tat aat gat aac att aag aag cct gaa ttt gtt ctt tgc gag tct gat 480
Tyr Asn Asp Asn Ile Lys Lys Pro Glu Phe Val Leu Cys Glu Ser Asp
145                 150                 155                 160

gac aag gaa gga gaa cgg att gat tca gat ttc tca cgt gat atc tgc 528
Asp Lys Glu Gly Glu Arg Ile Asp Ser Asp Phe Ser Arg Asp Ile Cys
                165                 170                 175

gtt ctc caa cct cat cgt ggg aag cgt gaa tgt ctt tat aca ggt ttc 576
Val Leu Gln Pro His Arg Gly Lys Arg Glu Cys Leu Tyr Thr Gly Phe
            180                 185                 190

caa ctc gcc aaa atg gac cca tca gtg aac gct gtg gtt ctt atc gat 624
Gln Leu Ala Lys Met Asp Pro Ser Val Asn Ala Val Val Leu Ile Asp
        195                 200                 205

agt gat act gtg ctg gag aaa gat gct atc ttg gag gtt gtt tac cct 672
Ser Asp Thr Val Leu Glu Lys Asp Ala Ile Leu Glu Val Val Tyr Pro
    210                 215                 220

ctt gcc tgt gat cct gaa att caa gct gtg gct gga gag tgc aag atc 720
Leu Ala Cys Asp Pro Glu Ile Gln Ala Val Ala Gly Glu Cys Lys Ile
225                 230                 235                 240

tgg aac aca gat act ctt ctt tct ctg ctt gtc gca tgg aga tat tac 768
Trp Asn Thr Asp Thr Leu Leu Ser Leu Leu Val Ala Trp Arg Tyr Tyr
                245                 250                 255

tcc gca ttc tgt gtg gag agg agc gct caa tcc ttt ttc cgt acc gtt 816
Ser Ala Phe Cys Val Glu Arg Ser Ala Gln Ser Phe Phe Arg Thr Val
            260                 265                 270

caa tgc gtt ggt ggt cct ttg gga gct tac aaa att gat atc atc aag 864
Gln Cys Val Gly Gly Pro Leu Gly Ala Tyr Lys Ile Asp Ile Ile Lys
        275                 280                 285

gag att aag gac cca tgg att agt caa agg ttt ctt ggt cag aag tgc 912
Glu Ile Lys Asp Pro Trp Ile Ser Gln Arg Phe Leu Gly Gln Lys Cys
    290                 295                 300

act tat ggc gat gat cgt aga ttg act aac gaa atc ctt atg agg ggc 960
Thr Tyr Gly Asp Asp Arg Arg Leu Thr Asn Glu Ile Leu Met Arg Gly
305                 310                 315                 320

aag aaa gtc gtt ttt act cca ttt gct gtc gga tgg tct gat tca cct 1008
Lys Lys Val Val Phe Thr Pro Phe Ala Val Gly Trp Ser Asp Ser Pro
                325                 330                 335

aca aat gtt ttc cgt tat att gtg caa caa aca cgt tgg agt aag agc 1056
Thr Asn Val Phe Arg Tyr Ile Val Gln Gln Thr Arg Trp Ser Lys Ser
            340                 345                 350

tgg tgt agg gag atc tgg tac act ttg ttc gct gct tgg aag cac ggg 1104
Trp Cys Arg Glu Ile Trp Tyr Thr Leu Phe Ala Ala Trp Lys His Gly
        355                 360                 365

ctt agc gga att tgg ctt gct ttt gaa tgc ctt tac cag att aca tac 1152
Leu Ser Gly Ile Trp Leu Ala Phe Glu Cys Leu Tyr Gln Ile Thr Tyr
    370                 375                 380

ttt ttc ttg gtg atc tat ttg ttt tca cgt ctt gcc gtc gag gct gac 1200
Phe Phe Leu Val Ile Tyr Leu Phe Ser Arg Leu Ala Val Glu Ala Asp
385                 390                 395                 400

cct aga gca cag act gca act gtg att gtt tct act aca gtc gca ctt 1248
Pro Arg Ala Gln Thr Ala Thr Val Ile Val Ser Thr Thr Val Ala Leu
                405                 410                 415

att aag tgt ggc tat ttc agt ttt aga gca aaa gat att aga gcc ttc 1296
Ile Lys Cys Gly Tyr Phe Ser Phe Arg Ala Lys Asp Ile Arg Ala Phe
            420                 425                 430

tat ttt gtt ttg tac aca ttt gtt tat ttc ttt tgc atg att cca gct 1344
Tyr Phe Val Leu Tyr Thr Phe Val Tyr Phe Phe Cys Met Ile Pro Ala
        435                 440                 445

cgt att acc gct atg atg acc ttg tgg gac atc gga tgg gga act aga 1392
Arg Ile Thr Ala Met Met Thr Leu Trp Asp Ile Gly Trp Gly Thr Arg
    450                 455                 460

ggt ggt aac gaa aag cct tct gtg gga aca agg gtg gcc ctt tgg gca 1440
Gly Gly Asn Glu Lys Pro Ser Val Gly Thr Arg Val Ala Leu Trp Ala
465                 470                 475                 480

aaa caa tat ctc atc gcc tac atg tgg tgg gcc gct gtc gtt ggt gcc 1488
Lys Gln Tyr Leu Ile Ala Tyr Met Trp Trp Ala Ala Val Val Gly Ala
                485                 490                 495

gga gtg tac tca atc gtt cat aac tgg atg ttt gac tgg aac tct ttg 1536
Gly Val Tyr Ser Ile Val His Asn Trp Met Phe Asp Trp Asn Ser Leu
            500                 505                 510

agc tat cgt ttc gct ctt gtg ggt att tgt tct tac att gtt ttc atc 1584
Ser Tyr Arg Phe Ala Leu Val Gly Ile Cys Ser Tyr Ile Val Phe Ile
        515                 520                 525

gtg att gtg ctc gtt gtg tat ttc act ggt aaa atc aca acc tgg aat 1632
Val Ile Val Leu Val Val Tyr Phe Thr Gly Lys Ile Thr Thr Trp Asn
    530                 535                 540

ttc act aaa ctt caa aag gaa ttg att gaa gac agg gtt ctg tat gat 1680
Phe Thr Lys Leu Gln Lys Glu Leu Ile Glu Asp Arg Val Leu Tyr Asp
545                 550                 555                 560

gct act acc aac gcc cag tca gtt taa 1707
Ala Thr Thr Asn Ala Gln Ser Val
                565

<210> 4

<211> 568

<212> PRT

<213> Artificial sequence

<220>

<223> Synthetic nucleotide sequence coding for a Paramecium bursaria Ch
lorella Virus 1 Hyaluronansynthase protein

<400> 4

Met Gly Lys Asn Ile Ile Ile Met Val Ser Trp Tyr Thr Ile Ile Thr
1               5                   10                  15

Ser Asn Leu Ile Ala Val Gly Gly Ala Ser Leu Ile Leu Ala Pro Ala
            20                  25                  30

Ile Thr Gly Tyr Val Leu His Trp Asn Ile Ala Leu Ser Thr Ile Trp
        35                  40                  45

Gly Val Ser Ala Tyr Gly Ile Phe Val Phe Gly Phe Phe Leu Ala Gln
    50                  55                  60

Val Leu Phe Ser Glu Leu Asn Arg Lys Arg Leu Arg Lys Trp Ile Ser
65                  70                  75                  80

Leu Arg Pro Lys Gly Trp Asn Asp Val Arg Leu Ala Val Ile Ile Ala
                85                  90                  95

Gly Tyr Arg Glu Asp Pro Tyr Met Phe Gln Lys Cys Leu Glu Ser Val
            100                 105                 110

Arg Asp Ser Asp Tyr Gly Asn Val Ala Arg Leu Ile Cys Val Ile Asp
        115                 120                 125

Gly Asp Glu Asp Asp Asp Met Arg Met Ala Ala Val Tyr Lys Ala Ile
    130                 135                 140

Tyr Asn Asp Asn Ile Lys Lys Pro Glu Phe Val Leu Cys Glu Ser Asp
145                 150                 155                 160

Asp Lys Glu Gly Glu Arg Ile Asp Ser Asp Phe Ser Arg Asp Ile Cys
                165                 170                 175

Val Leu Gln Pro His Arg Gly Lys Arg Glu Cys Leu Tyr Thr Gly Phe
            180                 185                 190

Gln Leu Ala Lys Met Asp Pro Ser Val Asn Ala Val Val Leu Ile Asp
        195                 200                 205

Ser Asp Thr Val Leu Glu Lys Asp Ala Ile Leu Glu Val Val Tyr Pro
    210                 215                 220

Leu Ala Cys Asp Pro Glu Ile Gln Ala Val Ala Gly Glu Cys Lys Ile
225                 230                 235                 240

Trp Asn Thr Asp Thr Leu Leu Ser Leu Leu Val Ala Trp Arg Tyr Tyr
                245                 250                 255

Ser Ala Phe Cys Val Glu Arg Ser Ala Gln Ser Phe Phe Arg Thr Val
            260                 265                 270

Gln Cys Val Gly Gly Pro Leu Gly Ala Tyr Lys Ile Asp Ile Ile Lys
        275                 280                 285

Glu Ile Lys Asp Pro Trp Ile Ser Gln Arg Phe Leu Gly Gln Lys Cys
    290                 295                 300

Thr Tyr Gly Asp Asp Arg Arg Leu Thr Asn Glu Ile Leu Met Arg Gly
305                 310                 315                 320

Lys Lys Val Val Phe Thr Pro Phe Ala Val Gly Trp Ser Asp Ser Pro
                325                 330                 335

Thr Asn Val Phe Arg Tyr Ile Val Gln Gln Thr Arg Trp Ser Lys Ser
            340                 345                 350

Trp Cys Arg Glu Ile Trp Tyr Thr Leu Phe Ala Ala Trp Lys His Gly
        355                 360                 365

Leu Ser Gly Ile Trp Leu Ala Phe Glu Cys Leu Tyr Gln Ile Thr Tyr
    370                 375                 380

Phe Phe Leu Val Ile Tyr Leu Phe Ser Arg Leu Ala Val Glu Ala Asp
385                 390                 395                 400

Pro Arg Ala Gln Thr Ala Thr Val Ile Val Ser Thr Thr Val Ala Leu
                405                 410                 415

Ile Lys Cys Gly Tyr Phe Ser Phe Arg Ala Lys Asp Ile Arg Ala Phe
            420                 425                 430

Tyr Phe Val Leu Tyr Thr Phe Val Tyr Phe Phe Cys Met Ile Pro Ala
        435                 440                 445

Arg Ile Thr Ala Met Met Thr Leu Trp Asp Ile Gly Trp Gly Thr Arg
    450                 455                 460

Gly Gly Asn Glu Lys Pro Ser Val Gly Thr Arg Val Ala Leu Trp Ala
465                 470                 475                 480

Lys Gln Tyr Leu Ile Ala Tyr Met Trp Trp Ala Ala Val Val Gly Ala
                485                 490                 495

Gly Val Tyr Ser Ile Val His Asn Trp Met Phe Asp Trp Asn Ser Leu
            500                 505                 510

Ser Tyr Arg Phe Ala Leu Val Gly Ile Cys Ser Tyr Ile Val Phe Ile
        515                 520                 525

Val Ile Val Leu Val Val Tyr Phe Thr Gly Lys Ile Thr Thr Trp Asn
    530                 535                 540

Phe Thr Lys Leu Gln Lys Glu Leu Ile Glu Asp Arg Val Leu Tyr Asp
545                 550                 555                 560

Ala Thr Thr Asn Ala Gln Ser Val
                565

<210>  5

<211>  1662

<212>  DNA

<213>  Homo sapiens

<220>

<221>  CDS

<222>  (1)..(1662)

<223>

<400>  5
atg ccg gtg cag ctg acg aca gcc ctg cgt gtg gtg ggc acc agc ctg       48
ttt gcc ctg gca gtg ctg ggt ggc atc ctg gca gcc tat gtg acg ggc       96
tac cag ttc atc cac acg gaa aag cac tac ctg tcc ttc ggc ctg tac      144
ggc gcc atc ctg ggc ctg cac ctg ctc att cag agc ctt ttt gcc ttc      192
ctg gag cac cgg cgc atg caa cgt gcc ggc cag gcc ctg aag ctg ccc      240
tcc ccg cgg cgg ggc tcg gtg gca ctg tgc att gcc gca tac cag gag      288
gac cct gac tac ttg cgc aag tgc ctg cgc tcg gcc cag cgc atc tcc      336
ttc cct gac ctc aag gtg gtc atg gtg gtg gat ggc aac cgc cag gag      384
gac gcc tac atg ctg gac atc ttc cac gag gtg ctg ggc ggc acc gag      432
cag gcc ggc ttc ttt gtg tgg cgc agc aac ttc cat gag gca ggc gag      480
ggt gag acg gag gcc agc ctg cag gag ggc atg gac cgt gtg cgg gat      528
gtg gtg cgg gcc agc acc ttc tcg tgc atc atg cag aag tgg gga ggc      576
aag cgc gag gtc atg tac acg gcc ttc aag gcc ctc ggc gat tcg gtg      624
gac tac atc cag gtg tgc gac tct gac act gtg ctg gat cca gcc tgc      672
acc atc gag atg ctt cga gtc ctg gag gag gat ccc caa gta ggg gga      720
gtc ggg gga gat gtc cag atc ctc aac aag tac gac tca tgg att tcc      768
ttc ctg agc agc gtg cgg tac tgg atg gcc ttc aac gtg gag cgg gcc      816
tgc cag tcc tac ttt ggc tgt gtg cag tgt att agt ggg ccc ttg ggc      864
atg tac cgc aac agc ctc ctc cag cag ttc ctg gag gac tgg tac cat      912
cag aag ttc cta ggc agc aag tgc agc ttc ggg gat gac cgg cac ctc      960
acc aac cga gtc ctg agc ctt ggc tac cga act aag tat acc gcg cgc     1008
tcc aag tgc ctc aca gag acc ccc act aag tac ctc cgg tgg ctc aac     1056
cag caa acc cgc tgg agc aag tct tac ttc cgg gag tgg ctc tac aac     1104
tct ctg tgg ttc cat aag cac cac ctc tgg atg acc tac gag tca gtg     1152
gtc acg ggt ttc ttc ccc ttc ttc ctc att gcc acg gtt ata cag ctt     1200
ttc tac cgg ggc cgc atc tgg aac att ctc ctc ttc ctg ctg acg gtg     1248
cag ctg gtg ggc att atc aag gcc acc tac gcc tgc ttc ctt cgg ggc     1296
aat gca gag atg atc ttc atg tcc ctc tac tcc ctc ctc tat atg tcc      1344
agc ctt ctg ccg gcc aag atc ttt gcc att gct acc atc aac aaa tct      1392
ggc tgg ggc acc tct ggc cga aaa acc att gtg gtg aac ttc att ggc      1440
ctc att cct gtg tcc atc tgg gtg gca gtt ctc ctg gga ggg ctg gcc      1488
tac aca gct tat tgc cag gac ctg ttc agt gag aca gag cta gcc ttc      1536
ctt gtc tct ggg gct ata ctg tat ggc tgc tac tgg gtg gcc ctc ctc      1584
atg cta tat ctg gcc atc atc gcc cgg cga tgt ggg aag aag ccg gag      1632
cag tca agc ttg gct ttt gct gag gtg tga                              1662

<210>  6

<211>  553

<212>  PRT

<213>  Homo sapiens

<400>  6

<210>  7

<211>  1662

<212>  DNA

<213>  Artificial sequence

<220>

<223>  Synthetic nucleotide sequence coding for a Homo sapiens Hyalurona
       nsynthase-3 protein

<220>

<221>  CDS

<222>  (1)..(1662)

<223>

<400>  7
atg cct gtt cag ctg act aca gca ctt aga gtc gta ggt aca agc ttg      48
ttc gct ctg gca gtg ttg ggc ggt att cta gct gca tat gta act gga      96
tat cag ttc att cat act gag aag cac tac cta tca ttc gga tta tac      144
ggt gca att ttg ggt ttg cac ttg cta att caa tct ttg ttt gct ttt      192
ctt gag cat cgt aga atg caa aga gcc gga caa gct ttg aaa ctt cca      240
tct cca agg aga ggg agt gtg gca tta tgc atc gct gcc tac caa gaa      288
gat cca gat tat ctt cgt aag tgt tta aga tca gca caa agg ata tct      336
ttc cct gat cta aaa gtc gtt atg gtt gtg gat ggg aat aga caa gag      384
gat gct tat atg ttg gat ata ttc cat gaa gtt tta ggt gga aca gaa      432
cag gct ggt ttc ttt gtt tgg agg tct aac ttc cac gag gct ggc gag      480
gga gaa act gag gct tct ttg cag gag ggg atg gat aga gtc agg gat      528
gtg gtt cga gcc agt acc ttt tca tgc att atg caa aaa tgg ggt gga      576
aaa agg gaa gtg atg tac act gct ttt aag gct ttg ggt gac tcc gtt      624
gat tat atc cag gta tgc gac tca gac act gtt ttg gac cca gca tgt      672
acc att gaa atg ctc cgt gtt ctt gag gaa gat cca cag gtg ggg ggt      720
gta gga ggt gat gta cag ata ctc aac aaa tat gac agt tgg atc tca      768
ttc ctt tcc tct gtc agg tac tgg atg gca ttt aat gtt gaa aga gcc      816
tgc caa tct tat ttt ggc tgt gtt caa tgt att tct gga cct ttg ggt      864
atg tac aga aat agt tta ctt caa cag ttc ctg gaa gat tgg tat cac      912
caa aaa ttt cta ggg agt aag tgt tct ttt gga gac gat aga cat cta      960
aca aat cgt gtc ctc agt ctt ggc tac agg act aag tat acc gct aga     1008
agt aaa tgc ctg act gaa act cct aca aag tat ctg aga tgg tta aat     1056
cag caa act aga tgg tca aag tct tac ttc agg gaa tgg ttg tac aat     1104
tca tta tgg ttt cat aaa cat cat ctg tgg atg aca tac gaa tcc gtt     1152
gtc aca ggt ttt ttc cca ttt ttc tta att gca aca gtt att cag ctt     1200
ttt tat aga gga agg atc tgg aac att ctt ttg ttt ctc ctt aca gtt     1248
caa ttg gtg ggc att ata aag gct aca tat gca tgt ttt ctc agg gga     1296
aac gca gaa atg att ttc atg tca ctt tac agc ctg tta tat atg tcc     1344
tca ctt tta ccc gca aag atc ttt gct att gct acc ata aat aag tct     1392
ggg tgg gga act tct gga cga aag aca atc gtt gtg aat ttt att ggc     1440
ttg ata ccc gtt tca att tgg gta gct gtt ctt ctc ggt gga ctt gca     1488
tat act gct tac tgt caa gat ctt ttt tca gag act gaa ctt gcc ttt     1536
ctc gtt agt gga gct att ttg tat gga tgt tat tgg gtg gct ctt cta      1584
atg ctt tat ctt gca atc ata gcc cgt cga tgt ggt aaa aaa cct gaa      1632
caa agc tcc ctc gct ttt gct gag gtg taa                              1662

<210>  8

<211>  553

<212>  PRT

<213>  Artificial sequence

<220>

<223>  Synthetic nucleotide sequence coding for a Homo sapiens Hyalurona
       nsynthase-3 protein

<400>  8

<210>  9

<211>  1632

<212>  DNA

<213>  Homo sapiens


<220>

<221>  CDS

<222>  (1)..(1632)

<223>


<300>

<308>  D84424.1

<309>  1996-07-02

<313>  (149)..(1780)


<400>  9
atg acc tgg gcc tac gcc gcc ggg gtg ccg ctg gcc tcc gat cgc tac        48
ggc ctc ctg gcc ttc ggc ctc tac ggg gcc ttc ctt tca gcg cac ctg      96
gtg gcg cag agc ctc ttc gcg tac ctg gag cac cgg cgg gtg gcg gcg      144
gcg gcg cgg ggg ccg ctg gat gca gcc acc gcg cgc agt gtg gcg ctg      192
acc atc tcc gcc tac cag gag gac ccc gcg tac ctg cgc cag tgc ctg      240
gcg tcc gcc cgc gcc ctg ctg tac ccg cgc gcg cgc gtg cgc gtc ctc      288
atg gtg gtg gat ggc aac cgc gcc gag gac ctc tac atg gtc gac atg      336
ttc cgc gag gtc ttc gct gac gag gac ccc gcc acg tac gtg tgg gac      384
ggc aac tac cac cag ccc tgg gaa ccc gcg gcg gcg ggc gcg gtg ggc      432
gcc gga gcc tat cgg gag gtg gag gcg gag gat cct ggg cgg ctg gca      480
gtg gag gcg ctg gtg agg act cgc agg tgc gtg tgc gtg gcg cag cgc      528
tgg ggc ggc aag cgc gag gtc atg tac aca gcc ttc aag gcg ctc gga      576
gat tcg gtg gac tac gtg cag gtc tgt gac tcg gac aca agg ttg gac      624
ccc atg gca ctg ctg gag ctc gtg cgg gta ctg gac gag gac ccc cgg      672
gta ggg gct gtt ggt ggg gat gtg cgg atc ctt aac cct ctg gac tcc      720
tgg gtc agc ttc cta agc agc ctg cga tac tgg gta gcc ttc aat gtg      768
gag cgg gct tgt cag agc tac ttc cac tgt gta tcc tgc atc agc ggt      816
cct cta ggc cta tat agg aat aac ctc ttg cag cag ttt ctt gag gcc      864
tgg tac aac cag aag ttc ctg ggt acc cac tgt act ttt ggg gat gac      912
cgg cac ctc acc aac cgc atg ctc agc atg ggt tat gct acc aag tac      960
acc tcc agg tcc cgc tgc tac tca gag acg ccc tcg tcc ttc ctg cgg     1008
tgg ctg agc cag cag aca cgc tgg tcc aag tcg tac ttc cgt gag tgg     1056
ctg tac aac gcg ctc tgg tgg cac cgg cac cat gcg tgg atg acc tac     1104
gag gcg gtg gtc tcc ggc ctg ttc ccc ttc ttc gtg gcg gcc act gtg     1152
ctg cgt ctg ttc tac gcg ggc cgc cct tgg gcg ctg ctg tgg gtg ctg     1200
ctg tgc gtg cag ggc gtg gca ctg gcc aag gcg gcc ttc gcg gcc tgg     1248
ctg cgg ggc tgc ctg cgc atg gtg ctt ctg tcg ctc tac gcg ccc ctc     1296
tac atg tgt ggc ctc ctg cct gcc aag ttc ctg gcg cta gtc acc atg     1344
aac cag agt ggc tgg ggc acc tcg ggc cgg cgg aag ctg gcc gct aac     1392
tac gtc cct ctg ctg ccc ctg gcg ctc tgg gcg ctg ctg ctg ctt ggg     1440
ggc ctg gtc cgc agc gta gca cac gag gcc agg gcc gac tgg agc ggc     1488
cct tcc cgc gca gcc gag gcc tac cac ttg gcc gcg ggg gcc ggc gcc     1536
tac gtg ggc tac tgg gtg gcc atg ttg acg ctg tac tgg gtg ggc gtg     1584
cgg agg ctt tgc cgg cgg cgg acc ggg ggc tac cgc gtc cag gtg tga     1632

<210> 10

<211> 543

<212> PRT

<213> Homo sapiens

<400> 10

<210>  11

<211>  1659

<212>  DNA

<213>  Homo sapiens

<220>

<221>  CDS

<222>  (1)..(1659)

<223>

<300>

<308>  U54804.1

<309>  1996-08-10

<313>  (536)..(2194)

<400>  11
atg cat tgt gag agg ttt cta tgt atc ctg aga ata att gga acc aca        48
Met His Cys Glu Arg Phe Leu Cys Ile Leu Arg Ile Ile Gly Thr Thr
1               5                   10                  15

ctc ttt gga gtc tct ctc ctc ctt gga atc aca gct gct tat att gtt        96
Leu Phe Gly Val Ser Leu Leu Leu Gly Ile Thr Ala Ala Tyr Ile Val
            20                  25                  30

ggc tac cag ttt atc caa acg gat aat tac tat ttc tct ttt gga ctg       144
Gly Tyr Gln Phe Ile Gln Thr Asp Asn Tyr Tyr Phe Ser Phe Gly Leu
        35                  40                  45

tat ggt gcc ttt ttg gca tca cac ctc atc atc caa agc ctg ttt gcc       192
Tyr Gly Ala Phe Leu Ala Ser His Leu Ile Ile Gln Ser Leu Phe Ala
    50                  55                  60

ttt ttg gag cac cga aaa atg aaa aaa tcc cta gaa acc ccc ata aag       240
Phe Leu Glu His Arg Lys Met Lys Lys Ser Leu Glu Thr Pro Ile Lys
65                  70                  75                  80

ttg aac aaa aca gtt gcc ctt tgc atc gct gcc tat caa gaa gat cca       288
Leu Asn Lys Thr Val Ala Leu Cys Ile Ala Ala Tyr Gln Glu Asp Pro
                85                  90                  95

gac tac tta agg aaa tgt ttg caa tct gtg aaa agg cta acc tac cct       336

Asp Tyr Leu Arg Lys Cys Leu Gln Ser Val Lys Arg Leu Thr Tyr Pro
            100                 105                 110

ggg att aaa gtt gtc atg gtc ata gat ggg aac tca gaa gat gac ctt 384
Gly Ile Lys Val Val Met Val Ile Asp Gly Asn Ser Glu Asp Asp Leu
        115                 120                 125

tac atg atg gac atc ttc agt gaa gtc atg ggc aga gac aaa tca gcc 432
Tyr Met Met Asp Ile Phe Ser Glu Val Met Gly Arg Asp Lys Ser Ala
    130                 135                 140

act tat atc tgg aag aac aac ttc cac gaa aag ggt ccc ggt gag aca 480
Thr Tyr Ile Trp Lys Asn Asn Phe His Glu Lys Gly Pro Gly Glu Thr
145                 150                 155                 160

gat gag tca cat aaa gaa agc tcg caa cac gta acg caa ttg gtc ttg 528
Asp Glu Ser His Lys Glu Ser Ser Gln His Val Thr Gln Leu Val Leu
                165                 170                 175

tcc aac aaa agt atc tgc atc atg caa aaa tgg ggt gga aaa aga gaa 576
Ser Asn Lys Ser Ile Cys Ile Met Gln Lys Trp Gly Gly Lys Arg Glu
            180                 185                 190

gtc atg tac aca gcc ttc aga gca ctg gga cga agt gtg gat tat gta 624
Val Met Tyr Thr Ala Phe Arg Ala Leu Gly Arg Ser Val Asp Tyr Val
        195                 200                 205

cag gtt tgt gat tca gac act atg ctt gac cca gcc tca tct gtg gag 672
Gln Val Cys Asp Ser Asp Thr Met Leu Asp Pro Ala Ser Ser Val Glu
    210                 215                 220

atg gta aaa gtt tta gaa gaa gat ccc atg gtt gga ggt gtt ggg gga 720
Met Val Lys Val Leu Glu Glu Asp Pro Met Val Gly Gly Val Gly Gly
225                 230                 235                 240

gat gtc cag att tta aac aag tac gat tcc tgg atc tca ttc ctc agc 768
Asp Val Gln Ile Leu Asn Lys Tyr Asp Ser Trp Ile Ser Phe Leu Ser
                245                 250                 255

agt gta aga tat tgg atg gct ttt aat ata gaa agg gcc tgt cag tct 816
Ser Val Arg Tyr Trp Met Ala Phe Asn Ile Glu Arg Ala Cys Gln Ser
            260                 265                 270

tat ttt ggg tgt gtt cag tgc att agt gga cct ctg gga atg tac aga 864
Tyr Phe Gly Cys Val Gln Cys Ile Ser Gly Pro Leu Gly Met Tyr Arg
        275                 280                 285

aac tcc ttg ttg cat gag ttt gtg gaa gat tgg tac aat caa gaa ttt 912
Asn Ser Leu Leu His Glu Phe Val Glu Asp Trp Tyr Asn Gln Glu Phe
    290                 295                 300

atg ggc aac caa tgt agc ttt ggt gat gac agg cat ctc acg aac cgg 960
Met Gly Asn Gln Cys Ser Phe Gly Asp Asp Arg His Leu Thr Asn Arg
305                 310                 315                 320

gtg ctg agc ctg ggc tat gca aca aaa tac aca gct cga tct aag tgc 1008
Val Leu Ser Leu Gly Tyr Ala Thr Lys Tyr Thr Ala Arg Ser Lys Cys
                325                 330                 335

ctt act gaa aca cct ata gag tat ctc aga tgg cta aac cag cag acc 1056
Leu Thr Glu Thr Pro Ile Glu Tyr Leu Arg Trp Leu Asn Gln Gln Thr
            340                 345                 350

cgt tgg agc aag tcc tac ttc cga gaa tgg ctg tac aat gca atg tgg 1104
Arg Trp Ser Lys Ser Tyr Phe Arg Glu Trp Leu Tyr Asn Ala Met Trp
        355                 360                 365

ttt cac aaa cat cac ttg tgg atg acc tac gaa gcg att atc act gga 1152

Phe His Lys His His Leu Trp Met Thr Tyr Glu Ala Ile Ile Thr Gly
    370                 375                 380

ttc ttt cct ttc ttt ctc att gcc aca gta atc cag ctc ttc tac cgg      1200
Phe Phe Pro Phe Phe Leu Ile Ala Thr Val Ile Gln Leu Phe Tyr Arg
385                 390                 395                 400

ggt aaa att tgg aac att ctc ctc ttc ttg tta act gtc cag cta gta      1248
Gly Lys Ile Trp Asn Ile Leu Leu Phe Leu Leu Thr Val Gln Leu Val
                405                 410                 415

ggt ctc ata aaa tca tct ttt gcc agc tgc ctt aga gga aat atc gtc      1296
Gly Leu Ile Lys Ser Ser Phe Ala Ser Cys Leu Arg Gly Asn Ile Val
            420                 425                 430

atg gtc ttc atg tct ctc tac tca gtg tta tac atg tcg agt tta ctt      1344
Met Val Phe Met Ser Leu Tyr Ser Val Leu Tyr Met Ser Ser Leu Leu
        435                 440                 445

ccc gcc aag atg ttt gca att gca aca ata aac aaa gct ggg tgg ggc      1392
Pro Ala Lys Met Phe Ala Ile Ala Thr Ile Asn Lys Ala Gly Trp Gly
    450                 455                 460

aca tca gga agg aaa acc att gtt gtt aat ttc ata gga ctc att cca      1440
Thr Ser Gly Arg Lys Thr Ile Val Val Asn Phe Ile Gly Leu Ile Pro
465                 470                 475                 480

gta tca gtt tgg ttt aca atc ctc ctg ggt ggt gtg att ttc acc att      1488
Val Ser Val Trp Phe Thr Ile Leu Leu Gly Gly Val Ile Phe Thr Ile
                485                 490                 495

tat aag gag tct aaa agg cca ttt tca gaa tcc aaa cag aca gtt cta      1536
Tyr Lys Glu Ser Lys Arg Pro Phe Ser Glu Ser Lys Gln Thr Val Leu
            500                 505                 510

att gtt gga acg ttg ctc tat gca tgc tat tgg gtc atg ctt ttg acg      1584
Ile Val Gly Thr Leu Leu Tyr Ala Cys Tyr Trp Val Met Leu Leu Thr
        515                 520                 525

ctg tat gta gtt ctc atc aat aag tgt ggc agg cgg aag aag gga caa      1632
Leu Tyr Val Val Leu Ile Asn Lys Cys Gly Arg Arg Lys Lys Gly Gln
    530                 535                 540

caa tat gac atg gtg ctt gat gta tga                                  1659
Gln Tyr Asp Met Val Leu Asp Val
545                 550

<210>  12

<211>  552

<212>  PRT

<213>  Homo sapiens


<400>  12

Met His Cys Glu Arg Phe Leu Cys Ile Leu Arg Ile Ile Gly Thr Thr
1               5                   10                  15


Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Glu Asp Asp Leu
        115                 120                 125

Tyr Met Met Asp Ile Phe Ser Glu Val Met Gly Arg Asp Lys Ser Ala
    130                 135                 140

Thr Tyr Ile Trp Lys Asn Asn Phe His Glu Lys Gly Pro Gly Glu Thr
145                 150                 155                 160

Asp Glu Ser His Lys Glu Ser Ser Gln His Val Thr Gln Leu Val Leu
                165                 170                 175

Ser Asn Lys Ser Ile Cys Ile Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Asn Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Ile Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Lys Glu Ser Lys Arg Pro Phe Ser Glu Ser Lys Gln Thr Val Leu
            500                 505                 510

Ile Val Gly Thr Leu Leu Tyr Ala Cys Tyr Trp Val Met Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  13

<211>  1752

<212>  DNA

<213>  Papio anubis

<220>

<221>  CDS

<222>  (1)..(1752)

<223>

<300>

<308>  AY463695

<309>  2003-12-08

<313>  (36)..(1787)

<400>  13
atg aca cag cgg gac acg ccc aag ccc act cct gca gcc cgc cgc tgc      48
Met Thr Gln Arg Asp Thr Pro Lys Pro Thr Pro Ala Ala Arg Arg Cys
1               5                   10                  15

tcc ggc ctg gcc cgg agg gtg ctg acc atc gcc ttc gcc ctg ctc atc      96
Ser Gly Leu Ala Arg Arg Val Leu Thr Ile Ala Phe Ala Leu Leu Ile
            20                  25                  30

ctg ggc ctc atg acc tgg gcc tac gcc gcc ggg gtg ccg ctg gcc tcc      144
Leu Gly Leu Met Thr Trp Ala Tyr Ala Ala Gly Val Pro Leu Ala Ser
        35                  40                  45

gat cgc tac ggc ctc ctg gcc ttc ggc ctc tac ggg gcc ttc ctc tcg      192
Asp Arg Tyr Gly Leu Leu Ala Phe Gly Leu Tyr Gly Ala Phe Leu Ser
    50                  55                  60

gcg cac ctg ttg gcg cag agc ctc ttc gcg tac ctg gag cat cgg cgg      240
Ala His Leu Leu Ala Gln Ser Leu Phe Ala Tyr Leu Glu His Arg Arg
65                  70                  75                  80

gtg gcg gcg gcg gcg cgg cgc gcg gcg gca cgg ggg cgc ctg gat gca      288
Val Ala Ala Ala Ala Arg Arg Ala Ala Ala Arg Gly Arg Leu Asp Ala
                85                  90                  95

gcc acg gcg cgc agc gtg gcg ctg acc att tcc gcc tac cag gag gac      336
Ala Thr Ala Arg Ser Val Ala Leu Thr Ile Ser Ala Tyr Gln Glu Asp
            100                 105                 110

ccc gcg tac ctg cgc cag tgc ctg gtg tcc gcc cgc gcc ctg ctg tac      384
Pro Ala Tyr Leu Arg Gln Cys Leu Val Ser Ala Arg Ala Leu Leu Tyr
        115                 120                 125

ccg cgc gcg cgg ctg cgc gtc ctc atg gtg gtg gac ggc aac cgc ccc      432
Pro Arg Ala Arg Leu Arg Val Leu Met Val Val Asp Gly Asn Arg Pro
    130                 135                 140

gag gac ctc tac atg gta gac atg ttc cgc gag gtc ttc gcc gac gag      480
Glu Asp Leu Tyr Met Val Asp Met Phe Arg Glu Val Phe Ala Asp Glu
145                 150                 155                 160

gac ccc gcc acg tac gtg tgg gac ggc aac tac cac cag ccc tgg gaa      528

Asp Pro Ala Thr Tyr Val Trp Asp Gly Asn Tyr His Gln Pro Trp Glu
                165                 170                 175

ccc gcg gcg gtg ggc gcg gtg ggc gtc gga gcc tac cgg gag gtg gag 576
Pro Ala Ala Val Gly Ala Val Gly Val Gly Ala Tyr Arg Glu Val Glu
            180                 185                 190

gcg gag gat ccc ggg cgg ttg gcg gtg gag gcg ctg gtg agg act cgc 624
Ala Glu Asp Pro Gly Arg Leu Ala Val Glu Ala Leu Val Arg Thr Arg
        195                 200                 205

agg tgc gtg tgc gtg gcg cag cgc tgg ggc ggc aag cgc gag gtc atg 672
Arg Cys Val Cys Val Ala Gln Arg Trp Gly Gly Lys Arg Glu Val Met
    210                 215                 220

tac acc gcc ttc aag gcg ctc gga gac tcg gtg gac tac gtg cag gtc 720
Tyr Thr Ala Phe Lys Ala Leu Gly Asp Ser Val Asp Tyr Val Gln Val
225                 230                 235                 240

tgt gac tcg gac aca agg ttg gac ccc atg gca ctg ctg gag ctc gtg 768
Cys Asp Ser Asp Thr Arg Leu Asp Pro Met Ala Leu Leu Glu Leu Val
                245                 250                 255

cag gtc ctg gat gag gac ccc cgg gta ggg gct gtt ggt ggg gac gtg 816
Gln Val Leu Asp Glu Asp Pro Arg Val Gly Ala Val Gly Gly Asp Val
            260                 265                 270

cgg atc ctt aac cct ctg gac tcc tgg gtc agc ttc cta agc agc ctg 864
Arg Ile Leu Asn Pro Leu Asp Ser Trp Val Ser Phe Leu Ser Ser Leu
        275                 280                 285

cga tac tgg gta gcc ttc aat gtg gag cgg gct tgt cag agc tac ttc 912
Arg Tyr Trp Val Ala Phe Asn Val Glu Arg Ala Cys Gln Ser Tyr Phe
    290                 295                 300

cac tgt gtg tcc tgc atc agt ggt cct cta ggc cta tat agg aac aac 960
His Cys Val Ser Cys Ile Ser Gly Pro Leu Gly Leu Tyr Arg Asn Asn
305                 310                 315                 320

ctc ttg cag cag ttt ctt gag gcc tgg tac aac cag aag ttc ctg gga 1008
Leu Leu Gln Gln Phe Leu Glu Ala Trp Tyr Asn Gln Lys Phe Leu Gly
                325                 330                 335

acc cac tgt act ttt ggg gac gac cgg cac ctc acc aac cgc atg ctc 1056
Thr His Cys Thr Phe Gly Asp Asp Arg His Leu Thr Asn Arg Met Leu
            340                 345                 350

agc atg ggt tat gct acc aag tac acc tcc agg tct cgt tgc tac tca 1104
Ser Met Gly Tyr Ala Thr Lys Tyr Thr Ser Arg Ser Arg Cys Tyr Ser
        355                 360                 365

gag aca ccc tcg tcc ttc ctg cgc tgg ctg agt cag cag act cgc tgg 1152
Glu Thr Pro Ser Ser Phe Leu Arg Trp Leu Ser Gln Gln Thr Arg Trp
    370                 375                 380

tcc aag tcg tac ttc cgt gaa tgg ctg tac aac gcg ctc tgg tgg cac 1200
Ser Lys Ser Tyr Phe Arg Glu Trp Leu Tyr Asn Ala Leu Trp Trp His
385                 390                 395                 400

cgg cat cac gcc tgg atg acc tac gag gcg gtg gtc tcg ggc ctg ttc 1248
Arg His His Ala Trp Met Thr Tyr Glu Ala Val Val Ser Gly Leu Phe
                405                 410                 415

ccc ttc ttc gtg gcg gcc acg gtg ctg cgt ctg ttc tat gcg ggc cgc 1296
Pro Phe Phe Val Ala Ala Thr Val Leu Arg Leu Phe Tyr Ala Gly Arg
            420                 425                 430

ccg tgg gcg ctg ctg tgg gtg ctg cta tgc gtg cag ggc gtg gca ctg 1344

Pro Trp Ala Leu Leu Trp Val Leu Leu Cys Val Gln Gly Val Ala Leu
        435                 440                 445

gcc aag gcg gcc ttt gcg gcc tgg ctg cgg ggc tgc ctg cgt atg gtg      1392
Ala Lys Ala Ala Phe Ala Ala Trp Leu Arg Gly Cys Leu Arg Met Val
    450                 455                 460

ctg ctg tcg ctc tac gcg ccc ctc tac atg tgt ggc ctc ctg ccc gcc      1440
Leu Leu Ser Leu Tyr Ala Pro Leu Tyr Met Cys Gly Leu Leu Pro Ala
465                 470                 475                 480

aag ttc ctg gcg ctg gtc acc atg aac cag agt ggc tgg ggc acc tcg      1488
Lys Phe Leu Ala Leu Val Thr Met Asn Gln Ser Gly Trp Gly Thr Ser
                485                 490                 495

ggc cgg cgg aag ctg gcc gct aat tac gtc cct ctg ctg ccc ctg gcg      1536
Gly Arg Arg Lys Leu Ala Ala Asn Tyr Val Pro Leu Leu Pro Leu Ala
            500                 505                 510

ctc tgg gcg ctg ctg ctg ctt ggg ggc ctg gtc cgc agt gtg gca cac      1584
Leu Trp Ala Leu Leu Leu Leu Gly Gly Leu Val Arg Ser Val Ala His
        515                 520                 525

gag gcc agg gcc gac tgg agc ggc cct tcc cgc gca gcg gag gcc tac      1632
Glu Ala Arg Ala Asp Trp Ser Gly Pro Ser Arg Ala Ala Glu Ala Tyr
    530                 535                 540

cac tta gcc gcg ggg gcc ggc gcc tac gtg ggc tac tgg gtg gtc atg      1680
His Leu Ala Ala Gly Ala Gly Ala Tyr Val Gly Tyr Trp Val Val Met
545                 550                 555                 560

ttg acg ctg tac tgg gtg ggc gtg cgg agg ctt tgc cgg cgg cgg acc      1728
Leu Thr Leu Tyr Trp Val Gly Val Arg Arg Leu Cys Arg Arg Arg Thr
                565                 570                 575

ggg ggc tac cgt gtc cag gtg tga                                      1752
Gly Gly Tyr Arg Val Gln Val
            580

<210>  14

<211>  583

<212>  PRT

<213>  Papio anubis

<400>  14

Met Thr Gln Arg Asp Thr Pro Lys Pro Thr Pro Ala Ala Arg Arg Cys
1               5                   10                  15

Ser Gly Leu Ala Arg Arg Val Leu Thr Ile Ala Phe Ala Leu Leu Ile
            20                  25                  30

Leu Gly Leu Met Thr Trp Ala Tyr Ala Ala Gly Val Pro Leu Ala Ser
        35                  40                  45

Asp Arg Tyr Gly Leu Leu Ala Phe Gly Leu Tyr Gly Ala Phe Leu Ser
    50                  55                  60

Ala His Leu Leu Ala Gln Ser Leu Phe Ala Tyr Leu Glu His Arg Arg
65                  70                  75                  80

Val Ala Ala Ala Ala Arg Arg Ala Ala Ala Arg Gly Arg Leu Asp Ala
                85                  90                  95

Ala Thr Ala Arg Ser Val Ala Leu Thr Ile Ser Ala Tyr Gln Glu Asp
            100                 105                 110

Pro Ala Tyr Leu Arg Gln Cys Leu Val Ser Ala Arg Ala Leu Leu Tyr
        115                 120                 125

Pro Arg Ala Arg Leu Arg Val Leu Met Val Val Asp Gly Asn Arg Pro
    130                 135                 140

Glu Asp Leu Tyr Met Val Asp Met Phe Arg Glu Val Phe Ala Asp Glu
145                 150                 155                 160

Asp Pro Ala Thr Tyr Val Trp Asp Gly Asn Tyr His Gln Pro Trp Glu
                165                 170                 175

Pro Ala Ala Val Gly Ala Val Gly Val Gly Ala Tyr Arg Glu Val Glu
            180                 185                 190

Ala Glu Asp Pro Gly Arg Leu Ala Val Glu Ala Leu Val Arg Thr Arg
        195                 200                 205

Arg Cys Val Cys Val Ala Gln Arg Trp Gly Gly Lys Arg Glu Val Met
    210                 215                 220

Tyr Thr Ala Phe Lys Ala Leu Gly Asp Ser Val Asp Tyr Val Gln Val
225                 230                 235                 240

Cys Asp Ser Asp Thr Arg Leu Asp Pro Met Ala Leu Leu Glu Leu Val
                245                 250                 255

Gln Val Leu Asp Glu Asp Pro Arg Val Gly Ala Val Gly Gly Asp Val
            260                 265                 270

Arg Ile Leu Asn Pro Leu Asp Ser Trp Val Ser Phe Leu Ser Ser Leu
        275                 280                 285

Arg Tyr Trp Val Ala Phe Asn Val Glu Arg Ala Cys Gln Ser Tyr Phe
    290                 295                 300

His Cys Val Ser Cys Ile Ser Gly Pro Leu Gly Leu Tyr Arg Asn Asn
305                 310                 315                 320

Leu Leu Gln Gln Phe Leu Glu Ala Trp Tyr Asn Gln Lys Phe Leu Gly
                325                 330                 335

Thr His Cys Thr Phe Gly Asp Asp Arg His Leu Thr Asn Arg Met Leu
            340                 345                 350

Ser Met Gly Tyr Ala Thr Lys Tyr Thr Ser Arg Ser Arg Cys Tyr Ser
        355                 360                 365

Glu Thr Pro Ser Ser Phe Leu Arg Trp Leu Ser Gln Gln Thr Arg Trp
    370                 375                 380

Ser Lys Ser Tyr Phe Arg Glu Trp Leu Tyr Asn Ala Leu Trp Trp His
385                 390                 395                 400

Arg His His Ala Trp Met Thr Tyr Glu Ala Val Val Ser Gly Leu Phe
                405                 410                 415

Pro Phe Phe Val Ala Ala Thr Val Leu Arg Leu Phe Tyr Ala Gly Arg
            420                 425                 430

Pro Trp Ala Leu Leu Trp Val Leu Leu Cys Val Gln Gly Val Ala Leu
        435                 440                 445

Ala Lys Ala Ala Phe Ala Ala Trp Leu Arg Gly Cys Leu Arg Met Val
    450                 455                 460

Leu Leu Ser Leu Tyr Ala Pro Leu Tyr Met Cys Gly Leu Leu Pro Ala
465                 470                 475                 480

Lys Phe Leu Ala Leu Val Thr Met Asn Gln Ser Gly Trp Gly Thr Ser
                485                 490                 495

Gly Arg Arg Lys Leu Ala Ala Asn Tyr Val Pro Leu Leu Pro Leu Ala
            500                 505                 510

Leu Trp Ala Leu Leu Leu Leu Gly Gly Leu Val Arg Ser Val Ala His
        515                 520                 525

Glu Ala Arg Ala Asp Trp Ser Gly Pro Ser Arg Ala Ala Glu Ala Tyr
    530                 535                 540

His Leu Ala Ala Gly Ala Gly Ala Tyr Val Gly Tyr Trp Val Val Met
545                 550                 555                 560

Leu Thr Leu Tyr Trp Val Gly Val Arg Arg Leu Cys Arg Arg Arg Thr
                565                 570                 575

Gly Gly Tyr Arg Val Gln Val
            580

<210>  15

<211>  1752

<212>  DNA

<213>  Mus musculus

<220>

<221>  CDS

<222>  (1)..(1752)

<223>

<300>

<308>  D82964.1

<309>  1996-05-31

<313>  (49)..(1800)

<400>  15
atg aga cag gac atg cca aag ccc tca gag gca gcg cgt tgc tgc tct      48
Met Arg Gln Asp Met Pro Lys Pro Ser Glu Ala Ala Arg Cys Cys Ser
1               5                   10                  15

ggc ctg gcc agg cga gca ctc acg atc atc ttt gcc ctg ctc atc ctg      96
Gly Leu Ala Arg Arg Ala Leu Thr Ile Ile Phe Ala Leu Leu Ile Leu
            20                  25                  30

ggc ctc atg acc tgg gcc tac gcc gca ggc gtt cct ctg gct tca gat      144
Gly Leu Met Thr Trp Ala Tyr Ala Ala Gly Val Pro Leu Ala Ser Asp
        35                  40                  45

cgc tat gga ctc ctg gcc ttt ggc ctc tat ggg gca ttc ctc agc gca      192
Arg Tyr Gly Leu Leu Ala Phe Gly Leu Tyr Gly Ala Phe Leu Ser Ala
    50                  55                  60

cac cta gtg gca cag agc ctc ttc gct tac ctg gag cac cga agg gtg      240
His Leu Val Ala Gln Ser Leu Phe Ala Tyr Leu Glu His Arg Arg Val
65                  70                  75                  80

gca gcg gct gcg cgg cgc tcc ttg gcg aag ggg ccc ctg gat gcg gcc      288
Ala Ala Ala Ala Arg Arg Ser Leu Ala Lys Gly Pro Leu Asp Ala Ala
                85                  90                  95

act gca cgc agc gtg gca ctc acc atc tca gcc tac caa gag gat ccc      336
Thr Ala Arg Ser Val Ala Leu Thr Ile Ser Ala Tyr Gln Glu Asp Pro
            100                 105                 110

gct tac ctg cgc cag tgc ttg acc tcc gcg cgc gcc ttg ctg tac ccg      384
Ala Tyr Leu Arg Gln Cys Leu Thr Ser Ala Arg Ala Leu Leu Tyr Pro
        115                 120                 125

cac acg agg tta cgc gtg ctc atg gtg gtg gac ggc aac cgc gct gag      432
His Thr Arg Leu Arg Val Leu Met Val Val Asp Gly Asn Arg Ala Glu
    130                 135                 140

gat ctg tac atg gtg gac atg ttc cga gaa gtc ttc gcc gat gag gac      480
Asp Leu Tyr Met Val Asp Met Phe Arg Glu Val Phe Ala Asp Glu Asp
145                 150                 155                 160

ccc gcc act tat gtg tgg gat ggc aac tac cat cag ccc tgg gaa cca      528

Pro Ala Thr Tyr Val Trp Asp Gly Asn Tyr His Gln Pro Trp Glu Pro
                165                 170                 175

gcg gag gct acg ggc gct gtc ggt gaa ggt gcc tac cgg gag gtg gag 576
Ala Glu Ala Thr Gly Ala Val Gly Glu Gly Ala Tyr Arg Glu Val Glu
            180                 185                 190

gcg gag gac ccc ggg cgg ttg gcg gtg gag gcg ctg gtg aga aca cgc 624
Ala Glu Asp Pro Gly Arg Leu Ala Val Glu Ala Leu Val Arg Thr Arg
        195                 200                 205

agg tgc gtg tgc gtg gct cag cgt tgg ggc ggc aaa cgt gag gtc atg 672
Arg Cys Val Cys Val Ala Gln Arg Trp Gly Gly Lys Arg Glu Val Met
    210                 215                 220

tac aca gct ttc aag gca ctg ggc gac tcc gtg gac tac gtg cag gtc 720
Tyr Thr Ala Phe Lys Ala Leu Gly Asp Ser Val Asp Tyr Val Gln Val
225                 230                 235                 240

tgt gac tca gac aca aga cta gac ccc atg gca ctg ctg gag ctt gtg 768
Cys Asp Ser Asp Thr Arg Leu Asp Pro Met Ala Leu Leu Glu Leu Val
                245                 250                 255

cga gtg ttg gat gaa gac ccc cgg gta ggg gct gtt gga ggg gat gtg 816
Arg Val Leu Asp Glu Asp Pro Arg Val Gly Ala Val Gly Gly Asp Val
            260                 265                 270

agg atc ctt aac cct ctg gac tcc tgg gtc agc ttc ttg agc agt ctt 864
Arg Ile Leu Asn Pro Leu Asp Ser Trp Val Ser Phe Leu Ser Ser Leu
        275                 280                 285

cga tac tgg gta gcc ttc aat gtg gaa cga gct tgt cag agc tac ttc 912
Arg Tyr Trp Val Ala Phe Asn Val Glu Arg Ala Cys Gln Ser Tyr Phe
    290                 295                 300

cac tgt gtg tcc tgc atc agt ggt cct ctg ggt cta tac aga aac aat 960
His Cys Val Ser Cys Ile Ser Gly Pro Leu Gly Leu Tyr Arg Asn Asn
305                 310                 315                 320

ctc ctg cag cag ttc ttg gag gcc tgg tac aac caa aag ttc ctg ggc 1008
Leu Leu Gln Gln Phe Leu Glu Ala Trp Tyr Asn Gln Lys Phe Leu Gly
                325                 330                 335

acc cac tgc aca ttt ggg gat gac agg cac ctc acc aac cga atg ctt 1056
Thr His Cys Thr Phe Gly Asp Asp Arg His Leu Thr Asn Arg Met Leu
            340                 345                 350

agc atg ggc tat gct acc aag tat acc tcg cgc tcc aga tgc tac tcg 1104
Ser Met Gly Tyr Ala Thr Lys Tyr Thr Ser Arg Ser Arg Cys Tyr Ser
        355                 360                 365

gag acg ccc tcc tcc ttc ctt cgt tgg ttg agc caa cag acc cgc tgg 1152
Glu Thr Pro Ser Ser Phe Leu Arg Trp Leu Ser Gln Gln Thr Arg Trp
    370                 375                 380

tcc aaa tct tac ttc cga gag tgg cta tac aat gct ctg tgg tgg cat 1200
Ser Lys Ser Tyr Phe Arg Glu Trp Leu Tyr Asn Ala Leu Trp Trp His
385                 390                 395                 400

cgc cac cac gca tgg atg acc tat gaa gcg gtg gtc tcg ggc ctc ttc 1248
Arg His His Ala Trp Met Thr Tyr Glu Ala Val Val Ser Gly Leu Phe
                405                 410                 415

cct ttc ttc gtg gct gcc acg gtg ttg agg ctc ttc tat gca ggg cgc 1296
Pro Phe Phe Val Ala Ala Thr Val Leu Arg Leu Phe Tyr Ala Gly Arg
            420                 425                 430

ccg tgg gct ctg ctc tgg gtg ctg ctc tgt gtg cag ggc gta gca ctg 1344

Pro Trp Ala Leu Leu Trp Val Leu Leu Cys Val Gln Gly Val Ala Leu
        435                 440                 445

gca aag gca gcc ttt gca gcc tgg ctg cgt ggc tgc gtg cgc atg gtg      1392
Ala Lys Ala Ala Phe Ala Ala Trp Leu Arg Gly Cys Val Arg Met Val
    450                 455                 460

ctg ctg tca ctc tat gca cca ctc tac atg tgc ggc ctc ctg cct gcc      1440
Leu Leu Ser Leu Tyr Ala Pro Leu Tyr Met Cys Gly Leu Leu Pro Ala
465                 470                 475                 480

aaa ttc cta gcg ttg gtt acc atg aat caa agt ggt tgg ggt acc tcg      1488
Lys Phe Leu Ala Leu Val Thr Met Asn Gln Ser Gly Trp Gly Thr Ser
                485                 490                 495

ggc cgg aag aaa ctg gct gct aac tat gtc ccc gtg ttg ccc ctg gca      1536
Gly Arg Lys Lys Leu Ala Ala Asn Tyr Val Pro Val Leu Pro Leu Ala
            500                 505                 510

ctc tgg gct cta ctg ctg ctt gga ggc ctg gcc cgc agt gtg gcc cag      1584
Leu Trp Ala Leu Leu Leu Leu Gly Gly Leu Ala Arg Ser Val Ala Gln
        515                 520                 525

gag gcc aga gct gac tgg agt ggc cca tcc cga gca gct gaa gcc tac      1632
Glu Ala Arg Ala Asp Trp Ser Gly Pro Ser Arg Ala Ala Glu Ala Tyr
    530                 535                 540

cac ctt gct gct ggg gct ggt gcc tat gtg gcc tac tgg gtg gta atg      1680
His Leu Ala Ala Gly Ala Gly Ala Tyr Val Ala Tyr Trp Val Val Met
545                 550                 555                 560

tta act atc tac tgg gta ggt gtg agg agg ctg tgc aga cgt cgg agc      1728
Leu Thr Ile Tyr Trp Val Gly Val Arg Arg Leu Cys Arg Arg Arg Ser
                565                 570                 575

ggt ggt tac cgt gtc caa gta tga                                      1752
Gly Gly Tyr Arg Val Gln Val
            580

<210>  16

<211>  583

<212>  PRT

<213>  Mus musculus

<400>  16

Met Arg Gln Asp Met Pro Lys Pro Ser Glu Ala Ala Arg Cys Cys Ser
1               5                   10                  15

Gly Leu Ala Arg Arg Ala Leu Thr Ile Ile Phe Ala Leu Leu Ile Leu
            20                  25                  30

Gly Leu Met Thr Trp Ala Tyr Ala Ala Gly Val Pro Leu Ala Ser Asp
        35                  40                  45

Arg Tyr Gly Leu Leu Ala Phe Gly Leu Tyr Gly Ala Phe Leu Ser Ala
    50                  55                  60

His Leu Val Ala Gln Ser Leu Phe Ala Tyr Leu Glu His Arg Arg Val
65                  70                  75                  80

Ala Ala Ala Ala Arg Arg Ser Leu Ala Lys Gly Pro Leu Asp Ala Ala
                85                  90                  95

Thr Ala Arg Ser Val Ala Leu Thr Ile Ser Ala Tyr Gln Glu Asp Pro
            100                 105                 110

Ala Tyr Leu Arg Gln Cys Leu Thr Ser Ala Arg Ala Leu Leu Tyr Pro
        115                 120                 125

His Thr Arg Leu Arg Val Leu Met Val Val Asp Gly Asn Arg Ala Glu
    130                 135                 140

Asp Leu Tyr Met Val Asp Met Phe Arg Glu Val Phe Ala Asp Glu Asp
145                 150                 155                 160

Pro Ala Thr Tyr Val Trp Asp Gly Asn Tyr His Gln Pro Trp Glu Pro
                165                 170                 175

Ala Glu Ala Thr Gly Ala Val Gly Glu Gly Ala Tyr Arg Glu Val Glu
            180                 185                 190

Ala Glu Asp Pro Gly Arg Leu Ala Val Glu Ala Leu Val Arg Thr Arg
        195                 200                 205

Arg Cys Val Cys Val Ala Gln Arg Trp Gly Gly Lys Arg Glu Val Met
    210                 215                 220

Tyr Thr Ala Phe Lys Ala Leu Gly Asp Ser Val Asp Tyr Val Gln Val
225                 230                 235                 240

Cys Asp Ser Asp Thr Arg Leu Asp Pro Met Ala Leu Leu Glu Leu Val
                245                 250                 255

Arg Val Leu Asp Glu Asp Pro Arg Val Gly Ala Val Gly Gly Asp Val
            260                 265                 270

Arg Ile Leu Asn Pro Leu Asp Ser Trp Val Ser Phe Leu Ser Ser Leu
        275                 280                 285

Arg Tyr Trp Val Ala Phe Asn Val Glu Arg Ala Cys Gln Ser Tyr Phe
    290                 295                 300

His Cys Val Ser Cys Ile Ser Gly Pro Leu Gly Leu Tyr Arg Asn Asn
305                 310                 315                 320

Leu Leu Gln Gln Phe Leu Glu Ala Trp Tyr Asn Gln Lys Phe Leu Gly
                325                 330                 335

Thr His Cys Thr Phe Gly Asp Asp Arg His Leu Thr Asn Arg Met Leu
            340                 345                 350

Ser Met Gly Tyr Ala Thr Lys Tyr Thr Ser Arg Ser Arg Cys Tyr Ser
        355                 360                 365

Glu Thr Pro Ser Ser Phe Leu Arg Trp Leu Ser Gln Gln Thr Arg Trp
    370                 375                 380

Ser Lys Ser Tyr Phe Arg Glu Trp Leu Tyr Asn Ala Leu Trp Trp His
385                 390                 395                 400

Arg His His Ala Trp Met Thr Tyr Glu Ala Val Val Ser Gly Leu Phe
                405                 410                 415

Pro Phe Phe Val Ala Ala Thr Val Leu Arg Leu Phe Tyr Ala Gly Arg
            420                 425                 430

Pro Trp Ala Leu Leu Trp Val Leu Leu Cys Val Gln Gly Val Ala Leu
        435                 440                 445

Ala Lys Ala Ala Phe Ala Ala Trp Leu Arg Gly Cys Val Arg Met Val
    450                 455                 460

Leu Leu Ser Leu Tyr Ala Pro Leu Tyr Met Cys Gly Leu Leu Pro Ala
465                 470                 475                 480

Lys Phe Leu Ala Leu Val Thr Met Asn Gln Ser Gly Trp Gly Thr Ser
                485                 490                 495

Gly Arg Lys Lys Leu Ala Ala Asn Tyr Val Pro Val Leu Pro Leu Ala
            500                 505                 510

Leu Trp Ala Leu Leu Leu Leu Gly Gly Leu Ala Arg Ser Val Ala Gln
        515                 520                 525

Glu Ala Arg Ala Asp Trp Ser Gly Pro Ser Arg Ala Ala Glu Ala Tyr
    530                 535                 540

His Leu Ala Ala Gly Ala Gly Ala Tyr Val Ala Tyr Trp Val Val Met
545                 550                 555                 560

Leu Thr Ile Tyr Trp Val Gly Val Arg Arg Leu Cys Arg Arg Arg Ser
                565                 570                 575

Gly Gly Tyr Arg Val Gln Val
            580

<210>  17

<211>  1659

<212>  DNA

<213>  Mus musculus

<220>

<221>  CDS

<222>  (1)..(1659)

<223>

<300>

<308>  U52524.2

<309>  1996-10-15

<313>  (508)..(2166)

<400>  17
atg cat tgt gag agg ttt cta tgt gtc ctg aga ata att gga act aca      48
Met His Cys Glu Arg Phe Leu Cys Val Leu Arg Ile Ile Gly Thr Thr
1               5                   10                  15

ctt ttt gga gtg tct ctc ctc ctc gga atc aca gct gct tat att gtt      96
Leu Phe Gly Val Ser Leu Leu Leu Gly Ile Thr Ala Ala Tyr Ile Val
            20                  25                  30

ggc tac cag ttt atc caa aca gat aat tac tac ttc tca ttt gga ctg      144
Gly Tyr Gln Phe Ile Gln Thr Asp Asn Tyr Tyr Phe Ser Phe Gly Leu
        35                  40                  45

tac ggt gcc ttt tta gcc tcg cat ctc atc atc caa agc ctc ttt gcc      192
Tyr Gly Ala Phe Leu Ala Ser His Leu Ile Ile Gln Ser Leu Phe Ala
    50                  55                  60

ttt ttg gaa cac cgg aaa atg aag aag tcc ctt gaa acc ccg att aaa      240
Phe Leu Glu His Arg Lys Met Lys Lys Ser Leu Glu Thr Pro Ile Lys
65                  70                  75                  80

ttg aac aaa acg gta gca ctc tgc atc gct gcg tac caa gag gac cct      288
Leu Asn Lys Thr Val Ala Leu Cys Ile Ala Ala Tyr Gln Glu Asp Pro
                85                  90                  95

gac tac tta cgg aaa tgt ttg caa tct gtg aaa agg ctg acc tac cct      336
Asp Tyr Leu Arg Lys Cys Leu Gln Ser Val Lys Arg Leu Thr Tyr Pro
            100                 105                 110

ggg att aaa gtc gtg atg gtc atc gat ggg aac tca gac gac gac ctt      384
Gly Ile Lys Val Val Met Val Ile Asp Gly Asn Ser Asp Asp Asp Leu
        115                 120                 125

tac atg atg gac ata ttc agc gaa gtt att ggc agg gac aaa tcg gcc      432
Tyr Met Met Asp Ile Phe Ser Glu Val Ile Gly Arg Asp Lys Ser Ala
    130                 135                 140

acg tac atc tgg aag aac aac ttt cat gaa aag gga cct ggt gag aca      480
Thr Tyr Ile Trp Lys Asn Asn Phe His Glu Lys Gly Pro Gly Glu Thr
145                 150                 155                 160

gaa gag tcc cat aaa gaa agt tca caa cat gtc acc caa ttg gtc ttg      528

Glu Glu Ser His Lys Glu Ser Ser Gln His Val Thr Gln Leu Val Leu
                165                 170                 175

tct aac aaa agt att tgc atc atg caa aaa tgg ggt gga aag aga gaa      576
Ser Asn Lys Ser Ile Cys Ile Met Gln Lys Trp Gly Gly Lys Arg Glu
            180                 185                 190

gtc atg tac aca gcc ttc aga gca ctg ggg cga agc gtg gat tat gta      624
Val Met Tyr Thr Ala Phe Arg Ala Leu Gly Arg Ser Val Asp Tyr Val
        195                 200                 205

cag gtg tgt gac tca gat act atg ctt gac cct gcc tca tct gtg gag      672
Gln Val Cys Asp Ser Asp Thr Met Leu Asp Pro Ala Ser Ser Val Glu
    210                 215                 220

atg gtg aag gtc tta gag gaa gac cct atg gtt gga ggt gtt gga gga      720
Met Val Lys Val Leu Glu Glu Asp Pro Met Val Gly Gly Val Gly Gly
225                 230                 235                 240

gat gtc cag att tta aac aag tat gat tcc tgg atc tcc ttc ctc agc      768
Asp Val Gln Ile Leu Asn Lys Tyr Asp Ser Trp Ile Ser Phe Leu Ser
                245                 250                 255

agc gtg aga tac tgg atg gct ttt aat ata gaa agg gcc tgc cag tct      816
Ser Val Arg Tyr Trp Met Ala Phe Asn Ile Glu Arg Ala Cys Gln Ser
            260                 265                 270

tat ttt ggc tgt gtc cag tgc ata agc ggt cct ctg gga atg tac aga      864
Tyr Phe Gly Cys Val Gln Cys Ile Ser Gly Pro Leu Gly Met Tyr Arg
        275                 280                 285

aac tcc ttg ctg cat gaa ttt gtg gaa gac tgg tac aat cag gaa ttc      912
Asn Ser Leu Leu His Glu Phe Val Glu Asp Trp Tyr Asn Gln Glu Phe
    290                 295                 300

atg ggt aac caa tgc agt ttt ggt gac gac agg cac ctt acc aac agg      960
Met Gly Asn Gln Cys Ser Phe Gly Asp Asp Arg His Leu Thr Asn Arg
305                 310                 315                 320

gtg ttg agt ctg ggc tat gca act aaa tac acg gct cgg tcc aag tgc     1008
Val Leu Ser Leu Gly Tyr Ala Thr Lys Tyr Thr Ala Arg Ser Lys Cys
                325                 330                 335

ctt act gaa act ccc ata gaa tat ctg aga tgg ctg aac cag cag acc     1056
Leu Thr Glu Thr Pro Ile Glu Tyr Leu Arg Trp Leu Asn Gln Gln Thr
            340                 345                 350

cga tgg agc aag tcc tac ttc cga gag tgg ctg tac aat gcc atg tgg     1104
Arg Trp Ser Lys Ser Tyr Phe Arg Glu Trp Leu Tyr Asn Ala Met Trp
        355                 360                 365

ttt cac aag cat cac ctg tgg atg acc tat gaa gct gtt atc act gga     1152
Phe His Lys His His Leu Trp Met Thr Tyr Glu Ala Val Ile Thr Gly
    370                 375                 380

ttc ttt cct ttc ttt ctc att gcc aca gtc atc cag ctc ttc tac agg     1200
Phe Phe Pro Phe Phe Leu Ile Ala Thr Val Ile Gln Leu Phe Tyr Arg
385                 390                 395                 400

ggt aaa atc tgg aac atc ctc ctc ttc ctg tta act gtc cag cta gtg     1248
Gly Lys Ile Trp Asn Ile Leu Leu Phe Leu Leu Thr Val Gln Leu Val
                405                 410                 415

ggt ctc atc aag tca tct ttt gcc agc tgc ctt aga gga aat atc gtc     1296
Gly Leu Ile Lys Ser Ser Phe Ala Ser Cys Leu Arg Gly Asn Ile Val
            420                 425                 430

atg gta ttc atg tct ctg tat tca gtg tta tac atg tca agt cta ctt     1344

Met Val Phe Met Ser Leu Tyr Ser Val Leu Tyr Met Ser Ser Leu Leu
        435                 440                 445

cct gcc aag atg ttt gca att gca acc ata aac aaa gct ggg tgg ggc      1392
Pro Ala Lys Met Phe Ala Ile Ala Thr Ile Asn Lys Ala Gly Trp Gly
    450                 455                 460

aca tct gga agg aag acc att gtt gtt aat ttc ata gga ctt att cca      1440
Thr Ser Gly Arg Lys Thr Ile Val Val Asn Phe Ile Gly Leu Ile Pro
465                 470                 475                 480

gtg tcc gtg tgg ttt aca atc ctt cta ggt ggt gta att ttc acc att      1488
Val Ser Val Trp Phe Thr Ile Leu Leu Gly Gly Val Ile Phe Thr Ile
                485                 490                 495

tat aag gaa tct aaa aag cca ttt tcc gaa tcc aaa cag act gtt ctc      1536
Tyr Lys Glu Ser Lys Lys Pro Phe Ser Glu Ser Lys Gln Thr Val Leu
            500                 505                 510

atc gtg gga act ttg atc tat gca tgc tac tgg gtc atg ctt ttg act      1584
Ile Val Gly Thr Leu Ile Tyr Ala Cys Tyr Trp Val Met Leu Leu Thr
        515                 520                 525

ctc tat gtg gtt ctc atc aat aag tgt ggc agg cgg aag aag gga caa      1632
Leu Tyr Val Val Leu Ile Asn Lys Cys Gly Arg Arg Lys Lys Gly Gln
    530                 535                 540

cag tat gac atg gtg ctt gat gta tga                                  1659
Gln Tyr Asp Met Val Leu Asp Val
545                 550

<210>  18

<211>  552

<212>  PRT

<213>  Mus musculus

<400>  18
   Met His Cys Glu Arg Phe Leu Cys Val Leu Arg Ile Ile Gly Thr Thr
   1               5                   10                  15
   Leu Phe Gly Val Ser Leu Leu Leu Gly Ile Thr Ala Ala Tyr Ile Val
               20                  25                  30
   Gly Tyr Gln Phe Ile Gln Thr Asp Asn Tyr Tyr Phe Ser Phe Gly Leu
           35                  40                  45
   Tyr Gly Ala Phe Leu Ala Ser His Leu Ile Ile Gln Ser Leu Phe Ala
       50                  55                  60
   Phe Leu Glu His Arg Lys Met Lys Lys Ser Leu Glu Thr Pro Ile Lys
   65                  70                  75                  80
   Leu Asn Lys Thr Val Ala Leu Cys Ile Ala Ala Tyr Gln Glu Asp Pro
                   85                  90                  95
   Asp Tyr Leu Arg Lys Cys Leu Gln Ser Val Lys Arg Leu Thr Tyr Pro
               100                 105                 110
   Gly Ile Lys Val Val Met Val Ile Asp Gly Asn Ser Asp Asp Asp Leu
           115                 120                 125
   Tyr Met Met Asp Ile Phe Ser Glu Val Ile Gly Arg Asp Lys Ser Ala
       130                 135                 140
   Thr Tyr Ile Trp Lys Asn Asn Phe His Glu Lys Gly Pro Gly Glu Thr
   145                 150                 155                 160
   Glu Glu Ser His Lys Glu Ser Ser Gln His Val Thr Gln Leu Val Leu
                   165                 170                 175
   Ser Asn Lys Ser Ile Cys Ile Met Gln Lys Trp Gly Gly Lys Arg Glu
               180                 185                 190
   Val Met Tyr Thr Ala Phe Arg Ala Leu Gly Arg Ser Val Asp Tyr Val
           195                 200                 205
   Gln Val Cys Asp Ser Asp Thr Met Leu Asp Pro Ala Ser Ser Val Glu
       210                 215                 220
   Met Val Lys Val Leu Glu Glu Asp Pro Met Val Gly Gly Val Gly Gly
   225                 230                 235                 240
   Asp Val Gln Ile Leu Asn Lys Tyr Asp Ser Trp Ile Ser Phe Leu Ser
                   245                 250                 255
   Ser Val Arg Tyr Trp Met Ala Phe Asn Ile Glu Arg Ala Cys Gln Ser
               260                 265                 270
   Tyr Phe Gly Cys Val Gln Cys Ile Ser Gly Pro Leu Gly Met Tyr Arg
           275                 280                 285
   Asn Ser Leu Leu His Glu Phe Val Glu Asp Trp Tyr Asn Gln Glu Phe
       290                 295                 300
   Met Gly Asn Gln Cys Ser Phe Gly Asp Asp Arg His Leu Thr Asn Arg
   305                 310                 315                 320
   Val Leu Ser Leu Gly Tyr Ala Thr Lys Tyr Thr Ala Arg Ser Lys Cys
                   325                 330                 335
   Leu Thr Glu Thr Pro Ile Glu Tyr Leu Arg Trp Leu Asn Gln Gln Thr
               340                 345                 350
   Arg Trp Ser Lys Ser Tyr Phe Arg Glu Trp Leu Tyr Asn Ala Met Trp
           355                 360                 365
   Phe His Lys His His Leu Trp Met Thr Tyr Glu Ala Val Ile Thr Gly
       370                 375                 380
   Phe Phe Pro Phe Phe Leu Ile Ala Thr Val Ile Gln Leu Phe Tyr Arg
   385                 390                 395                 400
   Gly Lys Ile Trp Asn Ile Leu Leu Phe Leu Leu Thr Val Gln Leu Val
                   405                 410                 415
   Gly Leu Ile Lys Ser Ser Phe Ala Ser Cys Leu Arg Gly Asn Ile Val
               420                 425                 430
   Met Val Phe Met Ser Leu Tyr Ser Val Leu Tyr Met Ser Ser Leu Leu
           435                 440                 445
   Pro Ala Lys Met Phe Ala Ile Ala Thr Ile Asn Lys Ala Gly Trp Gly
       450                 455                 460
   Thr Ser Gly Arg Lys Thr Ile Val Val Asn Phe Ile Gly Leu Ile Pro
   465                 470                 475                 480
   Val Ser Val Trp Phe Thr Ile Leu Leu Gly Gly Val Ile Phe Thr Ile
                   485                 490                 495
   Tyr Lys Glu Ser Lys Lys Pro Phe Ser Glu Ser Lys Gln Thr Val Leu
               500                 505                 510
   Ile Val Gly Thr Leu Ile Tyr Ala Cys Tyr Trp Val Met Leu Leu Thr
           515                 520                 525
   Leu Tyr Val Val Leu Ile Asn Lys Cys Gly Arg Arg Lys Lys Gly Gln
       530                 535                 540
   Gln Tyr Asp Met Val Leu Asp Val
   545                 550

<210>  19

<211>  1665

<212>  DNA

<213>  Mus musculus


<220>

<221>  CDS

<222>  (1)..(1665)

<223>

<300>

<308>  U86408.2

<309>  1997-04-16

<313>  (175)..(1839)

<400>  19
atg ccg gtg cag ctg act aca gcc ctg cgt gtg gtg ggc acc agt ctg      48
Met Pro Val Gln Leu Thr Thr Ala Leu Arg Val Val Gly Thr Ser Leu
1               5                   10                  15

ttt gcc ctg gta gtg ctg gga ggc atc ctg gcg gcc tat gtg aca ggc      96
Phe Ala Leu Val Val Leu Gly Gly Ile Leu Ala Ala Tyr Val Thr Gly
            20                  25                  30

tac cag ttt atc cac aca gaa aag cac tac ctg tcc ttt ggc ctc tac      144
Tyr Gln Phe Ile His Thr Glu Lys His Tyr Leu Ser Phe Gly Leu Tyr
        35                  40                  45

ggt gcc atc ctg ggt cta cat ctg ctc atc cag agc ctg ttt gcc ttc      192
Gly Ala Ile Leu Gly Leu His Leu Leu Ile Gln Ser Leu Phe Ala Phe
    50                  55                  60

ctg gag cac cgt cga atg cgc agg gca ggg cgc ccc ctc aag ctg cac      240
Leu Glu His Arg Arg Met Arg Arg Ala Gly Arg Pro Leu Lys Leu His
65                  70                  75                  80

tgc tcc cag agg tcg cgt tca gtg gca ctc tgc att gct gcc tac caa      288
Cys Ser Gln Arg Ser Arg Ser Val Ala Leu Cys Ile Ala Ala Tyr Gln
                85                  90                  95

gag gac ccc gaa tac ctg cgc aag tgc ctt cgc tca gct cag cgc att      336
Glu Asp Pro Glu Tyr Leu Arg Lys Cys Leu Arg Ser Ala Gln Arg Ile
            100                 105                 110

gcc ttt cca aac ctc aag gtg gtc atg gta gtg gat ggc aat cgc cag      384
Ala Phe Pro Asn Leu Lys Val Val Met Val Val Asp Gly Asn Arg Gln
        115                 120                 125

gaa gat acc tac atg ttg gac atc ttc cat gag gtg ctg ggt ggc act      432
Glu Asp Thr Tyr Met Leu Asp Ile Phe His Glu Val Leu Gly Gly Thr
    130                 135                 140

gag caa gct ggc ttc ttt gtg tgg cgt agc aat ttc cat gag gcg ggt      480
Glu Gln Ala Gly Phe Phe Val Trp Arg Ser Asn Phe His Glu Ala Gly
145                 150                 155                 160

gaa gga gag aca gag gcc agc ctg cag gaa ggc atg gag cgt gtg cga      528
Glu Gly Glu Thr Glu Ala Ser Leu Gln Glu Gly Met Glu Arg Val Arg
                165                 170                 175

gct gtg gtg tgg gcc agc acc ttc tca tgc atc atg cag aag tgg ggg      576
Ala Val Val Trp Ala Ser Thr Phe Ser Cys Ile Met Gln Lys Trp Gly
            180                 185                 190

ggc aag cgt gag gtc atg tac act gcc ttc aag gcc ctt ggc aac tca      624
Gly Lys Arg Glu Val Met Tyr Thr Ala Phe Lys Ala Leu Gly Asn Ser
        195                 200                 205

gtg gac tac atc cag gtg tgt gac tct gac act gtg ctg gac cca gcc      672
Val Asp Tyr Ile Gln Val Cys Asp Ser Asp Thr Val Leu Asp Pro Ala
    210                 215                 220

tgc acc att gag atg ctt cga gtc ttg gaa gaa gat ccc caa gta gga      720

Cys Thr Ile Glu Met Leu Arg Val Leu Glu Glu Asp Pro Gln Val Gly
225                 230                 235                 240

ggt gtt gga gga gat gtc caa atc ctc aac aag tat gat tca tgg atc      768
Gly Val Gly Gly Asp Val Gln Ile Leu Asn Lys Tyr Asp Ser Trp Ile
                245                 250                 255

tcc ttc ctg agc agt gtg agg tac tgg atg gct ttc aac gtg gag cgg      816
Ser Phe Leu Ser Ser Val Arg Tyr Trp Met Ala Phe Asn Val Glu Arg
            260                 265                 270

gcc tgc cag tcc tac ttt ggc tgt gtg caa tgt att agt ggg cct ttg      864
Ala Cys Gln Ser Tyr Phe Gly Cys Val Gln Cys Ile Ser Gly Pro Leu
        275                 280                 285

ggc atg tac cgc aac agc ctc ctt cag cag ttc ctg gag gat tgg tac      912
Gly Met Tyr Arg Asn Ser Leu Leu Gln Gln Phe Leu Glu Asp Trp Tyr
    290                 295                 300

cat cag aag ttc cta ggc agc aag tgc agc ttt ggg gat gat cgg cac      960
His Gln Lys Phe Leu Gly Ser Lys Cys Ser Phe Gly Asp Asp Arg His
305                 310                 315                 320

ctt acc aac cga gtc ctg agt ctt ggc tac cgg act aag tat aca gca     1008
Leu Thr Asn Arg Val Leu Ser Leu Gly Tyr Arg Thr Lys Tyr Thr Ala
                325                 330                 335

cgc tct aag tgc ctc aca gag acc ccc act agg tac ctt cga tgg ctc     1056
Arg Ser Lys Cys Leu Thr Glu Thr Pro Thr Arg Tyr Leu Arg Trp Leu
            340                 345                 350

aat cag caa acc cgc tgg agc aag tct tac ttt cgg gaa tgg ctc tac     1104
Asn Gln Gln Thr Arg Trp Ser Lys Ser Tyr Phe Arg Glu Trp Leu Tyr
        355                 360                 365

aat tct ctg tgg ttc cat aag cac cac ctc tgg atg acc tat gaa tca     1152
Asn Ser Leu Trp Phe His Lys His His Leu Trp Met Thr Tyr Glu Ser
    370                 375                 380

gtg gtc aca ggt ttc ttc cca ttc ttc ctc att gct aca gtc ata caa     1200
Val Val Thr Gly Phe Phe Pro Phe Phe Leu Ile Ala Thr Val Ile Gln
385                 390                 395                 400

ctt ttc tac cgt ggc cgc atc tgg aac att ctc ctc ttc ctg cta aca     1248
Leu Phe Tyr Arg Gly Arg Ile Trp Asn Ile Leu Leu Phe Leu Leu Thr
                405                 410                 415

gtg cag ctg gtg ggc att atc aag gct acc tat gcc tgc ttc ctt cga     1296
Val Gln Leu Val Gly Ile Ile Lys Ala Thr Tyr Ala Cys Phe Leu Arg
            420                 425                 430

ggc aat gca gag atg atc ttc atg tcc ctc tac tcc ctt ctc tat atg     1344
Gly Asn Ala Glu Met Ile Phe Met Ser Leu Tyr Ser Leu Leu Tyr Met
        435                 440                 445

tcc agc ctc ttg cca gcc aag atc ttt gct att gct acc atc aac aag     1392
Ser Ser Leu Leu Pro Ala Lys Ile Phe Ala Ile Ala Thr Ile Asn Lys
    450                 455                 460

tct ggc tgg ggc act tct ggc agg aaa acc att gtc gtg aac ttc att     1440
Ser Gly Trp Gly Thr Ser Gly Arg Lys Thr Ile Val Val Asn Phe Ile
465                 470                 475                 480

ggc cta atc ccc gtg tcc atc tgg gtg gca gtt ctt cta ggg ggg tta     1488
Gly Leu Ile Pro Val Ser Ile Trp Val Ala Val Leu Leu Gly Gly Leu
                485                 490                 495

gcc tac aca gct tat tgc cag gac ctg ttc agt gag acc gag cta gcc     1536

Ala Tyr Thr Ala Tyr Cys Gln Asp Leu Phe Ser Glu Thr Glu Leu Ala
            500                 505                 510

ttc cta gtc tct ggg gcc atc ctg tat ggc tgc tac tgg gtg gcc ctc      1584
Phe Leu Val Ser Gly Ala Ile Leu Tyr Gly Cys Tyr Trp Val Ala Leu
        515                 520                 525

ctc atg ctg tat ctg gcc att att gcc cgg agg tgt ggg aag aag cca      1632
Leu Met Leu Tyr Leu Ala Ile Ile Ala Arg Arg Cys Gly Lys Lys Pro
    530                 535                 540

gaa cag tat agc ctg gct ttt gcg gag gtg tga                          1665
Glu Gln Tyr Ser Leu Ala Phe Ala Glu Val
545                 550

<210>  20

<211>  554

<212>  PRT

<213>  Mus musculus

<400>  20

Met Pro Val Gln Leu Thr Thr Ala Leu Arg Val Val Gly Thr Ser Leu
1               5                   10                  15

Phe Ala Leu Val Val Leu Gly Gly Ile Leu Ala Ala Tyr Val Thr Gly
            20                  25                  30

Tyr Gln Phe Ile His Thr Glu Lys His Tyr Leu Ser Phe Gly Leu Tyr
        35                  40                  45

Gly Ala Ile Leu Gly Leu His Leu Leu Ile Gln Ser Leu Phe Ala Phe
    50                  55                  60

Leu Glu His Arg Arg Met Arg Arg Ala Gly Arg Pro Leu Lys Leu His
65                  70                  75                  80

Cys Ser Gln Arg Ser Arg Ser Val Ala Leu Cys Ile Ala Ala Tyr Gln
                85                  90                  95

Glu Asp Pro Glu Tyr Leu Arg Lys Cys Leu Arg Ser Ala Gln Arg Ile
            100                 105                 110

Ala Phe Pro Asn Leu Lys Val Val Met Val Val Asp Gly Asn Arg Gln
        115                 120                 125

Glu Asp Thr Tyr Met Leu Asp Ile Phe His Glu Val Leu Gly Gly Thr
    130                 135                 140

Glu Gln Ala Gly Phe Phe Val Trp Arg Ser Asn Phe His Glu Ala Gly
145                 150                 155                 160

Glu Gly Glu Thr Glu Ala Ser Leu Gln Glu Gly Met Glu Arg Val Arg
                165                 170                 175

Ala Val Val Trp Ala Ser Thr Phe Ser Cys Ile Met Gln Lys Trp Gly
            180                 185                 190

Gly Lys Arg Glu Val Met Tyr Thr Ala Phe Lys Ala Leu Gly Asn Ser
        195                 200                 205

Val Asp Tyr Ile Gln Val Cys Asp Ser Asp Thr Val Leu Asp Pro Ala
    210                 215                 220

Cys Thr Ile Glu Met Leu Arg Val Leu Glu Glu Asp Pro Gln Val Gly
225                 230                 235                 240

Gly Val Gly Gly Asp Val Gln Ile Leu Asn Lys Tyr Asp Ser Trp Ile
                245                 250                 255

Ser Phe Leu Ser Ser Val Arg Tyr Trp Met Ala Phe Asn Val Glu Arg
            260                 265                 270

Ala Cys Gln Ser Tyr Phe Gly Cys Val Gln Cys Ile Ser Gly Pro Leu
        275                 280                 285

Gly Met Tyr Arg Asn Ser Leu Leu Gln Gln Phe Leu Glu Asp Trp Tyr
    290                 295                 300

His Gln Lys Phe Leu Gly Ser Lys Cys Ser Phe Gly Asp Asp Arg His
305                 310                 315                 320

Leu Thr Asn Arg Val Leu Ser Leu Gly Tyr Arg Thr Lys Tyr Thr Ala
                325                 330                 335

Arg Ser Lys Cys Leu Thr Glu Thr Pro Thr Arg Tyr Leu Arg Trp Leu
            340                 345                 350

Asn Gln Gln Thr Arg Trp Ser Lys Ser Tyr Phe Arg Glu Trp Leu Tyr
        355                 360                 365

Asn Ser Leu Trp Phe His Lys His His Leu Trp Met Thr Tyr Glu Ser
    370                 375                 380

Val Val Thr Gly Phe Phe Pro Phe Phe Leu Ile Ala Thr Val Ile Gln
385                 390                 395                 400

Leu Phe Tyr Arg Gly Arg Ile Trp Asn Ile Leu Leu Phe Leu Leu Thr
                405                 410                 415

Val Gln Leu Val Gly Ile Ile Lys Ala Thr Tyr Ala Cys Phe Leu Arg
            420                 425                 430

Gly Asn Ala Glu Met Ile Phe Met Ser Leu Tyr Ser Leu Leu Tyr Met
        435                 440                 445

Ser Ser Leu Leu Pro Ala Lys Ile Phe Ala Ile Ala Thr Ile Asn Lys
    450                 455                 460

Ser Gly Trp Gly Thr Ser Gly Arg Lys Thr Ile Val Val Asn Phe Ile
465                 470                 475                 480

Gly Leu Ile Pro Val Ser Ile Trp Val Ala Val Leu Leu Gly Gly Leu
                485                 490                 495

Ala Tyr Thr Ala Tyr Cys Gln Asp Leu Phe Ser Glu Thr Glu Leu Ala
            500                 505                 510

Phe Leu Val Ser Gly Ala Ile Leu Tyr Gly Cys Tyr Trp Val Ala Leu
        515                 520                 525

Leu Met Leu Tyr Leu Ala Ile Ile Ala Arg Arg Cys Gly Lys Lys Pro
    530                 535                 540

Glu Gln Tyr Ser Leu Ala Phe Ala Glu Val
545                 550

<210>  21

<211>  1752

<212>  DNA

<213>  Rattus norvegicus


<220>

<221>  CDS

<222>  (1)..(1752)

<223>


<300>

<308>  AB097568

<309>  2004-04-28


<400>  21
atg aga cag gac atg cca aag ccc tca gag gca gca cgt tgc tgc tcc      48
Met Arg Gln Asp Met Pro Lys Pro Ser Glu Ala Ala Arg Cys Cys Ser
1               5                   10                  15

ggt ctg gcc agg cgg gtg ctc acg atc acc ttc gcc ctg ctc atc ctg      96
Gly Leu Ala Arg Arg Val Leu Thr Ile Thr Phe Ala Leu Leu Ile Leu
            20                  25                  30

ggc ctc atg acc tgg gcc tac gca gca gga gta cct ctg gct tct gat 144
Gly Leu Met Thr Trp Ala Tyr Ala Ala Gly Val Pro Leu Ala Ser Asp
        35                  40                  45

ccc tat ggc ctc ctg gcc ttt ggg ctc tat ggg gcg ttc ctc agt gcg 192
Pro Tyr Gly Leu Leu Ala Phe Gly Leu Tyr Gly Ala Phe Leu Ser Ala
    50                  55                  60

cac cta gtg gca cag agc ctc ttc gct tac ctg gag cac cga agg gtg 240
His Leu Val Ala Gln Ser Leu Phe Ala Tyr Leu Glu His Arg Arg Val
65                  70                  75                  80

acc gtg gct gcg cgg cgc gct ttt gcg aag gga ccc ctg gat gcg gcc 288
Thr Val Ala Ala Arg Arg Ala Phe Ala Lys Gly Pro Leu Asp Ala Ala
                85                  90                  95

act gcg cgc agc gtg gca ctc acc atc tca gcc tac cag gag gac ccc 336
Thr Ala Arg Ser Val Ala Leu Thr Ile Ser Ala Tyr Gln Glu Asp Pro
            100                 105                 110

act tac ctg cgc cag tgc ttg acc tcc gcg cgc gcc ttg ctg tac ccg 384
Thr Tyr Leu Arg Gln Cys Leu Thr Ser Ala Arg Ala Leu Leu Tyr Pro
        115                 120                 125

cgc acg cgg ctg cgc gtg ctt atg gtt gta gac ggc aat cgc gcg gag 432
Arg Thr Arg Leu Arg Val Leu Met Val Val Asp Gly Asn Arg Ala Glu
    130                 135                 140

gat ctg tac atg gtg gac atg ttc cga gaa gtc ttc gca gat gag gac 480
Asp Leu Tyr Met Val Asp Met Phe Arg Glu Val Phe Ala Asp Glu Asp
145                 150                 155                 160

cct gcc act tat gtg tgg gat ggc aac tac cat cag cct tgg gag cca 528
Pro Ala Thr Tyr Val Trp Asp Gly Asn Tyr His Gln Pro Trp Glu Pro
                165                 170                 175

gct gag gcg gcg ggt gct gtg ggt gaa ggt gcc tac cgc gag gtg gag 576
Ala Glu Ala Ala Gly Ala Val Gly Glu Gly Ala Tyr Arg Glu Val Glu
            180                 185                 190

gct gag gac cct ggg cgg ctg gcg gta gag gcg ctg gtg agg acc cgc 624
Ala Glu Asp Pro Gly Arg Leu Ala Val Glu Ala Leu Val Arg Thr Arg
        195                 200                 205

agg tgc gtg tgc gtg gct cag cgc tgg ggt ggc aag cgc gaa gtc atg 672
Arg Cys Val Cys Val Ala Gln Arg Trp Gly Gly Lys Arg Glu Val Met
    210                 215                 220

tac acg gct ttc aag gca ctg ggt gac tcc gtg gac tac gtg cag gtc 720
Tyr Thr Ala Phe Lys Ala Leu Gly Asp Ser Val Asp Tyr Val Gln Val
225                 230                 235                 240

tgt gac tca gac aca agg tta gac ccc atg gca ctg ctg gag ctt gtg 768
Cys Asp Ser Asp Thr Arg Leu Asp Pro Met Ala Leu Leu Glu Leu Val
                245                 250                 255

cga gtg ctg gat gaa gac ccc cgg gta ggt gct gtt gga gga gat gtg 816
Arg Val Leu Asp Glu Asp Pro Arg Val Gly Ala Val Gly Gly Asp Val
            260                 265                 270

aga atc ctt aac cct ctg gac tct tgg gtc agc ttc ttg agc agc ctt 864
Arg Ile Leu Asn Pro Leu Asp Ser Trp Val Ser Phe Leu Ser Ser Leu
        275                 280                 285

cga tac tgg gta gcc ttc aat gtg gag cga gct tgt cag agc tac ttt 912
Arg Tyr Trp Val Ala Phe Asn Val Glu Arg Ala Cys Gln Ser Tyr Phe
    290                 295                 300

cac tgt gtg tcc tgc atc agt ggt cct ctg ggt cta tac aga aac aat 960
His Cys Val Ser Cys Ile Ser Gly Pro Leu Gly Leu Tyr Arg Asn Asn
305                 310                 315                 320

ctc ctg cag cag ttc ctg gag gcc tgg tac aac cag aag ttc ctg ggc 1008
Leu Leu Gln Gln Phe Leu Glu Ala Trp Tyr Asn Gln Lys Phe Leu Gly
                325                 330                 335

acc cac tgc aca ttt ggg gat gac agg cac ctc acc aac cgc atg ctt 1056
Thr His Cys Thr Phe Gly Asp Asp Arg His Leu Thr Asn Arg Met Leu
            340                 345                 350

agc atg ggc tac gct acc aag tat acc tcg cgc tcc aga tgc tat tca 1104
Ser Met Gly Tyr Ala Thr Lys Tyr Thr Ser Arg Ser Arg Cys Tyr Ser
        355                 360                 365

gag acg ccc tcc tct ttc ctt cgt tgg ctg agc cag cag acc cgc tgg 1152
Glu Thr Pro Ser Ser Phe Leu Arg Trp Leu Ser Gln Gln Thr Arg Trp
    370                 375                 380

tcc aaa tct tat ttc cga gag tgg cta tac aac gcc ctg tgg tgg cac 1200
Ser Lys Ser Tyr Phe Arg Glu Trp Leu Tyr Asn Ala Leu Trp Trp His
385                 390                 395                 400

cgc cac cac gcg tgg atg acc tat gaa gcg gtg gtt tct ggc ctc ttc 1248
Arg His His Ala Trp Met Thr Tyr Glu Ala Val Val Ser Gly Leu Phe
                405                 410                 415

cct ttc ttt gtg gct gcc acg gtg ctg agg ctc ttc tat gca ggg cgc 1296
Pro Phe Phe Val Ala Ala Thr Val Leu Arg Leu Phe Tyr Ala Gly Arg
            420                 425                 430

cca tgg gct ctg ctc tgg gtg ctg ctc tgc gtg cag ggc gtg gca ctg 1344
Pro Trp Ala Leu Leu Trp Val Leu Leu Cys Val Gln Gly Val Ala Leu
        435                 440                 445

gca aag gca gcc ttt gca gcc tgg ctg cgt ggc tgc ctg cgc atg gtg 1392
Ala Lys Ala Ala Phe Ala Ala Trp Leu Arg Gly Cys Leu Arg Met Val
    450                 455                 460

ctg ctg tca ctc tat gca cca ctc tac atg tgc ggc ctg ctg cct gcc 1440
Leu Leu Ser Leu Tyr Ala Pro Leu Tyr Met Cys Gly Leu Leu Pro Ala
465                 470                 475                 480

aag ttc ctg gcg ttg gtt acc atg aat caa agt ggt tgg ggc acc tcg 1488
Lys Phe Leu Ala Leu Val Thr Met Asn Gln Ser Gly Trp Gly Thr Ser
                485                 490                 495

ggc cgg aag aaa ctg gct gct aac tat gta ccc gtg ttg ccc ctg gca 1536
Gly Arg Lys Lys Leu Ala Ala Asn Tyr Val Pro Val Leu Pro Leu Ala
            500                 505                 510

ctc tgg gct cta ctg ctg ctt gga ggc ctg atc cgc agt gtg gcc cag 1584
Leu Trp Ala Leu Leu Leu Leu Gly Gly Leu Ile Arg Ser Val Ala Gln
        515                 520                 525

gag gtc aga gct gac tgg agt ggc cca tca cga gca gct gaa gcc tac 1632
Glu Val Arg Ala Asp Trp Ser Gly Pro Ser Arg Ala Ala Glu Ala Tyr
    530                 535                 540

cac ctt gct gct ggg gcc agt gcc tat gtg gcc tac tgg gtg ata atg 1680
His Leu Ala Ala Gly Ala Ser Ala Tyr Val Ala Tyr Trp Val Ile Met
545                 550                 555                 560

ttg act atc tat tgg gta ggt gta agg aga ctg tgc aga cgt cgg agc 1728
Leu Thr Ile Tyr Trp Val Gly Val Arg Arg Leu Cys Arg Arg Arg Ser
                565                 570                 575

ggt ggc tac cgt gtc caa gta tga                                    1752
Gly Gly Tyr Arg Val Gln Val
            580

<210>  22

<211>  583

<212>  PRT

<213>  Rattus norvegicus

<400>  22

Met Arg Gln Asp Met Pro Lys Pro Ser Glu Ala Ala Arg Cys Cys Ser
1               5                   10                  15

Gly Leu Ala Arg Arg Val Leu Thr Ile Thr Phe Ala Leu Leu Ile Leu
            20                  25                  30

Gly Leu Met Thr Trp Ala Tyr Ala Ala Gly Val Pro Leu Ala Ser Asp
        35                  40                  45

Pro Tyr Gly Leu Leu Ala Phe Gly Leu Tyr Gly Ala Phe Leu Ser Ala
    50                  55                  60

His Leu Val Ala Gln Ser Leu Phe Ala Tyr Leu Glu His Arg Arg Val
65                  70                  75                  80

Thr Val Ala Ala Arg Arg Ala Phe Ala Lys Gly Pro Leu Asp Ala Ala
                85                  90                  95

Thr Ala Arg Ser Val Ala Leu Thr Ile Ser Ala Tyr Gln Glu Asp Pro
            100                 105                 110

Thr Tyr Leu Arg Gln Cys Leu Thr Ser Ala Arg Ala Leu Leu Tyr Pro
        115                 120                 125

Arg Thr Arg Leu Arg Val Leu Met Val Val Asp Gly Asn Arg Ala Glu
    130                 135                 140

Asp Leu Tyr Met Val Asp Met Phe Arg Glu Val Phe Ala Asp Glu Asp
145                 150                 155                 160

Pro Ala Thr Tyr Val Trp Asp Gly Asn Tyr His Gln Pro Trp Glu Pro
                165                 170                 175

Ala Glu Ala Ala Gly Ala Val Gly Glu Gly Ala Tyr Arg Glu Val Glu
            180                 185                 190

Ala Glu Asp Pro Gly Arg Leu Ala Val Glu Ala Leu Val Arg Thr Arg
        195                 200                 205

Arg Cys Val Cys Val Ala Gln Arg Trp Gly Gly Lys Arg Glu Val Met
    210                 215                 220

Tyr Thr Ala Phe Lys Ala Leu Gly Asp Ser Val Asp Tyr Val Gln Val
225                 230                 235                 240

Cys Asp Ser Asp Thr Arg Leu Asp Pro Met Ala Leu Leu Glu Leu Val
                245                 250                 255

Arg Val Leu Asp Glu Asp Pro Arg Val Gly Ala Val Gly Gly Asp Val
            260                 265                 270

Arg Ile Leu Asn Pro Leu Asp Ser Trp Val Ser Phe Leu Ser Ser Leu
        275                 280                 285

Arg Tyr Trp Val Ala Phe Asn Val Glu Arg Ala Cys Gln Ser Tyr Phe
    290                 295                 300

His Cys Val Ser Cys Ile Ser Gly Pro Leu Gly Leu Tyr Arg Asn Asn
305                 310                 315                 320

Leu Leu Gln Gln Phe Leu Glu Ala Trp Tyr Asn Gln Lys Phe Leu Gly
                325                 330                 335

Thr His Cys Thr Phe Gly Asp Asp Arg His Leu Thr Asn Arg Met Leu
            340                 345                 350

Ser Met Gly Tyr Ala Thr Lys Tyr Thr Ser Arg Ser Arg Cys Tyr Ser
        355                 360                 365

Glu Thr Pro Ser Ser Phe Leu Arg Trp Leu Ser Gln Gln Thr Arg Trp
    370                 375                 380

Ser Lys Ser Tyr Phe Arg Glu Trp Leu Tyr Asn Ala Leu Trp Trp His
385                 390                 395                 400

Arg His His Ala Trp Met Thr Tyr Glu Ala Val Val Ser Gly Leu Phe
                405                 410                 415

Pro Phe Phe Val Ala Ala Thr Val Leu Arg Leu Phe Tyr Ala Gly Arg
            420                 425                 430

Pro Trp Ala Leu Leu Trp Val Leu Leu Cys Val Gln Gly Val Ala Leu
        435                 440                 445

Ala Lys Ala Ala Phe Ala Ala Trp Leu Arg Gly Cys Leu Arg Met Val
    450                 455                 460

Leu Leu Ser Leu Tyr Ala Pro Leu Tyr Met Cys Gly Leu Leu Pro Ala
465                 470                 475                 480

Lys Phe Leu Ala Leu Val Thr Met Asn Gln Ser Gly Trp Gly Thr Ser
                485                 490                 495

Gly Arg Lys Lys Leu Ala Ala Asn Tyr Val Pro Val Leu Pro Leu Ala
            500                 505                 510

Leu Trp Ala Leu Leu Leu Leu Gly Gly Leu Ile Arg Ser Val Ala Gln
        515                 520                 525

Glu Val Arg Ala Asp Trp Ser Gly Pro Ser Arg Ala Ala Glu Ala Tyr
    530                 535                 540

His Leu Ala Ala Gly Ala Ser Ala Tyr Val Ala Tyr Trp Val Ile Met
545                 550                 555                 560

Leu Thr Ile Tyr Trp Val Gly Val Arg Arg Leu Cys Arg Arg Arg Ser
                565                 570                 575

Gly Gly Tyr Arg Val Gln Val
            580

<210> 23

<211> 1659

<212> DNA

<213> Rattus norvegicus

<220>

<221> CDS

<222> (1)..(1659)

<223>

<300>

<308> AF008201.1

<309> 1997-07-16

<313> (507)..(2165)

<400> 23
atg cat tgt gag agg ttt cta tgt gtc ctg aga ata atc gga acc aca 48
Met His Cys Glu Arg Phe Leu Cys Val Leu Arg Ile Ile Gly Thr Thr
1               5                   10                  15

ctg ttt gga gtg tct ctc ctc ctc gga atc aca gct gct tat att gtt 96
Leu Phe Gly Val Ser Leu Leu Leu Gly Ile Thr Ala Ala Tyr Ile Val
            20                  25                  30

ggc tac cag ttt atc caa aca gat aat tac tat ttc tcc ttt ggg ctg 144
Gly Tyr Gln Phe Ile Gln Thr Asp Asn Tyr Tyr Phe Ser Phe Gly Leu
        35                  40                  45

tat ggt gcc ttt tta gcc tca cac ctc atc atc caa agc ctc ttt gcc 192
Tyr Gly Ala Phe Leu Ala Ser His Leu Ile Ile Gln Ser Leu Phe Ala
    50                  55                  60

ttt ttg gaa cac cgg aaa atg aaa aag tcc ctt gaa acc ccc att aaa 240
Phe Leu Glu His Arg Lys Met Lys Lys Ser Leu Glu Thr Pro Ile Lys
65                  70                  75                  80

ttg aac aaa act gta gct ctc tgc att gct gcg tat caa gaa gac cct 288
Leu Asn Lys Thr Val Ala Leu Cys Ile Ala Ala Tyr Gln Glu Asp Pro
                85                  90                  95

gac tac tta cgg aaa tgt ttg caa tct gtg aaa agg ctg acc tac cct 336
Asp Tyr Leu Arg Lys Cys Leu Gln Ser Val Lys Arg Leu Thr Tyr Pro
            100                 105                 110

ggg atc aaa gtc gtg atg gtc ata gat gga aat tca gat gac gac ctt 384
Gly Ile Lys Val Val Met Val Ile Asp Gly Asn Ser Asp Asp Asp Leu
        115                 120                 125

tac atg atg gac atc ttc agt gaa gtc atg ggc agg gac aaa tca gtc 432
Tyr Met Met Asp Ile Phe Ser Glu Val Met Gly Arg Asp Lys Ser Val
    130                 135                 140

act tac atc tgg aag aac aac ttc cat gaa agg gga cct ggt gag aca 480
Thr Tyr Ile Trp Lys Asn Asn Phe His Glu Arg Gly Pro Gly Glu Thr
145                 150                 155                 160

gaa gag tcc cat aaa gaa agt tcg caa cat gta acc caa ttg gtc ttg 528
Glu Glu Ser His Lys Glu Ser Ser Gln His Val Thr Gln Leu Val Leu
                165                 170                 175

tct aac aag agt att tgc atc atg caa aaa tgg ggt gga aag aga gaa 576
Ser Asn Lys Ser Ile Cys Ile Met Gln Lys Trp Gly Gly Lys Arg Glu
            180                 185                 190

gtc atg tac acc gcc ttc aga gca ctg ggc aga agc gtg gat tat gta 624
Val Met Tyr Thr Ala Phe Arg Ala Leu Gly Arg Ser Val Asp Tyr Val
        195                 200                 205

cag gtg tgt gac tca gac acc atg ctt gac cct gcc tcg tct gtg gag 672
Gln Val Cys Asp Ser Asp Thr Met Leu Asp Pro Ala Ser Ser Val Glu
    210                 215                 220

atg gtg aag gtc tta gaa gaa gac ccg atg gtt gga ggt gtt ggg gga 720
Met Val Lys Val Leu Glu Glu Asp Pro Met Val Gly Gly Val Gly Gly
225                 230                 235                 240

gat gtt cag att tta aac aag tat gat tct tgg atc tcc ttc ctc agc 768
Asp Val Gln Ile Leu Asn Lys Tyr Asp Ser Trp Ile Ser Phe Leu Ser
                245                 250                 255

agt gtg aga tac tgg atg gct ttt aat ata gaa agg gcc tgc cag tct 816
Ser Val Arg Tyr Trp Met Ala Phe Asn Ile Glu Arg Ala Cys Gln Ser
            260                 265                 270

tat ttt ggc tgt gtc cag tgc ata agc ggt cct ctg gga atg tac aga 864
Tyr Phe Gly Cys Val Gln Cys Ile Ser Gly Pro Leu Gly Met Tyr Arg
        275                 280                 285

aac tcc ttg ttg cat gag ttt gtg gaa gac tgg tac aat cag gaa ttc 912
Asn Ser Leu Leu His Glu Phe Val Glu Asp Trp Tyr Asn Gln Glu Phe
    290                 295                 300

atg ggt aac caa tgc agt ttc ggt gat gat agg cac ctt acc aac agg 960
Met Gly Asn Gln Cys Ser Phe Gly Asp Asp Arg His Leu Thr Asn Arg
305                 310                 315                 320

gta ctg agt ctg ggc tat gca act aaa tac acg gct cgg tcc aag tgc 1008
Val Leu Ser Leu Gly Tyr Ala Thr Lys Tyr Thr Ala Arg Ser Lys Cys
                325                 330                 335

ctt act gaa aca ccc ata gaa tat ctg aga tgg ctg aac cag cag acc 1056
Leu Thr Glu Thr Pro Ile Glu Tyr Leu Arg Trp Leu Asn Gln Gln Thr
            340                 345                 350

cgt tgg agc aag tcc tac ttc cga gag tgg cta tac aat gcc atg tgg 1104
Arg Trp Ser Lys Ser Tyr Phe Arg Glu Trp Leu Tyr Asn Ala Met Trp
        355                 360                 365

ttt cac aag cat cac ttg tgg atg acc tac gaa gct gtt atc act gga 1152
Phe His Lys His His Leu Trp Met Thr Tyr Glu Ala Val Ile Thr Gly
    370                 375                 380

ttc ttt cct ttc ttt ctc att gcc aca gtc atc cag ctc ttc tac agg 1200
Phe Phe Pro Phe Phe Leu Ile Ala Thr Val Ile Gln Leu Phe Tyr Arg
385                 390                 395                 400

ggt aaa atc tgg aac atc ctc ctc ttc ctg tta act gtc cag cta gtg 1248
Gly Lys Ile Trp Asn Ile Leu Leu Phe Leu Leu Thr Val Gln Leu Val
                405                 410                 415

ggt ctc atc aag tcg tct ttc gcc agc tgc ctt aga gga aat atc gtc 1296
Gly Leu Ile Lys Ser Ser Phe Ala Ser Cys Leu Arg Gly Asn Ile Val
            420                 425                 430

atg gtc ttc atg tct ctc tac tca gtg ttg tac atg tca agt cta ctt 1344
Met Val Phe Met Ser Leu Tyr Ser Val Leu Tyr Met Ser Ser Leu Leu
        435                 440                 445

cct gcc aag atg ttt gca att gca acc ata aac aaa gct ggg tgg ggc 1392
Pro Ala Lys Met Phe Ala Ile Ala Thr Ile Asn Lys Ala Gly Trp Gly
    450                 455                 460

aca tct gga agg aaa acc att gtc gtt aat ttc ata gga ctt att cca 1440
Thr Ser Gly Arg Lys Thr Ile Val Val Asn Phe Ile Gly Leu Ile Pro
465                 470                 475                 480

gtg tcc gtg tgg ttt aca atc ctt cta ggt ggt gtg att ttc acc att 1488
Val Ser Val Trp Phe Thr Ile Leu Leu Gly Gly Val Ile Phe Thr Ile
                485                 490                 495

tat aag gaa tct aaa aag cca ttt tcc gaa tcc aaa cag act gtt ctc 1536
Tyr Lys Glu Ser Lys Lys Pro Phe Ser Glu Ser Lys Gln Thr Val Leu
            500                 505                 510

att gtg gga acc ttg atc tat gcg tgc tac tgg gtc gtg ctt ttg act 1584
Ile Val Gly Thr Leu Ile Tyr Ala Cys Tyr Trp Val Val Leu Leu Thr
        515                 520                 525

ctg tat gtg gtt ctc atc aat aag tgt ggc agg cgg aag aag gga caa 1632
Leu Tyr Val Val Leu Ile Asn Lys Cys Gly Arg Arg Lys Lys Gly Gln
    530                 535                 540

cag tac gac atg gtg ctt gat gta tga 1659
Gln Tyr Asp Met Val Leu Asp Val
545                 550

<210> 24

<211>  552

<212>  PRT

<213>  Rattus norvegicus

<400>  24

Met His Cys Glu Arg Phe Leu Cys Val Leu Arg Ile Ile Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Asp Asp Asp Leu
        115                 120                 125

Tyr Met Met Asp Ile Phe Ser Glu Val Met Gly Arg Asp Lys Ser Val
    130                 135                 140

Thr Tyr Ile Trp Lys Asn Asn Phe His Glu Arg Gly Pro Gly Glu Thr
145                 150                 155                 160

Glu Glu Ser His Lys Glu Ser Ser Gln His Val Thr Gln Leu Val Leu
                165                 170                 175

Ser Asn Lys Ser Ile Cys Ile Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Asn Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Lys Glu Ser Lys Lys Pro Phe Ser Glu Ser Lys Gln Thr Val Leu
            500                 505                 510

Ile Val Gly Thr Leu Ile Tyr Ala Cys Tyr Trp Val Val Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  25

<211>  1665

<212>  DNA

<213>  Rattus norvegicus

<220>

<221>  CDS

<222>  (1)..(1665)

<223>

<300>

<308>  NM 172319

<309>  2004-08-21

<400>  25
atg ccg gtg cag ctg act aca gcc ctt cgt gtg gtg ggc acc agt ctg      48
Met Pro Val Gln Leu Thr Thr Ala Leu Arg Val Val Gly Thr Ser Leu
1               5                   10                  15

ttt gcc ctg gta gtg ctg ggg ggc atc ctg gca gcc tat gtc aca ggc      96
Phe Ala Leu Val Val Leu Gly Gly Ile Leu Ala Ala Tyr Val Thr Gly
            20                  25                  30

tac cag ttt atc cac acg gaa aag cac tat ctg tcc ttc ggc ctc tac      144
Tyr Gln Phe Ile His Thr Glu Lys His Tyr Leu Ser Phe Gly Leu Tyr
        35                  40                  45

ggt gcc atc ctg ggt ctg cat ctg ctc atc cag agc ctg ttt gcc ttc      192
Gly Ala Ile Leu Gly Leu His Leu Leu Ile Gln Ser Leu Phe Ala Phe
    50                  55                  60

ctg gag cac cgt cgc atg cgc agg gca ggg cgc cca ctg aag ctg cac      240
Leu Glu His Arg Arg Met Arg Arg Ala Gly Arg Pro Leu Lys Leu His
65                  70                  75                  80

tgc tct cag aga cgg cgt tcg gtg gca ctc tgc atc gct gcc tac caa      288
Cys Ser Gln Arg Arg Arg Ser Val Ala Leu Cys Ile Ala Ala Tyr Gln
                85                  90                  95

gag gac cct gag tac ttg cgc aag tgc ctt cgc tca gct cag cgc att      336

Glu Asp Pro Glu Tyr Leu Arg Lys Cys Leu Arg Ser Ala Gln Arg Ile
            100                 105                 110

gcc ttc cca aac ctc aag gtg gtc atg gta gtg gat ggc aat cgc cag      384
Ala Phe Pro Asn Leu Lys Val Val Met Val Val Asp Gly Asn Arg Gln
        115                 120                 125

gaa gat gcc tac atg ctg gac atc ttc cat gag gtc ctg ggt ggc act      432
Glu Asp Ala Tyr Met Leu Asp Ile Phe His Glu Val Leu Gly Gly Thr
    130                 135                 140

gag caa gct ggc ttc ttt gtg tgg cgt agc aat ttc cat gag gcg ggt      480
Glu Gln Ala Gly Phe Phe Val Trp Arg Ser Asn Phe His Glu Ala Gly
145                 150                 155                 160

gaa gga gag aca gag gcc agc ttg cag gaa ggc atg gag cgt gtt cga      528
Glu Gly Glu Thr Glu Ala Ser Leu Gln Glu Gly Met Glu Arg Val Arg
                165                 170                 175

gct gtg gtg tgg gcc agc acc ttc tca tgc atc atg cag aag tgg ggg      576
Ala Val Val Trp Ala Ser Thr Phe Ser Cys Ile Met Gln Lys Trp Gly
            180                 185                 190

ggc aag cgt gaa gtc atg tac aca gct ttc aag gcc ctt ggc aac tca      624
Gly Lys Arg Glu Val Met Tyr Thr Ala Phe Lys Ala Leu Gly Asn Ser
        195                 200                 205

gtg gac tac atc cag gtg tgt gac tct gac act gtg ctg gac cca gcc      672
Val Asp Tyr Ile Gln Val Cys Asp Ser Asp Thr Val Leu Asp Pro Ala
    210                 215                 220

tgc acc att gag atg ctt cgg gtc ttg gag gaa gat ccc caa gta gga      720
Cys Thr Ile Glu Met Leu Arg Val Leu Glu Glu Asp Pro Gln Val Gly
225                 230                 235                 240

ggt gtt gga gga gat gtc caa atc ctc aac aaa tac gat tca tgg atc      768
Gly Val Gly Gly Asp Val Gln Ile Leu Asn Lys Tyr Asp Ser Trp Ile
                245                 250                 255

tcc ttc ctg agc agc gtg agg tac tgg atg gcc ttc aac gtg gag cgg      816
Ser Phe Leu Ser Ser Val Arg Tyr Trp Met Ala Phe Asn Val Glu Arg
            260                 265                 270

gcc tgc cag tcc tac ttt ggc tgt gtg cag tgt att agt ggg cct ctg      864
Ala Cys Gln Ser Tyr Phe Gly Cys Val Gln Cys Ile Ser Gly Pro Leu
        275                 280                 285

ggc atg tac cgc aac agc ctc ctt cag cag ttc ctg gag gac tgg tac      912
Gly Met Tyr Arg Asn Ser Leu Leu Gln Gln Phe Leu Glu Asp Trp Tyr
    290                 295                 300

cat cag aag ttc cta ggc agc aag tgc agc ttt ggg gat gat cgg cac      960
His Gln Lys Phe Leu Gly Ser Lys Cys Ser Phe Gly Asp Asp Arg His
305                 310                 315                 320

ctt acc aac cga gtc ctg agt ctt ggc tac cgg act aag tat aca gca     1008
Leu Thr Asn Arg Val Leu Ser Leu Gly Tyr Arg Thr Lys Tyr Thr Ala
                325                 330                 335

cgc tcc aag tgc ctc aca gag acc ccc act aag tat ctc cga tgg ctc     1056
Arg Ser Lys Cys Leu Thr Glu Thr Pro Thr Lys Tyr Leu Arg Trp Leu
            340                 345                 350

aac cag cag acc cgc tgg agc aag tct tac ttt cgg gag tgg ctc tac     1104
Asn Gln Gln Thr Arg Trp Ser Lys Ser Tyr Phe Arg Glu Trp Leu Tyr
        355                 360                 365

aac tct ctg tgg ttc cat aag cac cac ctg tgg atg acc tat gag tca     1152

Asn Ser Leu Trp Phe His Lys His His Leu Trp Met Thr Tyr Glu Ser
    370                 375                 380

gtg gtc aca ggc ttc ttc ccc ttc ttc ctc atc gcc aca gtc ata caa      1200
Val Val Thr Gly Phe Phe Pro Phe Phe Leu Ile Ala Thr Val Ile Gln
385                 390                 395                 400

ctt ttc tac cgt ggc cgc atc tgg aac att ctc ctc ttc cta cta acg      1248
Leu Phe Tyr Arg Gly Arg Ile Trp Asn Ile Leu Leu Phe Leu Leu Thr
                405                 410                 415

gtg cag ctg gtg ggc att atc aag gct acc tat gcc tgc ttc ctc cga      1296
Val Gln Leu Val Gly Ile Ile Lys Ala Thr Tyr Ala Cys Phe Leu Arg
            420                 425                 430

ggc aat gca gag atg atc ttc atg tcc ctc tac tcc ctt ctc tat atg      1344
Gly Asn Ala Glu Met Ile Phe Met Ser Leu Tyr Ser Leu Leu Tyr Met
        435                 440                 445

tcc agc ctg ctg cca gcc aag atc ttt gct att gct acc atc aac aag      1392
Ser Ser Leu Leu Pro Ala Lys Ile Phe Ala Ile Ala Thr Ile Asn Lys
    450                 455                 460

tct ggc tgg ggc act tct ggc agg aaa acc att gta gtg aac ttc att      1440
Ser Gly Trp Gly Thr Ser Gly Arg Lys Thr Ile Val Val Asn Phe Ile
465                 470                 475                 480

ggc cta atc cct gtg tcc atc tgg gtg gca gtt ctt cta ggg ggg tta      1488
Gly Leu Ile Pro Val Ser Ile Trp Val Ala Val Leu Leu Gly Gly Leu
                485                 490                 495

gcc tac aca gct tat tgt cag gac ctg ttc agt gag act gag cta gcc      1536
Ala Tyr Thr Ala Tyr Cys Gln Asp Leu Phe Ser Glu Thr Glu Leu Ala
            500                 505                 510

ttc ctg gtc tct ggg gcc atc ttg tat ggc tgc tac tgg gtg gcc ctc      1584
Phe Leu Val Ser Gly Ala Ile Leu Tyr Gly Cys Tyr Trp Val Ala Leu
        515                 520                 525

ctc atg ctg tat ttg gcc att att gcc cgg agg tgt ggg aaa aag cca      1632
Leu Met Leu Tyr Leu Ala Ile Ile Ala Arg Arg Cys Gly Lys Lys Pro
    530                 535                 540

gaa cag tat agc ctg gct ttt gct gag gtg tga                          1665
Glu Gln Tyr Ser Leu Ala Phe Ala Glu Val
545                 550

<210>  26

<211>  554

<212>  PRT

<213>  Rattus norvegicus

<400>  26

Met Pro Val Gln Leu Thr Thr Ala Leu Arg Val Val Gly Thr Ser Leu
1               5                   10                  15

Phe Ala Leu Val Val Leu Gly Gly Ile Leu Ala Ala Tyr Val Thr Gly
            20                  25                  30

Tyr Gln Phe Ile His Thr Glu Lys His Tyr Leu Ser Phe Gly Leu Tyr
        35                  40                  45

Gly Ala Ile Leu Gly Leu His Leu Leu Ile Gln Ser Leu Phe Ala Phe
    50                  55                  60

Leu Glu His Arg Arg Met Arg Arg Ala Gly Arg Pro Leu Lys Leu His
65                  70                  75                  80

Cys Ser Gln Arg Arg Arg Ser Val Ala Leu Cys Ile Ala Ala Tyr Gln
                85                  90                  95

Glu Asp Pro Glu Tyr Leu Arg Lys Cys Leu Arg Ser Ala Gln Arg Ile
            100                 105                 110

Ala Phe Pro Asn Leu Lys Val Val Met Val Val Asp Gly Asn Arg Gln
        115                 120                 125

Glu Asp Ala Tyr Met Leu Asp Ile Phe His Glu Val Leu Gly Gly Thr
    130                 135                 140

Glu Gln Ala Gly Phe Phe Val Trp Arg Ser Asn Phe His Glu Ala Gly
145                 150                 155                 160

Glu Gly Glu Thr Glu Ala Ser Leu Gln Glu Gly Met Glu Arg Val Arg
                165                 170                 175

Ala Val Val Trp Ala Ser Thr Phe Ser Cys Ile Met Gln Lys Trp Gly
            180                 185                 190

Gly Lys Arg Glu Val Met Tyr Thr Ala Phe Lys Ala Leu Gly Asn Ser
        195                 200                 205

Val Asp Tyr Ile Gln Val Cys Asp Ser Asp Thr Val Leu Asp Pro Ala
    210                 215                 220

Cys Thr Ile Glu Met Leu Arg Val Leu Glu Glu Asp Pro Gln Val Gly
225                 230                 235                 240

Gly Val Gly Gly Asp Val Gln Ile Leu Asn Lys Tyr Asp Ser Trp Ile
                245                 250                 255

Ser Phe Leu Ser Ser Val Arg Tyr Trp Met Ala Phe Asn Val Glu Arg
            260                 265                 270

Ala Cys Gln Ser Tyr Phe Gly Cys Val Gln Cys Ile Ser Gly Pro Leu
        275                 280                 285

Gly Met Tyr Arg Asn Ser Leu Leu Gln Gln Phe Leu Glu Asp Trp Tyr
    290                 295                 300

His Gln Lys Phe Leu Gly Ser Lys Cys Ser Phe Gly Asp Asp Arg His
305                 310                 315                 320

Leu Thr Asn Arg Val Leu Ser Leu Gly Tyr Arg Thr Lys Tyr Thr Ala
                325                 330                 335

Arg Ser Lys Cys Leu Thr Glu Thr Pro Thr Lys Tyr Leu Arg Trp Leu
            340                 345                 350

Asn Gln Gln Thr Arg Trp Ser Lys Ser Tyr Phe Arg Glu Trp Leu Tyr
        355                 360                 365

Asn Ser Leu Trp Phe His Lys His His Leu Trp Met Thr Tyr Glu Ser
    370                 375                 380

Val Val Thr Gly Phe Phe Pro Phe Phe Leu Ile Ala Thr Val Ile Gln
385                 390                 395                 400

Leu Phe Tyr Arg Gly Arg Ile Trp Asn Ile Leu Leu Phe Leu Leu Thr
                405                 410                 415

Val Gln Leu Val Gly Ile Ile Lys Ala Thr Tyr Ala Cys Phe Leu Arg
            420                 425                 430

Gly Asn Ala Glu Met Ile Phe Met Ser Leu Tyr Ser Leu Leu Tyr Met
        435                 440                 445

Ser Ser Leu Leu Pro Ala Lys Ile Phe Ala Ile Ala Thr Ile Asn Lys
    450                 455                 460

Ser Gly Trp Gly Thr Ser Gly Arg Lys Thr Ile Val Val Asn Phe Ile
465                 470                 475                 480

Gly Leu Ile Pro Val Ser Ile Trp Val Ala Val Leu Leu Gly Gly Leu
                485                 490                 495

Ala Tyr Thr Ala Tyr Cys Gln Asp Leu Phe Ser Glu Thr Glu Leu Ala
            500                 505                 510

Phe Leu Val Ser Gly Ala Ile Leu Tyr Gly Cys Tyr Trp Val Ala Leu
        515                 520                 525

Leu Met Leu Tyr Leu Ala Ile Ile Ala Arg Arg Cys Gly Lys Lys Pro
    530                 535                 540

Glu Gln Tyr Ser Leu Ala Phe Ala Glu Val
545                 550

<210>  27

<211>  1659

<212>  DNA

<213>  Oryctolagus cuniculus

<220>

<221>  CDS

<222>  (1)..(1659)

<223>

<300>

<308>  AB055978

<309>  2001-08-21

<313>  (1)..(1659)

<400>  27
atg cat tgt gag agg ttt ata tgt atc ctg aga ata att gga acc aca      48
Met His Cys Glu Arg Phe Ile Cys Ile Leu Arg Ile Ile Gly Thr Thr
1               5                   10                  15

ctg ttt gga gtc tct ctc ctt ctg gga atc aca gct gct tat att gtt      96
Leu Phe Gly Val Ser Leu Leu Leu Gly Ile Thr Ala Ala Tyr Ile Val
            20                  25                  30

gga tac cag ttt atc caa acg gat aat tac tat ttc tct ttt gga ctg      144
Gly Tyr Gln Phe Ile Gln Thr Asp Asn Tyr Tyr Phe Ser Phe Gly Leu
        35                  40                  45

tat ggt gcc ttt tta gca tca cac ctc atc atc caa agc ctg ttt gcc      192
Tyr Gly Ala Phe Leu Ala Ser His Leu Ile Ile Gln Ser Leu Phe Ala
    50                  55                  60

ttt ttg gag cac cga aaa atg aaa aaa tcc cta gaa acc ccc att aag      240
Phe Leu Glu His Arg Lys Met Lys Lys Ser Leu Glu Thr Pro Ile Lys
65                  70                  75                  80

ttg aac aaa aca gtt gct ctt tgc atc gct gcc tat caa gaa gac cca      288
Leu Asn Lys Thr Val Ala Leu Cys Ile Ala Ala Tyr Gln Glu Asp Pro
                85                  90                  95

gac tac tta agg aaa tgt tta caa tct gtg aaa agg ctg acc tac cct      336
Asp Tyr Leu Arg Lys Cys Leu Gln Ser Val Lys Arg Leu Thr Tyr Pro
            100                 105                 110

ggg att aaa gtt gtc atg gtc att gat ggg aac tca gaa gat gat gtt      384
Gly Ile Lys Val Val Met Val Ile Asp Gly Asn Ser Glu Asp Asp Val
        115                 120                 125

tac atg atg gac atc ttc agt gaa gtc atg ggc agg gaa aca tca gcc      432
Tyr Met Met Asp Ile Phe Ser Glu Val Met Gly Arg Glu Thr Ser Ala
    130                 135                 140

act tac atc tgg aag aac aac ttt cat gaa aag ggg cct ggg gag act      480
Thr Tyr Ile Trp Lys Asn Asn Phe His Glu Lys Gly Pro Gly Glu Thr
145                 150                 155                 160

gat gag tca cat aaa gaa agc tca caa cat gta acc caa ctg gtc ttg      528

Asp Glu Ser His Lys Glu Ser Ser Gln His Val Thr Gln Leu Val Leu
                165                 170                 175

tcg aac aaa agt gtt tgc atc atg cag aaa tgg ggt gga aag aga gaa      576
Ser Asn Lys Ser Val Cys Ile Met Gln Lys Trp Gly Gly Lys Arg Glu
            180                 185                 190

gtc atg tac aca gcc ttc aga gca ctg gga cga agc gtg gat tat gta      624
Val Met Tyr Thr Ala Phe Arg Ala Leu Gly Arg Ser Val Asp Tyr Val
        195                 200                 205

cag gtt tgt gat tca gat acc atg ctt gat cct gct tca tct gtg gag      672
Gln Val Cys Asp Ser Asp Thr Met Leu Asp Pro Ala Ser Ser Val Glu
    210                 215                 220

atg gtg aaa gtt tta gaa gaa gat ccc atg gtg gga ggt gtg ggg gga      720
Met Val Lys Val Leu Glu Glu Asp Pro Met Val Gly Gly Val Gly Gly
225                 230                 235                 240

gat gtc cag att tta aac aag tac gac tcc tgg atc tcc ttc ctc agc      768
Asp Val Gln Ile Leu Asn Lys Tyr Asp Ser Trp Ile Ser Phe Leu Ser
                245                 250                 255

agt gtg aga tac tgg atg gct ttt aat ata gaa agg gcc tgc cag tct      816
Ser Val Arg Tyr Trp Met Ala Phe Asn Ile Glu Arg Ala Cys Gln Ser
            260                 265                 270

tat ttt ggg tgt gtc cag tgc att agt gga cct ctt gga atg tac agg      864
Tyr Phe Gly Cys Val Gln Cys Ile Ser Gly Pro Leu Gly Met Tyr Arg
        275                 280                 285

aac tcc ttg ctg cat gag ttt gtg gaa gac tgg tac aat cag gaa ttt      912
Asn Ser Leu Leu His Glu Phe Val Glu Asp Trp Tyr Asn Gln Glu Phe
    290                 295                 300

atg ggc aac cag tgt agt ttt ggt gat gat agg cat ctg aca aac cga      960
Met Gly Asn Gln Cys Ser Phe Gly Asp Asp Arg His Leu Thr Asn Arg
305                 310                 315                 320

gtg ctg agt ctg ggc tac gca aca aaa tac aca gct cga tcc aag tgc     1008
Val Leu Ser Leu Gly Tyr Ala Thr Lys Tyr Thr Ala Arg Ser Lys Cys
                325                 330                 335

ctt act gaa acc cct ata gaa tat ctc cgg tgg tta aac cag cag acc     1056
Leu Thr Glu Thr Pro Ile Glu Tyr Leu Arg Trp Leu Asn Gln Gln Thr
            340                 345                 350

cgt tgg agc aag tcc tac ttc cga gag tgg ctg tac aat gca atg tgg     1104
Arg Trp Ser Lys Ser Tyr Phe Arg Glu Trp Leu Tyr Asn Ala Met Trp
        355                 360                 365

ttt cat aaa cat cac ttg tgg atg acc tat gaa gcg gtt atc act gga     1152
Phe His Lys His His Leu Trp Met Thr Tyr Glu Ala Val Ile Thr Gly
    370                 375                 380

ttc ttc ccc ttc ttt ctc att gcc aca gta atc cag ctc ttc tac cgg     1200
Phe Phe Pro Phe Phe Leu Ile Ala Thr Val Ile Gln Leu Phe Tyr Arg
385                 390                 395                 400

ggt aaa atc tgg aac atc ctc ctc ttc ttg tta act gtc cag cta gta     1248
Gly Lys Ile Trp Asn Ile Leu Leu Phe Leu Leu Thr Val Gln Leu Val
                405                 410                 415

ggt ctc ata aaa tca tct ttt gcc agt tgc ctt aga gga aat atc gtc     1296
Gly Leu Ile Lys Ser Ser Phe Ala Ser Cys Leu Arg Gly Asn Ile Val
            420                 425                 430

atg gtc ttt atg tct ctc tac tca gtg ctg tac atg tca agt tta ctt     1344

Met Val Phe Met Ser Leu Tyr Ser Val Leu Tyr Met Ser Ser Leu Leu
        435                 440                 445

ccg gcc aag atg ttt gca att gca aca ata aac aaa gct ggg tgg ggc      1392
Pro Ala Lys Met Phe Ala Ile Ala Thr Ile Asn Lys Ala Gly Trp Gly
    450                 455                 460

act tct gga agg aaa acc att gtt gtt aat ttc ata gga ctc att cca      1440
Thr Ser Gly Arg Lys Thr Ile Val Val Asn Phe Ile Gly Leu Ile Pro
465                 470                 475                 480

gta tca gtt tgg ttt aca att ctg ctg ggt ggt gtc att ttc acc att      1488
Val Ser Val Trp Phe Thr Ile Leu Leu Gly Gly Val Ile Phe Thr Ile
                485                 490                 495

tat aag gaa tct aaa aag cca ttt tca gaa tcc aaa cag aca gtt cta      1536
Tyr Lys Glu Ser Lys Lys Pro Phe Ser Glu Ser Lys Gln Thr Val Leu
            500                 505                 510

att gtt gga acg ttg ctc tat gca tgc tac tgg gtc atg ctt ttg acg      1584
Ile Val Gly Thr Leu Leu Tyr Ala Cys Tyr Trp Val Met Leu Leu Thr
        515                 520                 525

ctg tat gtg gtc ctc atc aat aag tgt ggc cgg cgg aag aag gga caa      1632
Leu Tyr Val Val Leu Ile Asn Lys Cys Gly Arg Arg Lys Lys Gly Gln
    530                 535                 540

cag tat gac atg gtg ctt gat gta tga                                  1659
Gln Tyr Asp Met Val Leu Asp Val
545                 550

<210>  28

<211>  552

<212>  PRT

<213>  Oryctolagus cuniculus

<400>  28

Met His Cys Glu Arg Phe Ile Cys Ile Leu Arg Ile Ile Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Glu Asp Asp Val
        115                 120                 125

Tyr Met Met Asp Ile Phe Ser Glu Val Met Gly Arg Glu Thr Ser Ala
    130                 135                 140

Thr Tyr Ile Trp Lys Asn Asn Phe His Glu Lys Gly Pro Gly Glu Thr
145                 150                 155                 160

Asp Glu Ser His Lys Glu Ser Ser Gln His Val Thr Gln Leu Val Leu
                165                 170                 175

Ser Asn Lys Ser Val Cys Ile Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Asn Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Lys Glu Ser Lys Lys Pro Phe Ser Glu Ser Lys Gln Thr Val Leu
            500                 505                 510

Ile Val Gly Thr Leu Leu Tyr Ala Cys Tyr Trp Val Met Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  29

<211>  1659

<212>  DNA

<213>  Oryctolagus cuniculus


<220>

<221>  CDS

<222>  (1)..(1659)

<223>

<300>

<308>  AB055979.1

<309>  2001-08-21

<313>  (1)..(1659)


<400>  29
atg cca gtg caa ctg aca aca gcc ttg cgt gtg gtg ggc acc agc ctg      48
Met Pro Val Gln Leu Thr Thr Ala Leu Arg Val Val Gly Thr Ser Leu
1               5                   10                  15

ttt gcc ttg gcg gtg ctg ggt ggc atc ctg gca gcc tat gtg aca ggc      96
Phe Ala Leu Ala Val Leu Gly Gly Ile Leu Ala Ala Tyr Val Thr Gly
            20                  25                  30

tac cag ttc atc cac aca gag aag cac tac ctg tcc ttc ggc ctg tac      144
Tyr Gln Phe Ile His Thr Glu Lys His Tyr Leu Ser Phe Gly Leu Tyr
        35                  40                  45

ggc gct atc ctg ggc ctg cac ctg ctc atc cag agc ctg ttt gcc ttc      192
Gly Ala Ile Leu Gly Leu His Leu Leu Ile Gln Ser Leu Phe Ala Phe
    50                  55                  60

ctg gag cac cgg cgc atg cgg cgg gcc agg cgg ccg ctg aag ctg ccc      240
Leu Glu His Arg Arg Met Arg Arg Ala Arg Arg Pro Leu Lys Leu Pro
65                  70                  75                  80

tca cgg cgg cgc tct gtg gcg ctc tgc atc gcc gcc tac cag gag gac      288
Ser Arg Arg Arg Ser Val Ala Leu Cys Ile Ala Ala Tyr Gln Glu Asp
                85                  90                  95

ccc gac tac ttg cgc aag tgc ctg cgc tca gcc cag cgc atc gcc ttc      336
Pro Asp Tyr Leu Arg Lys Cys Leu Arg Ser Ala Gln Arg Ile Ala Phe
            100                 105                 110

cct gac ctc aag gtg gtt atg gtg gtc gat ggc aac cgc cag gaa gac      384
Pro Asp Leu Lys Val Val Met Val Val Asp Gly Asn Arg Gln Glu Asp
        115                 120                 125

gcc tac atg ctg gac atc ttc cat gag gtg ctg ggt ggc act gag cag      432
Ala Tyr Met Leu Asp Ile Phe His Glu Val Leu Gly Gly Thr Glu Gln
    130                 135                 140

gcc ggc ttc ttt gtg tgg cgc agc aac ttc cac gag gca ggc gag ggc      480
Ala Gly Phe Phe Val Trp Arg Ser Asn Phe His Glu Ala Gly Glu Gly
145                 150                 155                 160

gag acc gag gcc agc ctg cag gaa ggc atg gag cgc gtg cgg gct gtg      528
Glu Thr Glu Ala Ser Leu Gln Glu Gly Met Glu Arg Val Arg Ala Val
                165                 170                 175

gtg cgg acc agc acc ttc tcg tgc atc atg cag aag tgg gga ggc aag      576
Val Arg Thr Ser Thr Phe Ser Cys Ile Met Gln Lys Trp Gly Gly Lys
            180                 185                 190

cgt gag gtc atg tac aca gcc ttc aag gcc ctc ggc gat tca gtg gac      624
Arg Glu Val Met Tyr Thr Ala Phe Lys Ala Leu Gly Asp Ser Val Asp
        195                 200                 205

tac atc cag gta tgt gac tcg gac acg gtg ctg gac cca gcc tgt acc      672
Tyr Ile Gln Val Cys Asp Ser Asp Thr Val Leu Asp Pro Ala Cys Thr
    210                 215                 220

atc gag atg ctt cgc gtc ctg gaa gag gat ccc caa gta ggg gga gtc      720

Ile Glu Met Leu Arg Val Leu Glu Glu Asp Pro Gln Val Gly Gly Val
225                 230                 235                 240

ggg gga gat gtc caa atc ctc aac aag tat gac tca tgg atc tcg ttc      768
Gly Gly Asp Val Gln Ile Leu Asn Lys Tyr Asp Ser Trp Ile Ser Phe
            245                 250                 255

ctg agc agt gtg cgg tac tgg atg gcc ttc aac gtg gag cgg gcg tgc      816
Leu Ser Ser Val Arg Tyr Trp Met Ala Phe Asn Val Glu Arg Ala Cys
        260                 265                 270

cag tcc tac ttt ggc tgt gtg cag tgt atc agt ggg ccc ttg ggc atg      864
Gln Ser Tyr Phe Gly Cys Val Gln Cys Ile Ser Gly Pro Leu Gly Met
    275                 280                 285

tac cgc aac agt ctc ctc cag caa ttc ctg gag gac tgg tac cat cag      912
Tyr Arg Asn Ser Leu Leu Gln Gln Phe Leu Glu Asp Trp Tyr His Gln
    290                 295                 300

aag ttc cta ggc agc aag tgc agc ttt ggg gat gac agg cac ctc acc      960
Lys Phe Leu Gly Ser Lys Cys Ser Phe Gly Asp Asp Arg His Leu Thr
305                 310                 315                 320

aac cga gtc ctg agt ctt ggc tac cga act aag tat aca gca cgc tcc      1008
Asn Arg Val Leu Ser Leu Gly Tyr Arg Thr Lys Tyr Thr Ala Arg Ser
                325                 330                 335

aag tgc ctc act gag acc cct acc aag tac cta cgg tgg ctc aac caa      1056
Lys Cys Leu Thr Glu Thr Pro Thr Lys Tyr Leu Arg Trp Leu Asn Gln
            340                 345                 350

cag acg cgc tgg agc aag tct tac ttc cgg gag tgg ctc tac aac tct      1104
Gln Thr Arg Trp Ser Lys Ser Tyr Phe Arg Glu Trp Leu Tyr Asn Ser
        355                 360                 365

ctg tgg ttc cat aag cac cat ctc tgg atg acc tac gag tcg gtg gtc      1152
Leu Trp Phe His Lys His His Leu Trp Met Thr Tyr Glu Ser Val Val
    370                 375                 380

aca ggt ttc ttc ccc ttc ttc ctc atc gcc aca gtc ata cag ctt ttc      1200
Thr Gly Phe Phe Pro Phe Phe Leu Ile Ala Thr Val Ile Gln Leu Phe
385                 390                 395                 400

tac cgt ggc cgc atc tgg aac atc ctc ctc ttc ctg ctg acc gtg cag      1248
Tyr Arg Gly Arg Ile Trp Asn Ile Leu Leu Phe Leu Leu Thr Val Gln
                405                 410                 415

ctg gtg ggc atc atc aaa gct acc tat gcc tgc ttc ctt cgg ggc aat      1296
Leu Val Gly Ile Ile Lys Ala Thr Tyr Ala Cys Phe Leu Arg Gly Asn
            420                 425                 430

gca gag atg atc ttc atg tcc ctc tac tcc ctt ctc tac atg tct agc      1344
Ala Glu Met Ile Phe Met Ser Leu Tyr Ser Leu Leu Tyr Met Ser Ser
        435                 440                 445

ctc ctg ccc gcc aag atc ttt gcc att gct acc atc aac aag tct ggc      1392
Leu Leu Pro Ala Lys Ile Phe Ala Ile Ala Thr Ile Asn Lys Ser Gly
    450                 455                 460

tgg ggc act tct ggc cga aaa aca att gtg gtg aac ttc att ggc ctc      1440
Trp Gly Thr Ser Gly Arg Lys Thr Ile Val Val Asn Phe Ile Gly Leu
465                 470                 475                 480

atc cct gtg tcc atc tgg gtg gca gtt ctt ttg ggg ggt ctg gcc tac      1488
Ile Pro Val Ser Ile Trp Val Ala Val Leu Leu Gly Gly Leu Ala Tyr
                485                 490                 495

acg gct tat tgc cag gac ctg ttc agt gag aca gag tta gcc ttc ctt      1536

Thr Ala Tyr Cys Gln Asp Leu Phe Ser Glu Thr Glu Leu Ala Phe Leu
            500                 505                 510

gtt tca ggg gcc att ctg tat ggc tgc tac tgg gtg gcc ctc ctc atg      1584
Val Ser Gly Ala Ile Leu Tyr Gly Cys Tyr Trp Val Ala Leu Leu Met
        515                 520                 525

ctg tat ctg gcc atc ata gcc cgg aga tgt ggg aag aag cca gaa caa      1632
Leu Tyr Leu Ala Ile Ile Ala Arg Arg Cys Gly Lys Lys Pro Glu Gln
    530                 535                 540

tat agc ttg gcc ttt gct gag gtg tga                                  1659
Tyr Ser Leu Ala Phe Ala Glu Val
545                 550

<210>  30

<211>  552

<212>  PRT

<213>  Oryctolagus cuniculus

<400>  30

Met Pro Val Gln Leu Thr Thr Ala Leu Arg Val Val Gly Thr Ser Leu
1               5                   10                  15

Phe Ala Leu Ala Val Leu Gly Gly Ile Leu Ala Ala Tyr Val Thr Gly
            20                  25                  30

Tyr Gln Phe Ile His Thr Glu Lys His Tyr Leu Ser Phe Gly Leu Tyr
        35                  40                  45

Gly Ala Ile Leu Gly Leu His Leu Leu Ile Gln Ser Leu Phe Ala Phe
    50                  55                  60

Leu Glu His Arg Arg Met Arg Arg Ala Arg Arg Pro Leu Lys Leu Pro
65                  70                  75                  80

Ser Arg Arg Arg Ser Val Ala Leu Cys Ile Ala Ala Tyr Gln Glu Asp
                85                  90                  95

Pro Asp Tyr Leu Arg Lys Cys Leu Arg Ser Ala Gln Arg Ile Ala Phe
            100                 105                 110

Pro Asp Leu Lys Val Val Met Val Val Asp Gly Asn Arg Gln Glu Asp
        115                 120                 125

Ala Tyr Met Leu Asp Ile Phe His Glu Val Leu Gly Gly Thr Glu Gln
    130                 135                 140

Ala Gly Phe Phe Val Trp Arg Ser Asn Phe His Glu Ala Gly Glu Gly
145                 150                 155                 160

Glu Thr Glu Ala Ser Leu Gln Glu Gly Met Glu Arg Val Arg Ala Val
                165                 170                 175

Val Arg Thr Ser Thr Phe Ser Cys Ile Met Gln Lys Trp Gly Gly Lys
            180                 185                 190

Arg Glu Val Met Tyr Thr Ala Phe Lys Ala Leu Gly Asp Ser Val Asp
        195                 200                 205

Tyr Ile Gln Val Cys Asp Ser Asp Thr Val Leu Asp Pro Ala Cys Thr
    210                 215                 220

Ile Glu Met Leu Arg Val Leu Glu Glu Asp Pro Gln Val Gly Gly Val
225                 230                 235                 240

Gly Gly Asp Val Gln Ile Leu Asn Lys Tyr Asp Ser Trp Ile Ser Phe
                245                 250                 255

Leu Ser Ser Val Arg Tyr Trp Met Ala Phe Asn Val Glu Arg Ala Cys
            260                 265                 270

Gln Ser Tyr Phe Gly Cys Val Gln Cys Ile Ser Gly Pro Leu Gly Met
        275                 280                 285

Tyr Arg Asn Ser Leu Leu Gln Gln Phe Leu Glu Asp Trp Tyr His Gln
    290                 295                 300

Lys Phe Leu Gly Ser Lys Cys Ser Phe Gly Asp Asp Arg His Leu Thr
305                 310                 315                 320

Asn Arg Val Leu Ser Leu Gly Tyr Arg Thr Lys Tyr Thr Ala Arg Ser
                325                 330                 335

Lys Cys Leu Thr Glu Thr Pro Thr Lys Tyr Leu Arg Trp Leu Asn Gln
            340                 345                 350

Gln Thr Arg Trp Ser Lys Ser Tyr Phe Arg Glu Trp Leu Tyr Asn Ser
        355                 360                 365

Leu Trp Phe His Lys His His Leu Trp Met Thr Tyr Glu Ser Val Val
    370                 375                 380

Thr Gly Phe Phe Pro Phe Phe Leu Ile Ala Thr Val Ile Gln Leu Phe
385                 390                 395                 400

Tyr Arg Gly Arg Ile Trp Asn Ile Leu Leu Phe Leu Leu Thr Val Gln
                405                 410                 415

Leu Val Gly Ile Ile Lys Ala Thr Tyr Ala Cys Phe Leu Arg Gly Asn
            420                 425                 430

Ala Glu Met Ile Phe Met Ser Leu Tyr Ser Leu Leu Tyr Met Ser Ser
        435                 440                 445

Leu Leu Pro Ala Lys Ile Phe Ala Ile Ala Thr Ile Asn Lys Ser Gly
    450                 455                 460

Trp Gly Thr Ser Gly Arg Lys Thr Ile Val Val Asn Phe Ile Gly Leu
465                 470                 475                 480

Ile Pro Val Ser Ile Trp Val Ala Val Leu Leu Gly Gly Leu Ala Tyr
                485                 490                 495

Thr Ala Tyr Cys Gln Asp Leu Phe Ser Glu Thr Glu Leu Ala Phe Leu
            500                 505                 510

Val Ser Gly Ala Ile Leu Tyr Gly Cys Tyr Trp Val Ala Leu Leu Met
        515                 520                 525

Leu Tyr Leu Ala Ile Ile Ala Arg Arg Cys Gly Lys Lys Pro Glu Gln
    530                 535                 540

Tyr Ser Leu Ala Phe Ala Glu Val
545                 550

<210>  31

<211>  1659

<212>  DNA

<213>  Equus caballus


<220>

<221>  CDS

<222>  (1)..(1659)

<223>


<300>

<308>  AY056582.1

<309>  2002-11-05

<313>  (437)..(2095)


<400>  31
atg cat tgt gag agg ttt ata tgt atc ctg aga ata att gga acc aca        48
Met His Cys Glu Arg Phe Ile Cys Ile Leu Arg Ile Ile Gly Thr Thr
1               5                   10                  15

ctt ttt gga gtc tct ctc cta ctt gga atc aca gct gct tat att gtt        96

Leu Phe Gly Val Ser Leu Leu Leu Gly Ile Thr Ala Ala Tyr Ile Val
            20                  25                  30

ggc tac caa ttt atc caa aca gat aat tac tat ttc tct ttt gga cta      144
Gly Tyr Gln Phe Ile Gln Thr Asp Asn Tyr Tyr Phe Ser Phe Gly Leu
        35                  40                  45

tat ggt gcc ttt tta gca tca cac ctc atc atc caa agc ctg ttt gcc      192
Tyr Gly Ala Phe Leu Ala Ser His Leu Ile Ile Gln Ser Leu Phe Ala
    50                  55                  60

ttt ttg gag cat cga aaa atg aaa aaa tcc cta gaa acc ccc att aag      240
Phe Leu Glu His Arg Lys Met Lys Lys Ser Leu Glu Thr Pro Ile Lys
65                  70                  75                  80

ttg aac aaa act gtt gct ctt tgc atc gct gcg tat caa gaa gat cca      288
Leu Asn Lys Thr Val Ala Leu Cys Ile Ala Ala Tyr Gln Glu Asp Pro
                85                  90                  95

gac tac tta cgg aaa tgc ttg caa tct gtg aaa agg cta acc tac cct      336
Asp Tyr Leu Arg Lys Cys Leu Gln Ser Val Lys Arg Leu Thr Tyr Pro
            100                 105                 110

ggg att aaa gtt gtc atg gtc ata gat ggg aac tca gaa gat gat ctt      384
Gly Ile Lys Val Val Met Val Ile Asp Gly Asn Ser Glu Asp Asp Leu
        115                 120                 125

tac atg atg gac atc ttc agt gaa gtc atg ggc agg gac aaa tca gcc      432
Tyr Met Met Asp Ile Phe Ser Glu Val Met Gly Arg Asp Lys Ser Ala
    130                 135                 140

act tac atc tgg aag aac aac ttc cac gag aag ggt cct ggt gag acg      480
Thr Tyr Ile Trp Lys Asn Asn Phe His Glu Lys Gly Pro Gly Glu Thr
145                 150                 155                 160

gat gag tca cat aaa gaa agc tct caa cat gtt acc caa ttg gtc ttg      528
Asp Glu Ser His Lys Glu Ser Ser Gln His Val Thr Gln Leu Val Leu
                165                 170                 175

tcc aac aaa agt att tgc atc atg caa aaa tgg ggt gga aaa aga gaa      576
Ser Asn Lys Ser Ile Cys Ile Met Gln Lys Trp Gly Gly Lys Arg Glu
            180                 185                 190

gtc atg tac acg gcc ttc aga gca ctg gga cga agt gtg gat tat gtg      624
Val Met Tyr Thr Ala Phe Arg Ala Leu Gly Arg Ser Val Asp Tyr Val
        195                 200                 205

cag gtt tgt gat tca gat acc atg ctt gac cct gcc tcg tct gtg gag      672
Gln Val Cys Asp Ser Asp Thr Met Leu Asp Pro Ala Ser Ser Val Glu
    210                 215                 220

atg gta aaa gtt tta gaa gaa gac ccc atg gtt gga ggt gtc gga gga      720
Met Val Lys Val Leu Glu Glu Asp Pro Met Val Gly Gly Val Gly Gly
225                 230                 235                 240

gat gtc cag att tta aac aag tac gat tcc tgg atc tcc ttc ctc agc      768
Asp Val Gln Ile Leu Asn Lys Tyr Asp Ser Trp Ile Ser Phe Leu Ser
                245                 250                 255

agt gtg aga tac tgg atg gct ttt aac ata gaa aga gcc tgt cag tct      816
Ser Val Arg Tyr Trp Met Ala Phe Asn Ile Glu Arg Ala Cys Gln Ser
            260                 265                 270

tat ttt ggg tgc gtc cag tgc att agt gga ccc ctg gga atg tac aga      864
Tyr Phe Gly Cys Val Gln Cys Ile Ser Gly Pro Leu Gly Met Tyr Arg
        275                 280                 285

aac tcc ttg ctg cat gaa ttc gta gaa gac tgg tac aat cag gaa ttt      912

Asn Ser Leu Leu His Glu Phe Val Glu Asp Trp Tyr Asn Gln Glu Phe
    290                 295                 300

atg ggc agc caa tgt agt ttt ggc gat gac cgg cat cta acg aac cga      960
Met Gly Ser Gln Cys Ser Phe Gly Asp Asp Arg His Leu Thr Asn Arg
305                 310                 315                 320

gtg ctg agt ctg ggt tat gca aca aaa tac aca gct cga tcc aag tgc      1008
Val Leu Ser Leu Gly Tyr Ala Thr Lys Tyr Thr Ala Arg Ser Lys Cys
                325                 330                 335

ctt act gaa aca cct ata gaa tat ctc aga tgg tta aac cag cag acc      1056
Leu Thr Glu Thr Pro Ile Glu Tyr Leu Arg Trp Leu Asn Gln Gln Thr
            340                 345                 350

cgt tgg agc aag tcc tac ttc cga gag tgg ctg tac aat gcg atg tgg      1104
Arg Trp Ser Lys Ser Tyr Phe Arg Glu Trp Leu Tyr Asn Ala Met Trp
        355                 360                 365

ttt cat aaa cat cac ttg tgg atg acc tac gaa gcg gtt atc act gga      1152
Phe His Lys His His Leu Trp Met Thr Tyr Glu Ala Val Ile Thr Gly
    370                 375                 380

ttc ttc cct ttc ttt ctc att gcc aca gta atc cag ctc ttc tac cgg      1200
Phe Phe Pro Phe Phe Leu Ile Ala Thr Val Ile Gln Leu Phe Tyr Arg
385                 390                 395                 400

ggt aaa att tgg aat atc ctc ctc ttc ttg tta aca gtc cag tta gta      1248
Gly Lys Ile Trp Asn Ile Leu Leu Phe Leu Leu Thr Val Gln Leu Val
                405                 410                 415

ggt ctc ata aag tct tcc ttt gcc agc tgc ctt aga gga aac atc gtc      1296
Gly Leu Ile Lys Ser Ser Phe Ala Ser Cys Leu Arg Gly Asn Ile Val
            420                 425                 430

atg gtc ttc atg tcc ctc tac tca gtg tta tac atg tca agt tta ctg      1344
Met Val Phe Met Ser Leu Tyr Ser Val Leu Tyr Met Ser Ser Leu Leu
        435                 440                 445

ccc gcc aaa atg ttt gct att gcc acg ata aac aaa gct ggg tgg ggc      1392
Pro Ala Lys Met Phe Ala Ile Ala Thr Ile Asn Lys Ala Gly Trp Gly
    450                 455                 460

aca tct gga agg aaa acc att gtt gtt aat ttc ata gga ctc att cca      1440
Thr Ser Gly Arg Lys Thr Ile Val Val Asn Phe Ile Gly Leu Ile Pro
465                 470                 475                 480

gta tca gtg tgg ttt aca atc ctc ctg ggt gga gtc att ttc acc att      1488
Val Ser Val Trp Phe Thr Ile Leu Leu Gly Gly Val Ile Phe Thr Ile
                485                 490                 495

tat atg gaa tct aaa aag cca ttc tca gaa tcc aaa cag aca gtt cta      1536
Tyr Met Glu Ser Lys Lys Pro Phe Ser Glu Ser Lys Gln Thr Val Leu
            500                 505                 510

atc gtt gga acg ttg ctc tat gca tgc tat tgg gtc atg ctt ttg acg      1584
Ile Val Gly Thr Leu Leu Tyr Ala Cys Tyr Trp Val Met Leu Leu Thr
        515                 520                 525

ctg tac gtg gtt ctc atc aac aaa tgt ggc agg cgg aag aag gga caa      1632
Leu Tyr Val Val Leu Ile Asn Lys Cys Gly Arg Arg Lys Lys Gly Gln
    530                 535                 540

cag tat gac atg gtg ctt gat gta tga                                  1659
Gln Tyr Asp Met Val Leu Asp Val
545                 550

<210> 32

<211> 552

<212> PRT

<213> Equus caballus

<400> 32

Met His Cys Glu Arg Phe Ile Cys Ile Leu Arg Ile Ile Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Glu Asp Asp Leu
        115                 120                 125

Tyr Met Met Asp Ile Phe Ser Glu Val Met Gly Arg Asp Lys Ser Ala
    130                 135                 140

Thr Tyr Ile Trp Lys Asn Asn Phe His Glu Lys Gly Pro Gly Glu Thr
145                 150                 155                 160

Asp Glu Ser His Lys Glu Ser Ser Gln His Val Thr Gln Leu Val Leu
                165                 170                 175

Ser Asn Lys Ser Ile Cys Ile Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Ser Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Met Glu Ser Lys Lys Pro Phe Ser Glu Ser Lys Gln Thr Val Leu
            500                 505                 510

Ile Val Gly Thr Leu Leu Tyr Ala Cys Tyr Trp Val Met Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  33

<211>  1659

<212>  DNA

<213>  Sus scrofa

<220>

<221>  CDS

<222>  (1)..(1659)

<223>

<300>

<308>  NM_214053.1

<309>  2004-08-23

<313>  (568)..(2226)

<400>  33
atg cat tgt gag agg ttt cta tgt atc ctg aga ata att gga acc aca        48
Met His Cys Glu Arg Phe Leu Cys Ile Leu Arg Ile Ile Gly Thr Thr
1               5                   10                  15

ctt ttt gga gtt tct ctc ctc ctt gga att acc gct gct tat atc gtt        96
Leu Phe Gly Val Ser Leu Leu Leu Gly Ile Thr Ala Ala Tyr Ile Val
            20                  25                  30

ggc tac caa ttt atc caa aca gat aat tac tat ttc ttt ttt ggg cta       144
Gly Tyr Gln Phe Ile Gln Thr Asp Asn Tyr Tyr Phe Phe Phe Gly Leu
        35                  40                  45

tat ggt gcc ttt tta gca tca cac ctt atc atc caa agc ttg ttt gcc       192
Tyr Gly Ala Phe Leu Ala Ser His Leu Ile Ile Gln Ser Leu Phe Ala
    50                  55                  60

ttt ttg gag cac cgg aaa atg aaa aaa tct cta gaa acc ccc att aaa       240
Phe Leu Glu His Arg Lys Met Lys Lys Ser Leu Glu Thr Pro Ile Lys
65                  70                  75                  80

ttg aac aaa act gtc gct ctt tgc atc gcg gcc tat caa gaa gat cca       288

Leu Asn Lys Thr Val Ala Leu Cys Ile Ala Ala Tyr Gln Glu Asp Pro
                85                  90                  95

gac tac ttg cga aag tgt ttg caa tct gtg aaa agg cta acc tac cct      336
Asp Tyr Leu Arg Lys Cys Leu Gln Ser Val Lys Arg Leu Thr Tyr Pro
            100                 105                 110

gga att aaa gtg gtc atg gtc ata gat ggg aac tcg gaa gat gac ctt      384
Gly Ile Lys Val Val Met Val Ile Asp Gly Asn Ser Glu Asp Asp Leu
        115                 120                 125

tac atg atg gac atc ttc agt gaa gtc atg ggc agg gac aat tca gcc      432
Tyr Met Met Asp Ile Phe Ser Glu Val Met Gly Arg Asp Asn Ser Ala
    130                 135                 140

act tat atc tgg aag aac aac ttc cac gaa aag ggc cct ggt gag acg      480
Thr Tyr Ile Trp Lys Asn Asn Phe His Glu Lys Gly Pro Gly Glu Thr
145                 150                 155                 160

gat gag tca cat aaa gaa agc tcc caa cat gtc acc caa ctg gtc ttg      528
Asp Glu Ser His Lys Glu Ser Ser Gln His Val Thr Gln Leu Val Leu
                165                 170                 175

tcc aac aaa agt att tgc atc atg caa aaa tgg ggt gga aaa aga gaa      576
Ser Asn Lys Ser Ile Cys Ile Met Gln Lys Trp Gly Gly Lys Arg Glu
            180                 185                 190

gtc atg tac acg gcc ttc aga gct ctg gga cga agt gtg gat tat gta      624
Val Met Tyr Thr Ala Phe Arg Ala Leu Gly Arg Ser Val Asp Tyr Val
        195                 200                 205

cag gtt tgt gat tca gac acc atg ctt gac cct gcc tca tct gtg gag      672
Gln Val Cys Asp Ser Asp Thr Met Leu Asp Pro Ala Ser Ser Val Glu
    210                 215                 220

atg gtg aaa gtt tta gaa gaa gac ccc atg gtt gga ggt gtc gga gga      720
Met Val Lys Val Leu Glu Glu Asp Pro Met Val Gly Gly Val Gly Gly
225                 230                 235                 240

gat gtc cag att tta aac aag tat gat tcc tgg atc tcc ttc ctc agc      768
Asp Val Gln Ile Leu Asn Lys Tyr Asp Ser Trp Ile Ser Phe Leu Ser
                245                 250                 255

agt gtg aga tac tgg atg gct ttt aac ata gaa agg gcc tgc cag tct      816
Ser Val Arg Tyr Trp Met Ala Phe Asn Ile Glu Arg Ala Cys Gln Ser
            260                 265                 270

tat ttt gga tgt gtc cag tgc att agt gga cct ctg ggg atg tac aga      864
Tyr Phe Gly Cys Val Gln Cys Ile Ser Gly Pro Leu Gly Met Tyr Arg
        275                 280                 285

aac tcc tta ctg cat gaa ttt gtg gaa gac tgg tac aat caa gag ttt      912
Asn Ser Leu Leu His Glu Phe Val Glu Asp Trp Tyr Asn Gln Glu Phe
    290                 295                 300

atg ggc agc caa tgt agt ttt gga gat gac agg cat cta acg aac cga      960
Met Gly Ser Gln Cys Ser Phe Gly Asp Asp Arg His Leu Thr Asn Arg
305                 310                 315                 320

gtg ctg agt ctg ggc tat gca aca aaa tac aca gct cgg tcc aag tgc     1008
Val Leu Ser Leu Gly Tyr Ala Thr Lys Tyr Thr Ala Arg Ser Lys Cys
                325                 330                 335

ctt act gag acg cct ata gaa tat ctc aga tgg tta aac cag cag acc     1056
Leu Thr Glu Thr Pro Ile Glu Tyr Leu Arg Trp Leu Asn Gln Gln Thr
            340                 345                 350

cgt tgg agc aag tcc tac ttc cga gag tgg ctg tac aat gcg atg tgg     1104

Arg Trp Ser Lys Ser Tyr Phe Arg Glu Trp Leu Tyr Asn Ala Met Trp
        355                 360                 365

ttt cat aag cat cat ttg tgg atg acc tat gag gca gtt atc acc ggg      1152
Phe His Lys His His Leu Trp Met Thr Tyr Glu Ala Val Ile Thr Gly
    370                 375                 380

ttc ttc cct ttc ttt ctc att gcc aca gta atc cag ctc ttc tac agg      1200
Phe Phe Pro Phe Phe Leu Ile Ala Thr Val Ile Gln Leu Phe Tyr Arg
385                 390                 395                 400

ggt aaa att tgg aac atc ctc ctc ttc ttg tta act gtc cag tta gta      1248
Gly Lys Ile Trp Asn Ile Leu Leu Phe Leu Leu Thr Val Gln Leu Val
                405                 410                 415

ggt ctc ata aaa tca tcc ttt gcc agc tgc ctt aga gga aat atc gtc      1296
Gly Leu Ile Lys Ser Ser Phe Ala Ser Cys Leu Arg Gly Asn Ile Val
            420                 425                 430

atg gtc ttc atg tcc ctc tac tcg gtg ttg tac atg tca agt tta ctt      1344
Met Val Phe Met Ser Leu Tyr Ser Val Leu Tyr Met Ser Ser Leu Leu
        435                 440                 445

ccc gcc aag atg ttt gcc att gca acg ata aac aaa gct ggc tgg ggc      1392
Pro Ala Lys Met Phe Ala Ile Ala Thr Ile Asn Lys Ala Gly Trp Gly
    450                 455                 460

aca tct gga agg aaa acc atc gtt gtg aat ttc ata gga ctc att cca      1440
Thr Ser Gly Arg Lys Thr Ile Val Val Asn Phe Ile Gly Leu Ile Pro
465                 470                 475                 480

gtg tca gtt tgg ttt aca atc ctc ctg ggt ggt gtg att ttc acc att      1488
Val Ser Val Trp Phe Thr Ile Leu Leu Gly Gly Val Ile Phe Thr Ile
                485                 490                 495

tat aag gaa tct aaa aag cca ttc tca gaa tcc aaa cag aca gtt tta      1536
Tyr Lys Glu Ser Lys Lys Pro Phe Ser Glu Ser Lys Gln Thr Val Leu
            500                 505                 510

att gtt gga acg ttg ctc tat gca tgc tat tgg gtc atg ctt ttg aca      1584
Ile Val Gly Thr Leu Leu Tyr Ala Cys Tyr Trp Val Met Leu Leu Thr
        515                 520                 525

ctg tat gtg gtt ctc atc aat aaa tgt ggc cgg cgg aag aag gga caa      1632
Leu Tyr Val Val Leu Ile Asn Lys Cys Gly Arg Arg Lys Lys Gly Gln
    530                 535                 540

cag tac gac atg gtg ctt gat gta tga                                  1659
Gln Tyr Asp Met Val Leu Asp Val
545                 550

<210>  34

<211>  552

<212>  PRT

<213>  Sus scrofa

<400>  34

Met His Cys Glu Arg Phe Leu Cys Ile Leu Arg Ile Ile Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Phe Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Glu Asp Asp Leu
        115                 120                 125

Tyr Met Met Asp Ile Phe Ser Glu Val Met Gly Arg Asp Asn Ser Ala
    130                 135                 140

Thr Tyr Ile Trp Lys Asn Asn Phe His Glu Lys Gly Pro Gly Glu Thr
145                 150                 155                 160

Asp Glu Ser His Lys Glu Ser Ser Gln His Val Thr Gln Leu Val Leu
                165                 170                 175

Ser Asn Lys Ser Ile Cys Ile Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Ser Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Lys Glu Ser Lys Lys Pro Phe Ser Glu Ser Lys Gln Thr Val Leu
            500                 505                 510

Ile Val Gly Thr Leu Leu Tyr Ala Cys Tyr Trp Val Met Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  35

<211>  1662

<212>  DNA

<213>  Sus scrofa


<220>

<221>  CDS

<222>  (1)..(1662)

<223>


<300>

<308>  AB159675

<309>  2004-05-08

<313>  (1)..(1662)


<400>  35
atg ccg gtt cag ctg acg aca gcc ctg cgt gtg gtg ggc acc agc ctg      48
Met Pro Val Gln Leu Thr Thr Ala Leu Arg Val Val Gly Thr Ser Leu
1               5                   10                  15

ttt gcc ctg gca gtg ctg ggc ggc att ctg gca gca tat gtg aca ggc      96
Phe Ala Leu Ala Val Leu Gly Gly Ile Leu Ala Ala Tyr Val Thr Gly
            20                  25                  30

tac cag ttc atc cac aca gaa aag cac tac ctg tcc ttt ggg ctg tat      144
Tyr Gln Phe Ile His Thr Glu Lys His Tyr Leu Ser Phe Gly Leu Tyr
        35                  40                  45

ggt gcc atc ctg ggc ctg cac ctg ctc atc cag agc ctg ttt gcc ttc      192
Gly Ala Ile Leu Gly Leu His Leu Leu Ile Gln Ser Leu Phe Ala Phe
    50                  55                  60

ctg gag cac cgg cgc atg cgg cgg gca ggc cgg cca ctg aag ctg ccc      240
Leu Glu His Arg Arg Met Arg Arg Ala Gly Arg Pro Leu Lys Leu Pro
65                  70                  75                  80

tcc ccg ctg cag cgc tca gtg gcg ctc tgc atc gcc gcg tac cag gag      288
Ser Pro Leu Gln Arg Ser Val Ala Leu Cys Ile Ala Ala Tyr Gln Glu
                85                  90                  95

gac ccc gac tac ttg cgc aag tgc ctg cgc tcg gcc cag cgc atc gcc      336
Asp Pro Asp Tyr Leu Arg Lys Cys Leu Arg Ser Ala Gln Arg Ile Ala
            100                 105                 110

ttc ccc gat ctc aag gtg gtc atg gtg gtg gac ggc aat cgc cag gag      384
Phe Pro Asp Leu Lys Val Val Met Val Val Asp Gly Asn Arg Gln Glu
        115                 120                 125

gat gcc tac atg ttg gac atc ttc cac gag gtg cta ggt ggc aac gag      432
Asp Ala Tyr Met Leu Asp Ile Phe His Glu Val Leu Gly Gly Asn Glu
    130                 135                 140

caa gcc ggc ttc ttt gtg tgg cgc agc aac ttc cat gag gcg ggt gag      480

Gln Ala Gly Phe Phe Val Trp Arg Ser Asn Phe His Glu Ala Gly Glu
145                 150                 155                 160

ggc gag acg gag gcc agc ctg cag gag ggc atg gac cgt gtg cgg aat     528
Gly Glu Thr Glu Ala Ser Leu Gln Glu Gly Met Asp Arg Val Arg Asn
                165                 170                 175

gtg gtg cgg gcc agc acc ttc tcc tgc atc atg cag aag tgg gga gga     576
Val Val Arg Ala Ser Thr Phe Ser Cys Ile Met Gln Lys Trp Gly Gly
            180                 185                 190

aag cga gag gtc atg tac acg gcc ttc aag gcc ctt ggt gac tcc gtg     624
Lys Arg Glu Val Met Tyr Thr Ala Phe Lys Ala Leu Gly Asp Ser Val
        195                 200                 205

gac tac att cag gtg tgt gac tct gac act gta ctg gat cca gcc tgt     672
Asp Tyr Ile Gln Val Cys Asp Ser Asp Thr Val Leu Asp Pro Ala Cys
    210                 215                 220

acc ttc gag atg ctt cga gtc ttg gag gag gac ccc caa gta ggg gga     720
Thr Phe Glu Met Leu Arg Val Leu Glu Glu Asp Pro Gln Val Gly Gly
225                 230                 235                 240

gtt ggg gga gat gtc caa atc ctc aat aag tat gac tcc tgg atc tcc     768
Val Gly Gly Asp Val Gln Ile Leu Asn Lys Tyr Asp Ser Trp Ile Ser
                245                 250                 255

ttc ctg agc agt gtg cgg tac tgg atg gcc ttc aac gtg gaa cgg gct     816
Phe Leu Ser Ser Val Arg Tyr Trp Met Ala Phe Asn Val Glu Arg Ala
            260                 265                 270

tgc cag tcc tac ttc ggc tgt gtg cag tgt atc agt ggg ccc ctg ggc     864
Cys Gln Ser Tyr Phe Gly Cys Val Gln Cys Ile Ser Gly Pro Leu Gly
        275                 280                 285

atg tac cgg aac agc ctg ctt cag cag ttc ttg gag gac tgg tac cat     912
Met Tyr Arg Asn Ser Leu Leu Gln Gln Phe Leu Glu Asp Trp Tyr His
    290                 295                 300

cag aag ttc cta ggt agc aag tgc agt ttt ggg gat gac cgg cac ctc     960
Gln Lys Phe Leu Gly Ser Lys Cys Ser Phe Gly Asp Asp Arg His Leu
305                 310                 315                 320

acc aac cga gtc ctg agt ctc ggc tac agg act aag tac aca gca cgc    1008
Thr Asn Arg Val Leu Ser Leu Gly Tyr Arg Thr Lys Tyr Thr Ala Arg
                325                 330                 335

tct aaa tgc ctc aca gag acc ccc acc aag tac ctc cgg tgg ctc aac    1056
Ser Lys Cys Leu Thr Glu Thr Pro Thr Lys Tyr Leu Arg Trp Leu Asn
            340                 345                 350

caa cag acc cgc tgg agc aag tct tac ttc cgg gag tgg ctc tac aac    1104
Gln Gln Thr Arg Trp Ser Lys Ser Tyr Phe Arg Glu Trp Leu Tyr Asn
        355                 360                 365

tct ctg tgg ttc cac aag cac cac ctc tgg atg acc tac gag tca gtg    1152
Ser Leu Trp Phe His Lys His His Leu Trp Met Thr Tyr Glu Ser Val
    370                 375                 380

gtc aca ggt ttt ttc ccc ttc ttc ctt att gcc aca gtc ata cag ctt    1200
Val Thr Gly Phe Phe Pro Phe Phe Leu Ile Ala Thr Val Ile Gln Leu
385                 390                 395                 400

ttc tac cgt ggc cgc atc tgg aac att ctc ctc ttc ctg ctg aca gtg    1248
Phe Tyr Arg Gly Arg Ile Trp Asn Ile Leu Leu Phe Leu Leu Thr Val
                405                 410                 415

cag ttg gta ggc atc atc aag gct acc tat gcc tgc ttt ctt cgg ggc    1296

Gln Leu Val Gly Ile Ile Lys Ala Thr Tyr Ala Cys Phe Leu Arg Gly
            420                 425                 430

aat gca gag atg atc ttc atg tct ctc tac tcc ctt ctc tac atg tcc      1344
Asn Ala Glu Met Ile Phe Met Ser Leu Tyr Ser Leu Leu Tyr Met Ser
        435                 440                 445

agc ctc ctg cca gcc aag atc ttc gcc att gct acc atc aac aaa tct      1392
Ser Leu Leu Pro Ala Lys Ile Phe Ala Ile Ala Thr Ile Asn Lys Ser
    450                 455                 460

ggc tgg ggc act tct ggc cga aaa acc att gtg gtg aac ttc att ggc      1440
Gly Trp Gly Thr Ser Gly Arg Lys Thr Ile Val Val Asn Phe Ile Gly
465                 470                 475                 480

ctc atc cct gtg tcc atc tgg gtg gca gtc ctt ctg ggg gga ctg gcc      1488
Leu Ile Pro Val Ser Ile Trp Val Ala Val Leu Leu Gly Gly Leu Ala
                485                 490                 495

tat aca gct tac tgt cag gac ctg ttc agt gag acg gag cta gcc ttc      1536
Tyr Thr Ala Tyr Cys Gln Asp Leu Phe Ser Glu Thr Glu Leu Ala Phe
            500                 505                 510

ctg gtc tca ggg gcc atc ctt tat ggc tgc tac tgg gtg gcc ctc ctt      1584
Leu Val Ser Gly Ala Ile Leu Tyr Gly Cys Tyr Trp Val Ala Leu Leu
        515                 520                 525

atg ctg tat ctg gcc atc atc gcc cgg cgg tgc ggg aag aag ccg gag      1632
Met Leu Tyr Leu Ala Ile Ile Ala Arg Arg Cys Gly Lys Lys Pro Glu
    530                 535                 540

cag tat agc tta gct ttt gct gag gtg tga                              1662
Gln Tyr Ser Leu Ala Phe Ala Glu Val
545                 550

<210>  36

<211>  553

<212>  PRT

<213>  Sus scrofa

<400>  36

Met Pro Val Gln Leu Thr Thr Ala Leu Arg Val Val Gly Thr Ser Leu
1               5                   10                  15

Phe Ala Leu Ala Val Leu Gly Gly Ile Leu Ala Ala Tyr Val Thr Gly
            20                  25                  30

Tyr Gln Phe Ile His Thr Glu Lys His Tyr Leu Ser Phe Gly Leu Tyr
        35                  40                  45

Gly Ala Ile Leu Gly Leu His Leu Leu Ile Gln Ser Leu Phe Ala Phe
    50                  55                  60

Leu Glu His Arg Arg Met Arg Arg Ala Gly Arg Pro Leu Lys Leu Pro
65                  70                  75                  80

Ser Pro Leu Gln Arg Ser Val Ala Leu Cys Ile Ala Ala Tyr Gln Glu
                85                  90                  95

Asp Pro Asp Tyr Leu Arg Lys Cys Leu Arg Ser Ala Gln Arg Ile Ala
            100                 105                 110

Phe Pro Asp Leu Lys Val Val Met Val Val Asp Gly Asn Arg Gln Glu
        115                 120                 125

Asp Ala Tyr Met Leu Asp Ile Phe His Glu Val Leu Gly Gly Asn Glu
    130                 135                 140

Gln Ala Gly Phe Phe Val Trp Arg Ser Asn Phe His Glu Ala Gly Glu
145                 150                 155                 160

Gly Glu Thr Glu Ala Ser Leu Gln Glu Gly Met Asp Arg Val Arg Asn
                165                 170                 175

Val Val Arg Ala Ser Thr Phe Ser Cys Ile Met Gln Lys Trp Gly Gly
            180                 185                 190

Lys Arg Glu Val Met Tyr Thr Ala Phe Lys Ala Leu Gly Asp Ser Val
        195                 200                 205

Asp Tyr Ile Gln Val Cys Asp Ser Asp Thr Val Leu Asp Pro Ala Cys
    210                 215                 220

Thr Phe Glu Met Leu Arg Val Leu Glu Glu Asp Pro Gln Val Gly Gly
225                 230                 235                 240

Val Gly Gly Asp Val Gln Ile Leu Asn Lys Tyr Asp Ser Trp Ile Ser
                245                 250                 255

Phe Leu Ser Ser Val Arg Tyr Trp Met Ala Phe Asn Val Glu Arg Ala
            260                 265                 270

Cys Gln Ser Tyr Phe Gly Cys Val Gln Cys Ile Ser Gly Pro Leu Gly
        275                 280                 285

Met Tyr Arg Asn Ser Leu Leu Gln Gln Phe Leu Glu Asp Trp Tyr His
    290                 295                 300

Gln Lys Phe Leu Gly Ser Lys Cys Ser Phe Gly Asp Asp Arg His Leu
305                 310                 315                 320

Thr Asn Arg Val Leu Ser Leu Gly Tyr Arg Thr Lys Tyr Thr Ala Arg
                325                 330                 335

Ser Lys Cys Leu Thr Glu Thr Pro Thr Lys Tyr Leu Arg Trp Leu Asn
            340                 345                 350

Gln Gln Thr Arg Trp Ser Lys Ser Tyr Phe Arg Glu Trp Leu Tyr Asn
        355                 360                 365

Ser Leu Trp Phe His Lys His His Leu Trp Met Thr Tyr Glu Ser Val
    370                 375                 380

Val Thr Gly Phe Phe Pro Phe Phe Leu Ile Ala Thr Val Ile Gln Leu
385                 390                 395                 400

Phe Tyr Arg Gly Arg Ile Trp Asn Ile Leu Leu Phe Leu Leu Thr Val
                405                 410                 415

Gln Leu Val Gly Ile Ile Lys Ala Thr Tyr Ala Cys Phe Leu Arg Gly
            420                 425                 430

Asn Ala Glu Met Ile Phe Met Ser Leu Tyr Ser Leu Leu Tyr Met Ser
        435                 440                 445

Ser Leu Leu Pro Ala Lys Ile Phe Ala Ile Ala Thr Ile Asn Lys Ser
    450                 455                 460

Gly Trp Gly Thr Ser Gly Arg Lys Thr Ile Val Val Asn Phe Ile Gly
465                 470                 475                 480

Leu Ile Pro Val Ser Ile Trp Val Ala Val Leu Leu Gly Gly Leu Ala
                485                 490                 495

Tyr Thr Ala Tyr Cys Gln Asp Leu Phe Ser Glu Thr Glu Leu Ala Phe
            500                 505                 510

Leu Val Ser Gly Ala Ile Leu Tyr Gly Cys Tyr Trp Val Ala Leu Leu
        515                 520                 525

Met Leu Tyr Leu Ala Ile Ile Ala Arg Arg Cys Gly Lys Lys Pro Glu
    530                 535                 540

Gln Tyr Ser Leu Ala Phe Ala Glu Val
545                 550

<210>  37

<211>  1659

<212>  DNA

<213>  Bos taurus


<220>

<221>  CDS

<222>  (1)..(1659)

<223>

<300>
<308> AJ004951.1
<309> 1998-11-24
<313> (545)..(2203)

<400> 37
atg cat tgt gag aga ttt cta tgt atc ctg aga ata att gga acc aca 48
Met His Cys Glu Arg Phe Leu Cys Ile Leu Arg Ile Ile Gly Thr Thr
1               5                   10                  15

ctt ttt gga gtc tct ctc ctc ctt gga atc aca gct gct tat att gtt 96
Leu Phe Gly Val Ser Leu Leu Leu Gly Ile Thr Ala Ala Tyr Ile Val
            20                  25                  30

ggc tat caa ttt atc caa aca gat aat tac tat ttc tct ttt gga ctg 144
Gly Tyr Gln Phe Ile Gln Thr Asp Asn Tyr Tyr Phe Ser Phe Gly Leu
        35                  40                  45

tat ggt gcc ttt tta gca tca cac ctc atc atc caa agc ctg ttt gcc 192
Tyr Gly Ala Phe Leu Ala Ser His Leu Ile Ile Gln Ser Leu Phe Ala
    50                  55                  60

ttt ttg gag cac cgg aaa atg aaa aaa tct cta gaa acc ccc att aag 240
Phe Leu Glu His Arg Lys Met Lys Lys Ser Leu Glu Thr Pro Ile Lys
65                  70                  75                  80

ttg aac aaa act gtt gct ctt tgc att gct gca tat caa gaa gat cca 288
Leu Asn Lys Thr Val Ala Leu Cys Ile Ala Ala Tyr Gln Glu Asp Pro
                85                  90                  95

gac tat ttg cgg aaa tgt ttg caa tct gtg aaa agg cta acc tac ccc 336
Asp Tyr Leu Arg Lys Cys Leu Gln Ser Val Lys Arg Leu Thr Tyr Pro
            100                 105                 110

gga att aaa gtt gtc atg gtc ata gat gga aac tcg gaa gat gac ctt 384
Gly Ile Lys Val Val Met Val Ile Asp Gly Asn Ser Glu Asp Asp Leu
        115                 120                 125

tac atg atg gac atc ttc agt gaa gtc atg ggc agg gac aag tca gcc 432
Tyr Met Met Asp Ile Phe Ser Glu Val Met Gly Arg Asp Lys Ser Ala
    130                 135                 140

act tat atc tgg aag aac aac tac cat gtg aag ggt cct gga gag acg 480
Thr Tyr Ile Trp Lys Asn Asn Tyr His Val Lys Gly Pro Gly Glu Thr
145                 150                 155                 160

gat gag tcg cac aaa gaa agc tca cag cat gtc acc cag ttg gtc ttg 528
Asp Glu Ser His Lys Glu Ser Ser Gln His Val Thr Gln Leu Val Leu
                165                 170                 175

tcc aac aag agt att tgc acc atg caa aaa tgg ggt gga aaa aga gaa 576
Ser Asn Lys Ser Ile Cys Thr Met Gln Lys Trp Gly Gly Lys Arg Glu
            180                 185                 190

gtc atg tac aca gcc ttc aga gca ctg ggg cga agt gtg gat tat gta 624
Val Met Tyr Thr Ala Phe Arg Ala Leu Gly Arg Ser Val Asp Tyr Val
        195                 200                 205

cag gtt tgt gat tca gac acc atg ctt gac cca gca tca tct gtg gag 672

Gln Val Cys Asp Ser Asp Thr Met Leu Asp Pro Ala Ser Ser Val Glu
    210                 215                 220

atg gta aaa gtt tta gaa gaa gat ccc atg gtt gga ggt gtc ggg gga      720
Met Val Lys Val Leu Glu Glu Asp Pro Met Val Gly Gly Val Gly Gly
225                 230                 235                 240

gat gtc cag att tta aac aag tat gat tcc tgg atc tcc ttc ctc agc      768
Asp Val Gln Ile Leu Asn Lys Tyr Asp Ser Trp Ile Ser Phe Leu Ser
                245                 250                 255

agt gtg aga tac tgg atg gct ttt aac ata gaa agg gcc tgt cag tct      816
Ser Val Arg Tyr Trp Met Ala Phe Asn Ile Glu Arg Ala Cys Gln Ser
            260                 265                 270

tat ttc gga tgt gtc cag tgc att agc gga cct ctg gga atg tac aga      864
Tyr Phe Gly Cys Val Gln Cys Ile Ser Gly Pro Leu Gly Met Tyr Arg
        275                 280                 285

aac tcc tta ctg cat gaa ttt gtg gaa gac tgg tac aat caa gaa ttt      912
Asn Ser Leu Leu His Glu Phe Val Glu Asp Trp Tyr Asn Gln Glu Phe
    290                 295                 300

atg ggc agc caa tgt agt ttt gga gat gac agg cat cta acg aac cga      960
Met Gly Ser Gln Cys Ser Phe Gly Asp Asp Arg His Leu Thr Asn Arg
305                 310                 315                 320

gtg ctg agt ctg ggc tat gca acg aaa tac aca gct cga tcc aag tgc     1008
Val Leu Ser Leu Gly Tyr Ala Thr Lys Tyr Thr Ala Arg Ser Lys Cys
                325                 330                 335

ctt act gaa aca cct ata gaa tat ctc aga tgg tta aac cag cag acc     1056
Leu Thr Glu Thr Pro Ile Glu Tyr Leu Arg Trp Leu Asn Gln Gln Thr
            340                 345                 350

cgc tgg agc aag tcg tac ttc cga gag tgg ctg tac aac gct atg tgg     1104
Arg Trp Ser Lys Ser Tyr Phe Arg Glu Trp Leu Tyr Asn Ala Met Trp
        355                 360                 365

ttt cat aaa cat cac ttg tgg atg acc tat gaa gcc gtc atc act ggg     1152
Phe His Lys His His Leu Trp Met Thr Tyr Glu Ala Val Ile Thr Gly
    370                 375                 380

ttc ttc cct ttc ttt ctc att gcc acg gta atc cag ctc ttc tac agg     1200
Phe Phe Pro Phe Phe Leu Ile Ala Thr Val Ile Gln Leu Phe Tyr Arg
385                 390                 395                 400

ggt aaa att tgg aac acc ctc ctc ttc ttg tta act gtc cag tta gtg     1248
Gly Lys Ile Trp Asn Thr Leu Leu Phe Leu Leu Thr Val Gln Leu Val
                405                 410                 415

ggt ctc ata aaa tca tct ttt gcc agc tgc ctt aga gga aac att gtc     1296
Gly Leu Ile Lys Ser Ser Phe Ala Ser Cys Leu Arg Gly Asn Ile Val
            420                 425                 430

atg gtc ttc atg tcc ctc tac tca gtg tta tac atg tca agt tta ctt     1344
Met Val Phe Met Ser Leu Tyr Ser Val Leu Tyr Met Ser Ser Leu Leu
        435                 440                 445

ccg gcc aag atg ttt gca att gca aca ata aac aaa gct ggg tgg ggc     1392
Pro Ala Lys Met Phe Ala Ile Ala Thr Ile Asn Lys Ala Gly Trp Gly
    450                 455                 460

aca tct gga agg aaa acc att gtc gtt aat ttc ata gga ctc att ccc     1440
Thr Ser Gly Arg Lys Thr Ile Val Val Asn Phe Ile Gly Leu Ile Pro
465                 470                 475                 480

gta tcg gtt tgg ttt aca ata ctc ctg ggt ggt gtg att ttc acc att     1488

Val Ser Val Trp Phe Thr Ile Leu Leu Gly Gly Val Ile Phe Thr Ile
                485                 490                 495

tat aag gaa tct aaa aag cca ttc tca gaa tcc aaa cag aca gtt tta      1536
Tyr Lys Glu Ser Lys Lys Pro Phe Ser Glu Ser Lys Gln Thr Val Leu
            500                 505                 510

att gtt gga acg ttg ctg tat gca tgc tat tgg gtc atg ctt ttg aca      1584
Ile Val Gly Thr Leu Leu Tyr Ala Cys Tyr Trp Val Met Leu Leu Thr
        515                 520                 525

ctg tac gtg gtt ctc atc aat aaa tgt ggc agg cgg aag aag gga caa      1632
Leu Tyr Val Val Leu Ile Asn Lys Cys Gly Arg Arg Lys Lys Gly Gln
    530                 535                 540

cag tac gac atg gtg ctt gat gta tga                                  1659
Gln Tyr Asp Met Val Leu Asp Val
545                 550

<210>  38

<211>  552

<212>  PRT

<213>  Bos taurus

<400>  38

Met His Cys Glu Arg Phe Leu Cys Ile Leu Arg Ile Ile Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Phe Leu Ala Ser His Leu Ile Ile Gln Ser Leu Phe Ala
    50                  55                  60

Phe Leu Glu His Arg Lys Met Lys Lys Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Gln Ser Val Lys Arg Leu Thr Tyr Pro
            100                 105                 110

Gly Ile Lys Val Val Met Val Ile Asp Gly Asn Ser Glu Asp Asp Leu
        115                 120                 125

Tyr Met Met Asp Ile Phe Ser Glu Val Met Gly Arg Asp Lys Ser Ala
    130                 135                 140

Thr Tyr Ile Trp Lys Asn Asn Tyr His Val Lys Gly Pro Gly Glu Thr
145                 150                 155                 160

Asp Glu Ser His Lys Glu Ser Ser Gln His Val Thr Gln Leu Val Leu
                165                 170                 175

Ser Asn Lys Ser Ile Cys Thr Met Gln Lys Trp Gly Gly Lys Arg Glu
            180                 185                 190

Val Met Tyr Thr Ala Phe Arg Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Ser Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Thr Leu Leu Phe Leu Leu Thr Val Gln Leu Val
                405                 410                 415

Gly Leu Ile Lys Ser Ser Phe Ala Ser Cys Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Val Trp Phe Thr Ile Leu Leu Gly Gly Val Ile Phe Thr Ile
                485                 490                 495

Tyr Lys Glu Ser Lys Lys Pro Phe Ser Glu Ser Lys Gln Thr Val Leu
            500                 505                 510

Ile Val Gly Thr Leu Leu Tyr Ala Cys Tyr Trp Val Met Leu Leu Thr
        515                 520                 525

Leu Tyr Val Val Leu Ile Asn Lys Cys Gly Arg Arg Lys Lys Gly Gln
    530                 535                 540

Gln Tyr Asp Met Val Leu Asp Val
545                 550

<210>  39

<211>  1659

<212>  DNA

<213>  Gallus gallus


<220>

<221>  CDS

<222>  (1)..(1659)

<223>


<300>

<308>  AF106940.1

<309>  1999-12-10

<313>  (563)..(2221)


<400>  39
atg tat tgt gag agg ttt ata tgt atc ctg aga ata ctt gga acc aca        48

Met Tyr Cys Glu Arg Phe Ile Cys Ile Leu Arg Ile Leu Gly Thr Thr
1               5                   10                  15

ctc ttt gga gtg tcc ctc ctg ctg gga atc acc gct gct tac att gtg     96
Leu Phe Gly Val Ser Leu Leu Leu Gly Ile Thr Ala Ala Tyr Ile Val
            20                  25                  30

ggc tac cag ttt atc caa aca gac aat tac tac ttc tcc ttt gga ctc     144
Gly Tyr Gln Phe Ile Gln Thr Asp Asn Tyr Tyr Phe Ser Phe Gly Leu
        35                  40                  45

tat ggt gct atc ctg gca tca cat ctc atc atc caa agc ctg ttt gcc     192
Tyr Gly Ala Ile Leu Ala Ser His Leu Ile Ile Gln Ser Leu Phe Ala
    50                  55                  60

tac cta gag cac agg aaa atg aag cgg tcg cta gag act cca atc aag     240
Tyr Leu Glu His Arg Lys Met Lys Arg Ser Leu Glu Thr Pro Ile Lys
65                  70                  75                  80

ctg aac aaa act gtt gcc ctt tgt att gct gcc tat caa gag gat cct     288
Leu Asn Lys Thr Val Ala Leu Cys Ile Ala Ala Tyr Gln Glu Asp Pro
                85                  90                  95

gac tac tta aga aaa tgt tta ctt tct gtg aaa aga ttg acc tac cct     336
Asp Tyr Leu Arg Lys Cys Leu Leu Ser Val Lys Arg Leu Thr Tyr Pro
            100                 105                 110

gga att aaa gtt gtt atg gtc att gat ggg aac tca gaa gat gac gtt     384
Gly Ile Lys Val Val Met Val Ile Asp Gly Asn Ser Glu Asp Asp Val
        115                 120                 125

tac atg atg gac att ttt act gaa atc atg ggg agg gac aaa tct gcc     432
Tyr Met Met Asp Ile Phe Thr Glu Ile Met Gly Arg Asp Lys Ser Ala
    130                 135                 140

act tat atc tgg agt aac aac ttc cat gac aaa ggt cca ggt gag acg     480
Thr Tyr Ile Trp Ser Asn Asn Phe His Asp Lys Gly Pro Gly Glu Thr
145                 150                 155                 160

gag gag tct cac aga gag agc atg cag cac gta tct cag ctg gtc ctg     528
Glu Glu Ser His Arg Glu Ser Met Gln His Val Ser Gln Leu Val Leu
                165                 170                 175

tcc aac aaa agt gtt tgc atc atg cag aaa tgg ggt gga aaa aga gaa     576
Ser Asn Lys Ser Val Cys Ile Met Gln Lys Trp Gly Gly Lys Arg Glu
            180                 185                 190

gta atg tac aca gca ttc aaa gca ctg gga gaa gcg tgg aat tat gta     624
Val Met Tyr Thr Ala Phe Lys Ala Leu Gly Glu Ala Trp Asn Tyr Val
        195                 200                 205

cag gtc tgt gat tca gac aca atg ctc gat cca gct tca tca gtg gaa     672
Gln Val Cys Asp Ser Asp Thr Met Leu Asp Pro Ala Ser Ser Val Glu
    210                 215                 220

atg gta aag gtc tta gaa gaa gat cca atg gtt gga gga gtt gga ggc     720
Met Val Lys Val Leu Glu Glu Asp Pro Met Val Gly Gly Val Gly Gly
225                 230                 235                 240

gat gtg cag att ttg aac aag tat gat tcc tgg atc tcc ttt ctg agc     768
Asp Val Gln Ile Leu Asn Lys Tyr Asp Ser Trp Ile Ser Phe Leu Ser
                245                 250                 255

agt gtg aga tac tgg atg gca ttt aac ata gaa aga gcc tgc cag tcc     816
Ser Val Arg Tyr Trp Met Ala Phe Asn Ile Glu Arg Ala Cys Gln Ser
            260                 265                 270

tat ttt ggc tgc gta cag tgc atc agt gga cct ctg gga atg tac aga     864

Tyr Phe Gly Cys Val Gln Cys Ile Ser Gly Pro Leu Gly Met Tyr Arg
        275                 280                 285

aac tct tta ctc cat gaa ttt gtg gaa gat tgg tac aat caa gaa ttt      912
Asn Ser Leu Leu His Glu Phe Val Glu Asp Trp Tyr Asn Gln Glu Phe
    290                 295                 300

atg ggc tcc cag tgc agc ttt gga gac gac agg cat cta act aac cga      960
Met Gly Ser Gln Cys Ser Phe Gly Asp Asp Arg His Leu Thr Asn Arg
305                 310                 315                 320

gtg cta agt ctg ggc tat gca aca aaa tac aca gct aga tcc aag tgc     1008
Val Leu Ser Leu Gly Tyr Ala Thr Lys Tyr Thr Ala Arg Ser Lys Cys
                325                 330                 335

ctt act gaa aca cca ata gag tat ctc agg tgg ctg aat cag cag acc     1056
Leu Thr Glu Thr Pro Ile Glu Tyr Leu Arg Trp Leu Asn Gln Gln Thr
            340                 345                 350

cgc tgg agt aaa tcg tat ttt aga gag tgg ctt tat aat gca atg tgg     1104
Arg Trp Ser Lys Ser Tyr Phe Arg Glu Trp Leu Tyr Asn Ala Met Trp
        355                 360                 365

ttc cac aag cac cat ttg tgg atg acc tat gaa gct gta atc act gga     1152
Phe His Lys His His Leu Trp Met Thr Tyr Glu Ala Val Ile Thr Gly
    370                 375                 380

ttc ttt cct ttc ttc ctt atc gct aca gtc att cag ctc ttc tac agg     1200
Phe Phe Pro Phe Phe Leu Ile Ala Thr Val Ile Gln Leu Phe Tyr Arg
385                 390                 395                 400

gga aaa atc tgg aac atc ctc ctt ttc ttg ttg aca gtt cag tta gtg     1248
Gly Lys Ile Trp Asn Ile Leu Leu Phe Leu Leu Thr Val Gln Leu Val
                405                 410                 415

ggc ctg ata aag tct tcc ttt gcc agt ttc ctt agg ggc aac att gtc     1296
Gly Leu Ile Lys Ser Ser Phe Ala Ser Phe Leu Arg Gly Asn Ile Val
            420                 425                 430

atg gtt ttc atg tca ctc tac tca gtg ttg tat atg tcg agt tta ctg     1344
Met Val Phe Met Ser Leu Tyr Ser Val Leu Tyr Met Ser Ser Leu Leu
        435                 440                 445

cca gca aag atg ttt gca atc gcc acg ata aac aaa gca ggg tgg ggc     1392
Pro Ala Lys Met Phe Ala Ile Ala Thr Ile Asn Lys Ala Gly Trp Gly
    450                 455                 460

aca tca gga aga aaa acc att gta gtt aat ttt ata gga ctc att cca     1440
Thr Ser Gly Arg Lys Thr Ile Val Val Asn Phe Ile Gly Leu Ile Pro
465                 470                 475                 480

gtc tcc att tgg ttt aca atc ctc cta ggt cgc gta att ttc act atc     1488
Val Ser Ile Trp Phe Thr Ile Leu Leu Gly Arg Val Ile Phe Thr Ile
                485                 490                 495

tac aag gaa tca aaa aag cca ttc tct gag tca aaa aca aca gtt ctc     1536
Tyr Lys Glu Ser Lys Lys Pro Phe Ser Glu Ser Lys Thr Thr Val Leu
            500                 505                 510

gtc att ggt aca atc ctc tat gca tgt tac tgg gtt ctt cta ttg act     1584
Val Ile Gly Thr Ile Leu Tyr Ala Cys Tyr Trp Val Leu Leu Leu Thr
        515                 520                 525

ttg tac ttg gtt ctc atc acc aaa tgt ggc agg cgg aag aaa gag caa     1632
Leu Tyr Leu Val Leu Ile Thr Lys Cys Gly Arg Arg Lys Lys Glu Gln
    530                 535                 540

cac tat gac atg gtg cta gat gta tga                                 1659

His Tyr Asp Met Val Leu Asp Val
545                 550

<210>  40

<211>  552

<212>  PRT

<213>  Gallus gallus

<400>  40

Met Tyr Cys Glu Arg Phe Ile Cys Ile Leu Arg Ile Leu Gly Thr Thr
1               5                   10                  15

Leu Phe Gly Val Ser Leu Leu Leu Gly Ile Thr Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Ile Leu Ala Ser His Leu Ile Ile Gln Ser Leu Phe Ala
    50                  55                  60

Tyr Leu Glu His Arg Lys Met Lys Arg Ser Leu Glu Thr Pro Ile Lys
65                  70                  75                  80

Leu Asn Lys Thr Val Ala Leu Cys Ile Ala Ala Tyr Gln Glu Asp Pro
                85                  90                  95

Asp Tyr Leu Arg Lys Cys Leu Leu Ser Val Lys Arg Leu Thr Tyr Pro
            100                 105                 110

Gly Ile Lys Val Val Met Val Ile Asp Gly Asn Ser Glu Asp Asp Val
        115                 120                 125

Tyr Met Met Asp Ile Phe Thr Glu Ile Met Gly Arg Asp Lys Ser Ala
    130                 135                 140

Thr Tyr Ile Trp Ser Asn Asn Phe His Asp Lys Gly Pro Gly Glu Thr
145                 150                 155                 160

Glu Glu Ser His Arg Glu Ser Met Gln His Val Ser Gln Leu Val Leu
                165                 170                 175

Ser Asn Lys Ser Val Cys Ile Met Gln Lys Trp Gly Gly Lys Arg Glu
            180                 185                 190

Val Met Tyr Thr Ala Phe Lys Ala Leu Gly Glu Ala Trp Asn Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Met Val Gly Gly Val Gly Gly
225                 230                 235                 240

Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Val Glu Asp Trp Tyr Asn Gln Glu Phe
    290                 295                 300

Met Gly Ser Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Glu Tyr Leu Arg Trp Leu Asn Gln Gln Thr
            340                 345                 350

Arg Trp Ser Lys Ser Tyr Phe Arg Glu Trp Leu Tyr Asn Ala Met Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Lys Ile Trp Asn Ile Leu Leu Phe Leu Leu Thr Val Gln Leu Val
                405                 410                 415

Gly Leu Ile Lys Ser Ser Phe Ala Ser Phe Leu Arg Gly Asn Ile Val
            420                 425                 430

Met Val Phe Met Ser Leu Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Val Ser Ile Trp Phe Thr Ile Leu Leu Gly Arg Val Ile Phe Thr Ile
                485                 490                 495

Tyr Lys Glu Ser Lys Lys Pro Phe Ser Glu Ser Lys Thr Thr Val Leu
            500                 505                 510

Val Ile Gly Thr Ile Leu Tyr Ala Cys Tyr Trp Val Leu Leu Leu Thr
        515                 520                 525

Leu Tyr Leu Val Leu Ile Thr Lys Cys Gly Arg Arg Lys Lys Glu Gln
    530                 535                 540

His Tyr Asp Met Val Leu Asp Val
545                 550

<210>  41

<211>  1767

<212>  DNA

<213>  Xenopus laevis

<220>

<221>  CDS

<222>  (1)..(1767)

<223>

<300>

<308>  M22249.1

<309>  1989-04-22

<313>  (527)..(2293)

<400>  41
atg aag gaa aaa gct gca gaa aca atg gag att cct gaa ggg atc ccc      48
Met Lys Glu Lys Ala Ala Glu Thr Met Glu Ile Pro Glu Gly Ile Pro
1               5                   10                  15

aaa gat cta gag cca aaa cac ccc acc ctt tgg agg ata att tat tat      96
Lys Asp Leu Glu Pro Lys His Pro Thr Leu Trp Arg Ile Ile Tyr Tyr
            20                  25                  30

tct ttt ggt gtg gtg cta tta gct acc att aca gca gcc tat gtg gca      144
Ser Phe Gly Val Val Leu Leu Ala Thr Ile Thr Ala Ala Tyr Val Ala
        35                  40                  45

gag ttc cag gtc ctc aaa cat gaa gcc att ctc ttc tcc ctt ggg ctt      192
Glu Phe Gln Val Leu Lys His Glu Ala Ile Leu Phe Ser Leu Gly Leu
    50                  55                  60

tat ggt ctt gca atg ctt ctc cac ctg atg atg cag agc ctc ttt gcc      240

Tyr Gly Leu Ala Met Leu Leu His Leu Met Met Gln Ser Leu Phe Ala
65                  70                  75                  80

ttc ctg gag ata cgc agg gta aat aag agt gag ctt cct tgc agc ttt      288
Phe Leu Glu Ile Arg Arg Val Asn Lys Ser Glu Leu Pro Cys Ser Phe
                85                  90                  95

aag aag aca gtg gct ctg acc att gct ggg tat cag gag aac cct gag      336
Lys Lys Thr Val Ala Leu Thr Ile Ala Gly Tyr Gln Glu Asn Pro Glu
            100                 105                 110

tac ctg ata aag tgc ttg gaa tcc tgc aag tat gtg aaa tac ccc aaa      384
Tyr Leu Ile Lys Cys Leu Glu Ser Cys Lys Tyr Val Lys Tyr Pro Lys
        115                 120                 125

gat aaa ctc aag atc att ttg gtc atc gat ggg aac aca gag gat gat      432
Asp Lys Leu Lys Ile Ile Leu Val Ile Asp Gly Asn Thr Glu Asp Asp
    130                 135                 140

gcc tac atg atg gag atg ttc aaa gac gtg ttc cac ggt gaa gat gta      480
Ala Tyr Met Met Glu Met Phe Lys Asp Val Phe His Gly Glu Asp Val
145                 150                 155                 160

ggc acc tac gta tgg aag gga aat tac cac act gtt aaa aag cct gag      528
Gly Thr Tyr Val Trp Lys Gly Asn Tyr His Thr Val Lys Lys Pro Glu
                165                 170                 175

gag acc aat aag gga tcc tgt cct gag gtt tct aag ccc ttg aat gaa      576
Glu Thr Asn Lys Gly Ser Cys Pro Glu Val Ser Lys Pro Leu Asn Glu
            180                 185                 190

gat gaa ggt atc aat atg gtg gaa gaa ctt gtt aga aac aag aga tgt      624
Asp Glu Gly Ile Asn Met Val Glu Glu Leu Val Arg Asn Lys Arg Cys
        195                 200                 205

gtg tgc atc atg caa cag tgg ggc gga aaa aga gag gtc atg tac aca      672
Val Cys Ile Met Gln Gln Trp Gly Gly Lys Arg Glu Val Met Tyr Thr
    210                 215                 220

gca ttc cag gcc att ggg act tct gtg gac tat gta cag gtc tgt gac      720
Ala Phe Gln Ala Ile Gly Thr Ser Val Asp Tyr Val Gln Val Cys Asp
225                 230                 235                 240

tcg gac acc aaa ctg gat gaa ctg gca aca gtg gaa atg gtg aag gtt      768
Ser Asp Thr Lys Leu Asp Glu Leu Ala Thr Val Glu Met Val Lys Val
                245                 250                 255

ctg gaa tcc aat gac atg tac ggc gca gtg gga gga gac gtt cgc att      816
Leu Glu Ser Asn Asp Met Tyr Gly Ala Val Gly Gly Asp Val Arg Ile
            260                 265                 270

ctg aac cct tat gat tcc ttc att agt ttc atg agc agc ctg cgt tac      864
Leu Asn Pro Tyr Asp Ser Phe Ile Ser Phe Met Ser Ser Leu Arg Tyr
        275                 280                 285

tgg atg gcg ttt aac gtg gag agg gcc tgc cag tct tac ttc gac tgc      912
Trp Met Ala Phe Asn Val Glu Arg Ala Cys Gln Ser Tyr Phe Asp Cys
    290                 295                 300

gtg tcc tgt ata agt gga cct ctg gga atg tac cgg aac aac att ctc      960
Val Ser Cys Ile Ser Gly Pro Leu Gly Met Tyr Arg Asn Asn Ile Leu
305                 310                 315                 320

cag gtg ttt ttg gaa gcc tgg tac aga cag aaa ttt ttg gga acc tat     1008
Gln Val Phe Leu Glu Ala Trp Tyr Arg Gln Lys Phe Leu Gly Thr Tyr
                325                 330                 335

tgt act ttg gga gat gat aga cat ctg aca aac cga gtg ctc agc atg     1056

Cys Thr Leu Gly Asp Asp Arg His Leu Thr Asn Arg Val Leu Ser Met
            340                 345                 350

gga tat cgc acc aaa tac acc cac aaa tcc aga gca ttc tcc gaa act      1104
Gly Tyr Arg Thr Lys Tyr Thr His Lys Ser Arg Ala Phe Ser Glu Thr
        355                 360                 365

cca tcc ctg tat ctc cgg tgg ttg aac cag caa acc cgg tgg acc aag      1152
Pro Ser Leu Tyr Leu Arg Trp Leu Asn Gln Gln Thr Arg Trp Thr Lys
    370                 375                 380

tcc tac ttc cga gag tgg ctg tat aat gcc cag tgg tgg cac aag cat      1200
Ser Tyr Phe Arg Glu Trp Leu Tyr Asn Ala Gln Trp Trp His Lys His
385                 390                 395                 400

cac atc tgg atg acc tat gag tct gtg gtg tcc ttc atc ttt ccc ttc      1248
His Ile Trp Met Thr Tyr Glu Ser Val Val Ser Phe Ile Phe Pro Phe
                405                 410                 415

ttc atc act gcc act gtt atc cgc ctc atc tat gcc ggc acc ata tgg      1296
Phe Ile Thr Ala Thr Val Ile Arg Leu Ile Tyr Ala Gly Thr Ile Trp
            420                 425                 430

aat gtt gtg tgg ctc ctc ctg tgc atc cag atc atg tct ctc ttc aaa      1344
Asn Val Val Trp Leu Leu Leu Cys Ile Gln Ile Met Ser Leu Phe Lys
        435                 440                 445

tcc atc tat gcc tgc tgg ctc cgc ggc aac ttc att atg ctc ctg atg      1392
Ser Ile Tyr Ala Cys Trp Leu Arg Gly Asn Phe Ile Met Leu Leu Met
    450                 455                 460

tct ctc tac tcc atg ctg tac atg act ggg ctt ctg cca tcc aag tac      1440
Ser Leu Tyr Ser Met Leu Tyr Met Thr Gly Leu Leu Pro Ser Lys Tyr
465                 470                 475                 480

ttt gcc ttg ttg acc tta aac aag acc ggt tgg gga aca tct ggg cgc      1488
Phe Ala Leu Leu Thr Leu Asn Lys Thr Gly Trp Gly Thr Ser Gly Arg
                485                 490                 495

aag aag ata gta ggc aac tac atg cca ata ctg ccc ctg tcc ata tgg      1536
Lys Lys Ile Val Gly Asn Tyr Met Pro Ile Leu Pro Leu Ser Ile Trp
            500                 505                 510

gca gct gtt ctg tgt gga ggg gtg ggt tat agt atc tat atg gac tgt      1584
Ala Ala Val Leu Cys Gly Gly Val Gly Tyr Ser Ile Tyr Met Asp Cys
        515                 520                 525

caa aat gac tgg agc acc cct gaa aag caa aag gag atg tac cat cta      1632
Gln Asn Asp Trp Ser Thr Pro Glu Lys Gln Lys Glu Met Tyr His Leu
    530                 535                 540

ttg tat ggg tgt gtg ggc tat gta atg tac tgg gta atc atg gct gtg      1680
Leu Tyr Gly Cys Val Gly Tyr Val Met Tyr Trp Val Ile Met Ala Val
545                 550                 555                 560

atg tac tgg gtc tgg gtg aag agg tgc tgc agg aag agg tcc caa act      1728
Met Tyr Trp Val Trp Val Lys Arg Cys Cys Arg Lys Arg Ser Gln Thr
                565                 570                 575

gtc acc ctg gtc cat gac att cct gat atg tgt gtt taa                  1767
Val Thr Leu Val His Asp Ile Pro Asp Met Cys Val
            580                 585

<210>  42

<211>  588

<212>  PRT

<213>  Xenopus laevis

<400>  42

Met Lys Glu Lys Ala Ala Glu Thr Met Glu Ile Pro Glu Gly Ile Pro
1               5                   10                  15

Lys Asp Leu Glu Pro Lys His Pro Thr Leu Trp Arg Ile Ile Tyr Tyr
            20                  25                  30

Ser Phe Gly Val Val Leu Leu Ala Thr Ile Thr Ala Ala Tyr Val Ala
        35                  40                  45

Glu Phe Gln Val Leu Lys His Glu Ala Ile Leu Phe Ser Leu Gly Leu
    50                  55                  60

Tyr Gly Leu Ala Met Leu Leu His Leu Met Met Gln Ser Leu Phe Ala
65                  70                  75                  80

Phe Leu Glu Ile Arg Arg Val Asn Lys Ser Glu Leu Pro Cys Ser Phe
                85                  90                  95

Lys Lys Thr Val Ala Leu Thr Ile Ala Gly Tyr Gln Glu Asn Pro Glu
            100                 105                 110

Tyr Leu Ile Lys Cys Leu Glu Ser Cys Lys Tyr Val Lys Tyr Pro Lys
        115                 120                 125

Asp Lys Leu Lys Ile Ile Leu Val Ile Asp Gly Asn Thr Glu Asp Asp
    130                 135                 140

Ala Tyr Met Met Glu Met Phe Lys Asp Val Phe His Gly Glu Asp Val
145                 150                 155                 160

Gly Thr Tyr Val Trp Lys Gly Asn Tyr His Thr Val Lys Lys Pro Glu
                165                 170                 175

Glu Thr Asn Lys Gly Ser Cys Pro Glu Val Ser Lys Pro Leu Asn Glu
            180                 185                 190

Asp Glu Gly Ile Asn Met Val Glu Glu Leu Val Arg Asn Lys Arg Cys
        195                 200                 205

Val Cys Ile Met Gln Gln Trp Gly Gly Lys Arg Glu Val Met Tyr Thr
    210                 215                 220

Ala Phe Gln Ala Ile Gly Thr Ser Val Asp Tyr Val Gln Val Cys Asp
225                 230                 235                 240

Ser Asp Thr Lys Leu Asp Glu Leu Ala Thr Val Glu Met Val Lys Val
                245                 250                 255

Leu Glu Ser Asn Asp Met Tyr Gly Ala Val Gly Gly Asp Val Arg Ile
            260                 265                 270

Leu Asn Pro Tyr Asp Ser Phe Ile Ser Phe Met Ser Ser Leu Arg Tyr
        275                 280                 285

Trp Met Ala Phe Asn Val Glu Arg Ala Cys Gln Ser Tyr Phe Asp Cys
    290                 295                 300

Val Ser Cys Ile Ser Gly Pro Leu Gly Met Tyr Arg Asn Asn Ile Leu
305                 310                 315                 320

Gln Val Phe Leu Glu Ala Trp Tyr Arg Gln Lys Phe Leu Gly Thr Tyr
                325                 330                 335

Cys Thr Leu Gly Asp Asp Arg His Leu Thr Asn Arg Val Leu Ser Met
            340                 345                 350

Gly Tyr Arg Thr Lys Tyr Thr His Lys Ser Arg Ala Phe Ser Glu Thr
        355                 360                 365

Pro Ser Leu Tyr Leu Arg Trp Leu Asn Gln Gln Thr Arg Trp Thr Lys
    370                 375                 380

Ser Tyr Phe Arg Glu Trp Leu Tyr Asn Ala Gln Trp Trp His Lys His
385                 390                 395                 400

His Ile Trp Met Thr Tyr Glu Ser Val Val Ser Phe Ile Phe Pro Phe
                405                 410                 415

Phe Ile Thr Ala Thr Val Ile Arg Leu Ile Tyr Ala Gly Thr Ile Trp
            420                 425                 430

Asn Val Val Trp Leu Leu Leu Cys Ile Gln Ile Met Ser Leu Phe Lys
        435                 440                 445

Ser Ile Tyr Ala Cys Trp Leu Arg Gly Asn Phe Ile Met Leu Leu Met
    450                 455                 460

Ser Leu Tyr Ser Met Leu Tyr Met Thr Gly Leu Leu Pro Ser Lys Tyr
465                 470                 475                 480

Phe Ala Leu Leu Thr Leu Asn Lys Thr Gly Trp Gly Thr Ser Gly Arg
                485                 490                 495

Lys Lys Ile Val Gly Asn Tyr Met Pro Ile Leu Pro Leu Ser Ile Trp
            500                 505                 510

Ala Ala Val Leu Cys Gly Gly Val Gly Tyr Ser Ile Tyr Met Asp Cys
        515                 520                 525

Gln Asn Asp Trp Ser Thr Pro Glu Lys Gln Lys Glu Met Tyr His Leu
    530                 535                 540

Leu Tyr Gly Cys Val Gly Tyr Val Met Tyr Trp Val Ile Met Ala Val
545                 550                 555                 560

Met Tyr Trp Val Trp Val Lys Arg Cys Cys Arg Lys Arg Ser Gln Thr
                565                 570                 575

Val Thr Leu Val His Asp Ile Pro Asp Met Cys Val
            580                 585

<210>  43

<211>  1656

<212>  DNA

<213>  Xenopus laevis

<220>

<221>  CDS

<222>  (1)..(1656)

<223>

<400>  43
atg cac tgt gaa cgg ttt ata tgc atc ctg aga ata att ggg aca act      48
Met His Cys Glu Arg Phe Ile Cys Ile Leu Arg Ile Ile Gly Thr Thr
1               5                   10                  15

ctc ttc gga gtc tcc ttg tta ctt gga atc tca gct gct tat atc gtt      96
Leu Phe Gly Val Ser Leu Leu Leu Gly Ile Ser Ala Ala Tyr Ile Val
            20                  25                  30

ggt tac caa ttt atc caa aca gac aac tat tat ttc tca ttt gga ctc      144
Gly Tyr Gln Phe Ile Gln Thr Asp Asn Tyr Tyr Phe Ser Phe Gly Leu
        35                  40                  45

tat ggg gcg att tta gcc ctc cac ctt att atc caa agc ctt ttt gcc      192
Tyr Gly Ala Ile Leu Ala Leu His Leu Ile Ile Gln Ser Leu Phe Ala
    50                  55                  60

ttt ctg gaa cac cga aaa atg aaa cga tct cta gaa acc ccc att aaa      240
Phe Leu Glu His Arg Lys Met Lys Arg Ser Leu Glu Thr Pro Ile Lys
65                  70                  75                  80

ctg aat aaa tca gtt gcc cta tgt att gct gca tat caa gag gac gaa      288
Leu Asn Lys Ser Val Ala Leu Cys Ile Ala Ala Tyr Gln Glu Asp Glu
                85                  90                  95

gac tac tta cgg aaa tgt tta ctt tcg gtc aag cgc ttg acg tac cca      336
Asp Tyr Leu Arg Lys Cys Leu Leu Ser Val Lys Arg Leu Thr Tyr Pro
            100                 105                 110

gga atg aaa gtc atc atg gtg atc gat gga aac tcg gac gat gat ctc 384
Gly Met Lys Val Ile Met Val Ile Asp Gly Asn Ser Asp Asp Asp Leu
        115                 120                 125

tac atg atg aat atc ttt cgt gag att atg ggg aat gac agc tgc gcc 432
Tyr Met Met Asn Ile Phe Arg Glu Ile Met Gly Asn Asp Ser Cys Ala
    130                 135                 140

act tac gta tgg aaa aat aac ttc cac atg aaa ggc ccc aac gag acg 480
Thr Tyr Val Trp Lys Asn Asn Phe His Met Lys Gly Pro Asn Glu Thr
145                 150                 155                 160

gac gaa acg cac aga gag agc atg cag cac gta acg cag atg gtt ctc 528
Asp Glu Thr His Arg Glu Ser Met Gln His Val Thr Gln Met Val Leu
                165                 170                 175

tcc aat aga aac gtg tgc atc atg cag aaa tgg aat ggg aag aga gaa 576
Ser Asn Arg Asn Val Cys Ile Met Gln Lys Trp Asn Gly Lys Arg Glu
            180                 185                 190

gtc atg tac acc gcg ttc aag gca ctg ggg aga agt gtg gat tat gtg 624
Val Met Tyr Thr Ala Phe Lys Ala Leu Gly Arg Ser Val Asp Tyr Val
        195                 200                 205

cag gta tgt gat tct gac aca gtg ctt gat ccg gcg tca tca gtg gag 672
Gln Val Cys Asp Ser Asp Thr Val Leu Asp Pro Ala Ser Ser Val Glu
    210                 215                 220

atg gtc aaa gta ctg gag gaa gac atc atg gtt gga gga gtg ggt gga 720
Met Val Lys Val Leu Glu Glu Asp Ile Met Val Gly Gly Val Gly Gly
225                 230                 235                 240

gat gtr cag att tta aac aag tac gac tca tgg att tcc ttc ctg agt 768
Asp Xaa Gln Ile Leu Asn Lys Tyr Asp Ser Trp Ile Ser Phe Leu Ser
                245                 250                 255

agc gtc aga tac tgg atg gcg ttt aac aty gag aga gca tgc cag tct 816
Ser Val Arg Tyr Trp Met Ala Phe Asn Xaa Glu Arg Ala Cys Gln Ser
            260                 265                 270

tac ttt ggc tgt gtg caa tgc att agc ggc ccg ttg ggg atg tac cgc 864
Tyr Phe Gly Cys Val Gln Cys Ile Ser Gly Pro Leu Gly Met Tyr Arg
        275                 280                 285

aat tcc ctt ctc cac gaa ttc att gaa gac tgg tac aac caa gaa ttt 912
Asn Ser Leu Leu His Glu Phe Ile Glu Asp Trp Tyr Asn Gln Glu Phe
    290                 295                 300

ttg ggt tcc cag tgc agt ttt ggg gat gac cgt cac cta acc aat cga 960
Leu Gly Ser Gln Cys Ser Phe Gly Asp Asp Arg His Leu Thr Asn Arg
305                 310                 315                 320

gtt ttg agt ctg ggc tat gca acc aaa tac acg gcc aga tcc aaa tgc 1008
Val Leu Ser Leu Gly Tyr Ala Thr Lys Tyr Thr Ala Arg Ser Lys Cys
                325                 330                 335

ctt act gaa aca ccc acc gag tac ctg cgg tgg ctc aac cag caa acg 1056
Leu Thr Glu Thr Pro Thr Glu Tyr Leu Arg Trp Leu Asn Gln Gln Thr
            340                 345                 350

cga tgg agc aag tcc tac ttc cga gaa tgg ctg tac aat tca ttg tgg 1104
Arg Trp Ser Lys Ser Tyr Phe Arg Glu Trp Leu Tyr Asn Ser Leu Trp
        355                 360                 365

ttc cat aaa cat cac tta tgg atg acc tac gaa gct gtg att act gga 1152
Phe His Lys His His Leu Trp Met Thr Tyr Glu Ala Val Ile Thr Gly
    370                 375                 380

ttc ttt cct ttc ttc ctc atc gcc act gtc atc cag ctc ttc tac cgt 1200
Phe Phe Pro Phe Phe Leu Ile Ala Thr Val Ile Gln Leu Phe Tyr Arg
385                 390                 395                 400

gga agg atc tgg aac atc ctc ctg ttc ttg ctg aca gta caa ctt gta 1248
Gly Arg Ile Trp Asn Ile Leu Leu Phe Leu Leu Thr Val Gln Leu Val
                405                 410                 415

ggc ctt atc aaa tct tcc ttt gct agt gcc ctc cga ggg aac ata gtc 1296
Gly Leu Ile Lys Ser Ser Phe Ala Ser Ala Leu Arg Gly Asn Ile Val
            420                 425                 430

atg gtc ttc atg tcc ttc tac tca gtg tta tac atg tcc agt tta cta 1344
Met Val Phe Met Ser Phe Tyr Ser Val Leu Tyr Met Ser Ser Leu Leu
        435                 440                 445

cct gcc aaa atg ttt gcc att gcc acc atc aac aag gca ggt tgg ggc 1392
Pro Ala Lys Met Phe Ala Ile Ala Thr Ile Asn Lys Ala Gly Trp Gly
    450                 455                 460

aca tca gga agg aag aca ata gtt gtg aat ttt ata gga ctg att cct 1440
Thr Ser Gly Arg Lys Thr Ile Val Val Asn Phe Ile Gly Leu Ile Pro
465                 470                 475                 480

ata acc gtt tgg ttt aca att ctc ctt ggg ggc gtc tgc tac act att 1488
Ile Thr Val Trp Phe Thr Ile Leu Leu Gly Gly Val Cys Tyr Thr Ile
                485                 490                 495

tgg agg gaa aca aaa aag cca ttt tca gaa tct gaa aag ata gtt ctc 1536
Trp Arg Glu Thr Lys Lys Pro Phe Ser Glu Ser Glu Lys Ile Val Leu
            500                 505                 510

gcc gtt ggt gca ata ctt tac gca tgc tac tgg gtc atg ctt ttg act 1584
Ala Val Gly Ala Ile Leu Tyr Ala Cys Tyr Trp Val Met Leu Leu Thr
        515                 520                 525

atg tat gtt tct ctc gtc atg aar tgt ggc agg cgg aga aag gaa cca 1632
Met Tyr Val Ser Leu Val Met Lys Cys Gly Arg Arg Arg Lys Glu Pro
    530                 535                 540

caa cat gac ttg gtg ctt gct tga 1656
Gln His Asp Leu Val Leu Ala
545                 550

<210> 44

<211> 551

<212> PRT

<213> Xenopus laevis

<220>

<221> misc_feature

<222> (242)..(242)

<223> The 'Xaa' at location 242 stands for Val.

<220>

<221> misc_feature

<222>  (266)..(266)

<223>  The 'Xaa' at location 266 stands for Ile.

<400>  44

Met His Cys Glu Arg Phe Ile Cys Ile Leu Arg Ile Ile Gly Thr Thr
1               5                   10                  15

Leu Phe Gly Val Ser Leu Leu Leu Gly Ile Ser Ala Ala Tyr Ile Val
            20                  25                  30

Gly Tyr Gln Phe Ile Gln Thr Asp Asn Tyr Tyr Phe Ser Phe Gly Leu
        35                  40                  45

Tyr Gly Ala Ile Leu Ala Leu His Leu Ile Ile Gln Ser Leu Phe Ala
    50                  55                  60

Phe Leu Glu His Arg Lys Met Lys Arg Ser Leu Glu Thr Pro Ile Lys
65                  70                  75                  80

Leu Asn Lys Ser Val Ala Leu Cys Ile Ala Ala Tyr Gln Glu Asp Glu
                85                  90                  95

Asp Tyr Leu Arg Lys Cys Leu Leu Ser Val Lys Arg Leu Thr Tyr Pro
            100                 105                 110

Gly Met Lys Val Ile Met Val Ile Asp Gly Asn Ser Asp Asp Asp Leu
        115                 120                 125

Tyr Met Met Asn Ile Phe Arg Glu Ile Met Gly Asn Asp Ser Cys Ala
    130                 135                 140

Thr Tyr Val Trp Lys Asn Asn Phe His Met Lys Gly Pro Asn Glu Thr
145                 150                 155                 160

Asp Glu Thr His Arg Glu Ser Met Gln His Val Thr Gln Met Val Leu
                165                 170                 175

Ser Asn Arg Asn Val Cys Ile Met Gln Lys Trp Asn Gly Lys Arg Glu
            180                 185                 190

Val Met Tyr Thr Ala Phe Lys Ala Leu Gly Arg Ser Val Asp Tyr Val
        195                 200                 205

Gln Val Cys Asp Ser Asp Thr Val Leu Asp Pro Ala Ser Ser Val Glu
    210                 215                 220

Met Val Lys Val Leu Glu Glu Asp Ile Met Val Gly Gly Val Gly Gly
225                 230                 235                 240

Asp Xaa Gln Ile Leu Asn Lys Tyr Asp Ser Trp Ile Ser Phe Leu Ser
                245                 250                 255

Ser Val Arg Tyr Trp Met Ala Phe Asn Xaa Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Ile Glu Asp Trp Tyr Asn Gln Glu Phe
    290                 295                 300

Leu Gly Ser Gln Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Thr Glu Tyr Leu Arg Trp Leu Asn Gln Gln Thr
            340                 345                 350

Arg Trp Ser Lys Ser Tyr Phe Arg Glu Trp Leu Tyr Asn Ser Leu Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Val Ile Gln Leu Phe Tyr Arg
385                 390                 395                 400

Gly Arg Ile Trp Asn Ile Leu Leu Phe Leu Leu Thr Val Gln Leu Val
                405                 410                 415

Gly Leu Ile Lys Ser Ser Phe Ala Ser Ala Leu Arg Gly Asn Ile Val
            420                 425                 430

Met Val Phe Met Ser Phe Tyr Ser Val Leu Tyr Met Ser Ser Leu Leu
        435                 440                 445

Pro Ala Lys Met Phe Ala Ile Ala Thr Ile Asn Lys Ala Gly Trp Gly
    450                 455                 460

Thr Ser Gly Arg Lys Thr Ile Val Val Asn Phe Ile Gly Leu Ile Pro
465                 470                 475                 480

Ile Thr Val Trp Phe Thr Ile Leu Leu Gly Gly Val Cys Tyr Thr Ile
                485                 490                 495

Trp Arg Glu Thr Lys Lys Pro Phe Ser Glu Ser Glu Lys Ile Val Leu
            500                 505                 510

Ala Val Gly Ala Ile Leu Tyr Ala Cys Tyr Trp Val Met Leu Leu Thr
        515                 520                 525

Met Tyr Val Ser Leu Val Met Lys Cys Gly Arg Arg Arg Lys Glu Pro
    530                 535                 540

Gln His Asp Leu Val Leu Ala
545                 550

<210>  45

<211>  1674

<212>  DNA

<213>  Xenopus laevis

<220>

<221>  CDS

<222>  (1)..(1674)

<223>

<300>

<308>  AY302252.1

<309>  2003-11-30

<313>  (102)..(1775)

<400>  45
atg cct ggg aag ttt cag acc ggc ctt agg gtg cta gcc act tgc ctc      48
Met Pro Gly Lys Phe Gln Thr Gly Leu Arg Val Leu Ala Thr Cys Leu
1               5                   10                  15

ttt gct ctg ctg gtg ttg ggg ggc atc ttg gtt gca tat gtg aca ggg      96
Phe Ala Leu Leu Val Leu Gly Gly Ile Leu Val Ala Tyr Val Thr Gly
            20                  25                  30

tac caa ttt att cat acc gat cgc cac cat ctc tca ttt ggc cta tac      144
Tyr Gln Phe Ile His Thr Asp Arg His His Leu Ser Phe Gly Leu Tyr
        35                  40                  45

gga gcc atc ctg ggt ctc cat tta ctc tct cag agc ctc ttt gct ttt      192
Gly Ala Ile Leu Gly Leu His Leu Leu Ser Gln Ser Leu Phe Ala Phe
    50                  55                  60

ttg gag cac agg aag atg cga gga ggt ggg cgg tgt cct tca gga aag      240
Leu Glu His Arg Lys Met Arg Gly Gly Gly Arg Cys Pro Ser Gly Lys
65                  70                  75                  80

tcc aca gtg gtg ctt tgt att gca gca tat caa gag gac cca gag tac      288
Ser Thr Val Val Leu Cys Ile Ala Ala Tyr Gln Glu Asp Pro Glu Tyr
                85                  90                  95

tta cgg aaa tgt ctg cga tca gtg cgc cgc ctc tca tat cct cac ctt      336
Leu Arg Lys Cys Leu Arg Ser Val Arg Arg Leu Ser Tyr Pro His Leu
            100                 105                 110

cgt gtg atc atg gtg gtg gat ggg aat aca gaa gag gac aga tat atg 384
Arg Val Ile Met Val Val Asp Gly Asn Thr Glu Glu Asp Arg Tyr Met
        115                 120                 125

atg gac ata ttc cga gag gtc atg gga tca gag gga acc tgc tgc tac 432
Met Asp Ile Phe Arg Glu Val Met Gly Ser Glu Gly Thr Cys Cys Tyr
    130                 135                 140

att tgg gac aaa aat tac cat gaa tca gag gag gga gga caa gag ggt 480
Ile Trp Asp Lys Asn Tyr His Glu Ser Glu Glu Gly Gly Gln Glu Gly
145                 150                 155                 160

gag agg gga gta cag gag atg gtg aag aac ttc cag tat gtc tgc atc 528
Glu Arg Gly Val Gln Glu Met Val Lys Asn Phe Gln Tyr Val Cys Ile
                165                 170                 175

atg cag aag tgg ggt gga aaa agg gaa gtc acg tat act gcg ttt cgt 576
Met Gln Lys Trp Gly Gly Lys Arg Glu Val Thr Tyr Thr Ala Phe Arg
            180                 185                 190

gca ctt gga gac agt gtg gct tat gtg cag gtc tgt gac tct gac act 624
Ala Leu Gly Asp Ser Val Ala Tyr Val Gln Val Cys Asp Ser Asp Thr
        195                 200                 205

gtg tta gac cca gct tgc acc gct gag atg ctg cgc att ttg gaa gaa 672
Val Leu Asp Pro Ala Cys Thr Ala Glu Met Leu Arg Ile Leu Glu Glu
    210                 215                 220

gat cct gaa gtg ggg gga gta ggt gga gat gtg cag atc ctg aat aag 720
Asp Pro Glu Val Gly Gly Val Gly Gly Asp Val Gln Ile Leu Asn Lys
225                 230                 235                 240

tac gaa tcg tgg atc tca ttt ctg agc agt ttc cgc tac tgg atg gca 768
Tyr Glu Ser Trp Ile Ser Phe Leu Ser Ser Phe Arg Tyr Trp Met Ala
                245                 250                 255

ttc aat gtg gaa cgg gcc tgc cag tcc tac ttt ggc tgt gtc cag tgc 816
Phe Asn Val Glu Arg Ala Cys Gln Ser Tyr Phe Gly Cys Val Gln Cys
            260                 265                 270

atc agt ggc cca ctg gga atg tat agg aac agt ctt ttg cag tac ttt 864
Ile Ser Gly Pro Leu Gly Met Tyr Arg Asn Ser Leu Leu Gln Tyr Phe
        275                 280                 285

tta gaa gat tgg tac cat caa aca ttt ttg ggg cag aag tgt agc ttt 912
Leu Glu Asp Trp Tyr His Gln Thr Phe Leu Gly Gln Lys Cys Ser Phe
    290                 295                 300

gga gat gac aga cat ctc acg aac cgt gta cta agc atg gga ttc cga 960
Gly Asp Asp Arg His Leu Thr Asn Arg Val Leu Ser Met Gly Phe Arg
305                 310                 315                 320

acg aag tat aca gct cgc tct cgt tgc ctg aca gag aca cca acg cgg 1008
Thr Lys Tyr Thr Ala Arg Ser Arg Cys Leu Thr Glu Thr Pro Thr Arg
                325                 330                 335

tat ttg cgc tgg ttg aac caa caa acg cgc tgg agc aaa tca tac ttc 1056
Tyr Leu Arg Trp Leu Asn Gln Gln Thr Arg Trp Ser Lys Ser Tyr Phe
            340                 345                 350

cgt gag tgg cta tac aat gca ctg tgg ttc cac aaa cac cac ctt tgg 1104
Arg Glu Trp Leu Tyr Asn Ala Leu Trp Phe His Lys His His Leu Trp
        355                 360                 365

atg acc tat gaa tct gtg gtt act ggc ttc ttt ccc ttc ttc ttg gtg 1152
Met Thr Tyr Glu Ser Val Val Thr Gly Phe Phe Pro Phe Phe Leu Val
    370                 375                 380

gcc aca gtg gtt caa cta ttt tat cgt ggc cgt gtt tgg aat att ctt 1200
Ala Thr Val Val Gln Leu Phe Tyr Arg Gly Arg Val Trp Asn Ile Leu
385                 390                 395                 400

ctt ttt cta ttg acc gta cag ctt gtt ggt att ttg aag gca acc tat 1248
Leu Phe Leu Leu Thr Val Gln Leu Val Gly Ile Leu Lys Ala Thr Tyr
                405                 410                 415

gcc tgt atc ctt cga ggt aat gct gaa atg att ttc atg tca ctt tat 1296
Ala Cys Ile Leu Arg Gly Asn Ala Glu Met Ile Phe Met Ser Leu Tyr
            420                 425                 430

tca ctt ctc tac atg act agc ctt ctg cct gcc aaa ata ttc gca gtg 1344
Ser Leu Leu Tyr Met Thr Ser Leu Leu Pro Ala Lys Ile Phe Ala Val
        435                 440                 445

atc acc atc aaa aag tct ggc tgg ggg act tca ggg cgc agg aag ctg 1392
Ile Thr Ile Lys Lys Ser Gly Trp Gly Thr Ser Gly Arg Arg Lys Leu
    450                 455                 460

gtg gtg aat ttt atg ggt atg gtg ccc gtg tct gtc tgg ttt tgc att 1440
Val Val Asn Phe Met Gly Met Val Pro Val Ser Val Trp Phe Cys Ile
465                 470                 475                 480

ctg ctg gga gga tta gta tac aca gca tat tgt cag agt cat gat ccg 1488
Leu Leu Gly Gly Leu Val Tyr Thr Ala Tyr Cys Gln Ser His Asp Pro
                485                 490                 495

ttc acc gaa aca gaa ctg tta ttc ctg ttg aca ggt gcc atc tta tac 1536
Phe Thr Glu Thr Glu Leu Leu Phe Leu Leu Thr Gly Ala Ile Leu Tyr
            500                 505                 510

gga tgt tat tgg gtg gca ctt ctc agt tta tat ctt gct ttg att gcc 1584
Gly Cys Tyr Trp Val Ala Leu Leu Ser Leu Tyr Leu Ala Leu Ile Ala
        515                 520                 525

cgg cgg tgt ggg aag aga caa gag ctg tac aac tta gca ttg gag gaa 1632
Arg Arg Cys Gly Lys Arg Gln Glu Leu Tyr Asn Leu Ala Leu Glu Glu
    530                 535                 540

gtc tca gaa cca gag cca gct gcc aaa gca ata aag cct taa 1674
Val Ser Glu Pro Glu Pro Ala Ala Lys Ala Ile Lys Pro
545                 550                 555

<210> 46

<211> 557

<212> PRT

<213> Xenopus laevis

<400> 46

Met Pro Gly Lys Phe Gln Thr Gly Leu Arg Val Leu Ala Thr Cys Leu
1               5                   10                  15

Phe Ala Leu Leu Val Leu Gly Gly Ile Leu Val Ala Tyr Val Thr Gly
            20                  25                  30

Tyr Gln Phe Ile His Thr Asp Arg His His Leu Ser Phe Gly Leu Tyr
        35                  40                  45

Gly Ala Ile Leu Gly Leu His Leu Leu Ser Gln Ser Leu Phe Ala Phe
    50                  55                  60

Leu Glu His Arg Lys Met Arg Gly Gly Gly Arg Cys Pro Ser Gly Lys
65                  70                  75                  80

Ser Thr Val Val Leu Cys Ile Ala Ala Tyr Gln Glu Asp Pro Glu Tyr
                85                  90                  95

Leu Arg Lys Cys Leu Arg Ser Val Arg Arg Leu Ser Tyr Pro His Leu
            100                 105                 110

Arg Val Ile Met Val Val Asp Gly Asn Thr Glu Glu Asp Arg Tyr Met
        115                 120                 125

Met Asp Ile Phe Arg Glu Val Met Gly Ser Glu Gly Thr Cys Cys Tyr
    130                 135                 140

Ile Trp Asp Lys Asn Tyr His Glu Ser Glu Glu Gly Gly Gln Glu Gly
145                 150                 155                 160

Glu Arg Gly Val Gln Glu Met Val Lys Asn Phe Gln Tyr Val Cys Ile
                165                 170                 175

Met Gln Lys Trp Gly Gly Lys Arg Glu Val Thr Tyr Thr Ala Phe Arg
            180                 185                 190

Ala Leu Gly Asp Ser Val Ala Tyr Val Gln Val Cys Asp Ser Asp Thr
        195                 200                 205

Val Leu Asp Pro Ala Cys Thr Ala Glu Met Leu Arg Ile Leu Glu Glu
    210                 215                 220

Asp Pro Glu Val Gly Gly Val Gly Gly Asp Val Gln Ile Leu Asn Lys
225                 230                 235                 240

Tyr Glu Ser Trp Ile Ser Phe Leu Ser Ser Phe Arg Tyr Trp Met Ala
                245                 250                 255

Phe Asn Val Glu Arg Ala Cys Gln Ser Tyr Phe Gly Cys Val Gln Cys
            260                 265                 270

Ile Ser Gly Pro Leu Gly Met Tyr Arg Asn Ser Leu Leu Gln Tyr Phe
        275                 280                 285

Leu Glu Asp Trp Tyr His Gln Thr Phe Leu Gly Gln Lys Cys Ser Phe
    290                 295                 300

Gly Asp Asp Arg His Leu Thr Asn Arg Val Leu Ser Met Gly Phe Arg
305                 310                 315                 320

Thr Lys Tyr Thr Ala Arg Ser Arg Cys Leu Thr Glu Thr Pro Thr Arg
                325                 330                 335

Tyr Leu Arg Trp Leu Asn Gln Gln Thr Arg Trp Ser Lys Ser Tyr Phe
            340                 345                 350

Arg Glu Trp Leu Tyr Asn Ala Leu Trp Phe His Lys His His Leu Trp
        355                 360                 365

Met Thr Tyr Glu Ser Val Val Thr Gly Phe Phe Pro Phe Phe Leu Val
    370                 375                 380

Ala Thr Val Val Gln Leu Phe Tyr Arg Gly Arg Val Trp Asn Ile Leu
385                 390                 395                 400

Leu Phe Leu Leu Thr Val Gln Leu Val Gly Ile Leu Lys Ala Thr Tyr
                405                 410                 415

Ala Cys Ile Leu Arg Gly Asn Ala Glu Met Ile Phe Met Ser Leu Tyr
            420                 425                 430

Ser Leu Leu Tyr Met Thr Ser Leu Leu Pro Ala Lys Ile Phe Ala Val
        435                 440                 445

Ile Thr Ile Lys Lys Ser Gly Trp Gly Thr Ser Gly Arg Arg Lys Leu
    450                 455                 460

Val Val Asn Phe Met Gly Met Val Pro Val Ser Val Trp Phe Cys Ile
465                 470                 475                 480

Leu Leu Gly Gly Leu Val Tyr Thr Ala Tyr Cys Gln Ser His Asp Pro
                485                 490                 495

Phe Thr Glu Thr Glu Leu Leu Phe Leu Leu Thr Gly Ala Ile Leu Tyr
            500                 505                 510

Gly Cys Tyr Trp Val Ala Leu Leu Ser Leu Tyr Leu Ala Leu Ile Ala
        515                 520                 525

Arg Arg Cys Gly Lys Arg Gln Glu Leu Tyr Asn Leu Ala Leu Glu Glu
    530                 535                 540

Val Ser Glu Pro Glu Pro Ala Ala Lys Ala Ile Lys Pro
545                 550                 555

<210>  47

<211>  1659

<212>  DNA

<213>  Danio rerio

<220>

<221>  CDS

<222>  (1)..(1659)

<223>

<300>

<308>  AF190742.1

<309>  2000-10-02

<313>  (34)..(1692)

<400>  47
atg aga tgt gat aaa gcg gtc agc tac tta agg att gtt ggg acg aca      48
Met Arg Cys Asp Lys Ala Val Ser Tyr Leu Arg Ile Val Gly Thr Thr
1               5                   10                  15

ctg ttc ggc att tca ctg ctt gtg gga atc tct act gct tat atc atg      96
Leu Phe Gly Ile Ser Leu Leu Val Gly Ile Ser Thr Ala Tyr Ile Met
            20                  25                  30

ggg tat aag ctt gta aca act ccg ggc aac tat ttg tcc ttt ggt ctt      144
Gly Tyr Lys Leu Val Thr Thr Pro Gly Asn Tyr Leu Ser Phe Gly Leu
        35                  40                  45

tat gga gca att ctt gtc atc cac ctc atc atc caa agt gtg ttt gcc      192
Tyr Gly Ala Ile Leu Val Ile His Leu Ile Ile Gln Ser Val Phe Ala
    50                  55                  60

cta ctg gaa cac agg aac atg aaa cgc tcc ctg gaa aca cca atc aaa      240
Leu Leu Glu His Arg Asn Met Lys Arg Ser Leu Glu Thr Pro Ile Lys
65                  70                  75                  80

ctc aac aag tca ctg gcc cta tgc atc gca gcc tat cag gaa gac ccc      288
Leu Asn Lys Ser Leu Ala Leu Cys Ile Ala Ala Tyr Gln Glu Asp Pro
                85                  90                  95

aac tac ctg aga aag tgt tta ata tca gtg aag agg ctc acg tat ccg      336
Asn Tyr Leu Arg Lys Cys Leu Ile Ser Val Lys Arg Leu Thr Tyr Pro
            100                 105                 110

ggt ata aag gtc ata atg gtt atc gat ggg aac aac gat gat gac tgc      384
Gly Ile Lys Val Ile Met Val Ile Asp Gly Asn Asn Asp Asp Asp Cys
        115                 120                 125

tac atg atg gag atc ttt cga gaa atc atg ggc cgg gac aag gca gcc      432
Tyr Met Met Glu Ile Phe Arg Glu Ile Met Gly Arg Asp Lys Ala Ala
    130                 135                 140

acg tac att tgg aaa agc aac tat cac cat aga gga ccc gaa gaa act      480
Thr Tyr Ile Trp Lys Ser Asn Tyr His His Arg Gly Pro Glu Glu Thr
145                 150                 155                 160

gaa gaa tca tac gca aca agc ttg cag cat gtt tct cac ctg gtt ctc      528
Glu Glu Ser Tyr Ala Thr Ser Leu Gln His Val Ser His Leu Val Leu
                165                 170                 175

aat aat aag tgt gtg tgc atc atg cag aag tgg ggc ggg aaa aga gag 576
Asn Asn Lys Cys Val Cys Ile Met Gln Lys Trp Gly Gly Lys Arg Glu
            180                 185                 190

gtc atg tac acc gcc ttc aaa gcc ctg gga aga agt gtt gac tat gta 624
Val Met Tyr Thr Ala Phe Lys Ala Leu Gly Arg Ser Val Asp Tyr Val
        195                 200                 205

cag gtt tgt gat tca gac acc atg ctg gac ccg gcc tcc tct gta gag 672
Gln Val Cys Asp Ser Asp Thr Met Leu Asp Pro Ala Ser Ser Val Glu
    210                 215                 220

atg gtg aag gtt cta gaa gaa gat ccc aat gtt gga gga gta ggt gga 720
Met Val Lys Val Leu Glu Glu Asp Pro Asn Val Gly Gly Val Gly Gly
225                 230                 235                 240

gat gtg cag ata ttg aac aaa tat gag tcg tgg gtc tcc ttc ctg agc 768
Asp Val Gln Ile Leu Asn Lys Tyr Glu Ser Trp Val Ser Phe Leu Ser
                245                 250                 255

agc gtg agg tat tgg atg gcg ttc aac att gaa aga gcc tgc cag tca 816
Ser Val Arg Tyr Trp Met Ala Phe Asn Ile Glu Arg Ala Cys Gln Ser
            260                 265                 270

tat ttc ggg tgc gtt caa tgt atc agc gga ccc ttg gga atg tac agg 864
Tyr Phe Gly Cys Val Gln Cys Ile Ser Gly Pro Leu Gly Met Tyr Arg
        275                 280                 285

aac tcc ctc ctc cat gag ttc ctg gag gac tgg tat gat cag aca ttc 912
Asn Ser Leu Leu His Glu Phe Leu Glu Asp Trp Tyr Asp Gln Thr Phe
    290                 295                 300

atg gga agc cac tgc agt ttt ggc gat gac cgc cat ctg acc aat cga 960
Met Gly Ser His Cys Ser Phe Gly Asp Asp Arg His Leu Thr Asn Arg
305                 310                 315                 320

gta ctg agc ttg gga tat gcc acg aaa tac acc gcg cgc tcc aag tgc 1008
Val Leu Ser Leu Gly Tyr Ala Thr Lys Tyr Thr Ala Arg Ser Lys Cys
                325                 330                 335

ttg acc gag acg ccc att acc tac ctg cgc tgg ctc aac caa caa acc 1056
Leu Thr Glu Thr Pro Ile Thr Tyr Leu Arg Trp Leu Asn Gln Gln Thr
            340                 345                 350

cgc tgg agt aaa tcc tat ttc aga gag tgg ctt tac aac tcc ttg tgg 1104
Arg Trp Ser Lys Ser Tyr Phe Arg Glu Trp Leu Tyr Asn Ser Leu Trp
        355                 360                 365

ttc cac aag cac cac ttg tgg atg acc tac gag gcc gtc atc acc ggc 1152
Phe His Lys His His Leu Trp Met Thr Tyr Glu Ala Val Ile Thr Gly
    370                 375                 380

ttc ttc ccg ttc ttc ctt atc gcc act gcc att caa ctg ttc tac cag 1200
Phe Phe Pro Phe Phe Leu Ile Ala Thr Ala Ile Gln Leu Phe Tyr Gln
385                 390                 395                 400

ggc agg atc tgg aat atc ctt ctg ttc ctg ctg atc gtc cag gtc gtg 1248
Gly Arg Ile Trp Asn Ile Leu Leu Phe Leu Leu Ile Val Gln Val Val
                405                 410                 415

gca ctc ata aag tcc tca ttt gcc agt tgc ctc cga ggc aac ata gtc 1296
Ala Leu Ile Lys Ser Ser Phe Ala Ser Cys Leu Arg Gly Asn Ile Val
            420                 425                 430

atg gtc ttc atg tca ttc tac tca gtg tta tac atg tca agt ctg cta 1344
Met Val Phe Met Ser Phe Tyr Ser Val Leu Tyr Met Ser Ser Leu Leu
        435                 440                 445

ccg gca aaa atg ttt gca ata gcc acc ata aac aaa tcc gga tgg gga          1392
Pro Ala Lys Met Phe Ala Ile Ala Thr Ile Asn Lys Ser Gly Trp Gly
    450                 455                 460

acg tct gga agg aag acc gtg gtg gtg aac ttc atc gga ctc att cca          1440
Thr Ser Gly Arg Lys Thr Val Val Val Asn Phe Ile Gly Leu Ile Pro
465                 470                 475                 480

atc tca att tgg ttc act att ctt ttc gtt ggc att att tat acc ata          1488
Ile Ser Ile Trp Phe Thr Ile Leu Phe Val Gly Ile Ile Tyr Thr Ile
                485                 490                 495

atc caa gag acg cga aaa ccc ttt cct gaa tcc gaa aag gtg gtt ttg          1536
Ile Gln Glu Thr Arg Lys Pro Phe Pro Glu Ser Glu Lys Val Val Leu
            500                 505                 510

ata att ggc gca atc gtt tat atc agc tac tgg gtt gtg ttt ttg act          1584
Ile Ile Gly Ala Ile Val Tyr Ile Ser Tyr Trp Val Val Phe Leu Thr
        515                 520                 525

ttg tac gct gtc ctc att atg aag tgt ggc aag agg aag aaa gga cag          1632
Leu Tyr Ala Val Leu Ile Met Lys Cys Gly Lys Arg Lys Lys Gly Gln
    530                 535                 540

cag tat gac atg gtt ctt gac gta tag                                      1659
Gln Tyr Asp Met Val Leu Asp Val
545                 550

<210>  48

<211>  552

<212>  PRT

<213>  Danio rerio

<400>  48

Met Arg Cys Asp Lys Ala Val Ser Tyr Leu Arg Ile Val Gly Thr Thr
1               5                   10                  15

Leu Phe Gly Ile Ser Leu Leu Val Gly Ile Ser Thr Ala Tyr Ile Met
            20                  25                  30

Gly Tyr Lys Leu Val Thr Thr Pro Gly Asn Tyr Leu Ser Phe Gly Leu
        35                  40                  45

Tyr Gly Ala Ile Leu Val Ile His Leu Ile Ile Gln Ser Val Phe Ala
    50                  55                  60

Leu Leu Glu His Arg Asn Met Lys Arg Ser Leu Glu Thr Pro Ile Lys
65                  70                  75                  80

Leu Asn Lys Ser Leu Ala Leu Cys Ile Ala Ala Tyr Gln Glu Asp Pro
                85                  90                  95

Asn Tyr Leu Arg Lys Cys Leu Ile Ser Val Lys Arg Leu Thr Tyr Pro
            100                 105                 110

Gly Ile Lys Val Ile Met Val Ile Asp Gly Asn Asn Asp Asp Asp Cys
        115                 120                 125

Tyr Met Met Glu Ile Phe Arg Glu Ile Met Gly Arg Asp Lys Ala Ala
    130                 135                 140

Thr Tyr Ile Trp Lys Ser Asn Tyr His His Arg Gly Pro Glu Glu Thr
145                 150                 155                 160

Glu Glu Ser Tyr Ala Thr Ser Leu Gln His Val Ser His Leu Val Leu
                165                 170                 175

Asn Asn Lys Cys Val Cys Ile Met Gln Lys Trp Gly Gly Lys Arg Glu
            180                 185                 190

Val Met Tyr Thr Ala Phe Lys Ala Leu Gly Arg Ser Val Asp Tyr Val
        195                 200                 205

Gln Val Cys Asp Ser Asp Thr Met Leu Asp Pro Ala Ser Ser Val Glu
    210                 215                 220

Met Val Lys Val Leu Glu Glu Asp Pro Asn Val Gly Gly Val Gly Gly
225                 230                 235                 240

Asp Val Gln Ile Leu Asn Lys Tyr Glu Ser Trp Val Ser Phe Leu Ser
                245                 250                 255

Ser Val Arg Tyr Trp Met Ala Phe Asn Ile Glu Arg Ala Cys Gln Ser
            260                 265                 270

Tyr Phe Gly Cys Val Gln Cys Ile Ser Gly Pro Leu Gly Met Tyr Arg
        275                 280                 285

Asn Ser Leu Leu His Glu Phe Leu Glu Asp Trp Tyr Asp Gln Thr Phe
    290                 295                 300

Met Gly Ser His Cys Ser Phe Gly Asp Asp Arg His Leu Thr Asn Arg
305                 310                 315                 320

Val Leu Ser Leu Gly Tyr Ala Thr Lys Tyr Thr Ala Arg Ser Lys Cys
                325                 330                 335

Leu Thr Glu Thr Pro Ile Thr Tyr Leu Arg Trp Leu Asn Gln Gln Thr
            340                 345                 350

Arg Trp Ser Lys Ser Tyr Phe Arg Glu Trp Leu Tyr Asn Ser Leu Trp
        355                 360                 365

Phe His Lys His His Leu Trp Met Thr Tyr Glu Ala Val Ile Thr Gly
    370                 375                 380

Phe Phe Pro Phe Phe Leu Ile Ala Thr Ala Ile Gln Leu Phe Tyr Gln
385                 390                 395                 400

Gly Arg Ile Trp Asn Ile Leu Leu Phe Leu Leu Ile Val Gln Val Val
                405                 410                 415

Ala Leu Ile Lys Ser Ser Phe Ala Ser Cys Leu Arg Gly Asn Ile Val
            420                 425                 430

Met Val Phe Met Ser Phe Tyr Ser Val Leu Tyr Met Ser Ser Leu Leu
        435                 440                 445

Pro Ala Lys Met Phe Ala Ile Ala Thr Ile Asn Lys Ser Gly Trp Gly
    450                 455                 460

Thr Ser Gly Arg Lys Thr Val Val Val Asn Phe Ile Gly Leu Ile Pro
465                 470                 475                 480

Ile Ser Ile Trp Phe Thr Ile Leu Phe Val Gly Ile Ile Tyr Thr Ile
                485                 490                 495

Ile Gln Glu Thr Arg Lys Pro Phe Pro Glu Ser Glu Lys Val Val Leu
            500                 505                 510

Ile Ile Gly Ala Ile Val Tyr Ile Ser Tyr Trp Val Val Phe Leu Thr
        515                 520                 525

Leu Tyr Ala Val Leu Ile Met Lys Cys Gly Lys Arg Lys Lys Gly Gln
    530                 535                 540

Gln Tyr Asp Met Val Leu Asp Val
545                 550

<210>  49

<211>  2994

<212>  DNA

<213>  Danio rerio


<220>

<221>  CDS

<222>  (1)..(633)

<223>


<220>

<221>  CDS
<222>  (1059)..(1160)
<223>

<220>
<221>  CDS
<222>  (2065)..(2991)
<223>

<220>
<221>  misc_feature
<222>  (1425)..(1425)
<223>  1425 = any

<220>
<221>  misc_feature
<222>  (1481)..(1481)
<223>  1481= any

<220>
<221>  misc_feature
<222>  (1632)..(1632)
<223>  1632 = any

<220>
<221>  misc_feature
<222>  (1834)..(1834)
<223>  1834 = any

<220>
<221>  misc_feature
<222>  (2295)..(2295)
<223>  2295 = any

<400> 49
atg ccc tct cgc ttt ggc act gcg gtg cgg atc ttc atc acc acc tta 48
Met Pro Ser Arg Phe Gly Thr Ala Val Arg Ile Phe Ile Thr Thr Leu
1               5                   10                  15

ttt gca gca gtg gtg ctt ttc gca atc cta cta gcc tat gtg aca ggt 96
Phe Ala Ala Val Val Leu Phe Ala Ile Leu Leu Ala Tyr Val Thr Gly
            20                  25                  30

tac cag ttc atc cac aca gag cag cac cat ctg tct ttt ggc ttg tac 144
Tyr Gln Phe Ile His Thr Glu Gln His His Leu Ser Phe Gly Leu Tyr
        35                  40                  45

ggt gca ttt cta tcc ctc cac ctt ctc ctg cag agt ctc ttc gcc tac 192
Gly Ala Phe Leu Ser Leu His Leu Leu Leu Gln Ser Leu Phe Ala Tyr
    50                  55                  60

ctg gag cac aga caa atg cga ggc ccc tcc aga ccg cag cac ctg cgc 240
Leu Glu His Arg Gln Met Arg Gly Pro Ser Arg Pro Gln His Leu Arg
65                  70                  75                  80

cgc act gtg gcc ctc tgc att gca gcc tat cag gaa gat cca gac tac 288
Arg Thr Val Ala Leu Cys Ile Ala Ala Tyr Gln Glu Asp Pro Asp Tyr
                85                  90                  95

ctt cgc aag tgt ctg cgg agt tcg cgc att tct ttt ccc ggg ctg aaa 336
Leu Arg Lys Cys Leu Arg Ser Ser Arg Ile Ser Phe Pro Gly Leu Lys
            100                 105                 110

gtg gtg ttg gtg gtg gat ggc aat cgg cag gag gat gcc tac atg atg 384
Val Val Leu Val Val Asp Gly Asn Arg Gln Glu Asp Ala Tyr Met Met
        115                 120                 125

gat atc ttc cag gag gtg atg ggg gga gtg gag cag aca ggc tgt gtg 432
Asp Ile Phe Gln Glu Val Met Gly Gly Val Glu Gln Thr Gly Cys Val
    130                 135                 140

gtg tgg aaa ggg aat tac cac agt aac ggg gat gga gga gga gga gga 480
Val Trp Lys Gly Asn Tyr His Ser Asn Gly Asp Gly Gly Gly Gly Gly
145                 150                 155                 160

ggg aaa ggc tcg gtg cat gcc gaa gag gct gca cga gtg gcc aga gtg 528
Gly Lys Gly Ser Val His Ala Glu Glu Ala Ala Arg Val Ala Arg Val
                165                 170                 175

gtg cgg agc tgc cgt tac tcc tgc atc atg cag gaa tgg ggt ggc aag 576
Val Arg Ser Cys Arg Tyr Ser Cys Ile Met Gln Glu Trp Gly Gly Lys
            180                 185                 190

agg gaa gtg atg tat aca gcc ttc aaa gca ctt gga gat aca gtg gat 624
Arg Glu Val Met Tyr Thr Ala Phe Lys Ala Leu Gly Asp Thr Val Asp
        195                 200                 205

tac atg cag gtaagtctaa acctcattta acaaccaaaa aaaaaaatcg 673
Tyr Met Gln
    210

gaaacatacc tctatgcttt ttgatcttgt tactcttttt tttatgccta caattaacca 733

acggttcata ctctagtcat ctcaatgtcc ctgtaatctg aatccttgca acactaaggt 793

caaaggtcag tcctttaaat tcacctgtag ttccgagcac acctgtgtgc tgtcatcaac 853

tattcattgt ttcggcgtat aatgtgtatg ttcagaaaca gaaatgacct tgaaacatct 913

gtgaacctct ctttacaaca gtccttgcat tgttcacaca gtgattgtta acatgagcct 973

taccttctgg aaattagtca atatttgtat acgagttagt gaggaagtct tccaagtaag 1033

tatctcttgc tttgtttgtt tgcag gta tgt gat tca gac act gta ctt gat     1085
                          Val Cys Asp Ser Asp Thr Val Leu Asp
                                      215                 220

cca gca tgc act att gag atg ttg aag att cta gag gag gat cct gat     1133
Pro Ala Cys Thr Ile Glu Met Leu Lys Ile Leu Glu Glu Asp Pro Asp
            225                 230                 235

gtg gga gga gtt gga gga gat gtt cag gtaataattt taaggcaatt           1180
Val Gly Gly Val Gly Gly Asp Val Gln
        240                 245

tgtaaatatg tagaaacatt tcaacaaatt aatttgagag gattacattt gatctcatag   1240

atatatacat tagatatcgc ctggcctatt gtctattgga cggaatccaa agccagagat   1300

atacacatat atctatgatc gggagttttc ctggatgtta gtatgtaatt ttttttttct    1360

aattacgaaa attataattt tacatcactt ttctacattg atggatcagt caccgaacgg   1420

gcatncctga caaaaagctt gtgtttgtgt gtacagaaat gtactattca ccctccctat   1480

naaatttgat ctaatcatgt cctgaaacac agctcctctc ctgctttcac ttctcatact   1540

gacggaggga gcgattcgtt tgtgaatgaa tccccggaac gactctttca ctaacgttag   1600

ccgacaataa tacgagtttc tggcagcgca gnatctcgtt gtcatatttc ttttgcattg   1660

tttgctgatt ttattcaaca aaactagcat aagccgagtg tttaatgcga gttggagctg   1720

ctttgcctta tatggtgaat gcagtaagtg actgttatca tcaataacgt tacctgatta   1780

gcacaaaagt tcagaacata caaaaacaga aaaaaacgta atattaccta tganatgttc   1840

tgcctttgtg ctttgttttc tttgtttgct cgttactaca accgtagaca gcgctaaagt   1900

cccgcatctt cacgtaataa cactgtcttg actagtgcgg ttgaatgaca ttctcctggg   1960

agcactgtac caatgtggct gtgctattga cgcatgctca gggtccctat gcgatatcta   2020

gtgtatatat ctatgatttg ttctctgttc attgtttcct tcag att ctg aac aag    2076
                                                 Ile Leu Asn Lys

tat gac tcc tgg atc tcc ttc ctg agc agt gtc cgc tat tgg atg gcc     2124
Tyr Asp Ser Trp Ile Ser Phe Leu Ser Ser Val Arg Tyr Trp Met Ala
250                 255                 260                 265

ttc aat gtg gag cgg gcc tgc cag tct tat ttc gga tgt gtg cag tgc     2172
Phe Asn Val Glu Arg Ala Cys Gln Ser Tyr Phe Gly Cys Val Gln Cys
                270                 275                 280

atc agt gga cct cta gga atg tac cgt aac tct ctc ctt cag cag ttc     2220
Ile Ser Gly Pro Leu Gly Met Tyr Arg Asn Ser Leu Leu Gln Gln Phe
            285                 290                 295

tta gag ccc tgg tac cac cag acc ttc cta gga agc aag tgc agt ttt     2268
Leu Glu Pro Trp Tyr His Gln Thr Phe Leu Gly Ser Lys Cys Ser Phe
        300                 305                 310

ggt gat gat cga cat ctc acc aac cgn gtc ctt agc ttt ggc ttc aaa     2316
Gly Asp Asp Arg His Leu Thr Asn Arg Val Leu Ser Phe Gly Phe Lys
    315                 320                 325

acc aag ttc acg gca cgc tct caa tgc cag acc gag acc cca aca caa     2364
Thr Lys Phe Thr Ala Arg Ser Gln Cys Gln Thr Glu Thr Pro Thr Gln
330                 335                 340                 345

tac ctg cgt tgg ctc aat cag cag acc cgc tgg agc aag tct tac ttt 2412
Tyr Leu Arg Trp Leu Asn Gln Gln Thr Arg Trp Ser Lys Ser Tyr Phe
                350                 355                 360

cga gag tgg ctc tac aat gct ctt tgg ttc cac aag cac agc ctg tgg 2460
Arg Glu Trp Leu Tyr Asn Ala Leu Trp Phe His Lys His Ser Leu Trp
            365                 370                 375

atg acc tat gaa tct gtg gtt act ggg ttc ttc ccc ttc ttt ctg gtg 2508
Met Thr Tyr Glu Ser Val Val Thr Gly Phe Phe Pro Phe Phe Leu Val
        380                 385                 390

gct aca gtc atc cac ctg ttc tac aga ggc cgg ttg tgg aac atc tta 2556
Ala Thr Val Ile His Leu Phe Tyr Arg Gly Arg Leu Trp Asn Ile Leu
    395                 400                 405

ctc ttc ttg ttg acg gtc cag ctg gtg ggt atg gtg aag gcc acc tac 2604
Leu Phe Leu Leu Thr Val Gln Leu Val Gly Met Val Lys Ala Thr Tyr
410                 415                 420                 425

gcc tgc ttc ctg cgg ggc agc ctt gtc atg atc ttc atg tca ctc tac 2652
Ala Cys Phe Leu Arg Gly Ser Leu Val Met Ile Phe Met Ser Leu Tyr
                430                 435                 440

tca ctg ctc tac atg tca agc ctg ctc cct gct aag atc ttt gcc ctg 2700
Ser Leu Leu Tyr Met Ser Ser Leu Leu Pro Ala Lys Ile Phe Ala Leu
            445                 450                 455

ctg act att aac aaa gca gga tgg ggc aca tca ggc agg aaa aag atg 2748
Leu Thr Ile Asn Lys Ala Gly Trp Gly Thr Ser Gly Arg Lys Lys Met
        460                 465                 470

gta gta aac ctt att gga gct gtg cct gtt act gtg tgg aca gcc att 2796
Val Val Asn Leu Ile Gly Ala Val Pro Val Thr Val Trp Thr Ala Ile
    475                 480                 485

ctg ctt ggt gga gtg gtt tac acc att tac tgc gag gtt caa gaa cct 2844
Leu Leu Gly Gly Val Val Tyr Thr Ile Tyr Cys Glu Val Gln Glu Pro
490                 495                 500                 505

ttt acc gcg act gag aag gct ctt ctt atc gca ggc acc att gtc tat 2892
Phe Thr Ala Thr Glu Lys Ala Leu Leu Ile Ala Gly Thr Ile Val Tyr
                510                 515                 520

gcc tct tac tgg ctt ata ctc ctg gtg ctc tac ctg gcc ata gtg gcc 2940
Ala Ser Tyr Trp Leu Ile Leu Leu Val Leu Tyr Leu Ala Ile Val Ala
            525                 530                 535

aaa cgt tgt aac aaa aga gaa gaa cag ttc cac ctt tcc tat gcc gaa 2988
Lys Arg Cys Asn Lys Arg Glu Glu Gln Phe His Leu Ser Tyr Ala Glu
        540                 545                 550

gcc tag 2994
Ala

<210> 50

<211> 554

<212> PRT

<213> Danio rerio

<220>

<221> misc_feature

<222> (1425)..(1425)

<223> 1425 = any

<220>

<221> misc_feature

<222> (1481)..(1481)

<223> 1481= any

<220>

<221> misc_feature

<222> (1632)..(1632)

<223> 1632 = any

<220>

<221> misc_feature

<222> (1834)..(1834)

<223> 1834 = any

<220>

<221> misc_feature

<222> (2295)..(2295)

<223> 2295 = any

<400> 50

Met Pro Ser Arg Phe Gly Thr Ala Val Arg Ile Phe Ile Thr Thr Leu
1               5                   10                  15

Phe Ala Ala Val Val Leu Phe Ala Ile Leu Leu Ala Tyr Val Thr Gly
            20                  25                  30

Tyr Gln Phe Ile His Thr Glu Gln His His Leu Ser Phe Gly Leu Tyr
        35                  40                  45

Gly Ala Phe Leu Ser Leu His Leu Leu Leu Gln Ser Leu Phe Ala Tyr
    50                  55                  60

Leu Glu His Arg Gln Met Arg Gly Pro Ser Arg Pro Gln His Leu Arg
65                  70                  75                  80

Arg Thr Val Ala Leu Cys Ile Ala Ala Tyr Gln Glu Asp Pro Asp Tyr
                85                  90                  95

Leu Arg Lys Cys Leu Arg Ser Ser Arg Ile Ser Phe Pro Gly Leu Lys
            100                 105                 110

Val Val Leu Val Val Asp Gly Asn Arg Gln Glu Asp Ala Tyr Met Met
        115                 120                 125

Asp Ile Phe Gln Glu Val Met Gly Gly Val Glu Gln Thr Gly Cys Val
    130                 135                 140

Val Trp Lys Gly Asn Tyr His Ser Asn Gly Asp Gly Gly Gly Gly Gly
145                 150                 155                 160

Gly Lys Gly Ser Val His Ala Glu Glu Ala Ala Arg Val Ala Arg Val
                165                 170                 175

Val Arg Ser Cys Arg Tyr Ser Cys Ile Met Gln Glu Trp Gly Gly Lys
            180                 185                 190

Arg Glu Val Met Tyr Thr Ala Phe Lys Ala Leu Gly Asp Thr Val Asp
        195                 200                 205

Tyr Met Gln Val Cys Asp Ser Asp Thr Val Leu Asp Pro Ala Cys Thr
    210                 215                 220

Ile Glu Met Leu Lys Ile Leu Glu Glu Asp Pro Asp Val Gly Gly Val
225                 230                 235                 240

Gly Gly Asp Val Gln Ile Leu Asn Lys Tyr Asp Ser Trp Ile Ser Phe
                245                 250                 255

Leu Ser Ser Val Arg Tyr Trp Met Ala Phe Asn Val Glu Arg Ala Cys
            260                 265                 270

Gln Ser Tyr Phe Gly Cys Val Gln Cys Ile Ser Gly Pro Leu Gly Met
        275                 280                 285

Tyr Arg Asn Ser Leu Leu Gln Gln Phe Leu Glu Pro Trp Tyr His Gln
    290                 295                 300

Thr Phe Leu Gly Ser Lys Cys Ser Phe Gly Asp Asp Arg His Leu Thr
305                 310                 315                 320

Asn Arg Val Leu Ser Phe Gly Phe Lys Thr Lys Phe Thr Ala Arg Ser
                325                 330                 335

Gln Cys Gln Thr Glu Thr Pro Thr Gln Tyr Leu Arg Trp Leu Asn Gln
            340                 345                 350

Gln Thr Arg Trp Ser Lys Ser Tyr Phe Arg Glu Trp Leu Tyr Asn Ala
        355                 360                 365

Leu Trp Phe His Lys His Ser Leu Trp Met Thr Tyr Glu Ser Val Val
    370                 375                 380

Thr Gly Phe Phe Pro Phe Phe Leu Val Ala Thr Val Ile His Leu Phe
385                 390                 395                 400

Tyr Arg Gly Arg Leu Trp Asn Ile Leu Leu Phe Leu Leu Thr Val Gln
                405                 410                 415

Leu Val Gly Met Val Lys Ala Thr Tyr Ala Cys Phe Leu Arg Gly Ser
            420                 425                 430

Leu Val Met Ile Phe Met Ser Leu Tyr Ser Leu Leu Tyr Met Ser Ser
        435                 440                 445

Leu Leu Pro Ala Lys Ile Phe Ala Leu Leu Thr Ile Asn Lys Ala Gly
    450                 455                 460

Trp Gly Thr Ser Gly Arg Lys Lys Met Val Val Asn Leu Ile Gly Ala
465                 470                 475                 480

Val Pro Val Thr Val Trp Thr Ala Ile Leu Leu Gly Gly Val Val Tyr
                485                 490                 495

Thr Ile Tyr Cys Glu Val Gln Glu Pro Phe Thr Ala Thr Glu Lys Ala
            500                 505                 510

Leu Leu Ile Ala Gly Thr Ile Val Tyr Ala Ser Tyr Trp Leu Ile Leu
        515                 520                 525

Leu Val Leu Tyr Leu Ala Ile Val Ala Lys Arg Cys Asn Lys Arg Glu
    530                 535                 540

Glu Gln Phe His Leu Ser Tyr Ala Glu Ala
545                 550

<210>  51

<211>  2919

<212>  DNA

<213>  Pasteurella multocida


<220>

<221>  CDS

<222>  (1)..(2919)

<223>


<300>

<308>  AF036004.2

<309>  1998-04-15

<313>  (1)..(2919)

<400>  51
atg aat aca tta tca caa gca ata aaa gca tat aac agc aat gac tat      48
Met Asn Thr Leu Ser Gln Ala Ile Lys Ala Tyr Asn Ser Asn Asp Tyr
1               5                   10                  15

caa tta gca ctc aaa tta ttt gaa aag tcg gcg gaa atc tat gga cgg      96
Gln Leu Ala Leu Lys Leu Phe Glu Lys Ser Ala Glu Ile Tyr Gly Arg
            20                  25                  30

aaa att gtt gaa ttt caa att acc aaa tgc aaa gaa aaa ctc tca gca      144
Lys Ile Val Glu Phe Gln Ile Thr Lys Cys Lys Glu Lys Leu Ser Ala
        35                  40                  45

cat cct tct gtt aat tca gca cat ctt tct gta aat aaa gaa gaa aaa      192
His Pro Ser Val Asn Ser Ala His Leu Ser Val Asn Lys Glu Glu Lys
    50                  55                  60

gtc aat gtt tgc gat agt ccg tta gat att gca aca caa ctg tta ctt      240
Val Asn Val Cys Asp Ser Pro Leu Asp Ile Ala Thr Gln Leu Leu Leu
65                  70                  75                  80

tcc aac gta aaa aaa tta gta ctt tct gac tcg gaa aaa aac acg tta      288
Ser Asn Val Lys Lys Leu Val Leu Ser Asp Ser Glu Lys Asn Thr Leu
                85                  90                  95

aaa aat aaa tgg aaa ttg ctc act gag aag aaa tct gaa aat gcg gag      336
Lys Asn Lys Trp Lys Leu Leu Thr Glu Lys Lys Ser Glu Asn Ala Glu
            100                 105                 110

gta aga gcg gtc gcc ctt gta cca aaa gat ttt ccc aaa gat ctg gtt      384
Val Arg Ala Val Ala Leu Val Pro Lys Asp Phe Pro Lys Asp Leu Val
        115                 120                 125

tta gcg cct tta cct gat cat gtt aat gat ttt aca tgg tac aaa aag      432
Leu Ala Pro Leu Pro Asp His Val Asn Asp Phe Thr Trp Tyr Lys Lys
    130                 135                 140

cga aag aaa aga ctt ggc ata aaa cct gaa cat caa cat gtt ggt ctt      480
Arg Lys Lys Arg Leu Gly Ile Lys Pro Glu His Gln His Val Gly Leu
145                 150                 155                 160

tct att atc gtt aca aca ttc aat cga cca gca att tta tcg att aca      528
Ser Ile Ile Val Thr Thr Phe Asn Arg Pro Ala Ile Leu Ser Ile Thr
                165                 170                 175

tta gcc tgt tta gta aac caa aaa aca cat tac ccg ttt gaa gtt atc      576
Leu Ala Cys Leu Val Asn Gln Lys Thr His Tyr Pro Phe Glu Val Ile
            180                 185                 190

gtg aca gat gat ggt agt cag gaa gat cta tca ccg atc att cgc caa      624
Val Thr Asp Asp Gly Ser Gln Glu Asp Leu Ser Pro Ile Ile Arg Gln
        195                 200                 205

tat gaa aat aaa ttg gat att cgc tac gtc aga caa aaa gat aac ggt      672
Tyr Glu Asn Lys Leu Asp Ile Arg Tyr Val Arg Gln Lys Asp Asn Gly
    210                 215                 220

ttt caa gcc agt gcc gct cgg aat atg gga tta cgc tta gca aaa tat      720
Phe Gln Ala Ser Ala Ala Arg Asn Met Gly Leu Arg Leu Ala Lys Tyr
225                 230                 235                 240

gac ttt att ggc tta ctc gac tgt gat atg gcg cca aat cca tta tgg 768
Asp Phe Ile Gly Leu Leu Asp Cys Asp Met Ala Pro Asn Pro Leu Trp
                245                 250                 255

gtt cat tct tat gtt gca gag cta tta gaa gat gat gat tta aca atc 816
Val His Ser Tyr Val Ala Glu Leu Leu Glu Asp Asp Asp Leu Thr Ile
            260                 265                 270

att ggt cca aga aaa tac atc gat aca caa cat att gac cca aaa gac 864
Ile Gly Pro Arg Lys Tyr Ile Asp Thr Gln His Ile Asp Pro Lys Asp
        275                 280                 285

ttc tta aat aac gcg agt ttg ctt gaa tca tta cca gaa gtg aaa acc 912
Phe Leu Asn Asn Ala Ser Leu Leu Glu Ser Leu Pro Glu Val Lys Thr
    290                 295                 300

aat aat agt gtt gcc gca aaa ggg gaa gga aca gtt tct ctg gat tgg 960
Asn Asn Ser Val Ala Ala Lys Gly Glu Gly Thr Val Ser Leu Asp Trp
305                 310                 315                 320

cgc tta gaa caa ttc gaa aaa aca gaa aat ctc cgc tta tcc gat tcg 1008
Arg Leu Glu Gln Phe Glu Lys Thr Glu Asn Leu Arg Leu Ser Asp Ser
                325                 330                 335

cct ttc cgt ttt ttt gcg gcg ggt aat gtt gct ttc gct aaa aaa tgg 1056
Pro Phe Arg Phe Phe Ala Ala Gly Asn Val Ala Phe Ala Lys Lys Trp
            340                 345                 350

cta aat aaa tcc ggt ttc ttt gat gag gaa ttt aat cac tgg ggt gga 1104
Leu Asn Lys Ser Gly Phe Phe Asp Glu Glu Phe Asn His Trp Gly Gly
        355                 360                 365

gaa gat gtg gaa ttt gga tat cgc tta ttc cgt tac ggt agt ttc ttt 1152
Glu Asp Val Glu Phe Gly Tyr Arg Leu Phe Arg Tyr Gly Ser Phe Phe
    370                 375                 380

aaa act att gat ggc att atg gcc tac cat caa gag cca cca ggt aaa 1200
Lys Thr Ile Asp Gly Ile Met Ala Tyr His Gln Glu Pro Pro Gly Lys
385                 390                 395                 400

gaa aat gaa acc gat cgt gaa gcg gga aaa aat att acg ctc gat att 1248
Glu Asn Glu Thr Asp Arg Glu Ala Gly Lys Asn Ile Thr Leu Asp Ile
                405                 410                 415

atg aga gaa aag gtc cct tat atc tat aga aaa ctt tta cca ata gaa 1296
Met Arg Glu Lys Val Pro Tyr Ile Tyr Arg Lys Leu Leu Pro Ile Glu
            420                 425                 430

gat tcg cat atc aat aga gta cct tta gtt tca att tat atc cca gct 1344
Asp Ser His Ile Asn Arg Val Pro Leu Val Ser Ile Tyr Ile Pro Ala
        435                 440                 445

tat aac tgt gca aac tat att caa cgt tgc gta gat agt gca ctg aat 1392
Tyr Asn Cys Ala Asn Tyr Ile Gln Arg Cys Val Asp Ser Ala Leu Asn
    450                 455                 460

cag act gtt gtt gat ctc gag gtt tgt att tgt aac gat ggt tca aca 1440
Gln Thr Val Val Asp Leu Glu Val Cys Ile Cys Asn Asp Gly Ser Thr
465                 470                 475                 480

gat aat acc tta gaa gtg atc aat aag ctt tat ggt aat aat cct agg 1488
Asp Asn Thr Leu Glu Val Ile Asn Lys Leu Tyr Gly Asn Asn Pro Arg
                485                 490                 495

gta cgc atc atg tct aaa cca aat ggc gga ata gcc tca gca tca aat 1536
Val Arg Ile Met Ser Lys Pro Asn Gly Gly Ile Ala Ser Ala Ser Asn
            500                 505                 510

gca gcc gtt tct ttt gct aaa ggt tat tac att ggg cag tta gat tca     1584
Ala Ala Val Ser Phe Ala Lys Gly Tyr Tyr Ile Gly Gln Leu Asp Ser
        515                 520                 525

gat gat tat ctt gag cct gat gca gtt gaa ctg tgt tta aaa gaa ttt     1632
Asp Asp Tyr Leu Glu Pro Asp Ala Val Glu Leu Cys Leu Lys Glu Phe
    530                 535                 540

tta aaa gat aaa acg cta gct tgt gtt tat acc act aat aga aac gtc     1680
Leu Lys Asp Lys Thr Leu Ala Cys Val Tyr Thr Thr Asn Arg Asn Val
545                 550                 555                 560

aat ccg gat ggt agc tta atc gct aat ggt tac aat tgg cca gaa ttt     1728
Asn Pro Asp Gly Ser Leu Ile Ala Asn Gly Tyr Asn Trp Pro Glu Phe
                565                 570                 575

tca cga gaa aaa ctc aca acg gct atg att gct cac cac ttt aga atg     1776
Ser Arg Glu Lys Leu Thr Thr Ala Met Ile Ala His His Phe Arg Met
            580                 585                 590

ttc acg att aga gct tgg cat tta act gat gga ttc aat gaa aaa att     1824
Phe Thr Ile Arg Ala Trp His Leu Thr Asp Gly Phe Asn Glu Lys Ile
        595                 600                 605

gaa aat gcc gta gac tat gac atg ttc ctc aaa ctc agt gaa gtt gga     1872
Glu Asn Ala Val Asp Tyr Asp Met Phe Leu Lys Leu Ser Glu Val Gly
    610                 615                 620

aaa ttt aaa cat ctt aat aaa atc tgc tat aac cgt gta tta cat ggt     1920
Lys Phe Lys His Leu Asn Lys Ile Cys Tyr Asn Arg Val Leu His Gly
625                 630                 635                 640

gat aac aca tca att aag aaa ctt ggc att caa aag aaa aac cat ttt     1968
Asp Asn Thr Ser Ile Lys Lys Leu Gly Ile Gln Lys Lys Asn His Phe
                645                 650                 655

gtt gta gtc aat cag tca tta aat aga caa ggc ata act tat tat aat     2016
Val Val Val Asn Gln Ser Leu Asn Arg Gln Gly Ile Thr Tyr Tyr Asn
            660                 665                 670

tat gac gaa ttt gat gat tta gat gaa agt aga aag tat att ttc aat     2064
Tyr Asp Glu Phe Asp Asp Leu Asp Glu Ser Arg Lys Tyr Ile Phe Asn
        675                 680                 685

aaa acc gct gaa tat caa gaa gag att gat atc tta aaa gat att aaa     2112
Lys Thr Ala Glu Tyr Gln Glu Glu Ile Asp Ile Leu Lys Asp Ile Lys
    690                 695                 700

atc atc cag aat aaa gat gcc aaa atc gca gtc agt att ttt tat ccc     2160
Ile Ile Gln Asn Lys Asp Ala Lys Ile Ala Val Ser Ile Phe Tyr Pro
705                 710                 715                 720

aat aca tta aac ggc tta gtg aaa aaa cta aac aat att att gaa tat     2208
Asn Thr Leu Asn Gly Leu Val Lys Lys Leu Asn Asn Ile Ile Glu Tyr
                725                 730                 735

aat aaa aat ata ttc gtt att gtt cta cat gtt gat aag aat cat ctt     2256
Asn Lys Asn Ile Phe Val Ile Val Leu His Val Asp Lys Asn His Leu
            740                 745                 750

aca cca gat atc aaa aaa gaa ata cta gcc ttc tat cat aaa cat caa     2304
Thr Pro Asp Ile Lys Lys Glu Ile Leu Ala Phe Tyr His Lys His Gln
        755                 760                 765

gtg aat att tta cta aat aat gat atc tca tat tac acg agt aat aga     2352
Val Asn Ile Leu Leu Asn Asn Asp Ile Ser Tyr Tyr Thr Ser Asn Arg
    770                 775                 780

tta ata aaa act gag gcg cat tta agt aat att aat aaa tta agt cag          2400
Leu Ile Lys Thr Glu Ala His Leu Ser Asn Ile Asn Lys Leu Ser Gln
785                 790                 795                 800

tta aat cta aat tgt gaa tac atc att ttt gat aat cat gac agc cta          2448
Leu Asn Leu Asn Cys Glu Tyr Ile Ile Phe Asp Asn His Asp Ser Leu
                805                 810                 815

ttc gtt aaa aat gac agc tat gct tat atg aaa aaa tat gat gtc ggc          2496
Phe Val Lys Asn Asp Ser Tyr Ala Tyr Met Lys Lys Tyr Asp Val Gly
            820                 825                 830

atg aat ttc tca gca tta aca cat gat tgg atc gag aaa atc aat gcg          2544
Met Asn Phe Ser Ala Leu Thr His Asp Trp Ile Glu Lys Ile Asn Ala
        835                 840                 845

cat cca cca ttt aaa aag ctc att aaa act tat ttt aat gac aat gac          2592
His Pro Pro Phe Lys Lys Leu Ile Lys Thr Tyr Phe Asn Asp Asn Asp
    850                 855                 860

tta aaa agt atg aat gtg aaa ggg gca tca caa ggt atg ttt atg acg          2640
Leu Lys Ser Met Asn Val Lys Gly Ala Ser Gln Gly Met Phe Met Thr
865                 870                 875                 880

tat gcg cta gcg cat gag ctt ctg acg att att aaa gaa gtc atc aca          2688
Tyr Ala Leu Ala His Glu Leu Leu Thr Ile Ile Lys Glu Val Ile Thr
                885                 890                 895

tct tgc cag tca att gat agt gtg cca gaa tat aac act gag gat att          2736
Ser Cys Gln Ser Ile Asp Ser Val Pro Glu Tyr Asn Thr Glu Asp Ile
            900                 905                 910

tgg ttc caa ttt gca ctt tta atc tta gaa aag aaa acc ggc cat gta          2784
Trp Phe Gln Phe Ala Leu Leu Ile Leu Glu Lys Lys Thr Gly His Val
        915                 920                 925

ttt aat aaa aca tcg acc ctg act tat atg cct tgg gaa cga aaa tta          2832
Phe Asn Lys Thr Ser Thr Leu Thr Tyr Met Pro Trp Glu Arg Lys Leu
    930                 935                 940

caa tgg aca aat gaa caa att gaa agt gca aaa aga gga gaa aat ata          2880
Gln Trp Thr Asn Glu Gln Ile Glu Ser Ala Lys Arg Gly Glu Asn Ile
945                 950                 955                 960

cct gtt aac aag ttc att att aat agt ata act cta taa                      2919
Pro Val Asn Lys Phe Ile Ile Asn Ser Ile Thr Leu
                965                 970

<210>  52

<211>  972

<212>  PRT

<213>  Pasteurella multocida

<400>  52

Met Asn Thr Leu Ser Gln Ala Ile Lys Ala Tyr Asn Ser Asn Asp Tyr
1               5                   10                  15

Gln Leu Ala Leu Lys Leu Phe Glu Lys Ser Ala Glu Ile Tyr Gly Arg
            20                  25                  30

Lys Ile Val Glu Phe Gln Ile Thr Lys Cys Lys Glu Lys Leu Ser Ala
        35                  40                  45

His Pro Ser Val Asn Ser Ala His Leu Ser Val Asn Lys Glu Glu Lys
    50                  55                  60

Val Asn Val Cys Asp Ser Pro Leu Asp Ile Ala Thr Gln Leu Leu Leu
65                  70                  75                  80

Ser Asn Val Lys Lys Leu Val Leu Ser Asp Ser Glu Lys Asn Thr Leu
                85                  90                  95

Lys Asn Lys Trp Lys Leu Leu Thr Glu Lys Lys Ser Glu Asn Ala Glu
            100                 105                 110

Val Arg Ala Val Ala Leu Val Pro Lys Asp Phe Pro Lys Asp Leu Val
        115                 120                 125

Leu Ala Pro Leu Pro Asp His Val Asn Asp Phe Thr Trp Tyr Lys Lys
    130                 135                 140

Arg Lys Lys Arg Leu Gly Ile Lys Pro Glu His Gln His Val Gly Leu
145                 150                 155                 160

Ser Ile Ile Val Thr Thr Phe Asn Arg Pro Ala Ile Leu Ser Ile Thr
                165                 170                 175

Leu Ala Cys Leu Val Asn Gln Lys Thr His Tyr Pro Phe Glu Val Ile
            180                 185                 190

Val Thr Asp Asp Gly Ser Gln Glu Asp Leu Ser Pro Ile Ile Arg Gln
        195                 200                 205

Tyr Glu Asn Lys Leu Asp Ile Arg Tyr Val Arg Gln Lys Asp Asn Gly
    210                 215                 220

Phe Gln Ala Ser Ala Ala Arg Asn Met Gly Leu Arg Leu Ala Lys Tyr
225                 230                 235                 240

Asp Phe Ile Gly Leu Leu Asp Cys Asp Met Ala Pro Asn Pro Leu Trp
                245                 250                 255

Val His Ser Tyr Val Ala Glu Leu Leu Glu Asp Asp Asp Leu Thr Ile
            260                 265                 270

Ile Gly Pro Arg Lys Tyr Ile Asp Thr Gln His Ile Asp Pro Lys Asp
        275                 280                 285

Phe Leu Asn Asn Ala Ser Leu Leu Glu Ser Leu Pro Glu Val Lys Thr
    290                 295                 300

Asn Asn Ser Val Ala Ala Lys Gly Glu Gly Thr Val Ser Leu Asp Trp
305                 310                 315                 320

Arg Leu Glu Gln Phe Glu Lys Thr Glu Asn Leu Arg Leu Ser Asp Ser
                325                 330                 335

Pro Phe Arg Phe Phe Ala Ala Gly Asn Val Ala Phe Ala Lys Lys Trp
            340                 345                 350

Leu Asn Lys Ser Gly Phe Phe Asp Glu Glu Phe Asn His Trp Gly Gly
        355                 360                 365

Glu Asp Val Glu Phe Gly Tyr Arg Leu Phe Arg Tyr Gly Ser Phe Phe
    370                 375                 380

Lys Thr Ile Asp Gly Ile Met Ala Tyr His Gln Glu Pro Pro Gly Lys
385                 390                 395                 400

Glu Asn Glu Thr Asp Arg Glu Ala Gly Lys Asn Ile Thr Leu Asp Ile
                405                 410                 415

Met Arg Glu Lys Val Pro Tyr Ile Tyr Arg Lys Leu Leu Pro Ile Glu
            420                 425                 430

Asp Ser His Ile Asn Arg Val Pro Leu Val Ser Ile Tyr Ile Pro Ala
        435                 440                 445

Tyr Asn Cys Ala Asn Tyr Ile Gln Arg Cys Val Asp Ser Ala Leu Asn
    450                 455                 460

Gln Thr Val Val Asp Leu Glu Val Cys Ile Cys Asn Asp Gly Ser Thr
465                 470                 475                 480

Asp Asn Thr Leu Glu Val Ile Asn Lys Leu Tyr Gly Asn Asn Pro Arg
                485                 490                 495

Val Arg Ile Met Ser Lys Pro Asn Gly Gly Ile Ala Ser Ala Ser Asn
            500                 505                 510

Ala Ala Val Ser Phe Ala Lys Gly Tyr Tyr Ile Gly Gln Leu Asp Ser
        515                 520                 525

Asp Asp Tyr Leu Glu Pro Asp Ala Val Glu Leu Cys Leu Lys Glu Phe
    530                 535                 540

Leu Lys Asp Lys Thr Leu Ala Cys Val Tyr Thr Thr Asn Arg Asn Val
545                 550                 555                 560

Asn Pro Asp Gly Ser Leu Ile Ala Asn Gly Tyr Asn Trp Pro Glu Phe
                565                 570                 575

Ser Arg Glu Lys Leu Thr Thr Ala Met Ile Ala His His Phe Arg Met
            580                 585                 590

Phe Thr Ile Arg Ala Trp His Leu Thr Asp Gly Phe Asn Glu Lys Ile
        595                 600                 605

Glu Asn Ala Val Asp Tyr Asp Met Phe Leu Lys Leu Ser Glu Val Gly
    610                 615                 620

Lys Phe Lys His Leu Asn Lys Ile Cys Tyr Asn Arg Val Leu His Gly
625                 630                 635                 640

Asp Asn Thr Ser Ile Lys Lys Leu Gly Ile Gln Lys Lys Asn His Phe
                645                 650                 655

Val Val Val Asn Gln Ser Leu Asn Arg Gln Gly Ile Thr Tyr Tyr Asn
            660                 665                 670

Tyr Asp Glu Phe Asp Asp Leu Asp Glu Ser Arg Lys Tyr Ile Phe Asn
        675                 680                 685

Lys Thr Ala Glu Tyr Gln Glu Glu Ile Asp Ile Leu Lys Asp Ile Lys
    690                 695                 700

Ile Ile Gln Asn Lys Asp Ala Lys Ile Ala Val Ser Ile Phe Tyr Pro
705                 710                 715                 720

Asn Thr Leu Asn Gly Leu Val Lys Lys Leu Asn Asn Ile Ile Glu Tyr
                725                 730                 735

Asn Lys Asn Ile Phe Val Ile Val Leu His Val Asp Lys Asn His Leu
            740                 745                 750

Thr Pro Asp Ile Lys Lys Glu Ile Leu Ala Phe Tyr His Lys His Gln
        755                 760                 765

Val Asn Ile Leu Leu Asn Asn Asp Ile Ser Tyr Tyr Thr Ser Asn Arg
    770                 775                 780

Leu Ile Lys Thr Glu Ala His Leu Ser Asn Ile Asn Lys Leu Ser Gln
785                 790                 795                 800

Leu Asn Leu Asn Cys Glu Tyr Ile Ile Phe Asp Asn His Asp Ser Leu
                805                 810                 815

Phe Val Lys Asn Asp Ser Tyr Ala Tyr Met Lys Lys Tyr Asp Val Gly
            820                 825                 830

Met Asn Phe Ser Ala Leu Thr His Asp Trp Ile Glu Lys Ile Asn Ala
        835                 840                 845

His Pro Pro Phe Lys Lys Leu Ile Lys Thr Tyr Phe Asn Asp Asn Asp
    850                 855                 860

Leu Lys Ser Met Asn Val Lys Gly Ala Ser Gln Gly Met Phe Met Thr
865                 870                 875                 880

Tyr Ala Leu Ala His Glu Leu Leu Thr Ile Ile Lys Glu Val Ile Thr
                885                 890                 895

Ser Cys Gln Ser Ile Asp Ser Val Pro Glu Tyr Asn Thr Glu Asp Ile
            900                 905                 910

Trp Phe Gln Phe Ala Leu Leu Ile Leu Glu Lys Lys Thr Gly His Val
        915                 920                 925

Phe Asn Lys Thr Ser Thr Leu Thr Tyr Met Pro Trp Glu Arg Lys Leu
    930                 935                 940

Gln Trp Thr Asn Glu Gln Ile Glu Ser Ala Lys Arg Gly Glu Asn Ile
945                 950                 955                 960

Pro Val Asn Lys Phe Ile Ile Asn Ser Ile Thr Leu
                965                 970

<210>  53

<211>  1260

<212>  DNA

<213>  Streptococcus pyogenes


<220>

<221>  CDS

<222>  (1)..(1260)

<223>


<300>

<308>  L21187.1

<309>  1993-07-23

<313>  (79)..(1338)


<400>  53
gtg cct att ttt aaa aaa act tta att gtt tta tcc ttt att ttt ttg        48
Met Pro Ile Phe Lys Lys Thr Leu Ile Val Leu Ser Phe Ile Phe Leu
1               5                   10                  15

ata tct atc ttg att tat cta aat atg tat cta ttt gga aca tca act 96
Ile Ser Ile Leu Ile Tyr Leu Asn Met Tyr Leu Phe Gly Thr Ser Thr
            20                  25                  30

gta gga att tat gga gta ata tta ata acc tat cta gtt atc aaa ctt 144
Val Gly Ile Tyr Gly Val Ile Leu Ile Thr Tyr Leu Val Ile Lys Leu
        35                  40                  45

gga tta tct ttc ctt tat gag cca ttt aaa gga aag cca cat gac tat 192
Gly Leu Ser Phe Leu Tyr Glu Pro Phe Lys Gly Lys Pro His Asp Tyr
    50                  55                  60

aaa gtt gct gct gta att cct tct tat aat gaa gat gcc gag tca tta 240
Lys Val Ala Ala Val Ile Pro Ser Tyr Asn Glu Asp Ala Glu Ser Leu
65                  70                  75                  80

tta gaa aca ctt aaa agt gtg tta gca cag acc tat ccg tta tca gaa 288
Leu Glu Thr Leu Lys Ser Met Leu Ala Gln Thr Tyr Pro Leu Ser Glu
                85                  90                  95

att tat att gtt gat gat ggg agt tca aac aca gat gca ata caa tta 336
Ile Tyr Ile Val Asp Asp Gly Ser Ser Asn Thr Asp Ala Ile Gln Leu
            100                 105                 110

att gaa gag tat gta aat aga gaa gtg gat att tgt cga aac gtt atc 384
Ile Glu Glu Tyr Val Asn Arg Glu Met Asp Ile Cys Arg Asn Val Ile
        115                 120                 125

gtt cac cgt tcc ctt gtc aat aaa gga aaa cgc cat gct caa gcg tgg 432
Val His Arg Ser Leu Val Asn Lys Gly Lys Arg His Ala Gln Ala Trp
    130                 135                 140

gca ttt gaa aga tct gac gct gac gtt ttt tta acc gta gat tca gat 480
Ala Phe Glu Arg Ser Asp Ala Asp Val Phe Leu Thr Val Asp Ser Asp
145                 150                 155                 160

act tat atc tat cca aat gcc tta gaa gaa ctc cta aaa agc ttc aat 528
Thr Tyr Ile Tyr Pro Asn Ala Leu Glu Glu Leu Leu Lys Ser Phe Asn
                165                 170                 175

gat gag aca gtt tat gct gca aca gga cat ttg aat gct aga aac aga 576
Asp Glu Thr Val Tyr Ala Ala Thr Gly His Leu Asn Ala Arg Asn Arg
            180                 185                 190

caa act aat cta tta acg cga ctt aca gat atc cgt tac gat aat gcc 624
Gln Thr Asn Leu Leu Thr Arg Leu Thr Asp Ile Arg Tyr Asp Asn Ala
        195                 200                 205

ttt ggg gtg gag cgt gct gct caa tca tta aca ggt aat att tta gtt 672
Phe Gly Met Glu Arg Ala Ala Gln Ser Leu Thr Gly Asn Ile Leu Val
    210                 215                 220

tgc tca gga cca ttg agt att tat cga cgt gaa gtg att att cct aac 720
Cys Ser Gly Pro Leu Ser Ile Tyr Arg Arg Glu Met Ile Ile Pro Asn
225                 230                 235                 240

tta gag cgc tat aaa aat caa aca ttc cta ggt tta cct gtt agc att 768
Leu Glu Arg Tyr Lys Asn Gln Thr Phe Leu Gly Leu Pro Val Ser Ile
                245                 250                 255

ggg gat gat cga tgt tta aca aat tat gct att gat tta gga cgc act 816
Gly Asp Asp Arg Cys Leu Thr Asn Tyr Ala Ile Asp Leu Gly Arg Thr
            260                 265                 270

gtc tac caa tca aca gct aga tgt gat act gat gta cct ttc caa tta 864
Val Tyr Gln Ser Thr Ala Arg Cys Asp Thr Asp Val Pro Phe Gln Leu
        275                 280                 285

aaa agt tat tta aag caa caa aat cga tgg aat aaa tct ttt ttt aga 912
Lys Ser Tyr Leu Lys Gln Gln Asn Arg Trp Asn Lys Ser Phe Phe Arg
    290                 295                 300

gaa tct att att tct gtt aaa aaa att ctt tct aat ccc atc gtt gcc 960
Glu Ser Ile Ile Ser Val Lys Lys Ile Leu Ser Asn Pro Ile Val Ala
305                 310                 315                 320

tta tgg act att ttc gaa gtc gtt atg ttt atg atg ttg att gtc gca 1008
Leu Trp Thr Ile Phe Glu Val Val Met Phe Met Met Leu Ile Val Ala
                325                 330                 335

att ggg aat ctt ttg ttt aat caa gct att caa tta gac ctt att aaa 1056
Ile Gly Asn Leu Leu Phe Asn Gln Ala Ile Gln Leu Asp Leu Ile Lys
            340                 345                 350

ctt ttt gcc ttt tta tcc atc atc ttt atc gtt gct tta tgt cgt aat 1104
Leu Phe Ala Phe Leu Ser Ile Ile Phe Ile Val Ala Leu Cys Arg Asn
        355                 360                 365

gtt cat tat atg gtc aaa cat cct gct agt ttt ttg tta tct cct ctg 1152
Val His Tyr Met Val Lys His Pro Ala Ser Phe Leu Leu Ser Pro Leu
    370                 375                 380

tat gga ata tta cac ttg ttt gtc tta cag ccc cta aaa ctt tat tct 1200
Tyr Gly Ile Leu His Leu Phe Val Leu Gln Pro Leu Lys Leu Tyr Ser
385                 390                 395                 400

tta tgc acc att aaa aat acg gaa tgg gga aca cgt aaa aag gtc act 1248
Leu Cys Thr Ile Lys Asn Thr Glu Trp Gly Thr Arg Lys Lys Val Thr
                405                 410                 415

att ttt aaa taa 1260
Ile Phe Lys

<210> 54

<211> 419

<212> PRT

<213> Streptococcus pyogenes

<400> 54

Met Pro Ile Phe Lys Lys Thr Leu Ile Val Leu Ser Phe Ile Phe Leu
1               5                   10                  15

Ile Ser Ile Leu Ile Tyr Leu Asn Met Tyr Leu Phe Gly Thr Ser Thr
            20                  25                  30

Val Gly Ile Tyr Gly Val Ile Leu Ile Thr Tyr Leu Val Ile Lys Leu
        35                  40                  45

Gly Leu Ser Phe Leu Tyr Glu Pro Phe Lys Gly Lys Pro His Asp Tyr
    50                  55                  60

Lys Val Ala Ala Val Ile Pro Ser Tyr Asn Glu Asp Ala Glu Ser Leu
65                  70                  75                  80

Leu Glu Thr Leu Lys Ser Met Leu Ala Gln Thr Tyr Pro Leu Ser Glu
                85                  90                  95

Ile Tyr Ile Val Asp Asp Gly Ser Ser Asn Thr Asp Ala Ile Gln Leu
            100                 105                 110

Ile Glu Glu Tyr Val Asn Arg Glu Met Asp Ile Cys Arg Asn Val Ile
        115                 120                 125

Val His Arg Ser Leu Val Asn Lys Gly Lys Arg His Ala Gln Ala Trp
    130                 135                 140

Ala Phe Glu Arg Ser Asp Ala Asp Val Phe Leu Thr Val Asp Ser Asp
145                 150                 155                 160

Thr Tyr Ile Tyr Pro Asn Ala Leu Glu Glu Leu Leu Lys Ser Phe Asn
                165                 170                 175

Asp Glu Thr Val Tyr Ala Ala Thr Gly His Leu Asn Ala Arg Asn Arg
            180                 185                 190

Gln Thr Asn Leu Leu Thr Arg Leu Thr Asp Ile Arg Tyr Asp Asn Ala
        195                 200                 205

Phe Gly Met Glu Arg Ala Ala Gln Ser Leu Thr Gly Asn Ile Leu Val
    210                 215                 220

Cys Ser Gly Pro Leu Ser Ile Tyr Arg Arg Glu Met Ile Ile Pro Asn
225                 230                 235                 240

Leu Glu Arg Tyr Lys Asn Gln Thr Phe Leu Gly Leu Pro Val Ser Ile
                245                 250                 255

Gly Asp Asp Arg Cys Leu Thr Asn Tyr Ala Ile Asp Leu Gly Arg Thr
            260                 265                 270

Val Tyr Gln Ser Thr Ala Arg Cys Asp Thr Asp Val Pro Phe Gln Leu
        275                 280                 285

Lys Ser Tyr Leu Lys Gln Gln Asn Arg Trp Asn Lys Ser Phe Phe Arg
    290                 295                 300

Glu Ser Ile Ile Ser Val Lys Lys Ile Leu Ser Asn Pro Ile Val Ala
305                 310                 315                 320

Leu Trp Thr Ile Phe Glu Val Val Met Phe Met Met Leu Ile Val Ala
                325                 330                 335

Ile Gly Asn Leu Leu Phe Asn Gln Ala Ile Gln Leu Asp Leu Ile Lys
            340                 345                 350

Leu Phe Ala Phe Leu Ser Ile Ile Phe Ile Val Ala Leu Cys Arg Asn
        355                 360                 365

Val His Tyr Met Val Lys His Pro Ala Ser Phe Leu Leu Ser Pro Leu
    370                 375                 380

Tyr Gly Ile Leu His Leu Phe Val Leu Gln Pro Leu Lys Leu Tyr Ser
385                 390                 395                 400

Leu Cys Thr Ile Lys Asn Thr Glu Trp Gly Thr Arg Lys Lys Val Thr
                405                 410                 415

Ile Phe Lys

<210>  55

<211>  1254

<212>  DNA

<213>  Streptococcus equi

<220>

<221>  CDS

<222>  (1)..(1254)

<223>

<300>

<308>  AF347022.1

<309>  2002-11-13

<313>  (531)..(1784)

<400>  55
atg aga aca tta aaa aac ctc ata act gtt gtg gcc ttt agt att ttt      48
Met Arg Thr Leu Lys Asn Leu Ile Thr Val Val Ala Phe Ser Ile Phe
1               5                   10                  15

tgg gta ctg ttg att tac gtc aat gtt tat ctc ttt ggt gct aaa gga      96
Trp Val Leu Leu Ile Tyr Val Asn Val Tyr Leu Phe Gly Ala Lys Gly
            20                  25                  30

agc ttg tca att tat ggc ttt ttg ctg ata gct tac cta tta gtc aaa      144
Ser Leu Ser Ile Tyr Gly Phe Leu Leu Ile Ala Tyr Leu Leu Val Lys
        35                  40                  45

atg tcc tta tcc ttt ttt tac aag cca ttt aag gga agg gct ggg caa      192
Met Ser Leu Ser Phe Phe Tyr Lys Pro Phe Lys Gly Arg Ala Gly Gln
    50                  55                  60

tat aag gtt gca gcc att att ccc tct tat aac gaa gat gct gag tca 240
Tyr Lys Val Ala Ala Ile Ile Pro Ser Tyr Asn Glu Asp Ala Glu Ser
65                  70                  75                  80

ttg cta gag acc tta aaa agt gtt cag cag caa acc tat ccc cta gca 288
Leu Leu Glu Thr Leu Lys Ser Val Gln Gln Gln Thr Tyr Pro Leu Ala
                85                  90                  95

gaa att tat gtt gtt gac gat gga agt gct gat gag aca ggt att aag 336
Glu Ile Tyr Val Val Asp Asp Gly Ser Ala Asp Glu Thr Gly Ile Lys
            100                 105                 110

cgc att gaa gac tat gtg cgt gac act ggt gac cta tca agc aat gtc 384
Arg Ile Glu Asp Tyr Val Arg Asp Thr Gly Asp Leu Ser Ser Asn Val
        115                 120                 125

att gtt cac cgg tca gaa aaa aat caa gga aag cgt cat gca cag gcc 432
Ile Val His Arg Ser Glu Lys Asn Gln Gly Lys Arg His Ala Gln Ala
    130                 135                 140

tgg gcc ttt gaa aga tca gac gct gat gtc ttt ttg acc gtt gac tca 480
Trp Ala Phe Glu Arg Ser Asp Ala Asp Val Phe Leu Thr Val Asp Ser
145                 150                 155                 160

gat act tat atc tac cct gat gct tta gag gag ttg tta aaa acc ttt 528
Asp Thr Tyr Ile Tyr Pro Asp Ala Leu Glu Glu Leu Leu Lys Thr Phe
                165                 170                 175

aat gac cca act gtt ttt gct gcg acg ggt cac ctt aat gtc aga aat 576
Asn Asp Pro Thr Val Phe Ala Ala Thr Gly His Leu Asn Val Arg Asn
            180                 185                 190

aga caa acc aat ctc tta aca cgc ttg aca gat att cgc tat gat aat 624
Arg Gln Thr Asn Leu Leu Thr Arg Leu Thr Asp Ile Arg Tyr Asp Asn
        195                 200                 205

gct ttt ggc gtt gaa cga gct gcc caa tcc gtt aca ggt aat att ctc 672
Ala Phe Gly Val Glu Arg Ala Ala Gln Ser Val Thr Gly Asn Ile Leu
    210                 215                 220

gtt tgc tca ggc ccg ctt agc gtt tac aga cgc gag gtg gtt gtt cct 720
Val Cys Ser Gly Pro Leu Ser Val Tyr Arg Arg Glu Val Val Val Pro
225                 230                 235                 240

aac ata gat aga tac atc aac cag acc ttc ctg ggt att cct gta agt 768
Asn Ile Asp Arg Tyr Ile Asn Gln Thr Phe Leu Gly Ile Pro Val Ser
                245                 250                 255

atc ggt gat gac agg tgc ttg acc aac tat gca act gat tta gga aag 816
Ile Gly Asp Asp Arg Cys Leu Thr Asn Tyr Ala Thr Asp Leu Gly Lys
            260                 265                 270

act gtt tat caa tcc act gct aaa tgt att aca gat gtt cct gac aag 864
Thr Val Tyr Gln Ser Thr Ala Lys Cys Ile Thr Asp Val Pro Asp Lys
        275                 280                 285

atg tct act tac ttg aag cag caa aac cgc tgg aac aag tcc ttc ttt 912
Met Ser Thr Tyr Leu Lys Gln Gln Asn Arg Trp Asn Lys Ser Phe Phe
    290                 295                 300

aga gag tcc att att tct gtt aag aaa atc atg aac aat cct ttt gta 960
Arg Glu Ser Ile Ile Ser Val Lys Lys Ile Met Asn Asn Pro Phe Val
305                 310                 315                 320

gcc cta tgg acc ata ctt gag gtg tct atg ttt atg atg ctt gtt tat 1008
Ala Leu Trp Thr Ile Leu Glu Val Ser Met Phe Met Met Leu Val Tyr
                325                 330                 335

tct gtg gtg gat ttc ttt gta gac aat gtc aga gaa ttt gat tgg ctc      1056
Ser Val Val Asp Phe Phe Val Asp Asn Val Arg Glu Phe Asp Trp Leu
            340                 345                 350

agg gtt ttg gcc ttt ctg gtg att atc ttc att gtt gct ctt tgt cgt      1104
Arg Val Leu Ala Phe Leu Val Ile Ile Phe Ile Val Ala Leu Cys Arg
        355                 360                 365

aat att cac tat atg ctt aag cac ccg ctg tcc ttc ttg tta tct ccg      1152
Asn Ile His Tyr Met Leu Lys His Pro Leu Ser Phe Leu Leu Ser Pro
    370                 375                 380

ttt tat ggg gta ctg cat ttg ttt gtc cta cag ccc ttg aaa ttg tat      1200
Phe Tyr Gly Val Leu His Leu Phe Val Leu Gln Pro Leu Lys Leu Tyr
385                 390                 395                 400

tct ctt ttt act att aga aat gct gac tgg gga aca cgt aaa aaa tta      1248
Ser Leu Phe Thr Ile Arg Asn Ala Asp Trp Gly Thr Arg Lys Lys Leu
                405                 410                 415

tta taa                                                              1254
Leu

<210>  56

<211>  417

<212>  PRT

<213>  Streptococcus equi

<400>  56

Met Arg Thr Leu Lys Asn Leu Ile Thr Val Val Ala Phe Ser Ile Phe
1               5                   10                  15

Trp Val Leu Leu Ile Tyr Val Asn Val Tyr Leu Phe Gly Ala Lys Gly
            20                  25                  30

Ser Leu Ser Ile Tyr Gly Phe Leu Leu Ile Ala Tyr Leu Leu Val Lys
        35                  40                  45

Met Ser Leu Ser Phe Phe Tyr Lys Pro Phe Lys Gly Arg Ala Gly Gln
    50                  55                  60

Tyr Lys Val Ala Ala Ile Ile Pro Ser Tyr Asn Glu Asp Ala Glu Ser
65                  70                  75                  80

Leu Leu Glu Thr Leu Lys Ser Val Gln Gln Gln Thr Tyr Pro Leu Ala
                85                  90                  95

Glu Ile Tyr Val Val Asp Asp Gly Ser Ala Asp Glu Thr Gly Ile Lys
            100                 105                 110

Arg Ile Glu Asp Tyr Val Arg Asp Thr Gly Asp Leu Ser Ser Asn Val
        115                 120                 125

Ile Val His Arg Ser Glu Lys Asn Gln Gly Lys Arg His Ala Gln Ala
    130                 135                 140

Trp Ala Phe Glu Arg Ser Asp Ala Asp Val Phe Leu Thr Val Asp Ser
145                 150                 155                 160

Asp Thr Tyr Ile Tyr Pro Asp Ala Leu Glu Glu Leu Leu Lys Thr Phe
                165                 170                 175

Asn Asp Pro Thr Val Phe Ala Ala Thr Gly His Leu Asn Val Arg Asn
            180                 185                 190

Arg Gln Thr Asn Leu Leu Thr Arg Leu Thr Asp Ile Arg Tyr Asp Asn
        195                 200                 205

Ala Phe Gly Val Glu Arg Ala Ala Gln Ser Val Thr Gly Asn Ile Leu
    210                 215                 220

Val Cys Ser Gly Pro Leu Ser Val Tyr Arg Arg Glu Val Val Val Pro
225                 230                 235                 240

Asn Ile Asp Arg Tyr Ile Asn Gln Thr Phe Leu Gly Ile Pro Val Ser
                245                 250                 255

Ile Gly Asp Asp Arg Cys Leu Thr Asn Tyr Ala Thr Asp Leu Gly Lys
            260                 265                 270

Thr Val Tyr Gln Ser Thr Ala Lys Cys Ile Thr Asp Val Pro Asp Lys
        275                 280                 285

Met Ser Thr Tyr Leu Lys Gln Gln Asn Arg Trp Asn Lys Ser Phe Phe
    290                 295                 300

Arg Glu Ser Ile Ile Ser Val Lys Lys Ile Met Asn Asn Pro Phe Val
305                 310                 315                 320

Ala Leu Trp Thr Ile Leu Glu Val Ser Met Phe Met Met Leu Val Tyr
                325                 330                 335

Ser Val Val Asp Phe Phe Val Asp Asn Val Arg Glu Phe Asp Trp Leu
            340                 345                 350

Arg Val Leu Ala Phe Leu Val Ile Ile Phe Ile Val Ala Leu Cys Arg
        355                 360                 365

Asn Ile His Tyr Met Leu Lys His Pro Leu Ser Phe Leu Leu Ser Pro
    370                 375                 380

Phe Tyr Gly Val Leu His Leu Phe Val Leu Gln Pro Leu Lys Leu Tyr
385                 390                 395                 400

Ser Leu Phe Thr Ile Arg Asn Ala Asp Trp Gly Thr Arg Lys Lys Leu
                405                 410                 415

Leu

<210> 57

<211> 1254

<212> DNA

<213> Streptococcus uberis

<220>

<221> CDS

<222> (1)..(1254)

<223>

<300>

<308> AJ242946.2

<309> 1999-07-07

<313> (23)..(1276)

<400> 57
atg gaa aaa cta aaa aat ctc att aca ttt atg act ttt att ttc ctg 48
Met Glu Lys Leu Lys Asn Leu Ile Thr Phe Met Thr Phe Ile Phe Leu
1               5                   10                  15

tgg ctc ata att att ggg ctt aat gtt ttt gta ttt gga act aaa gga 96
Trp Leu Ile Ile Ile Gly Leu Asn Val Phe Val Phe Gly Thr Lys Gly
            20                  25                  30

agt cta aca gtg tat ggg att att cta tta acc tat ttg tcg ata aaa 144
Ser Leu Thr Val Tyr Gly Ile Ile Leu Leu Thr Tyr Leu Ser Ile Lys
        35                  40                  45

atg gga tta tct ttt ttt tat cgt ccc tat aaa gga agt gta ggt caa 192
Met Gly Leu Ser Phe Phe Tyr Arg Pro Tyr Lys Gly Ser Val Gly Gln
    50                  55                  60

tat aag gta gca gct att atc cca tct tat aat gag gat ggt gtc ggt 240
Tyr Lys Val Ala Ala Ile Ile Pro Ser Tyr Asn Glu Asp Gly Val Gly
65                  70                  75                  80

tta cta gaa act cta aag agt gtt caa aaa caa aca tat cca att gca 288
Leu Leu Glu Thr Leu Lys Ser Val Gln Lys Gln Thr Tyr Pro Ile Ala
                85                  90                  95

gaa att ttc gta att gac gat ggg tca gta gat aaa aca ggt ata aaa 336
Glu Ile Phe Val Ile Asp Asp Gly Ser Val Asp Lys Thr Gly Ile Lys
            100                 105                 110

ttg gtc gaa gac tat gtg aag tta aat ggc ttt gga gac caa gtt atc     384
Leu Val Glu Asp Tyr Val Lys Leu Asn Gly Phe Gly Asp Gln Val Ile
        115                 120                 125

gtt cat cag atg cct gaa aat gtt ggt aaa aga cat gct cag gct tgg     432
Val His Gln Met Pro Glu Asn Val Gly Lys Arg His Ala Gln Ala Trp
    130                 135                 140

gca ttt gaa agg tct gat gct gat gtt ttc tta aca gtg gat tca gat     480
Ala Phe Glu Arg Ser Asp Ala Asp Val Phe Leu Thr Val Asp Ser Asp
145                 150                 155                 160

acc tac atc tat cct gat gct ctt gaa gaa tta tta aag aca ttt aat     528
Thr Tyr Ile Tyr Pro Asp Ala Leu Glu Glu Leu Leu Lys Thr Phe Asn
                165                 170                 175

gat cca gag gtc tac gct gca act ggt cat tta aat gca aga aat aga     576
Asp Pro Glu Val Tyr Ala Ala Thr Gly His Leu Asn Ala Arg Asn Arg
            180                 185                 190

caa act aat ctc tta act aga ctg act gat att cgt tac gat aat gca     624
Gln Thr Asn Leu Leu Thr Arg Leu Thr Asp Ile Arg Tyr Asp Asn Ala
        195                 200                 205

ttt ggt gta gaa cgt gct gct cag tct gtt acg gga aat att ttg gtt     672
Phe Gly Val Glu Arg Ala Ala Gln Ser Val Thr Gly Asn Ile Leu Val
    210                 215                 220

tgt tcc gga cct tta agt att tat aga cgt tcc gtc ggt att cca aat     720
Cys Ser Gly Pro Leu Ser Ile Tyr Arg Arg Ser Val Gly Ile Pro Asn
225                 230                 235                 240

ctt gaa cgc tat acc tca caa aca ttt ctt ggt gtc cct gta agc ata     768
Leu Glu Arg Tyr Thr Ser Gln Thr Phe Leu Gly Val Pro Val Ser Ile
                245                 250                 255

ggg gat gac cgt tgt ttg aca aat tat gca act gat ttg gga aaa acg     816
Gly Asp Asp Arg Cys Leu Thr Asn Tyr Ala Thr Asp Leu Gly Lys Thr
            260                 265                 270

gtt tat cag tca act gca aga tgt gat act gac gtt cca gat aag ttt     864
Val Tyr Gln Ser Thr Ala Arg Cys Asp Thr Asp Val Pro Asp Lys Phe
        275                 280                 285

aag gtt ttc atc aaa caa caa aat cgt tgg aat aag tca ttt ttt agg     912
Lys Val Phe Ile Lys Gln Gln Asn Arg Trp Asn Lys Ser Phe Phe Arg
    290                 295                 300

gag tct att atc tct gtt aag aag tta tta gcc aca cca agt gtt gct     960
Glu Ser Ile Ile Ser Val Lys Lys Leu Leu Ala Thr Pro Ser Val Ala
305                 310                 315                 320

gtt tgg act att aca gaa gtt tcc atg ttc atc atg cta gtt tat tct    1008
Val Trp Thr Ile Thr Glu Val Ser Met Phe Ile Met Leu Val Tyr Ser
                325                 330                 335

atc ttt agc tta ttg ata gga gag gct caa gaa ttt aat ctc ata aaa    1056
Ile Phe Ser Leu Leu Ile Gly Glu Ala Gln Glu Phe Asn Leu Ile Lys
            340                 345                 350

ctg gtt gct ttt tta gtt att att ttc ata gta gct ctt tgt aga aat    1104
Leu Val Ala Phe Leu Val Ile Ile Phe Ile Val Ala Leu Cys Arg Asn
        355                 360                 365

gtt cat tac atg gtt aag cat cca ttt gct ttt tta ttg tca ccg ttt    1152
Val His Tyr Met Val Lys His Pro Phe Ala Phe Leu Leu Ser Pro Phe
    370                 375                 380

tat gga ttg ata cat cta ttc gtt ttg caa cct ctt aag ata tat tcg      1200
Tyr Gly Leu Ile His Leu Phe Val Leu Gln Pro Leu Lys Ile Tyr Ser
385                 390                 395                 400

tta ttt act ata aga aat gct aca tgg gga act cgt aaa aag aca agt      1248
Leu Phe Thr Ile Arg Asn Ala Thr Trp Gly Thr Arg Lys Lys Thr Ser
                405                 410                 415

aaa taa                                                              1254
Lys

<210>  58

<211>  417

<212>  PRT

<213>  Streptococcus uberis

<400>  58

Met Glu Lys Leu Lys Asn Leu Ile Thr Phe Met Thr Phe Ile Phe Leu
1               5                   10                  15

Trp Leu Ile Ile Ile Gly Leu Asn Val Phe Val Phe Gly Thr Lys Gly
            20                  25                  30

Ser Leu Thr Val Tyr Gly Ile Ile Leu Leu Thr Tyr Leu Ser Ile Lys
        35                  40                  45

Met Gly Leu Ser Phe Phe Tyr Arg Pro Tyr Lys Gly Ser Val Gly Gln
    50                  55                  60

Tyr Lys Val Ala Ala Ile Ile Pro Ser Tyr Asn Glu Asp Gly Val Gly
65                  70                  75                  80

Leu Leu Glu Thr Leu Lys Ser Val Gln Lys Gln Thr Tyr Pro Ile Ala
                85                  90                  95

Glu Ile Phe Val Ile Asp Asp Gly Ser Val Asp Lys Thr Gly Ile Lys
            100                 105                 110

Leu Val Glu Asp Tyr Val Lys Leu Asn Gly Phe Gly Asp Gln Val Ile
        115                 120                 125

Val His Gln Met Pro Glu Asn Val Gly Lys Arg His Ala Gln Ala Trp
    130                 135                 140

Ala Phe Glu Arg Ser Asp Ala Asp Val Phe Leu Thr Val Asp Ser Asp
145                 150                 155                 160

Thr Tyr Ile Tyr Pro Asp Ala Leu Glu Glu Leu Leu Lys Thr Phe Asn
                165                 170                 175

Asp Pro Glu Val Tyr Ala Ala Thr Gly His Leu Asn Ala Arg Asn Arg
            180                 185                 190

Gln Thr Asn Leu Leu Thr Arg Leu Thr Asp Ile Arg Tyr Asp Asn Ala
        195                 200                 205

Phe Gly Val Glu Arg Ala Ala Gln Ser Val Thr Gly Asn Ile Leu Val
    210                 215                 220

Cys Ser Gly Pro Leu Ser Ile Tyr Arg Arg Ser Val Gly Ile Pro Asn
225                 230                 235                 240

Leu Glu Arg Tyr Thr Ser Gln Thr Phe Leu Gly Val Pro Val Ser Ile
                245                 250                 255

Gly Asp Asp Arg Cys Leu Thr Asn Tyr Ala Thr Asp Leu Gly Lys Thr
            260                 265                 270

Val Tyr Gln Ser Thr Ala Arg Cys Asp Thr Asp Val Pro Asp Lys Phe
        275                 280                 285

Lys Val Phe Ile Lys Gln Gln Asn Arg Trp Asn Lys Ser Phe Phe Arg
    290                 295                 300

Glu Ser Ile Ile Ser Val Lys Lys Leu Leu Ala Thr Pro Ser Val Ala
305                 310                 315                 320

Val Trp Thr Ile Thr Glu Val Ser Met Phe Ile Met Leu Val Tyr Ser
                325                 330                 335

Ile Phe Ser Leu Leu Ile Gly Glu Ala Gln Glu Phe Asn Leu Ile Lys
            340                 345                 350

Leu Val Ala Phe Leu Val Ile Ile Phe Ile Val Ala Leu Cys Arg Asn
        355                 360                 365

Val His Tyr Met Val Lys His Pro Phe Ala Phe Leu Leu Ser Pro Phe
    370                 375                 380

Tyr Gly Leu Ile His Leu Phe Val Leu Gln Pro Leu Lys Ile Tyr Ser
385                 390                 395                 400

Leu Phe Thr Ile Arg Asn Ala Thr Trp Gly Thr Arg Lys Lys Thr Ser
                405                 410                 415

Lys

<210>  59

<211>  1254

<212>  DNA

<213>  Streptococcus equisimilis


<220>

<221>  CDS

<222>  (1)..(1254)

<223>


<300>

<308>  AF023876.1

<309>  1997-12-04

<313>  (1)..(1254)


<400>  59
atg aga aca tta aaa aac ctc ata act gtt gtg gcc ttt agt att ttt      48
Met Arg Thr Leu Lys Asn Leu Ile Thr Val Val Ala Phe Ser Ile Phe
1               5                   10                  15

tgg gta ctg ttg att tac gtc aat gtt tat ctc ttt ggt gct aaa gga      96
Trp Val Leu Leu Ile Tyr Val Asn Val Tyr Leu Phe Gly Ala Lys Gly
            20                  25                  30

agc ttg tca att tat ggc ttt ttg ctg ata gct tac cta tta gtc aaa      144
Ser Leu Ser Ile Tyr Gly Phe Leu Leu Ile Ala Tyr Leu Leu Val Lys
        35                  40                  45

atg tcc tta tcc ttt ttt tac aag cca ttt aag gga agg gct ggg caa      192
Met Ser Leu Ser Phe Phe Tyr Lys Pro Phe Lys Gly Arg Ala Gly Gln
    50                  55                  60

tat aag gtt gca gcc att att ccc tct tat aac gaa gat gct gag tca      240
Tyr Lys Val Ala Ala Ile Ile Pro Ser Tyr Asn Glu Asp Ala Glu Ser
65                  70                  75                  80

ttg cta gag acc tta aaa agt gtt cag cag caa acc tat ccc cta gca      288
Leu Leu Glu Thr Leu Lys Ser Val Gln Gln Gln Thr Tyr Pro Leu Ala
                85                  90                  95

gaa att tat gtt gtt gac gat gga agt gct gat gag aca ggt att aag      336
Glu Ile Tyr Val Val Asp Asp Gly Ser Ala Asp Glu Thr Gly Ile Lys
            100                 105                 110

cgc att gaa gac tat gtg cgt gac act ggt gac cta tca agc aat gtc      384
Arg Ile Glu Asp Tyr Val Arg Asp Thr Gly Asp Leu Ser Ser Asn Val
        115                 120                 125

att gtt cat cgg tca gag aaa aat caa gga aag cgt cat gca cag gcc      432
Ile Val His Arg Ser Glu Lys Asn Gln Gly Lys Arg His Ala Gln Ala
    130                 135                 140

tgg gcc ttt gaa aga tca gac gct gat gtc ttt ttg acc gtt gac tca      480
Trp Ala Phe Glu Arg Ser Asp Ala Asp Val Phe Leu Thr Val Asp Ser
145                 150                 155                 160

gat act tat atc tac cct gat gct tta gag gag ttg tta aaa acc ttt 528
Asp Thr Tyr Ile Tyr Pro Asp Ala Leu Glu Glu Leu Leu Lys Thr Phe
                165                 170                 175

aat gac cca act gtt ttt gct gcg acg ggt cac ctt aat gtc aga aat 576
Asn Asp Pro Thr Val Phe Ala Ala Thr Gly His Leu Asn Val Arg Asn
            180                 185                 190

aga caa acc aat ctc tta aca cgc ttg aca gat att cgc tat gat aat 624
Arg Gln Thr Asn Leu Leu Thr Arg Leu Thr Asp Ile Arg Tyr Asp Asn
        195                 200                 205

gct ttt ggc gtt gaa cga gct gcc caa tcc gtt aca ggt aat atc ctt 672
Ala Phe Gly Val Glu Arg Ala Ala Gln Ser Val Thr Gly Asn Ile Leu
    210                 215                 220

gtt tgc tca ggt ccg ctt agc gtt tac aga cgc gag gtg gtt gtt cct 720
Val Cys Ser Gly Pro Leu Ser Val Tyr Arg Arg Glu Val Val Val Pro
225                 230                 235                 240

aac ata gat aga tac atc aac cag acc ttc ctg ggt att cct gta agt 768
Asn Ile Asp Arg Tyr Ile Asn Gln Thr Phe Leu Gly Ile Pro Val Ser
                245                 250                 255

att ggt gat gac agg tgc ttg acc aac tat gca act gat tta gga aag 816
Ile Gly Asp Asp Arg Cys Leu Thr Asn Tyr Ala Thr Asp Leu Gly Lys
            260                 265                 270

act gtt tat caa tcc act gct aaa tgt att aca gat gtt cct gac aag 864
Thr Val Tyr Gln Ser Thr Ala Lys Cys Ile Thr Asp Val Pro Asp Lys
        275                 280                 285

atg tct act tac ttg aag cag caa aac cgc tgg aac aag tcc ttc ttt 912
Met Ser Thr Tyr Leu Lys Gln Gln Asn Arg Trp Asn Lys Ser Phe Phe
    290                 295                 300

aga gag tcc att att tct gtt aag aaa atc atg aac aat cct ttt gta 960
Arg Glu Ser Ile Ile Ser Val Lys Lys Ile Met Asn Asn Pro Phe Val
305                 310                 315                 320

gcc cta tgg acc ata ctt gag gtg tct atg ttt atg atg ctt gtt tat 1008
Ala Leu Trp Thr Ile Leu Glu Val Ser Met Phe Met Met Leu Val Tyr
                325                 330                 335

tct gtg gtg gat ttc ttt gta ggc aat gtc aga gaa ttt gat tgg ctc 1056
Ser Val Val Asp Phe Phe Val Gly Asn Val Arg Glu Phe Asp Trp Leu
            340                 345                 350

agg gtt tta gcc ttt ctg gtg att atc ttc att gtt gcc ctg tgt cgg 1104
Arg Val Leu Ala Phe Leu Val Ile Ile Phe Ile Val Ala Leu Cys Arg
        355                 360                 365

aac att cat tac atg ctt aag cac ccg ctg tcc ttc ttg tta tct ccg 1152
Asn Ile His Tyr Met Leu Lys His Pro Leu Ser Phe Leu Leu Ser Pro
    370                 375                 380

ttt tat ggg gtg ctg cat ttg ttt gtc cta cag ccc ttg aaa tta tat 1200
Phe Tyr Gly Val Leu His Leu Phe Val Leu Gln Pro Leu Lys Leu Tyr
385                 390                 395                 400

tct ctt ttt act att aga aat gct gac tgg gga aca cgt aaa aaa tta 1248
Ser Leu Phe Thr Ile Arg Asn Ala Asp Trp Gly Thr Arg Lys Lys Leu
                405                 410                 415

tta taa 1254
Leu

<210> 60

<211> 417

<212> PRT

<213> Streptococcus equisimilis

<400> 60

Met Arg Thr Leu Lys Asn Leu Ile Thr Val Val Ala Phe Ser Ile Phe
1               5                   10                  15

Trp Val Leu Leu Ile Tyr Val Asn Val Tyr Leu Phe Gly Ala Lys Gly
            20                  25                  30

Ser Leu Ser Ile Tyr Gly Phe Leu Leu Ile Ala Tyr Leu Leu Val Lys
        35                  40                  45

Met Ser Leu Ser Phe Phe Tyr Lys Pro Phe Lys Gly Arg Ala Gly Gln
    50                  55                  60

Tyr Lys Val Ala Ala Ile Ile Pro Ser Tyr Asn Glu Asp Ala Glu Ser
65                  70                  75                  80

Leu Leu Glu Thr Leu Lys Ser Val Gln Gln Gln Thr Tyr Pro Leu Ala
                85                  90                  95

Glu Ile Tyr Val Val Asp Asp Gly Ser Ala Asp Glu Thr Gly Ile Lys
            100                 105                 110

Arg Ile Glu Asp Tyr Val Arg Asp Thr Gly Asp Leu Ser Ser Asn Val
        115                 120                 125

Ile Val His Arg Ser Glu Lys Asn Gln Gly Lys Arg His Ala Gln Ala
    130                 135                 140

Trp Ala Phe Glu Arg Ser Asp Ala Asp Val Phe Leu Thr Val Asp Ser
145                 150                 155                 160

Asp Thr Tyr Ile Tyr Pro Asp Ala Leu Glu Glu Leu Leu Lys Thr Phe
                165                 170                 175

Asn Asp Pro Thr Val Phe Ala Ala Thr Gly His Leu Asn Val Arg Asn
            180                 185                 190

Arg Gln Thr Asn Leu Leu Thr Arg Leu Thr Asp Ile Arg Tyr Asp Asn
        195                 200                 205

Ala Phe Gly Val Glu Arg Ala Ala Gln Ser Val Thr Gly Asn Ile Leu
    210                 215                 220

Val Cys Ser Gly Pro Leu Ser Val Tyr Arg Arg Glu Val Val Val Pro
225                 230                 235                 240

Asn Ile Asp Arg Tyr Ile Asn Gln Thr Phe Leu Gly Ile Pro Val Ser
                245                 250                 255

Ile Gly Asp Asp Arg Cys Leu Thr Asn Tyr Ala Thr Asp Leu Gly Lys
            260                 265                 270

Thr Val Tyr Gln Ser Thr Ala Lys Cys Ile Thr Asp Val Pro Asp Lys
        275                 280                 285

Met Ser Thr Tyr Leu Lys Gln Gln Asn Arg Trp Asn Lys Ser Phe Phe
    290                 295                 300

Arg Glu Ser Ile Ile Ser Val Lys Lys Ile Met Asn Asn Pro Phe Val
305                 310                 315                 320

Ala Leu Trp Thr Ile Leu Glu Val Ser Met Phe Met Met Leu Val Tyr
                325                 330                 335

Ser Val Val Asp Phe Phe Val Gly Asn Val Arg Glu Phe Asp Trp Leu
            340                 345                 350

Arg Val Leu Ala Phe Leu Val Ile Ile Phe Ile Val Ala Leu Cys Arg
        355                 360                 365

Asn Ile His Tyr Met Leu Lys His Pro Leu Ser Phe Leu Leu Ser Pro
    370                 375                 380

Phe Tyr Gly Val Leu His Leu Phe Val Leu Gln Pro Leu Lys Leu Tyr
385                 390                 395                 400

Ser Leu Phe Thr Ile Arg Asn Ala Asp Trp Gly Thr Arg Lys Lys Leu
                405                 410                 415

Leu

<210>  61
<211>  1248
<212>  DNA
<213>  Sulfolobus tokodaii str. 7

<220>
<221>  CDS

<222>  (1)..(1248)

<223>

<300>

<308>  AP000988

<309>  2001-09-17

<313>  (173392)..(174639)

<400>  61
gtg gtg att atg ttt cac tta ttt cac gga gtc tca tat ttc att tat      48
Met Met Ile Met Phe His Leu Phe His Gly Val Ser Tyr Phe Ile Tyr
1               5                   10                  15

tct ctt tca ttc aca att ata act att ctt tat ttc ttt ttg aat tca      96
Ser Leu Ser Phe Thr Ile Ile Thr Ile Leu Tyr Phe Phe Leu Asn Ser
            20                  25                  30

ttt ttt gca gta ata agt aat aat aga aaa act caa cac tca agt ttt      144
Phe Phe Ala Val Ile Ser Asn Asn Arg Lys Thr Gln His Ser Ser Phe
        35                  40                  45

tat aat ctc tct gat ctt aca gtt gtg ata cca gtt tat aag gag gaa      192
Tyr Asn Leu Ser Asp Leu Thr Val Met Ile Pro Val Tyr Lys Glu Glu
    50                  55                  60

ata gat att ttt gaa aaa gtg ata agg act tta tat gac aca agg tta      240
Ile Asp Ile Phe Glu Lys Met Ile Arg Thr Leu Tyr Asp Thr Arg Leu
65                  70                  75                  80

gaa ttt att gtt gta ggg gat agt gtt cta gaa cca tac aaa tca att      288
Glu Phe Ile Val Val Gly Asp Ser Val Leu Glu Pro Tyr Lys Ser Ile
                85                  90                  95

acg gaa aga tat ggt ggt aaa ttt att tat atg cgt gaa cat aag ggg      336
Thr Glu Arg Tyr Gly Gly Lys Phe Ile Tyr Met Arg Glu His Lys Gly
            100                 105                 110

aaa aga tac gcg tta gcc gag gga gtt aag tat gta aga tct cct cta      384
Lys Arg Tyr Ala Leu Ala Glu Gly Val Lys Tyr Val Arg Ser Pro Leu
        115                 120                 125

gtg atg ttt cta gat agt gat acg att att tat aaa gac tct ata cta      432
Met Met Phe Leu Asp Ser Asp Thr Ile Ile Tyr Lys Asp Ser Ile Leu
    130                 135                 140

aag atg tta agt gtt ttt gat gag tca gta ggt gga gta ggg cca aat      480
Lys Met Leu Ser Val Phe Asp Glu Ser Val Gly Gly Val Gly Pro Asn
145                 150                 155                 160

att aga att atg tat gac gag aaa aat aaa tat gca tat tat tat ggt      528
Ile Arg Ile Met Tyr Asp Glu Lys Asn Lys Tyr Ala Tyr Tyr Tyr Gly
                165                 170                 175

gaa ttc ttt gag aga ata agt gag ata gta aac agg gcg gta aac tat      576
Glu Phe Phe Glu Arg Ile Ser Glu Ile Val Asn Arg Ala Val Asn Tyr
            180                 185                 190

ttt gga agt gct ata ata tta agt gga caa tgt gta ata tat agg acc      624
Phe Gly Ser Ala Ile Ile Leu Ser Gly Gln Cys Val Ile Tyr Arg Thr
        195                 200                 205

gaa ctc gta aaa cca tat ata tta tct aaa gag ttt tta gag ccg aaa     672
Glu Leu Val Lys Pro Tyr Ile Leu Ser Lys Glu Phe Leu Glu Pro Lys
    210                 215                 220

atg ttt gga aga cca att aaa att tcc gat gat aga gat tta acc gat     720
Met Phe Gly Arg Pro Ile Lys Ile Ser Asp Asp Arg Asp Leu Thr Asp
225                 230                 235                 240

ttt gtt ata aaa aaa ggg tat agg gct gta aaa gtc ttt gat gca gtg     768
Phe Val Ile Lys Lys Gly Tyr Arg Ala Val Lys Val Phe Asp Ala Met
                245                 250                 255

gca tat aca aaa ccc cct aga gac ata aaa atg ttt acg aaa caa gta     816
Ala Tyr Thr Lys Pro Pro Arg Asp Ile Lys Met Phe Thr Lys Gln Val
            260                 265                 270

act aga tgg aca aga gca aat tat ctt aat ttt ata agg gag ata gct     864
Thr Arg Trp Thr Arg Ala Asn Tyr Leu Asn Phe Ile Arg Glu Ile Ala
        275                 280                 285

gac ggt agt ata agt aaa agg ggt tca tta tac gtt ttt aat atg ata     912
Asp Gly Ser Ile Ser Lys Arg Gly Ser Leu Tyr Val Phe Asn Met Ile
    290                 295                 300

tac acc aat ctg tta cca tta ttt acg ctc ttg ttc ctt tat atg agt     960
Tyr Thr Asn Leu Leu Pro Leu Phe Thr Leu Leu Phe Leu Tyr Met Ser
305                 310                 315                 320

ttc act aga att ctt aag atc tat tcc tca att aat gta att aat act     1008
Phe Thr Arg Ile Leu Lys Ile Tyr Ser Ser Ile Asn Val Ile Asn Thr
                325                 330                 335

aag ctc tta tta cta ttg tat ctg cca acc cgt tac cat tcc gac ttc     1056
Lys Leu Leu Leu Leu Leu Tyr Leu Pro Thr Arg Tyr His Ser Asp Phe
            340                 345                 350

ttt atc ttt tat tta ttc ttg cat tac gga gga ttt ata gct ata ata     1104
Phe Ile Phe Tyr Leu Phe Leu His Tyr Gly Gly Phe Ile Ala Ile Ile
        355                 360                 365

ccc ttt gta atg acc atg att tat tta att cca gaa gat aaa ttg aaa     1152
Pro Phe Val Met Thr Met Ile Tyr Leu Ile Pro Glu Asp Lys Leu Lys
    370                 375                 380

act cta ata tac ggt tct atc gca cta gca gtg caa tat att gct tcc     1200
Thr Leu Ile Tyr Gly Ser Ile Ala Leu Ala Met Gln Tyr Ile Ala Ser
385                 390                 395                 400

cta tat gct atg ata act ttc tgg tgg caa gat tgg tta act aga taa     1248
Leu Tyr Ala Met Ile Thr Phe Trp Trp Gln Asp Trp Leu Thr Arg
                405                 410                 415

<210>  62

<211>  415

<212>  PRT

<213>  Sulfolobus tokodaii str. 7

<400>  62

Met Met Ile Met Phe His Leu Phe His Gly Val Ser Tyr Phe Ile Tyr
1               5                   10                  15

Ser Leu Ser Phe Thr Ile Ile Thr Ile Leu Tyr Phe Phe Leu Asn Ser
            20                  25                  30

Phe Phe Ala Val Ile Ser Asn Asn Arg Lys Thr Gln His Ser Ser Phe
        35                  40                  45

Tyr Asn Leu Ser Asp Leu Thr Val Met Ile Pro Val Tyr Lys Glu Glu
    50                  55                  60

Ile Asp Ile Phe Glu Lys Met Ile Arg Thr Leu Tyr Asp Thr Arg Leu
65                  70                  75                  80

Glu Phe Ile Val Val Gly Asp Ser Val Leu Glu Pro Tyr Lys Ser Ile
                85                  90                  95

Thr Glu Arg Tyr Gly Gly Lys Phe Ile Tyr Met Arg Glu His Lys Gly
            100                 105                 110

Lys Arg Tyr Ala Leu Ala Glu Gly Val Lys Tyr Val Arg Ser Pro Leu
        115                 120                 125

Met Met Phe Leu Asp Ser Asp Thr Ile Ile Tyr Lys Asp Ser Ile Leu
    130                 135                 140

Lys Met Leu Ser Val Phe Asp Glu Ser Val Gly Gly Val Gly Pro Asn
145                 150                 155                 160

Ile Arg Ile Met Tyr Asp Glu Lys Asn Lys Tyr Ala Tyr Tyr Tyr Gly
                165                 170                 175

Glu Phe Phe Glu Arg Ile Ser Glu Ile Val Asn Arg Ala Val Asn Tyr
            180                 185                 190

Phe Gly Ser Ala Ile Ile Leu Ser Gly Gln Cys Val Ile Tyr Arg Thr
        195                 200                 205

Glu Leu Val Lys Pro Tyr Ile Leu Ser Lys Glu Phe Leu Glu Pro Lys
    210                 215                 220

Met Phe Gly Arg Pro Ile Lys Ile Ser Asp Asp Arg Asp Leu Thr Asp
225                 230                 235                 240

Phe Val Ile Lys Lys Gly Tyr Arg Ala Val Lys Val Phe Asp Ala Met
                245                 250                 255

Ala Tyr Thr Lys Pro Pro Arg Asp Ile Lys Met Phe Thr Lys Gln Val
            260                 265                 270

Thr Arg Trp Thr Arg Ala Asn Tyr Leu Asn Phe Ile Arg Glu Ile Ala
        275                 280                 285

Asp Gly Ser Ile Ser Lys Arg Gly Ser Leu Tyr Val Phe Asn Met Ile
    290                 295                 300

Tyr Thr Asn Leu Leu Pro Leu Phe Thr Leu Leu Phe Leu Tyr Met Ser
305                 310                 315                 320

Phe Thr Arg Ile Leu Lys Ile Tyr Ser Ser Ile Asn Val Ile Asn Thr
                325                 330                 335

Lys Leu Leu Leu Leu Leu Tyr Leu Pro Thr Arg Tyr His Ser Asp Phe
            340                 345                 350

Phe Ile Phe Tyr Leu Phe Leu His Tyr Gly Gly Phe Ile Ala Ile Ile
        355                 360                 365

Pro Phe Val Met Thr Met Ile Tyr Leu Ile Pro Glu Asp Lys Leu Lys
    370                 375                 380

Thr Leu Ile Tyr Gly Ser Ile Ala Leu Ala Met Gln Tyr Ile Ala Ser
385                 390                 395                 400

Leu Tyr Ala Met Ile Thr Phe Trp Trp Gln Asp Trp Leu Thr Arg
                405                 410                 415

<210>  63

<211>  1767

<212>  DNA

<213>  Artificial Sequence

<220>

<223>  Synthetic sequence of HAS 1 from Xenopus laevis

<220>

<221>  CDS

<222>  (1)..(1767)

<223>

<400>  63
atg aaa gag aaa gcc gct gaa aca atg gaa ata ccg gaa gga atc cct      48
Met Lys Glu Lys Ala Ala Glu Thr Met Glu Ile Pro Glu Gly Ile Pro
1               5                   10                  15

aaa gat tta gaa ccc aag cac cct acg ttg tgg cgc att atc tac tat      96
Lys Asp Leu Glu Pro Lys His Pro Thr Leu Trp Arg Ile Ile Tyr Tyr
            20                  25                  30

tcc ttc ggt gtt gtc ctc ctt gcg acg att act gct gcc tac gtt gca      144

Ser Phe Gly Val Val Leu Leu Ala Thr Ile Thr Ala Ala Tyr Val Ala
        35                      40                      45

gag ttc caa gtc ctc aaa cat gaa gcc atc ctg ttc tcc ctt ggc ctg      192
Glu Phe Gln Val Leu Lys His Glu Ala Ile Leu Phe Ser Leu Gly Leu
    50                  55                  60

tat ggg ctc gca atg ttg ctc cat ctc atg atg caa tca cta ttc gcg      240
Tyr Gly Leu Ala Met Leu Leu His Leu Met Met Gln Ser Leu Phe Ala
65                  70                  75                  80

ttc ttg gag atc cgt cgc gtc aac aaa tcg gag ctc cct tgc tca ttc      288
Phe Leu Glu Ile Arg Arg Val Asn Lys Ser Glu Leu Pro Cys Ser Phe
                85                  90                  95

aag aag acg gtc gcc ctc act atc gca ggc tat caa gag aat ccc gaa      336
Lys Lys Thr Val Ala Leu Thr Ile Ala Gly Tyr Gln Glu Asn Pro Glu
            100                 105                 110

tac ctt atc aag tgc ctc gaa agt tgt aag tac gtc aag tac cca aag      384
Tyr Leu Ile Lys Cys Leu Glu Ser Cys Lys Tyr Val Lys Tyr Pro Lys
        115                 120                 125

gac aaa ttg aag atc atc ttg gtt atc gac ggc aac aca gag gat gac      432
Asp Lys Leu Lys Ile Ile Leu Val Ile Asp Gly Asn Thr Glu Asp Asp
    130                 135                 140

gct tac atg atg gaa atg ttc aag gat gtg ttc cac gga gag gat gta      480
Ala Tyr Met Met Glu Met Phe Lys Asp Val Phe His Gly Glu Asp Val
145                 150                 155                 160

ggt act tac gtc tgg aag gga aac tac cat acc gtg aag aaa ccc gag      528
Gly Thr Tyr Val Trp Lys Gly Asn Tyr His Thr Val Lys Lys Pro Glu
                165                 170                 175

gag aca aac aag gga agc tgt ccc gag gta tcg aaa cct ttg aac gaa      576
Glu Thr Asn Lys Gly Ser Cys Pro Glu Val Ser Lys Pro Leu Asn Glu
            180                 185                 190

gat gaa ggt atc aac atg gtt gaa gag tta gtc cgt aac aag agg tgc      624
Asp Glu Gly Ile Asn Met Val Glu Glu Leu Val Arg Asn Lys Arg Cys
        195                 200                 205

gtt tgc att atg caa cag tgg gga ggt aaa cga gaa gtg atg tat act      672
Val Cys Ile Met Gln Gln Trp Gly Gly Lys Arg Glu Val Met Tyr Thr
    210                 215                 220

gcc ttc caa gca ata ggt act tcc gtt gac tac gtt caa gtc tgt gac      720
Ala Phe Gln Ala Ile Gly Thr Ser Val Asp Tyr Val Gln Val Cys Asp
225                 230                 235                 240

agc gac acc aag ctc gac gag ctt gcc acc gtt gag atg gtg aaa gtt      768
Ser Asp Thr Lys Leu Asp Glu Leu Ala Thr Val Glu Met Val Lys Val
                245                 250                 255

ctg gag tca aac gac atg tat ggt gcg gtc ggc ggt gat gtt cga atc      816
Leu Glu Ser Asn Asp Met Tyr Gly Ala Val Gly Gly Asp Val Arg Ile
            260                 265                 270

ttg aat cca tac gac tcg ttc atc agt ttc atg agc tca ctt agg tac      864
Leu Asn Pro Tyr Asp Ser Phe Ile Ser Phe Met Ser Ser Leu Arg Tyr
        275                 280                 285

tgg atg gct ttc aat gtc gag cgc gct tgt caa agc tat ttc gat tgc      912
Trp Met Ala Phe Asn Val Glu Arg Ala Cys Gln Ser Tyr Phe Asp Cys
    290                 295                 300

gta tct tgc att agt gga cct ctc ggt atg tat aga aat aac att ctt      960

Val Ser Cys Ile Ser Gly Pro Leu Gly Met Tyr Arg Asn Asn Ile Leu
305                 310                 315                 320

cag gtg ttc ctc gaa gct tgg tat cgg cag aag ttc ttg ggc acc tac      1008
Gln Val Phe Leu Glu Ala Trp Tyr Arg Gln Lys Phe Leu Gly Thr Tyr
                325                 330                 335

tgt acc cta ggc gat gac cgt cac ctc acc aat cgt gtt ctc tcc atg      1056
Cys Thr Leu Gly Asp Asp Arg His Leu Thr Asn Arg Val Leu Ser Met
            340                 345                 350

gga tac aga act aag tat acc cat aag tct cgt gct ttc tcc gag acc      1104
Gly Tyr Arg Thr Lys Tyr Thr His Lys Ser Arg Ala Phe Ser Glu Thr
        355                 360                 365

ccg tct ctt tat ctc aga tgg ctg aac cag caa act cgg tgg acc aag      1152
Pro Ser Leu Tyr Leu Arg Trp Leu Asn Gln Gln Thr Arg Trp Thr Lys
    370                 375                 380

tct tac ttc cgc gaa tgg ctc tac aac gca caa tgg tgg cac aaa cac      1200
Ser Tyr Phe Arg Glu Trp Leu Tyr Asn Ala Gln Trp Trp His Lys His
385                 390                 395                 400

cat atc tgg atg aca tat gaa tcc gtc gtt agc ttt atc ttc ccc ttc      1248
His Ile Trp Met Thr Tyr Glu Ser Val Val Ser Phe Ile Phe Pro Phe
                405                 410                 415

ttc atc acg gcg act gtt atc cga ctc ata tac gcc ggc act att tgg      1296
Phe Ile Thr Ala Thr Val Ile Arg Leu Ile Tyr Ala Gly Thr Ile Trp
            420                 425                 430

aat gtc gta tgg ctc tta ctt tgt atc cag att atg tct ctg ttc aag      1344
Asn Val Val Trp Leu Leu Leu Cys Ile Gln Ile Met Ser Leu Phe Lys
        435                 440                 445

agc atc tac gct tgc tgg cta cgc gga aac ttc att atg ctc ctt atg      1392
Ser Ile Tyr Ala Cys Trp Leu Arg Gly Asn Phe Ile Met Leu Leu Met
    450                 455                 460

tct ctt tac tcg atg ctc tat atg acc ggc ctg ctc cca tct aag tac      1440
Ser Leu Tyr Ser Met Leu Tyr Met Thr Gly Leu Leu Pro Ser Lys Tyr
465                 470                 475                 480

ttc gcc ctg ttg act ttg aac aag aca ggt tgg ggt act tcc ggt cgc      1488
Phe Ala Leu Leu Thr Leu Asn Lys Thr Gly Trp Gly Thr Ser Gly Arg
                485                 490                 495

aag aag att gtc gga aac tat atg cct att ctg ccg ctt agt atc tgg      1536
Lys Lys Ile Val Gly Asn Tyr Met Pro Ile Leu Pro Leu Ser Ile Trp
            500                 505                 510

gct gct gtc cta tgc ggt ggc gtc ggg tat tcg atc tat atg gat tgc      1584
Ala Ala Val Leu Cys Gly Gly Val Gly Tyr Ser Ile Tyr Met Asp Cys
        515                 520                 525

cag aac gat tgg tcc acg ccc gag aag cag aaa gaa atg tat cac ctt      1632
Gln Asn Asp Trp Ser Thr Pro Glu Lys Gln Lys Glu Met Tyr His Leu
    530                 535                 540

ctc tat ggg tgc gtg ggt tac gtc atg tac tgg gtc atc atg gct gtc      1680
Leu Tyr Gly Cys Val Gly Tyr Val Met Tyr Trp Val Ile Met Ala Val
545                 550                 555                 560

atg tac tgg gtt tgg gtc aaa cgt tgt tgc cgt aag agg tct cag act      1728
Met Tyr Trp Val Trp Val Lys Arg Cys Cys Arg Lys Arg Ser Gln Thr
                565                 570                 575

gtt acc ctt gtg cat gac atc cct gat atg tgt gtt taa                  1767

Val Thr Leu Val His Asp Ile Pro Asp Met Cys Val
            580                 585

<210> 64

<211> 588

<212> PRT

<213> Artificial Sequence

<220>

<223> Synthetic sequence of HAS 1 from Xenopus laevis

<400> 64

Met Lys Glu Lys Ala Ala Glu Thr Met Glu Ile Pro Glu Gly Ile Pro
1               5                   10                  15

Lys Asp Leu Glu Pro Lys His Pro Thr Leu Trp Arg Ile Ile Tyr Tyr
            20                  25                  30

Ser Phe Gly Val Val Leu Leu Ala Thr Ile Thr Ala Ala Tyr Val Ala
        35                  40                  45

Glu Phe Gln Val Leu Lys His Glu Ala Ile Leu Phe Ser Leu Gly Leu
    50                  55                  60

Tyr Gly Leu Ala Met Leu Leu His Leu Met Met Gln Ser Leu Phe Ala
65                  70                  75                  80

Phe Leu Glu Ile Arg Arg Val Asn Lys Ser Glu Leu Pro Cys Ser Phe
                85                  90                  95

Lys Lys Thr Val Ala Leu Thr Ile Ala Gly Tyr Gln Glu Asn Pro Glu
            100                 105                 110

Tyr Leu Ile Lys Cys Leu Glu Ser Cys Lys Tyr Val Lys Tyr Pro Lys
        115                 120                 125

Asp Lys Leu Lys Ile Ile Leu Val Ile Asp Gly Asn Thr Glu Asp Asp
    130                 135                 140

Ala Tyr Met Met Glu Met Phe Lys Asp Val Phe His Gly Glu Asp Val
145                 150                 155                 160

Gly Thr Tyr Val Trp Lys Gly Asn Tyr His Thr Val Lys Lys Pro Glu
                165                 170                 175

Glu Thr Asn Lys Gly Ser Cys Pro Glu Val Ser Lys Pro Leu Asn Glu
            180                 185                 190

Asp Glu Gly Ile Asn Met Val Glu Glu Leu Val Arg Asn Lys Arg Cys
        195                 200                 205

Val Cys Ile Met Gln Gln Trp Gly Gly Lys Arg Glu Val Met Tyr Thr
    210                 215                 220

Ala Phe Gln Ala Ile Gly Thr Ser Val Asp Tyr Val Gln Val Cys Asp
225                 230                 235                 240

Ser Asp Thr Lys Leu Asp Glu Leu Ala Thr Val Glu Met Val Lys Val
                245                 250                 255

Leu Glu Ser Asn Asp Met Tyr Gly Ala Val Gly Gly Asp Val Arg Ile
            260                 265                 270

Leu Asn Pro Tyr Asp Ser Phe Ile Ser Phe Met Ser Ser Leu Arg Tyr
        275                 280                 285

Trp Met Ala Phe Asn Val Glu Arg Ala Cys Gln Ser Tyr Phe Asp Cys
    290                 295                 300

Val Ser Cys Ile Ser Gly Pro Leu Gly Met Tyr Arg Asn Asn Ile Leu
305                 310                 315                 320

Gln Val Phe Leu Glu Ala Trp Tyr Arg Gln Lys Phe Leu Gly Thr Tyr
                325                 330                 335

Cys Thr Leu Gly Asp Asp Arg His Leu Thr Asn Arg Val Leu Ser Met
            340                 345                 350

Gly Tyr Arg Thr Lys Tyr Thr His Lys Ser Arg Ala Phe Ser Glu Thr
        355                 360                 365

Pro Ser Leu Tyr Leu Arg Trp Leu Asn Gln Gln Thr Arg Trp Thr Lys
    370                 375                 380

Ser Tyr Phe Arg Glu Trp Leu Tyr Asn Ala Gln Trp Trp His Lys His
385                 390                 395                 400

His Ile Trp Met Thr Tyr Glu Ser Val Val Ser Phe Ile Phe Pro Phe
                405                 410                 415

Phe Ile Thr Ala Thr Val Ile Arg Leu Ile Tyr Ala Gly Thr Ile Trp
            420                 425                 430

Asn Val Val Trp Leu Leu Leu Cys Ile Gln Ile Met Ser Leu Phe Lys
        435                 440                 445

Ser Ile Tyr Ala Cys Trp Leu Arg Gly Asn Phe Ile Met Leu Leu Met
    450                 455                 460

Ser Leu Tyr Ser Met Leu Tyr Met Thr Gly Leu Leu Pro Ser Lys Tyr
465                 470                 475                 480

Phe Ala Leu Leu Thr Leu Asn Lys Thr Gly Trp Gly Thr Ser Gly Arg
                485                 490                 495

Lys Lys Ile Val Gly Asn Tyr Met Pro Ile Leu Pro Leu Ser Ile Trp
            500                 505                 510

Ala Ala Val Leu Cys Gly Gly Val Gly Tyr Ser Ile Tyr Met Asp Cys
        515                 520                 525

Gln Asn Asp Trp Ser Thr Pro Glu Lys Gln Lys Glu Met Tyr His Leu
    530                 535                 540

Leu Tyr Gly Cys Val Gly Tyr Val Met Tyr Trp Val Ile Met Ala Val
545                 550                 555                 560

Met Tyr Trp Val Trp Val Lys Arg Cys Cys Arg Lys Arg Ser Gln Thr
                565                 570                 575

Val Thr Leu Val His Asp Ile Pro Asp Met Cys Val
            580                 585

<210>  65

<211>  658

<212>  DNA

<213>  Artificial

<220>

<221>  promoter

<222>  (16)..(269)

<223>

<220>

<221>  Polylinker

<222>  (271)..(296)

<223>

<220>

<221>  Intron

<222>  (298)..(352)

<223>

<220>
<221>  polyA_signal
<222>  (356)..(491)
<223>

<220>
<221>  polyA_signal
<222>  (492)..(540)
<223>

<220>
<221>  pause_signal
<222>  (541)..(632)
<223>

<400>  65
gtcgacgtta actttaagag gtccgcaagt agattgaaag ttcagtacgt ttttaacaat      60
agagcatttt cgaggcttgc gtcattctgt gtcaggctag cagtttataa gcgttgagga     120
tctagagctg ctgttcccgc gtctcgaatg ttctcggtgt ttaggggtta gcaatctgat     180
atgataataa tttgtgatga catcgatagt acaaaaaccc caattccggt cacatccacc     240
atctccgttt tctcccatct acacacaaca ttaattaagg taccactagt ggatccggtc     300
agtacaccac acagcccgac cgcgacgacc gcgtgctgac ttcgctttcc aggtcattca     360
tattccacgc ggtttcttac ctggtcacgg ctactcgttg atggattaaa ggtcttcgct     420
tgtttttctg tacgctgccc tggattgttg gaaactggtc ttttggtaat acatgaggtt     480
catctctgtt caataaaata tctttatttt cattacatct gtgtgttggt tttttgtgtg     540
aacatacgct ctccatcaaa acaaaacgaa acaaaacaaa ctagcaaaat aggctgtccc     600
cagtgcaagt gcaggtgcca gaacatttct ctatcgaaag gcctcctgca gggttaac       658

## Beschreibung

Die vorliegende Erfindung betrifft Pilzzellen und Pilze, die Hyaluronan synthetisieren, sowie Verfahren zur Herstellung solcher Pilze, als auch Verfahren zur Herstellung von Hyaluronan mit Hilfe dieser Pilzzellen oder Pilze. Weiterhin betrifft die vorliegende Erfindung die Verwendung von Pilzen zur Herstellung von Hyaluronan und Lebens- oder Futtermitteln, die Hyaluronan enthalten.

Hyaluronan ist ein in der Natur vorkommendes, unverzweigtes, lineares Mucopolysaccharid (Glucosaminoglucan), welches aus alternierenden Molekülen von Glucuronsäure und N-Acetyl-Glucosamin zusammengesetzt ist. Der Grundbaustein von Hyaluronan besteht aus dem Disaccharid Glucuronsäure-beta-1,3-N-Acetyl-Glucosamin. Diese Wiederholungseinheit ist im Hyaluronan durch beta-1,4-Verknüpfungen miteinander verbunden.
Im Bereich der Pharmazie wird häufig der Begriff Hyaluronsäure verwendet. Da Hyaluronan meist als Polyanion und nicht als freie Säure vorliegt, wird im Folgenden der Begriff Hyaluronan bevorzugt verwendet, wobei beide Molekülformen von der jeweiligen Bezeichnung als umfasst gelten.

Hyaluronan besitzt außergewöhnliche physico-chemische Eigenschaften, wie z.B. Eigenschaften von Polyelektrolyten, viskoelastische Eigenschaften, eine hohe Wasserbindekapazität, Eigenschaften der Gelbildung, die, neben weiteren Eigenschaften von Hyaluronan, in einem Übersichtsartikel von Lapcik et al. (1998, Chemical Reviews 98(8), 2663-2684) beschrieben sind. Die spezifischen Eigenschaften des Hyaluronans werden u.a durch das Molekulargewicht und die Molekulargewichtsverteilung bestimmt, welches das betreffende Hyaluronan aufweist.

Hyaluronan ist Bestandteil von extrazellulärem Bindegewebe und Körperflüssigkeiten von Vertebraten. Beim Menschen wird Hyaluronsäure von der Zellmembran aller Körperzellen, vor allem der mesenchymalen Zellen, synthetisiert und kommt ubiquitär im Körper vor, mit einer besonders hohen Konzentration in den Bindegeweben, der extrazellulären Matrix, der Nabelschnur, der Gelenkflüssigkeit, dem Knorpelgewebe, der Haut und dem Glaskörper des Auges (Bernhard Gebauer, 1998, Inaugural-Dissertation, Virchow-Klinikum Medizinische Fakultät Charité der Humboldt Universität zu Berlin; Fraser et al., 1997, Journal of Internal Medicine 242, 27-33). Kürzlich konnte Hyaluronan auch in tierischen nicht-Vertebraten Organismen (Mollusken) nachgewiesen werden (Volpi und Maccari, 2003, Biochimie 85, 619-625).
Weiterhin synthetisieren einige pathogene gram-positive Bakterien (Gruppe A und C *Streptococcus)* und gram-negative Bakterien *(Pasteurella)* Hyaluronan als Exopolysaccharide, die diese Bakterien vor dem Zugriff des Immunsystems ihres Wirtes bewahren, da Hyaluronan eine nicht immunogene Substanz darstellt.
Viren, welche einzellige und teilweise als Endosymbionten in *Paramecium* Spezies vorkommende Grünalgen der Gattung *Chlorella* infizieren, vermitteln den einzelligen Grünalgen nach Virusinfektion die Fähigkeit, Hyaluronan zu synthetisieren (Graves et al., 1999, Virology 257, 15-23). Dieses ist bisher das einzige Beispiel aus dem systematischen Reich der Pflanzen, für welches die Synthese von Hyaluronan nachgewiesen wurde.
Organismen aus dem Reich der Pilze (Mycota), die Hyaluronan synthetisieren, sind bisher nicht beschrieben. In WO 03 060063 wird zwar die Verwendung von *Saccharomyces cerevisiae* zur Herstellung einer rekombinant exprimierten Hyaluronansynthase beschrieben, jedoch nicht die Herstellung von Hyaluronan mit Hilfe transgener Hefen. Die Synthese von Hyaluronan mit Hilfe von gentechnisch veränderten *Saccharomyces cerevisiae* Zellen scheint sogar unmöglich, da ihnen offenbar das Enzym UDP-Glucose-6-Dehydrogenase, welches zur Herstellung eines Substrates der Hyaluronansynthase (UDP-Glucuronsäure) notwendig ist, fehlt (DeAngelis und Achyuthan, 1996, J Biological Chemistry 271 (39), 23657-23660).

Die Katalyse der Hyaluronansynthese wird durch ein einziges, Membran integriertes oder Membran assoziiertes Enzym, der Hyaluronansynthase bewerkstelligt. Die bisher untersuchten Hyaluronansynthasen können in zwei Gruppen eingeteilt werden: Hyaluronansynthasen der Klasse I und Hyaluronansynthasen der Klasse II (DeAngelis, 1999, CMLS, Cellular and Molecular Life Sciences 56, 670-682).
Eine weitere Unterscheidung der Hyaluronansynthasen aus Vertebraten erfolgt an Hand der identifizierten Isoenzyme. Die verschiedenen Isoenzyme werden in der Reihenfolge ihrer Identifizierung mit arabischen Nummern bezeichnet (z.B. hsHAS1, hsHAS2, hsHAS3).
Der Mechanismus des Transfers von synthetisierten Hyaluronanmoleküen über die Cytoplasmamembran hinweg in das die Zelle umgebende Medium, ist noch nicht vollständig geklärt. Frühere Hypothesen, gingen davon aus, dass der Transport über die Zellmembran hinweg von der Hyaluronansynthase selbst bewerkstelligt würde. Neuere Ergebnisse deuten jedoch darauf hin, dass der Transport von Hyaluronanmolekülen über die Cytoplasmamembran durch einen energieabhängigen Transport mittels dafür zuständiger Transportproteine stattfindet. So wurden durch Mutagenese *Streptococcus* Stämme erzeugt, bei welchen die Synthese eines aktiven Transportproteins inhibiert war. Diese Stämme synthetisierten weniger Hyaluronan als entsprechende wildtyp Bakterienstämme (Ouskova et al., 2004, Glycobiology 14(10), 931-938). In humanen Fibroblastenzellen konnte mit Hilfe von auf bekannte Transportproteine spezifisch inhibierend wirkenden Agentien gezeigt werden, dass sowohl die Menge an produziertem Hyaluronan, als auch die Aktivität von Hyaluronansynthasen reduziert werden kann (Prehm und Schumacher, 2004, Biochemical Pharmacology 68, 1401-1410).

Die außergewöhnlichen Eigenschaften des Hyaluronans eröffnen eine Vielfalt an Möglichkeiten für die Anwendung auf verschiedensten Gebieten, wie z.B. der Pharmazie, der Kosmetikindustrie, in der Lebens- und Futtermittel Herstellung, bei technischen Anwendungen (z.B: als Schmierstoffe) etc.. Die wichtigsten Anwendungen, bei denen Hyaluronan zurzeit verwendet wird, liegen im medizinischen und kosmetischen Bereich (siehe z.B. Lapcik et al., 1998, Chemical Reviews 98(8), 2663-2684, Goa und Benfield, 1994, Drugs 47(3), 536-566).
Im medizinischen Bereich werden Hyaluronan enthaltende Produkte derzeit zur intraartikulären Arthrosebehandlung sowie bei den Ophthalmika, die bei Operationen am Auge zum Einsatz kommen, verwendet. Derivastisiertes, so genanntes kreuz vernetztes (cross-linked) Hyaluronan wird zur Behandlung von Gelenkerkrankungen verwendet (Fong Chong et al., 2005), Appl Microbiol Biotechnol 66, 341-351).
Auch für die Behandlung von Gelenkerkrankungen bei Rennpferden wird Hyaluronan eingesetzt. Daneben ist Hyaluronsäure Bestandteil einiger Rhinologica, die z.B. in Form von Augentropfen und Nasalia der Befeuchtung trockener Schleimhäute dienen. Hyaluronan enthaltende Injektionslösungen werden als Analgetika und Antirheumatica verwendet. Hyaluronan oder derivatisiertes Hyaluronan enthaltende Auflagen werden bei der Wundheilung eingesetzt. Als Dermatika werden Hyaluronan enthaltende Gelimplantate zur Korrektur von Hautdeformationen in der plastischen Chirugie verwendet.
Für pharmakologische Anwendungen wird Hyaluronan mit einem hohen Molekulargewicht bevorzugt.
In der Schönheitsmedizin zählen Hyaluronpräparate zu den geeigneten Hautfüllmaterialien. Durch Einspritzen von Hyaluronan erreicht man für eine begrenzte Zeit die Glättung von Falten oder ein vergrößertes Lippenvolumen.
In kosmetischen Produkten, insbesondere in Hautcremes und Lotionen findet Hyaluronan wegen seiner hohen Wasserbindungskapazität häufig Anwendung als Feuchtigkeitsspender.
Weitere Anwendungsmöglichkeiten im medizinischen und kosmetischen Bereich, wie z.B. die Verwendung von Hyaluronan als Wirkstoffträger, der eine kontrollierte Abgabe des Wirkstoffes über einen langen Zeitraum gewährleistet, als Wirkstoffträger, der Wirkstoffe gezielt in das lymphatische System transportiert oder als Wirkstoff, der nach Applikation als Salbe einen über einen längeren Zeitraum andauernden Verbleib des Wirkstoffes in der Haut gewährleistet, werden bei Lapcik et al. (1998, Chemical Reviews 98(8), 2663-2684) beschrieben. Zur Verwendung von Hyaluronan Derivaten im medizinischen Bereich sind noch weitere Forschungsarbeiten notwendig, jedoch zeigen erste Ergebnisse bereits ein großes Potential (Lapcik et al. 1998, Chemical Reviews 98(8), 2663-2684
Weiterhin werden Hyaluronan enthaltende Präparate als so genannte Nutraceuticals (Nahrungsergänzungsmittel) vertrieben, die auch bei Tieren (z.B. Hunde, Pferde) zur Vorbeugung und Linderung von Arthrose angewandt werden.

Für gewerbliche Zwecke verwendetes Hyaluronan wird zurzeit aus tierischen Geweben (Hahnenkämmen) isoliert oder fermentativ mittels Bakterienkulturen hergestellt.
Ein Verfahren zur Isolierung von Hyaluronan aus Hahnenkämmen oder alternativ aus Nabelschnüren ist beschrieben in US 4,141,973. Neben Hyaluronan kommen in tierischen Geweben (z.B. Hahnenkämme, Nabelschnüre) noch weitere, mit Hyaluronan verwandte Mucopolysaccharide, wie Chondrotinsulfat, Dermatansulfat, Keratansulfat, Heparansulfat und Heparin vor. Tierische Organismen weisen weiterhin Proteine (Hyaladherine) auf, die spezifisch an Hyaluronan binden und für verschiedenste Funktionen im Organismus, wie z.B. dem Abbau von Hyaluronan in der Leber, der Funktion von Hyaluronan als Leitstruktur für die Zellwanderung, die Regulierung der Endocytose, die Verankerung von Hyaluronan an der Zelloberfläche oder die Ausbildung von Hyaluronan Netzwerken notwendig sind (Turley, 1991, Adv Drug Delivery Rev 7, 257 ff.; Laurent und Fraser, 1992, FASEB J. 6, 183 ff.; Stamenkovic und Aruffo, 1993, Methods Enzymol. 245, 195 ff; Knudson und Knudson, 1993, FASEB 7, 1233 ff.).

Die zur bakteriellen Produktion von Hyaluronan verwendeten *Streptococcus* Stämme gehören ausschließlich zu den pathogenen Bakterien. Diese Bakterien produzieren auch beim Kultivieren (pyrogene) Exotoxine und Haemolysine (Streptolysin, (insbesondere alpha- und beta-Haemolysin) (Kilian, M.: *Streptococcus* and *Enterococcus.* In: *Medical Microbiology.* Greenwood, D.; Slack, RCA; Peutherer, J.F. (Eds.). Kapitel 16. Churchill Livingstone, Edingburgh, UK: pp. 174-188, 2002, ISBN 0443070776), die ins Kulturmedium abgegeben werden. Dieses erschwert die Reinigung und Isolierung des Hyaluronans, welches mit Hilfe von *Streptococcus* Stämmen hergestellt wurde. Besonders für phamazeutische Anwendungen stellt das Vorliegen von Exotoxinen und Haemolysinen in den Präparaten ein Problem dar.
US 4,801,539 beschreibt die Herstellung von Hyaluronan durch Fermentation eines mutagenisierten Bakterienstammes *(Streptococcus zooedemicus).* Der verwendete mutagenisierte Bakterienstamm synthetisiert kein beta-Haemolysin mehr. Es wurde dabei eine Ausbeute von 3,6 g Hyaluronan pro Liter Kultur erreicht.

EP 0694616 beschreibt ein Verfahren zur Kultivierung von *Streptococcus zooedemicus* oder *Streptococcus equi,* worin unter den verwendeten Kulturbedingungen kein Streptolysin, aber erhöhte Mengen an Hyaluronan synthetisiert werden. Es wurde dabei eine Ausbeute von 3,5 g Hyaluronan pro Liter Kultur erreicht.
*Streptococcus* Stämme geben während der Kultur das Enzym Hyaluronidase in das Kulturmedium ab, was dazu führt, dass auch bei diesem Produktionssystem das Molekulargewicht während der Aufreinigung reduziert wird. Die Verwendung von Hyaluronidase negativen *Streptococcus* Stämmen oder von Produktionsverfahren, bei welchen die Produktion von Hyaluronidase während der Kultur inhibiert wird, zur Produktion von Hyaluronan, sind in US 4,782,046 beschrieben. Es wurde dabei eine Ausbeute von bis zu 2,5 g Hyaluronan pro Liter Kultur erreicht, wobei ein maximales mittleres Molekulargewicht von 3,8×10⁶ Da bei einer Molekulargewichtsverteilung von 2,4x10⁶ bis 4,0x10⁶ erhalten wurde.
US 20030175902 und WO 03 054163 beschreiben die Herstellung von Hyaluronan mit Hilfe der heterologen Expression einer Hyaluronansynthase aus *Streptococcus equisimilis* in *Bacillus subtilis.* Um die Produktion von ausreichenden Mengen an Hyaluronan zu erlangen ist neben der heterologen Expression einer Hyaluronansynthase auch die gleichzeitige Expression einer UDP-Glucose-Dehydrogenase in den *Bacillus* Zellen notwendig. Die absolute Menge an Hyaluronan, die bei der Produktion mit Hilfe von *Bacillus subtilis* erhalten wird, ist in US 20030175902 und WO 03 054163 nicht angegeben. Jedoch sind die erhaltenen Mengen an Hyaluronan nicht höher, als die Mengen, die mittels Fermentation von *Streptococcus* Stämmen erhalten werden. (Fong Chong et al., 2005), Appl Microbiol Biotechnol 66, 341-351). Es wurde bei der Produktion von Hyaluronan mit Hilfe von *Bacillus subtilis* ein maximales mittleres Molekulargewicht von ca. 4,2x10⁶ Da erhalten. Dieses mittlere Molekulargewicht wurde jedoch nur für den rekombinanten *Bacillus* Stamm erhalten, bei welchem ein Gen codierend das Hyaluronansynthase Gen aus *Streptococcus equisimilis* und das Gen codierend die UDP-Glucose-Dehydrogenase aus *Bacillus subtilis* unter Kontrolle des *amyQ* Promotors in das *Bacillus subtilis* Genom integriert war und gleichzeitig das *Bacillus subtilis* endogene cxpY Gen (codierend eine P450 Cytochromoxidase) inaktiviert war. Auch das Molekulargewicht des mit Hilfe von *Bacillus* Stämmen produzierten Hyaluronans konnte gegenüber dem von mittels *Streptococcus* Stämmen produzierten Hyaluronans nicht gesteigert werden (Fong Chong et al., 2005), Appl Microbiol Biotechnol 66, 341-351).

Die Produktion von Hyaluronan mit Hilfe der Fermentation von Bakterien Stämmen ist mit hohen Kosten verbunden, da die Bakterien in geschlossenen, sterilen Behältnissen, unter aufwendigen, kontrollierten Kulturbedingungen (siehe z.B. US 4,897,349) fermentiert werden müssen. Weiterhin ist die Menge an Hyaluronan, die mittels Fermentation von Bakterien Stämmen produziert werden kann, beschränkt durch die jeweils bestehenden Produktionsanlagen. Dabei ist auch zu berücksichtigen, dass Fermenter wegen physikalischer Gesetzmäßigkeiten nicht für übermäßig große Kulturvolumen gebaut werden können. Insbesondere ist hier die für eine effiziente Produktion gleichmäßige Durchmischung von durch von außen zugeführten Substanzen (z.B. essentielle Nährstoffquellen für Bakterien, Reagenzien zur pH Regulierung, Sauerstoff) mit dem Kulturmedium zu erwähnen, die in großen Fermentern, wenn überhaupt, nur mit großem technischen Aufwand gewährleistet werden kann.

Das Vorhandensein von anderen Mucopolysacchariden und von spezifisch an Hyaluronan bindenden Proteinen in tierischen Geweben macht die Aufreinigung von Hyaluronan aus tierischen Organismen aufwendig. Die Verwendung von Hyaluronan haltigen medizinischen Präparaten, die mit tierischen Proteinen verunreinigt sind, kann bei Patienten zu unerwünschten immunologischen Reaktionen des Körpers führen (US 4,141,973), insbesondere dann, wenn der Patient eine Allergie gegen tierische Proteine (z.B. Hühnereiweiß) aufweist. Weiterhin sind die Mengen (Ausbeuten) an Hyaluronan, die aus tierischen Geweben in ausreichender Qualität und Reinheit gewonnen werden können, gering (Hahnenkamm: 0,079% w/w, EP 0144019, US 4,782,046), was dazu führt, dass große Mengen an tierischen Geweben verarbeitet werden müssen. Ein weiteres Problem bei der Isolierung von Hyaluronan aus tierischen Geweben besteht darin, dass das Molekulargewicht des Hyaluronans während der Aufreinigung verringert wird, weil tierische Gewebe auch ein Hyaluronan abbauendes Enzym (Hyaluronidase) aufweisen.

*Streptococcus* Stämme produzieren neben den bereits erwähnten Hyaluronidasen und Exotoxinen auch Endotoxine, die, wenn in phamakologischen Produkten vorhanden, Risiken für die Gesundheit des Patienten darstellen. In einer wissenschaftlichen Studie wurde gezeigt, dass selbst auf dem Markt befindliche Hyaluronan enthaltende medizinische Präparate noch nachweisbare Mengen an bakteriellen Endotoxinen aufweisen (Dick et al., 2003, Eur J Opthalmol. 13(2), 176-184). Ein weiterer Nachteil des mit Hilfe von *Streptococcus* Stämmen produzierten Hyaluronans besteht darin, dass das isolierte Hyaluronan ein geringeres Molekulargewicht aufweist, als Hyaluronan, welches aus Hahnenkämmen isoliert wurde (Lapcik et al. 1998, Chemical Reviews 98(8), 2663-2684). US 20030134393 beschreibt die Verwendung eines *Streptococcus* Stammes zur Produktion von Hyaluronan, welcher eine besonders ausgeprägte Hyaluronan Kapsel (supercapsulated) synthetisiert. Das nach Fermentation isolierte Hyaluronan wies ein Molekulargewicht von 9,1x10⁶ Da auf. Jedoch betrug die Ausbeute lediglich 350 mg pro Liter.

Obwohl Hyaluronan außergewöhnliche Eigenschaften aufweist, wird es für technische Anwendungen wegen seiner geringen Verfügbarkeit und des hohen Preises bisher, wenn überhaupt, selten eingesetzt.

Es ist daher Aufgabe der vorliegenden Erfindung, Mittel und Verfahren zur Verfügung zu stellen, die es erlauben, Hyaluronan in ausreichender Menge und Qualität zur Verfügung zu stellen, sowie die Möglichkeit zu eröffnen, Hyaluronan auch für technische Anwendungen und Anwendungen im Lebensmittel- und Futtermittelbereich zur Verfügung zu stellen.

Diese Aufgabe wird durch die in den Ansprüchen bezeichneten Ausführungsformen gelöst.

Somit betrifft die vorliegende Erfindung Pilzzellen oder Pilze, bevorzugt Pilzzellen oder Pilze der systematischen Abteilung *Basidiomycota* dadurch gekennzeichnet, dass sie ein Nucleinsäuremolekül, codierend eine Hyaluronansynthase, aufweisen.

In einer bevorzugten Ausführungsform ist das Nucleinsäuremolekül, codierend eine Hyaluronansynthase in das Genom erfindungsgemäßer Pilzzellen oder erfindungsgemäßer Pilze integriert.

Gegenstand der vorliegenden Erfindung sind auch Pilzzellen oder Pilze, bevorzugt Pilzzellen oder Pilze der systematischen Abteilung *Basidiomycota,* die Hyaluronan synthetisieren. Eine bevorzugte Ausführungsform stellen erfindungsgemäße Pilzzellen oder erfindungsgemäße Pilze, die Hyaluronan synthetisieren, dar.

Hyaluronan kann aus erfindungsgemäßen Pilzzellen oder erfindungsgemäßen Pilzen isoliert werden. Erfindungsgemäße Pilzzellen oder erfindungsgemäße Pilze bieten daher gegenüber dem Stand der Technik den Vorteil, dass sie großflächig für die Hyaluronan Produktion mit geringem Aufwand kultiviert werden können. Dieses führt zu der Möglichkeit, Hyaluronan in ausreichender Menge auch für technische Anwendungen zur Verfügung zu stellen, bei welchen es wegen der geringen Verfügbarkeit und des hohen Preises zurzeit keine Verwendung findet.
Ein weiterer Vorteil der vorliegenden Erfindung liegt darin, dass Pilze auf Substraten kultiviert werden können, die relativ günstig sind und häufig als Abfallprodukte z.B. in der Land- und/oder Forstwirtschaft anfallen.

Unter dem Begriff "Hyaluronan" soll im Zusammenhang mit der vorliegenden Erfindung sowohl eine freie Säure (Hyaluronsäure), als auch die Polyanion Form eines linearen Glucosamins verstanden werden, die aus durch beta-1,4-Verknüpfungen miteinander verknüpften, mehreren Grundbausteinen des Disaccharids Glucuronsäure-beta-1,3-N-Acetyl-Glucosamin besteht.

Unter dem Begriff "Hyaluronansynthase" (EC 2.4.1.212) soll im Zusammenhang mit der vorliegenden Erfindung ein Protein verstanden werden, welches ausgehend von den Substraten UDP-Glucuronsäure (UDP-GlcA) und N-Acetyl-Glucosamin (UDP-GlcNAc) Hyaluronan synthetisiert. Die Katalyse der Hyaluronsynthese folgt dabei folgenden Reaktionsschemen:

nUDP-GlcA + nUDP-GlcNAc → [GlcA-beta-1,3-GlcNAc]ₙ + 2 nUDP

Nucleinsäuremoleküle und korrespondierende Proteinsequenzen, codierend Hyaluronansynthasen sind u.a. für folgende Organismen beschrieben: Kaninchen *(Oryctolagus cuniculus)* ocHas2 (EMBL AB055978.1, US 20030235893), ocHas3 (EMBL AB055979.1, US 20030235893); Pavian *(Papio anubis)* paHas1 (EMBL AY463695.1); Frosch *(Xenopus laevis)* xlHas1 (DG42) (EMBL M22249.1, US 20030235893), xlHas2 (EMBL AF168465.1), xlHas3 (EMBL AY302252.1); Mensch (*Homo sapiens*) hsHAS1 (EMBL D84424.1, US 20030235893), hsHAS2 (EMBL U54804.1, US 20030235893), hsHAS3 (EMBL AF232772.1, US 20030235893); Maus *(Mus musculus),* mmHas1 (EMBL D82964.1, US 20030235893), mmHAS2 (EMBL U52524.2, US 20030235893), mmHas3 (EMBL U86408.2, US 20030235893); Rind *(Bos taurus)* btHas2 (EMBL AJ004951.1, US 20030235893); Huhn *(Gallus gallus)* ggHas2 (EMBL AF106940.1, US 20030235893); Ratte *(Rattus norvegicus)* rnHas 1 (EMBL AB097568.1, Itano et al., 2004, J. Biol. Chem. 279(18) 18679-18678), rnHas2 (EMBL AF008201.1); rnHas 3 (NCBI NM_172319.1, Itano et al., 2004, J. Biol. Chem. 279(18) 18679-18678), Pferd *(Equus caballus)* ecHAS2 (EMBL AY056582.1, GI:23428486), Schwein (*Sus scrofa*) sscHAS2 (NCBI NM_214053.1, GI:47522921), sscHas 3 (EMBLAB159675), Zebrafisch (Danio *rerio)* brHas1 (EMBL AY437407), brHas2 (EMBL AF190742.1) brHas3 (EMBL AF190743.1); *Pasteurella multocida* pmHas (EMBL AF036004.2); *Streptococcus pyogenes* spHas (EMBL, L20853.1, L21187.1, US 6,455,304, US 20030235893); *Streptococcus equis* seHas (EMBL AF347022.1, AY173078.1), *Streptococcus uberis* suHasA (EMBL AJ242946.2, US 20030235893), *Streptococcus equisimilis* seqHas (EMBL AF023876.1, US 20030235893); *Sulfolobus solfataricus* ssHAS (US 20030235893), *Sulfolobus tokodaii* stHas (AP000988.1), *Paramecium bursaria Chlorella* Virus 1, cvHAS (EMBL U42580.3, PB42580, US 20030235893).

Unter dem Begriff "Genom" soll im Zusammenhang mit der vorliegenden Erfindung die Gesamtheit des in einer Pilzzelle vorliegenden Erbmaterials verstanden werden. Dem Fachmann ist bekannt, dass neben dem Zellkern auch andere Kompartimente (z.B. Mitochondrien) Erbmaterial enthalten.

Unter dem Begriff "stabil integriertes Nucleinsäuremolekül" soll im Zusammenhang mit der vorliegenden Erfindung die integration eines Nukieinsäuremoleküls in das Genom des Pilzes verstanden werden. Ein stabil integriertes Nucleinsäuremolekül zeichnet sich dadurch aus, dass es bei der Replikation des entsprechenden Integrationsortes zusammen mit den wirtseigenen, den Integrationsort flankierenden Nucleinsäuresequenzen vervielfältigt wird, so dass der Integrationsort in dem replizierten DNA Strang von den selben Nucleinsäuresequenzen umgeben ist, wie auf dem abgelesenen Strang, der als Matrize für die Replikation dient. Bevorzugt ist das Nucleinsäuremolekül stabil in das Kerngenom integriert.

Nachgewiesen werden kann die stabile Integration eines Nucleinsäuremoleküls in das Genom einer Pilzzelle oder eines Pilzes durch genetische und/oder molekularbiologische Methoden. Eine stabile Integration eines Nucleinsäuremoleküls in das Genom einer Pilzzelle oder in das Genom eines Pilzes zeichnet sich dadurch aus, dass das stabil integrierte Nucleinsäuremolekül bei der Nachkommenschaft, der besagtes Nucleinsäuremolekül vererbt wurde, in derselben genomischen Umgebung vorliegt wie in der Elterngeneration. Das Vorliegen einer stabilen Integration einer Nucleinsäuresequenz im Genom einer Pilzzelle oder im Genom eines Pilzes kann mit dem Fachmann bekannten Methoden, u.a. mit Hilfe der Southern-Blot Analyse der RFLP-Analyse (Restriction Fragment Length Polymorphism) (Nam et al., 1989, The Plant Cell 1, 699-705; Leister and Dean, 1993, The Plant Journal 4 (4), 745-750), auf PCR basierenden Methoden, wie z.B. die Analyse von amplifizierten Fragment Längenunterschieden (Amplified Fragment Length Polymorphism, AFLP) (Castiglioni et al., 1998, Genetics 149, 2039-2056; Meksem et al., 2001, Molecular Genetics and Genomics 265, 207-214; Meyer et al., 1998, Molecular and General Genetics 259 150-160) oder der Verwendung von mit Restriktionsendonucleasen geschnittenen amplifizierten Fragmenten (Cleaved Amplified Polymorphic Sequences, CAPS) (Konieczny und Ausubel, 1993, The Plant Journal 4, 403-410; Jarvis et al., 1994, Plant Molecular Biology 24, 685-687; Bachem et al., 1996, The Plant Journal 9 (5), 745-753) nachgewiesen werden.

In einer weiteren bevorzugten Ausführungsform betrifft die vorliegende Erfindung erfindungsgemäße Pilzzellen oder erfindungsgemäße Pilze, wobei das Hyaluronansynthase codierende Nucleinsäuremolekül dadurch gekennzeichnet ist, dass es eine Hyaluronansynthase Klasse I codiert.
Die bisher untersuchten Hyaluronansynthasen können in zwei Gruppen eingeteilt werden: Hyaluronansynthasen der Klasse I und Hyaluronansynthasen der Klasse II (DeAngelis, 1999, CMLS, Cellular and Molecular Life Sciences 56, 670-682). Diese Klassifizierung basiert im Wesentlichen auf biochemischen Untersuchungen des Reaktionsmechanismus und der Analyse der Aminosäuresequenzen, codierend die betreffenden Hyaluronansynthasen. Zur Klasse I gehören u.a. die Hyaluronansynthasen aus *Streptococcus pyogenes* (spHas), *Streptococcus equisimilis* (seHas), *Paramecium bursaria Chlorella* Virus 1 (cvHas) und die bekannten Hyaluronansynthasen der Vertebraten *(Xenopus laevis,* xlHas; *Homo sapiens;* hsHAS, *Mus musculus,* mmHas). Klasse I Hyaluronansynthasen weisen eine Aminosäuresequenz von 417 bis 588 Aminosäuren auf. Klasse I Hyaluronansynthasen sind Proteine, die in die Cytoplasmamembran integriert sind und weisen multiple (fünf bis sieben) Membran assoziierte Regionen auf. Die Verlängerung des Hyaluronans mit weiteren Molekülbausteinen erfolgt wahrscheinlich am reduzierenden Ende des Polymers. Geeignete Akzeptormoleküle, die von Hyaluronansynthasen der Klasse I verwendet werden, sind bisher nicht bekannt.
Die Hyaluronansynthase aus *Pasteurella* ist der bisher einzige bekannte Vertreter der Klasse II Hyaluronansynthasen. Seine Proteinsequenz weist 972 Aminosäuren auf. Es handelt sich um ein lösliches Protein, welches an seinem C-Terminus Aminosäuresequenzen enthält, die für die Lokalisation an der Cytoplasmamembran verantwortlich sind (Jing und DeAngelis, 2000, Glycobiology 10, 883-889). Wahrscheinlich findet die Interaktion über mit der Cytoplasmamembran assoziierte Moleküle statt. Die Synthese des Hyaluronans findet bei dem Enzym der Klasse II durch Verlängerung am nicht-reduzierenden Ende statt (DeAngelis, 1999, J. Biol. Chem 274, 26557-26562). Obwohl ein Akzeptormolekül für die Synthese von Hyaluronan durch das Klasse II Enzym nicht notwendig ist, konnte gezeigt werden, dass Hyaluronan Oligomere (DP4) als Akzeptor verwendet werden und die Syntheserate durch Zugabe der Akzeptoren gesteigert wird (DeAngelis, 1999, J. Biol. Chem 274, 26557-26562).

In einer vorzugsweisen Ausführungsform betrifft die vorliegende Erfindung erfindungsgemäße Pilzzellen oder erfindungsgemäße Pilze, wobei das Hyaluronansynthase codierende Nucleinsäuremolekül dadurch gekennzeichnet ist, dass es eine Hyaluronansynthase aus Vertebraten oder eine virale Hyaluronansynthase codiert. Bevorzugt codiert das Hyaluronansynthase codierende Nucleinsäuremolekül eine Hyaluronansynthase aus Amphibien oder eine Hyaluronansynthase eines Virus, der Algen infiziert.
Bezüglich eines Virus, der Algen infiziert, codiert das Hyaluronansynthase codierende Nucleinsäuremolekül besonders bevorzugt eine Hyaluronansynthase eines *Chlorella* infizierenden Virus, insbesondere bevorzugt eine Hyaluronansynthase eines *Paramecium bursaria Chlorella* Virus 1.
Bezüglich des Nucleinsäuremoleküls, welches eine Hyaluronansynthase aus Amphibien codiert, handelt es sich bevorzugt um eine Hyaluronansynthase eines Frosches, insbesondere um eine Hyaluronansynthase 1 aus *Xenopus laevis.*

In einer weiteren bevorzugten Ausführungsform betrifft die vorliegende Erfindung erfindungsgemäße Pilzzellen oder erfindungsgemäße Pilze, wobei das Hyaluronansynthase codierende Nucleinsäuremolekül dadurch gekennzeichnet, dass die Codons des Nucleinsäuremoleküls codierend eine Hyaluronansynthase verändert sind, im Vergleich zu den Codons des Nucleinsäuremoleküls, welches die Hyaluronansynthase des Ursprungsorganismus der Hyaluronansynthase codieren. Besonders bevorzugt sind die Codons der Hyaluronansynthase dahingehend verändert, dass sie an die Häufigkeit der Verwendung der Codons der Pilzzelle oder des Pilzes, in dessen Genom sie integriert sind oder werden, angepasst sind. Insbesondere bevorzugt sind die Codons der Hyaluronansynthase dahingehend verändert, dass die Nucleinsäuresequenz, codierend die Hyaluronansynthase, keine AT-reichen Regionen aufweisen. Dem Fachmann ist bekannt, dass AT-reiche Regionen, die innerhalb einer codierenden Nucleinsäureseqeunz vorliegen, bei der Expression in Pilzen zu einer verringerten Expressionsrate führen können (Scholtmeijer et al., 2001, Applied and Environmental Micobiology 67(1), 481-483).
Aminosäuren können auf Grund der Degeneration des genetischen Codes durch ein oder mehrere Codons codiert werden. Unterschiedliche Organismen verwenden die jeweils für eine Aminosäure codierenden Codons mit unterschiedlicher Häufigkeit. Das Anpassen der Codons einer codierenden Nucleinsäuresequenz an die Häufigkeit ihrer Verwendung in der Pilzzelle oder in dem Pilz, in dessen Genom die zu exprimierende Sequenz integriert werden soll, kann zu einer erhöhten Menge an translatiertem Protein und/oder zur Stabilität der betreffenden mRNA in den betreffenden Pilzzellen oder Pilzen beitragen. Der Fachmann kann die Häufigkeit der Verwendung von Codons in betreffenden Pilzzellen oder Pilzen ermitteln, indem er möglichst viele codierende Nucleinsäuresequenzen des betreffenden Organismus dahingehend untersucht, wie häufig bestimmte Codons für die Codierung einer bestimmten Aminosäure verwendet werden. Die Häufigkeit der Verwendung von Codons bestimmter Organismen ist dem Fachmann geläufig und kann mit Hilfe von Computerprogrammen einfach und schnell durchgeführt werden. Entsprechende Computerprogramme sind öffentlich zugänglich und werden u.a. im Internet frei zur Verfügung gestellt (z.B. http://gcua.schoedl.de/; http://www.kazusa.or.jp/codon/; http://www.entelechon.com/eng/cutanalysis.html).
Das Anpassen der Codons einer codierenden Nucleinsäuresequenz an die Häufigkeit ihrer Verwendung in der Pilzzelle oder in dem Pilz, in dessen Genom die zu exprimierende Sequenz integriert werden soll, kann durch *in vitro* Mutagenese oder bevorzugt durch Neusynthese der Gensequenz erfolgen. Methoden zur Neusynthese von Nucleinsäuresequenzen sind dem Fachmann bekannt. Eine Neusynthese kann z.B. dadurch erfolgen, dass zunächst einzelne Nucleinsäureoligonucleotide synthetisiert werden, diese mit zu ihnen komplementären Oligonucleotiden hybridisiert werden, so dass sie einen DNA-Doppelstrang ausbilden, bevor die einzelnen doppelsträngigen Oligonucleotide so miteinander ligiert werden, dass die gewünschte Nucleinsäuresequenz erhalten wird.

Die Neusynthese von Nucleinsäuresequenzen inklusive der Anpassung der Häufigkeit der Verwendung der Codons an einen bestimmten Zielorganismus kann auch als Auftrag an Firmen, die dieses als Dienstleistung anbieten, vergeben werden (z.B. Entelechon GmbH, Regensburg, Germany).

In einer weiteren bevorzugten Ausführungsform betrifft die vorliegende Erfindung erfindungsgemäße Pilzzellen oder erfindungsgemäße Pilze, wobei das Hyaluronansynthase codierende Nucleinsäuremolekül dadurch gekennzeichnet ist, dass es eine Hyaluronansynthase mit der unter SEQ ID NO 2, SEQ ID NO 4, SEQ ID NO 6, SEQ ID NO 8, SEQ ID NO 10, SEQ ID NO 12, SEQ ID NO 14, SEQ ID NO 16, SEQ ID NO 18, SEQ ID NO 20, SEQ ID NO 22, SEQ ID NO 24, SEQ ID NO 26, SEQ ID NO 28, SEQ ID NO 30, SEQ ID NO 32, SEQ ID NO 34, SEQ ID NO 36, SEQ ID NO 38, SEQ ID NO 40, SEQ ID NO 42, SEQ ID NO 44, SEQ ID NO 46, SEQ ID NO 48, SEQ ID NO 50, SEQ ID NO 52, SEQ ID NO 54, SEQ ID NO 56, SEQ ID NO 58, SEQ ID NO 60, SEQ ID NO 62 oder SEQ ID NO 64 dargestellten Aminosäuresequenz codiert. Besonders bevorzugt ist das Hyaluronansynthase codierende Nucleinsäuremolekül dadurch gekennzeichnet, dass es eine Hyaluronansynthase mit der unter SEQ ID NO 2 oder SEQ ID NO 42 dargestellten Aminosäuresequenz codiert.

In einer weiteren bevorzugten Ausführungsform betrifft die vorliegende Erfindung erfindungsgemäße Pilzzellen oder erfindungsgemäße Pilze, wobei das Hyaluronansynthase codierende Nucleinsäuremolekül dadurch gekennzeichnet ist, dass es eine unter SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 53, SEQ ID NO 55, SEQ ID NO 57, SEQ ID NO 59, SEQ ID NO 61 oder SEQ ID NO 63 dargestellte Nucleinsäuresequenz umfasst. Besonders bevorzugt ist das Hyaluronansynthase codierende Nucleinsäuremolekül dadurch gekennzeichnet, dass es eine unter SEQ ID NO 1 oder SEQ ID NO 41, insbesondere bevorzugt eine Hyaluronansynthase mit der unter SEQ ID NO 3 oder SEQ ID NO 63 dargestellten Nucleinsäuresequenz umfasst.

Das Plasmid IC 341-222, enthaltend ein synthetisches Nucleinsäuremolekül, das eine *Paramecium bursaria Chlorella* Virus Hyaluronansynthase codiert, wurde am 25.08.2004 unter der Nummer DSM16664 nach dem Budapester Vertrag hinterlegt bei der Deutschen Sammiung von Mikroorganismen und Zellkulturen GmbH, Mascheroder Weg 1b, 38124 Braunschweig, Deutschland. Die in SEQ ID NO 4 dargestellte Aminosäuresequenz kann von der codierenden Region der in Plasmid IC 341-222 integrierten Nucleinsäuresequenz abgeleitet werden und codiert für eine *Paramecium bursaria Chlorella* Virus Hyaluronansynthase.

Die vorliegende Erfindung betrifft daher auch erfindungsgemäße Pilzzellen oder erfindungsgemäße Pilze, wobei das Hyaluronansynthase codierende Nucleinsäuremolekül dadurch gekennzeichnet ist, dass es ein Protein codiert, dessen Aminosäuresequenz von der codierenden Region der im Plasmid DSM16664 inserierten Nucleinsäuresequenz abgeleitet werden kann.

Für die (stabile) Integration von Nucleinsäuren in eine Pilzzelle stehen verschiedene Techniken zur Verfügung (Übersicht Olmedo-Monfil et al., 2004, Methods in Molecular Biology Series 267, 297-314 und Casas-Flores et al., 2004, Methods in Molecular Biology Series 267, 315-326 in "Recombinant Gene Expression", 2nd Edition, 2004, Balbas und Lorenz eds, Humana Press, ISBN: 1-59259-774-2) Die dem Fachmann bekannten Methoden umfassen z.B. das Einbringen fremder DNA in Pilzzellen, d.h. von Nucleinsäuresequenzen, die zusätzlich zu den in der betreffenden Pilzzelle vorliegenden Nucleinsäuresequenzen eingebracht werden, mit Hilfe der Elektroporation von Protoplasten (WO 95 02691; *Agaricus bisporus;*Van de Rhee et al., 1996, Mol Gen Gent 250, 252-258, *Agaricus bisporus;* Noel und Labarere, 1994, Current Genetics 25(5), 432-437, *Agrocybe aegerita),* die Transformation von Protoplasten mit Hilfe von Polyethylenglycol (Ogawa et al., 1998, Appl Microbiol Biotechnol 49, 285-289, *Coprinus cinereus;* Shuren und Wessels, 1994, Curr Genet 26(2), 179-183, *Schizophytium commune),* die Transformation von Protoplasten mit Hilfe von Polyethylenglycol unter Zugabe von Restriktionsenzymen (REMI = Restriction enzyme-mediated DNA integration, Sato et al., 1998, Biosci. Biotechnol. Biochem. 62(12), 2346-2350, *Lentinus eodes),* die Transformation von Protoplasten mit Hilfe von Polyethylenglycol unter Zugabe von Kalziumchlorid (Yanai et al., 1996, Biosci. Biotechnol. Biochem. 60(3), 472-475, *Pleurotus ostreatus;* Honda et al., 2000, Curr Genet 37, 209.212, *Pleurotus ostreatus)* und die Transformation von Protopiasten, wobei die integration der DNA in das Pilzgenom mittels homologer Rekombination stattfindet (van de Rhee et al., 1996, Curr Genet 30, 166-173, *Agaricus bisporus).* Auch die Transformation von Pilzen mit Hilfe von Agrobacterium vermitteltem Gentransfer konnte erfolgreich durchgeführt werden (Godio et al., 2004 Curr Genet 46, 287-294, *Hypholoma sublateritium;* Mikosch et al., 2001, Curr Genet 39, 35-39, *Agaricus bisporus;* Chen et al., 2000, Applied and Environmental Microbiology 66(10), 4510-4513; US 2002 0016982; WO 02 00896, *Agaricus bisporus,* WO 98 45455; US 6,436,643, *Agaricus bisporus, Pleurotus ostreatus;* Hanif et al., 2002, Curr Genet 41(3), 183-188 *Suillus bovinus).*

Erfindungsgemäße Pilzzellen und erfindungsgemäße Pilze, die ein Nucleinsäuremolekül, codierend eine Hyaluronansynthase, stabil in ihr Genom integriert haben, lassen sich unter anderem daran erkennen, dass sie mindestens eine Kopie eines Nucleinsäuremoleküls, codierend eine Hyaluronansynthase, stabil integriert in ihr Genom enthalten. Dies lässt sich beispielsweise mit Hilfe einer Southern Blot-Analyse nachprüfen.
Weiterhin lassen sich die erfindungsgemäßen Pilzzellen und die erfindungsgemäßen Pilze vorzugsweise durch mindestens eines der folgenden Merkmale unterscheiden: Die erfindungsgemäßen Pilzzellen oder erfindungsgemäßen Pilze weisen Transkripte der stabil in das Genom integrierten Nucleinsäuremoleküle, codierend eine Hyaluronansynthase, auf. Diese lassen sich z. B. durch Northern-Blot-Analyse oder durch RT-PCR (Reverse Transcription Polymerase Chain Reaction) nachweisen. Vorzugsweise enthalten die erfindungsgemäßen Pilzzellen und die erfindungsgemäßen Pilze ein Protein, das durch stabil in das Genom integrierte Nucleinsäuremoleküle, codierend eine Hyaluronansynthase, codiert wird. Dies kann z. B. durch immunologische Methoden, insbesondere durch eine Western-Blot-Analyse nachgewiesen werden. Dass erfindungsgemäße Pilzzellen oder erfindungsgemäße Pilze eine aktive Hyaluronansynthase aufweisen, kann bevorzugt dadurch nachgewiesen werden, dass man die Aktivität einer Hyaluronansynthase in rekonstituierten Membranen, die aus von erfindungsgemäßen Pilzzellen oder erfindungsgemäßen Pilzen isolierten Membranenfraktionen hergestellt werden, nachweist. Eine für den Nachweis der Aktivität einer Hyaluronansynthase geeignete Methode ist beschrieben bei DeAngeiis und Achyuthan (1996, J Biol. Chem. 271(39), 23657-23660) beschrieben.

Methoden zur Herstellung von Antikörpern, die spezifisch mit einem bestimmten Protein reagieren, d.h. die spezifisch an besagtes Protein binden, sind dem Fachmann bekannt (siehe z.B. Lottspeich und Zorbas (Eds.), 1998, Bioanalytik, Spektrum akad, Verlag, Heidelberg, Berlin, ISBN 3-8274-0041-4). Die Herstellung solcher Antikörper wird von einigen Firmen (z.B. Eurogentec, Belgien) als Auftragsservice angeboten. Antikörper, die spezifisch Hyaluronansynthasen erkennen sind z.B. beschrieben in Jacobson et al., 2000, Biochem J. 348, 29-35.

Erfindungsgemäße Pilzzellen oder erfindungsgemäße Pilze, die Hyaluronan synthetisieren, lassen sich auch dadurch nachweisen, dass man das von ihnen synthetisierte Hyaluronan isoliert und dessen Struktur nachweist.
Da Gewebe von Pilzen den Vorteil aufweisen, dass sie keine Hyaluronidasen enthalten, kann zum Nachweis des Vorliegens von Hyaluronan in erfindungsgemäßen Pilzzellen oder erfindungsgemäßen Pilzen eine einfache und schnelle Isolierungsmethode verwendet werden. Dazu wird zu dem zu untersuchenden Pilzgewebe Wasser hinzu gegeben, bevor das Pilzgewebe mechanisch (z.B. unter zu Hilfenahme einer Kugelmühle, eines Warring Blendors etc.) zerkleinert wird. Anschließend kann bei Bedarf erneut Wasser zu der Suspension hinzu gegeben werden, bevor Zelltrümmer und Wasser unlösliche Bestandteile durch Zentrifugation abgetrennt werden. Der Nachweis des Vorliegens von Hyaluronan kann anschließend im nach Zentrifugation erhaltenen Überstand z.B. mittels eines spezifisch an Hyaluronan bindenden Proteins erfolgen. Ein Verfahren zum Nachweis von Hyaluronan mit Hilfe eines spezifisch an Hyaluronan bindenden Proteins ist z.B. in US 5,019,498 beschrieben. Testkits, (z.B. das Hyaluronic Acid (HA) Test Kit der Firma Corgenix, Inc., Colorado, USA, Prod. Nr. 029-001) zur Durchführung der in US 5,019,498 beschrieben Methode, sind käuflich erwerbbar (z.B. das Hyaluronic Acid (HA) Test Kit der Firma Corgenix, Inc., Colorado, USA, Prod. Nr. 029-001; siehe auch Allgemeine Methoden Punkt 6.). Parallel dazu kann ein Aliquot des erhaltenen Überstandes der Zentrifugation zunächst mit einer Hyaluronidase verdaut werden, bevor der Nachweis des Vorliegens von Hyaluronan mit Hilfe des spezifisch an Hyaluronan bindenden Proteins, wie oben beschrieben, erfolgt. Durch die Einwirkung der Hyaluronidase in dem Parallelansatz wird das darin vorliegende Hyaluronan abgebaut, so dass nach vollständigem Verdau keine signifikanten Mengen an Hyaluronan mehr nachweisbar sind.
Weiterhin kann der Nachweis des Vorliegens von Hyaluronan im Überstand der Zentrifugation auch mit anderen Analysemethoden, wie z.B. der IR-, NMR- oder Massen-Spektroskopie, erfolgen.

Ein weiterer Gegenstand der vorliegenden Erfindung betrifft erfindungsgemäße Pilzzellen oder erfindungsgemäße Pilze, dadurch gekennzeichnet, dass das stabil in das Genom der Pilzzelle oder des Pilzes integrierte Nucleinsäuremolekül, codierend eine Hyaluronansynthase, mit regulatorischen Elementen verknüpft ist, die die Transkription in Pilzzellen initiieren (Promotoren). In einer bevorzugten Ausführungsform handelt es sich bei den Promotoren um gewebespezifische Promotoren, besonders bevorzugt sind Promotoren, die die Initiation der Transkription spezifisch in Fruchtkörpern initiieren.

Zur Expression von erfindungsgemäßen Nucleinsäuremolekülen, die eine Hyaluronansynthase codieren, werden diese vorzugsweise mit regulatorischen DNA-Sequenzen verknüpft, die die Transkription in Pilzzellen gewährleisten. Hierzu zählen insbesondere Promotoren. Generell kommt für die Expression jeder in Pilzzellen aktive Promotor in Frage.
Der Promotor kann dabei so gewählt sein, dass die Expression konstitutiv erfolgt oder nur in einem bestimmten Gewebe, zu einem bestimmten Zeitpunkt der Pilzentwicklung oder zu einem durch äußere Einflüsse determinierten Zeitpunkt.

Sowohl in Bezug auf den Pilz als auch in Bezug auf das Nucleinsäuremolekül, codierend eine Hyaluronansynthase, kann der Promotor homolog oder heterolog sein.
Für die Initiation der Transkription in Pilzzellen geeignete Promotoren sind z.B. der Promotor des Glycerinaldehyd-3-Phosphat-Dehydrogenase Gens aus *Agaricus bisporus* (Van de Rhee et al., 1996, Mol Gen Genet 250, 252-258; Chen et al., 2000; Applied and Environmental Microbiology 66(10), 4510-4513; US 2002 0016982) und aus *Lentinus edodes* (Hirano et al., 2000, Mol Gen Genet 263, 1047-1052), der Promotor des *priA* Gens aus *Lentinus edodes* (Yanai et al., 1996, Biosci. Biotech. Biochem 60(3), 472-475; Kajiwara et al., 1992, Gene 114(2), 173-178) ,oder der Promotor des *ras* Gens aus *Lentinus edodes* (Yanai et al., 1996, Biosci. Biotech. Biochem 60(3), 472-475) Auch für den 35S Promotor des Blumenkohlmosaikvirus, der für die Expression von fremden Nucleinsäuren in Pflanzen häufig Verwendung findet, konnte die Initiation der Transkription in Plizzellen nachgewiesen werden (Sun et al., 2002, Molecular Biotechnology 20(3), 239-244). Eine Übersicht über weitere Promotoren, die in Pilzzellen die Innitiation der Transkription vermitteln, ist in Burns et al. (2005, Fungal Gentics and Biology, in Press, prebubished online at www.sciencedirect.com, 05.01.2005, doi:10.1016/j.fgb.2004.11.005) beschrieben.
Ein Beispiel für einen in Pilzen durch äußere Einflüsse induzierbaren Promotor ist der Promotor des Manganperoxidase 1 *(mnp* 1) Gens aus *Phanerochaete chrysosporium* (Ma et al., Applied and Environmental Microbiology 67(2), 948-955; Godfrey et al., 1990, Gene 93(1), 119-124).
Beispiele für fruchtkörperspezifische Promotoren sind z.B. Promotoren der Hydrophobin A (*hypA*)*,* Hydrophobin B (*hypB*) und Hydrophobin C (*hypC*) Gene aus *Agararicus bisporus* (De Groot et al., 1999, Microbiology 145, 1105-1113), des Promotors des Hydrophobin Gens *(fvh1)* aus *Flammulina velutipes* (Ando et al., 2001, Curr Genet 39(3), 190-197), die Promotoren der Hydrophobin Gene Sc1 und *Sc4* aus *Schizophyllum commune* (Schuren und Wessels, 1990, Gene 90(2), 199-205), die Promotoren der Hydrophobin Gene fbh1 und poh1 aus Pleurotus ostreatus (Penas et al., 2004, Mycologica 96(1), 75-82), die Promotoren des *abst1* Gens (auch als *mag1* Gen bezeichnet) aus *Agaricus bisporus* (WO 04 039985, EMBL Acc. No.: AJ299400.1) oder der Promotor des *rafe* Gens aus *Agaricus bisporus* (WO 04 039985, EMBL Acc. No.: AJ853495.1). Weitere Gene, die fruchtköperspezifische Promotoren aufweisen, und Möglichkeiten zur deren Identifizierung und Isolierung sind z.B. beschrieben in De Groot et al. (1997, Microbiology 143, 1993-2001) oder Hirano et al. (2004, Biosci. Biotechnol. Biochem. 68, 468-472.

Unter dem Begriff "gewebespezifisch" soll im Zusammenhang mit der vorliegenden Erfindung die überwiegende Beschränkung einer Ausprägung (z.B. die Initiation der Transkription) auf ein bestimmtes Gewebe verstanden werden. Insbesondere sind bei Pilzen der Abteilung *Basidiomycota* die folgenden Gewebe zu unterscheiden: Mycel und Fruchtkörper, wobei das Mycel weiter unterteilt werden kann in ein haploides und ein dikaryontisches Mycel und der Fruchtköper weiter unterteilt werden kann in einen Stamm, eine Kappe, Lamellen und Basidien.

Ferner kann eine Terminationssequenz vorhanden sein, die der Addition eines Poly-A-Schwanzes an das Transkript dient.

Unter dem Begriff "Terminationssequenz" sollen im Zusammenhang mit der vorliegenden Erfindung Nucleinsäuresequenzen, die eine oder mehrere Erkennungssequenz(en) für die Polyadenylierung eines RNA Transkriptes (Polyadenylierungssignal) und/oder eine oder mehrere Nucleinsäuresequenz(en), die die Funktion eines Trankriptionsblockers (Pause Signal) aufweisen, verstanden werden.

Dem Polyadenylierungssignal wird eine Funktion bei der Stabilisierung der Transkripte beigemessen. Weiterhin dienen Polyadenylierungssignale der Vermeidung von zu langen Transkripten, d.h. es wird verhindert, dass Transkripte eines ersten Genes entstehen, die zusätzlich Sequenzen eines zweiten Genes (z.B. Promotorsequenzen) enthalten (Eggermont und Proudfoot, 1993, EMBO J. 12(5), 2539-2548). Derartige Elemente sind in der Literatur beschrieben (siehe z.B. Schuren und Wessels, 1990, Gene 90, 199-205; Penas et al., 2004, Mycologica 96(1), 75-82;,Ando et al., 2001, Curr Genet 39(2), 190-197Sirand-Pugnet et al., 2003, Curr Genet 44, 124-131; Sirand-Pugnet und Labarere, 2002, Curr Genet 41, 31-42; Yanai et al., 1996, Biosci. Biotech. Biochem. 60(3), 472-475; Godio et al., 2004, Curr Genet 46, 287-294;Hirano et al., 2000, Mol Gen Genet 263, 1047-1052; Chen et al.,2000, Applied and Environmental Microbiology 66(10), 4510-4513).

Dem Terminationsblocker wird die Funktion der effizienten Verwendung von Polyadenylierungssignalen zugeschrieben, d.h. dass das vorliegen eines Transkriptionsbiockers dazu führt, dass ein erstes schwächeres Polyadenylierungssignal verwendet wird, wenn zusätzlich ein zweites stärkeres Polyadenylierungssignal stromabwärts (downstream) des Transkriptionsblockers vorhanden ist (Enriquez-Harris et al., 1991, EMBO J. 10(7), 1833-1842).

Zur Stabilisierung der transkribierten RNA kann es notwendig sein, dass das zu transkribierende fremde Nucleinsäuremolekül Intronsequenzen aufweist (Lugones et al., 1999, Molecular Microbiology 32(4), 681-689; Scholtmeijer et al., 2001, Applied and Environmental Microbiology 67(1), 481-483; Ma et al., Applied and Environmental Microbiology 67(2), 948-955, Burns et al. (2005, Fungal Gentics and Biology, in Press, prebubished online at www.sciencedirect.com, 05.01.2005, doi:10.1016/j.fgb.2004.11.005). Es können daher auch Intronsequenzen zwischen dem Promotor und der codierenden Region und/oder der Terminationssequenz und der codierenden Region und/oder innerhalb der codierenden Region des fremden Nucleinsäuremoleküls vorhanden sein. Es kann sich dabei um eine einzelne Intronsequenz oder um mehrere Intronsequenzen handeln. Bevorzugt werden Introns aus entsprechenden natürlich vorkommenden Pilzgenen verwendet.

Ein weiterer Gegenstand der vorliegenden Erfindung betrifft erfindungsgemäße Pilzzellen oder erfindungsgemäße Pilze, dadurch gekennzeichnet, dass das stabil in das Genom der Pilzzelle oder des Pilzes integrierte Nucleinsäuremolekül, umfassend Sequenzen, codierend eine Hyaluronansynthase, Intronsequenzen aufweist. Bevorzugt handelt es sich dabei um Intronssequenzen, die natürlicherweise in Genen von Pilzzellen vorkommen.

Pilzzellen können mit dem Fachmann bekannten Methoden transformiert und/oder vermehrt werden.

Ein weiterer Gegenstand der vorliegenden Erfindung betrifft daher Pilzzellen, die von einer erfindungsgemäßen Pilzzelle oder einem erfindugsgemäßen Pilz abstammen, dadurch gekennzeichnet, dass sie das Nucleinsäuremolekül, codierend eine Hyaluronansynthase aufweisen, welches ihre Mutterzelle, von der sie abstammen, aufweist.

Dem Fachmann ist bekannt, dass Pilze während ihres Lebenszyklus verschiedene Entwicklungsphasen durchlaufen. Pilze der systematischen Abteilung *Basidiomycota* weisen dabei im Allgemeinen die morphologisch unterscheidbaren Gewebe eines Mycels und eines Fruchtkörpers auf. Prinzipiell kann das aus Hyphen gebildete Mycel dabei in unterschiedlichen genetischen Varianten (haploid oder dikaryontisch) vorliegen. Die dikaryontische Lebensphase geht normalerweise durch Verschmelzung bezüglich des Paarungsfaktors genetisch unterschiedlicher haploider Mycelzellen hervor (Somatogamie). Sowohl das haploide Mycel, als auch das als Dikayon vorliegende Mycel sind dabei unbegrenzt wachstumsfähig, d.h. sie sind vegetativ vermehrungsfähig.
Der von dem dikaryontischen Mycel gebildete Fruchtkörper besteht größtenteils aus dikaryontischen Zellen. In den Basidien, die spezialisierte Zellen des Fruchtkörpers darstellen, findet normalerweise eine Verschmelzung der betreffenden Kerne (Karyogamie), gefolgt von einer Meiose statt (sexuelle Vermehrung). Die daraus hervorgehenden haploiden Kerne stellen dass Genom der anschließend gebildeten Basidiosporen dar, die nach Auskeimung auf geeignetem Substrat wieder ein haploides Mycel beilden.
Abweichungen von diesem grundlegenden Lebenszyklus sind bekannt. So enthalten z:B. die Basidiosporen aller kultivierter *Agaricus bisporus* Vertreter meistens jeweils zwei haploide Kerne, die anschließend zu Hyphen auskeimen, welche direkt ein dikaryontisches Mycel bilden. Bei Vorliegen von dikaryontischen Mycelien können ohne vorherige Verschmelzung mit einem zweiten Mycel direkt Fruchtkörper gebildet werden. In seltenen Fällen können jedoch auch einkernige Basidiosporen von *Agaricus bisporus* gebildet werden. Außerdem kommt es bei *Agaricus bisporus* Vertretern häufig vor, dass die das Mycel oder den Fruchtkörper bildenden Zellen vielkernig sind, d.h. dass sie mehr als zwei Kerne aufweisen (Kothe, 2001, Appl Microbiol Biotechnol 56, 602-612).

Wie bereits ausgeführt, können erfindungsgemäße Pilzzellen zu Pilzen regeneriert werden.

Ein weiterer Gegenstand der vorliegenden Erfindung betrifft daher Pilze, enthaltend erfindungsgemäße Pilzzellen.

Unter dem Bergriff "Pilz" sollen im Zusammenhang mit der vorliegenden Erfindung alle Erscheinungsformen der verschiedenen Entwicklungsphasen eines betreffenden Pilzes verstanden werden. Es kann sich dabei um Mycelien, die haploide, dikaryontische oder vielkernige Pilzzellen aufweisen und/oder Fruchtkörper, die dikaryontische oder vielkernige Pilzzellen aufwiesen und/oder um Basidiosporen handeln.

Die vorliegende Erfindung betrifft daher auch Pilzmycelein, die haploide, dikaryontische oder vielkernige erfindungsgemäße Pilzzellen aufweisen und/oder Fruchtkörper, die dikaryontische oder vielkernige erfindungsgemäße Pilzzellen aufweisen.

Vorzugsweise handelt es sich bei erfindungsgemäßen Pilzzellen oder erfindungsgemäßen Pilzen um Pilzzellen oder Pilze der systematischen Klasse *Basidiomycetes* (Ständerpilze), bevorzugt um Pilze der systematischen Unterklasse *Hymenomycotidae* (Hutpilze, in neuerer Nomenklatur auch als *Agaricomycotidae* bezeichnet), besonders bevorzugt um Pilze der systematischen Ordnung *Agaricales* (Blätterpilze), insbesondere bevorzugt um Pilze der systematischen Familie *Agaricaceae* (Champignonartige). Bei einer im speziellen bevorzugten Ausführungsform handelt es sich bei den erfindungsgemäßen Pilzzellen oder erfindungsgemäßen Pilzen um Pilzzellen oder Pilze der Gattung *Agaricus* insbesondere speziell bevorzugt handelt es sich um Pilzzellen oder Pilze der Spezies *Agaricus bisporus.*
Die im Zusammenhang mit der vorliegenden Erfindung verwendete Systematik der Pilze basiert auf neueren Kenntnissen und richtet sich nach Ainsworth and Bisby's *Dictionary of the Fungi* (9th Edition, Utrecht NL, 2001, ISBN 085199377X).

Wie bereits ausgeführt, können Pilze sowohl sexuell, als auch vegetativ mit dem Fachmann bekannten Methoden vermehrt werden.

Die vorliegende Erfindung betrifft daher auch Vermehrungsmaterial eines erfindungsgemäßen Pilzes, dadurch gekennzeichnet, dass es das Nucleinsäuremolekül, codierend eine Hyaluronansynthase aufweist, welches der erfindungsgemäße Pilz aufweist.

Der Begriff "Vermehrungsmaterial" umfasst dabei jene Bestandteile des Pilzes, die geeignet sind zur Erzeugung von Nachkommen auf vegetativem oder sexuellem Weg. Für die vegetative Vermehrung eignen sich Pilzmycelein, die haploide, dikaryontische oder vielkernige erfindungsgemäße Pilzzellen aufweisen oder Zellen von Fruchtkörpern, die dikaryontische oder vielkernige erfindungsgemäße Pilzzellen aufweisen. Sexuelles Vermehrungsmaterial betrifft Sporen (Bsidiosporen), die einen oder mehrere Kerne (Nuclei) aufweisen.

Die vorliegende Erfindung betrifft auch verarbeitbare oder konsumierbare Teile von erfindungsgemäßen Pilzen, enthaltend erfindungsgemäße Pilzzellen und/oder enthaltend Hyaluronan.

Unter dem Begriff "verarbeitbare Teile" sollen im Zusammenhang mit der vorliegenden Erfindung Gewebe von Pilzen verstanden werden, die Verwendung in der Herstellung von Nahrungs- oder Futtermitteln finden, die als Rohstoffquelle für industrielle Prozesse, als Rohstoffquelle für die Herstellung pharmazeutischer oder als Rohstoffquelle für die Herstellung kosmetischer Produkte eingesetzt werden. Bevorzugte verarbeitbare Teile stellen Fruchtkörper erfindungsgemäßer Pilze dar.

Unter dem Begriff "konsumierbare Teile" sollen im Zusammenhang mit der vorliegenden Erfindung Gewebe von Pilzen verstanden werden, die dem Menschen als Nahrungsmittel dienen oder als Tierfutter verwendet werden. Bevorzugte konsumierbare Teile stellen Fruchtkörper erfindungsgemäßer Pilze dar.

Bevorzugt betrifft die vorliegende Erfindung Vermehrungsmateriai, verarbeitbare oder konsumierbare Teile von Pilzen, enthaltend Hyaluronan. Besonders bevorzugt handelt es sich dabei um Vermehrungsmaterial, verarbeitbare oder konsumierbare Teile von Pilzen, die Hyaluronan synthetisieren.

Ein weiterer Vorteil der vorliegenden Erfindung liegt darin, dass verarbeitbare Teile oder konsumierbare Teile von erfindungsgemäßen Pilzen Hyaluronan enthalten. Diese eignen sich daher nicht nur als Rohmaterial, aus dem Hyaluronan isoliert werden kann, sondern auch als direkte Verwendung als Nahrungs-/Futtermittel oder zur Herstellung von Nahrungs-/Futtermitteln, welche einen vorbeugenden oder therapeutischen Charakter aufweisen (z.B. zur Vorbeugung gegen Osteoarthritis, US 6,607,745). Es ist also z.B. nicht mehr notwendig, so genannten Nutraceuticals fermentativ hergestelltes oder aus tierischen Geweben isoliertes Hyaluronan zuzusetzen, wenn erfindungsgemäße Pilze oder Teile von erfindungsgemäßen Pilzen für die Herstellung von Nutraceuticals verwendet oder direkt als Nahrungs-/Futtermittel eingesetzt werden. Durch die hohe Wasserbindungskapazität von Hyaluronan weisen verarbeitbare Teile oder konsumierbare Teile von erfindungsgemäßen Pilzen weiterhin den Vorteil auf, dass bei der Herstellung von verfestigten Nahrungs-/Futtermitteln weniger Dickungsmittel eingesetzt werden müssen. So kann für die Herstellung von getrockneten so genannten Fertignahrungsmitteln (Convenience), wie z.B. Tütensuppen, die Verwendung erfindungsgemäßer verarbeitbarer Teile oder konsumierbarer Teile von erfindungsgemäßen Pilzen dazu führen, dass weniger Dickungsmittel (z.B. Stärke) zugegeben werden müssen. Dieses führt z.B. zu geringeren Kosten bei der Herstellung solcher Produkte.

Ein weiterer Gegenstand der vorliegenden Erfindung ist ein Verfahren zur Herstellung eines Pilzes, der Hyaluronan synthetisiert, worin
a) ein Nucleinsäuremolekül, codierend eine Hyaluronansynthase in das Genom einer Pilzzelle integriert wird
b) aus Pilzzellen von Schritt a) ein Pilz regeneriert wird; und
c) gegebenenfalls weitere Pilze mit Hilfe der Pilze nach Schritt b) erzeugt werden.

Die Regeneration der Pilze gemäß Schritt b) kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pilze gemäß Schritt c) des erfindungsgemäßen Verfahrens zur Herstellung eines Pilzes kann z.B. erfolgen durch vegetative Vermehrung oder durch sexuelle Vermehrung. Die sexuelle Vermehrung findet dabei vorzugsweise kontrolliert statt, d.h. es werden ausgewählte Pilze mit bestimmten Eigenschaften miteinander gekreuzt und vermehrt. Die Auswahl erfolgt dabei in der Weise, dass die weiteren Pilze, die nach Schritt c) erzeugt werden, das ins Genom des Pilzes integrierte Nucleinsäuremolekül, codierend eine Hyaluronansynthase, aufweisen und/oder dass sie Hyaluronan synthetisieren.

In einer bevorzugten Ausführungsform erfindungsgemäßer Verfahren zur Herstellung eines Pilzes werden in einem zusätzlichen Verfahrensschritt b)-1 folgend auf Verfahrensschritt b) Pilze selektiert, die ein Nucleinsäuremolekül, codierend eine Hyaluronansynthase stabil in ihr Genom integriert haben.

In einer weiteren bevorzugten Ausführungsform enthalten die erfindungsgemäßen Verfahren zur Herstellung eines Pilzes einen auf die Verfahrensschritte b) oder b)-1 folgenden Verfahrensschritt, worin Pilze identifiziert werden, die Hyaluronan synthetisieren.

In weiteren Ausführungsformen betrifft die vorliegende Erfindung erfindungsgemäße Verfahren zur Herstellung eines Pilzes, worin das Nucleinsäuremolekül, codierend eine Hyaluronansynthase in Schritt a) ausgesucht ist aus der Gruppe bestehend aus:
a) Nucleinsäuremoleküle, dadurch gekennzeichnet, dass sie eine Hyaluronansynthase Klasse I codieren,
b) Nucleinsäuremoleküle, dadurch gekennzeichnet, dass sie eine vertebraten Hyaluronansynthase oder eine virale Hyaluronansynthase codieren,
c) Nucleinsäuremoleküle, dadurch gekennzeichnet, dass sie eine Hyaluronansynthase aus Amphibien oder eine Hyaluronansynthase eines Virus, der Algen infiziert codieren,
d) Nucleinsäuremoleküle, dadurch gekennzeichnet, dass sie eine Hyaluronansynthase eines *Chlorella* infizierenden Virus oder eine Hyaluronansynthase aus einem Frosch codieren,
e) Nucleinsäuremoleküle, dadurch gekennzeichnet, dass sie eine Hyaluronansynthase eines *Paramecium bursaria Chlorella* Virus 1 oder eine Hyaluronansynthase 1 aus *Xenopus laevis* codieren,
f) Nucleinsäuremoleküle, dadurch gekennzeichnet, dass die Codons des Nucleinsäuremoleküls codierend eine Hyaluronansynthase verändert sind, im Vergleich zu den Codons des Nucleinsäuremoleküls, welches die Hyaluronansynthase des Ursprungsorganismus der Hyaluronansynthase codieren,
g) Nucleinsäuremoleküle, dadurch gekennzeichnet, dass die Codons der Hyaluronansynthase dahingehend verändert sind, dass sie an die Häufigkeit der Verwendung der Codons der Pilzzelle, in dessen Genom sie integriert werden oder sind, angepasst sind,
h) Nucleinsäuremoleküle, dadurch gekennzeichnet, dass sie eine Hyaluronansynthase mit der unter SEQ ID NO 2, SEQ ID NO 4, SEQ ID NO 6, SEQ ID NO 8, SEQ ID NO 10, SEQ ID NO 12, SEQ ID NO 14, SEQ ID NO 16, SEQ ID NO 18, SEQ ID NO 20, SEQ ID NO 22, SEQ ID NO 24, SEQ ID NO 26, SEQ ID NO 28, SEQ ID NO 30, SEQ ID NO 32, SEQ ID NO 34, SEQ ID NO 36, SEQ ID NO 38, SEQ ID NO 40, SEQ ID NO 42, SEQ ID NO 44, SEQ ID NO 46, SEQ ID NO 48, SEQ ID NO 50, SEQ ID NO 52, SEQ ID NO 54, SEQ ID NO 56, SEQ ID NO 58, SEQ ID NO 60, SEQ ID NO 62 oder SEQ ID NO 64 dargestellten Aminosäuresequenz codieren,
i) Nucleinsäuremoleküle, dadurch gekennzeichnet , dass sie ein Protein codieren, dessen Aminosäuresequenz von der codierenden Region der im Plasmid DSM16664 inserierten Nucleinsäuresequenz abgeleitet werden kann,
j) Nucleinsäuremoleküle, die eine unter SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 53 SEQ ID NO 55, SEQ ID NO 57, SEQ ID NO 59, SEQ ID NO 61 oder SEQ ID NO 63 dargestellte Nucleinsäuresequenz umfassen,
k) Nucleinsäuremoleküle, die die in Plasmid DSM16664 inserierte Nucleinsäuresequenz umfassen,
l) Nucleinsäuremoleküle, codierend eine Hyaluronansynthase, wobei die Hyaluronansynthase codierenden Nucleinsäuresequenzen mit regulatorischen Elementen (Promotor) verknüpft sind, die die Transkription in Pilzzellen initiieren, oder
m) Nucleinsäuremoleküle, nach j), wobei die Promotoren gewebespezifische Promotoren, besonders bevorzugt Promotoren, die die Initiation der Transkription spezifisch in Fruchtkörpern von Pilzen initiieren, darstellen.

In einer weiteren Ausführungsform dienen erfindungsgemäße Verfahren zur Herstellung eines Pilzes, der Herstellung eines erfindungsgemäßen Pilzes.

Pilze erhältlich nach erfindungsgemäßen Verfahren zur Herstellung eines Pilzes, sind ebenfalls Gegenstand der vorliegenden Erfindung.

Weiterhin sind Verfahren zur Herstellung von Hyaluronan Gegenstand der vorliegenden Erfindung, umfassend den Schritt der Extraktion von Hyaluronan aus erfindungsgemäßen Pilzzellen, aus erfindungsgemäßen Pilzen, aus erfindungsgemäßem Vermehrungsmaterial, aus erfindungsgemäßen konsumierbaren Pilzteilen, aus verarbeitbaren Pilzteilen oder aus Pilzen, erhältlich nach einem erfindungsgemäßen Verfahren. Vorzugsweise umfasst ein solches Verfahren auch den Schritt des Erntens der kultivierten erfindungsgemäßen Pilzzellen, der erfindungsgemäßen Pilze, des erfindungsgemäßen Vermehrungsmaterials, der erfindungsgemäßen konsumierbaren Pilzteile, der erfindungsgemäßen verarbeitbaren Pilzteile vor der Extraktion des Hyaiuronans und besonders bevorzugt ferner den Schritt der Kultivierung erfindungsgemäßer Pilzzellen oder erfindungsgemäßer Pilze vor dem Ernten.

Im Gegensatz zu bakteriellen oder tierischen Geweben weisen Pilzgewebe keine Hyaluronidasen auf und enthalten keine Hyaladherine. Daher ist wie oben bereits beschrieben die Extraktion von Hyaluronan aus Pilzgeweben mit Hilfe relativ einfacher Verfahren möglich. Die oben beschriebenen wässrigen Extrakte aus pilzlichen Zellen oder Geweben, enthaltend Hyaluronan, können bei Bedarf mit dem Fachmann bekannten Methoden, wie z.B. von mehrfach hintereinander folgenden Fällungen mit Ethanol, weiter aufgereinigt werden

Gegenstand der vorliegenden Erfindung ist auch die Verwendung erfindungsgemäßer Pilzzellen, erfindungsgemäßer Pilze, erfindungsgemäßen Vermehrungsmaterials, ,erfindungsgemäßen verarbeitbaren Pilzteilen, erfindungsgemäßen konsumierbaren Pilzteilen oder Pilzen, erhältlich nach einem erfindungsgemäßen Verfahren, zur Herstellung von Hyaluronan.

Ein weiterer Gegenstand der vorliegenden Erfindung betrifft Zusammensetzungen, enthaltend Bestandteile erfindungsgemäßer Pilzzellenzellen, erfindungsgemäßer Pilze, erfindungsgemäßen Vermehrungsmaterials, erfindungsgemäßer verarbeitbarer Pilzteile, erfindungsgemäßer konsumierbarer Pilzteile oder von Pilzen, erhältlich nach einem erfindungsgemäßen Verfahren. Bevorzugt handelt es sich bei den Zusammensetzungen um Nahrungs-, Nahrungsergänzungs- oder Futtermittel, um pharmazeutische oder um kosmetische Produkte.

Wie oben bereits ausgeführt können erfindungsgemäße Pilzzellen, erfindungsgemäße Pilze, erfindungsgemäßes Vermehrungsmaterial, erfindungsgemäße erntebare Pilzteile, erfindungsgemäße verarbeitbare Pilzteile, erfindungsgemäße konsumierbare Pilzteile oder Pilze, erhältlich nach einem erfindungsgemäßen Verfahren verwendet werden, um Nahrungs- oder Futtermittel herzustellen. Aber auch die Verwendung als Rohstoff für technische Anwendungen ist möglich, ohne dass Hyaluronan isoliert werden muss. So können z.B. erfindungsgemäße Pilze bzw. Teile von erfindungsgemäßen Pilzen auf Ackerflächen aufgebracht werden, um eine erhöhte Wasserbindung des Bodens zu erreichen. Weiterhin ist eine Verwendung von erfindungsgemäßen Pilzen oder erfindungsgemäßen Pilzzellen zur Herstellung von Trocknungsmitteln (z.B. für die Verwendung beim Versand von Gegenständen, die empfindlich auf Feuchtigkeit reagieren) oder als Absorber von Flüssigkeiten (z.B. in Babywindeln, oder zum Aufsaugen ausgelaufener wässriger Flüssigkeiten) möglich. Für solche Anwendungen können je nach Bedarf ganze erfindungsgemäße Pilze, Teile von erfindungsgemäßen Pilzen oder zerkleinerte (z.B. zermahlene) erfindungsgemäße Pilze oder erfindungsgemäße Pilzteile Verwendung finden.

Weiterhin sind Gegenstand der vorliegenden Erfindung Verfahren zur Herstellung einer erfindungsgemäßen Zusammensetzung, worin erfindungsgemäße Pilzzellen, erfindungsgemäße Pilze, erfindungsgemäßes Vermehrungsmaterial, erfindungsgemäße verarbeitbare Pilzteile, erfindungsgemäße konsumierbare Pilzteile oder Pilze, erhältlich nach einem erfindungsgemäßen Verfahren, verwendet werden. Bevorzugt handelt es sich bei den Verfahren zur Herstellung einer erfindungsgemäßen Zusammensetzung um Verfahren zur Herstellung von Nahrungs- oder Futtermitteln, Verfahren zur Herstellung zur Herstellung eines pharmazeutischen oder Verfahren zur Herstellung zur Herstellung eines kosmetischen Produktes.

Verfahren zur Herstellung von Lebens- oder Futtermitteln sind dem Fachmann bekannt. Verfahren für die Verwendung von Pilzen oder Pilzteilen auf technischen Gebieten sind dem Fachmann ebenfalls bekannt. Einige Vorteile, die sich aus der Verwendung erfindungsgemäßer Gegenstände für die Herstellung von Nahrungs-/Futtermitteln oder für den Einsatz in technischen Gebieten ergeben, sind bereits oben beschrieben worden.

Die vorliegende Erfindung betrifft auch die Verwendung von erfindungsgemäßen Pilzzellen, erfindungsgemäßen Pilzen, erfindungsgemäßem Vermehrungsmaterial, erfindungsgemäßen verarbeitbaren Pilzteilen, erfindungsgemäßen konsumierbaren Pilzteilen oder Pilzen, erhältlich nach einem erfindungsgemäßen Verfahren zur Herstellung eines Pilzes, zur Herstellung einer erfindungsgemäßen Zusammensetzung. Bevorzugt handelt es sich um die Verwendung von erfindungsgemäßen Pilzzellen, erfindungsgemäßen Pilzen, erfindungsgemäßem Vermehrungsmaterial, erfindungsgemäßen verarbeitbaren Pilzteilen, erfindungsgemäßen konsumierbaren Pilzteilen oder von Pilzen, erhältlich nach einem erfindungsgemäßen Verfahren zur Herstellung eines erfindungsgemäßen Pilzes, für die Herstellung von Lebens- oder Futtermitteln, für die Herstellung eines Pharmazeutikums oder für die Herstellung eines kosmetischen Produktes.

Es ist auch Aufgabe der vorliegenden Erfindung, Mittel, wie z.B. DNA Moleküle zur Erzeugung von erfindungsgemäßen Pilzzellen und erfindungsgemäßen Pilzen, zur Verfügung zu stellen.

Daher sind weiterhin Gegenstand der vorliegenden Erfindung rekombinante Nucleinsäuremoleküle, umfassend eine Nucleinsäuresequenz, die eine Hyaluronansynthase codiert und eine Nucleinsäuresequenz, die die Initiation der Transkription (Promotor) in einer Pilzzelle der systematischen Abteilung Basidiomycota bewirkt.

Unter dem Begriff "rekombinantes Nukleinsäuremolekül" soll im Zusammenhang mit der vorliegenden Erfindung ein Nukleinsäuremolekül verstanden werden, welches neben Nucleinsäuremolekülen, die eine Hyaluronansynthase codieren, zusätzliche Sequenzen enthält, welche natürlicherweise nicht in einer Kombination vorliegen, wie sie in erfindungsgemäßen rekombinanten Nucleinsäuren vorliegen. Die genannten zusätzlichen Sequenzen können dabei beliebige Sequenzen sein, bevorzugt handelt es sich dabei um Sequenzen verschiedener funktionaler Elemente (Promotoren, Terminationssequenzen bestehend aus Polyadenylierungssequenzen und/oder Transkriptionsblockern, Intronsequenzen, Enhancer,), besonders bevorzugt um regulatorische Sequenzen (Promotoren), die in Pilzzellen aktiv sind, insbesondere bevorzugt um gewebespezifische regulatorische Sequenzen die in Fruchtkörper Gewebe von Pilzen aktiv sind. Methoden zur Erzeugung erfindungsgemäßer rekombinanter Nucleinsäuremoleküle sind dem Fachmann bekannt und umfassen gentechnische Methoden wie z.B. die Verbindung von Nucleinsäuremolekülen durch Ligation, genetische Rekombination oder die Neusynthese von Nucleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition ( 2002),ISBN: 0471250929).

In einer bevorzugten Ausführungsform umfasst das rekombinante Nucleinsäuremolekül einen fruchtkörperspezifischen Promotor und/oder mindestens eine Intronsequenz.

In einer weiteren bevorzugten Ausführungsform umfassen rekombinante erfindungsgemäße Nucleinsäuremoleküle auch Terminationssequenzen. Die Terminationssequernzen können dabei Nucleinsäuresequenzen, die eine oder mehrere Erkennungssequenz(en) für die Polyadenylierung eines RNA Transkriptes (Polyadenylierungssignal) und/oder eine oder mehrere Nucleinsäuresequenz(en), die die Funktion eines Trankriptionsblockers (Pause Signal) aufweisen, enthalten.

Eine weitere Ausführungsform von erfindungsgemäßen rekombinanten Nucleinsäuremolekülen der vorliegenden Erfindung sind Vektoren, insbesondere Plasmide, Cosmide, Virengenome, Bacteriophagengenome und andere in der Gentechnik gängige Vektoren, die erfindungsgemäße Nucleinsäuremoleküle enthalten. Bevorzugt handelt es sich hierbei um Vektoren, Plasmide, Cosmide oder Virengenome, die zur Transformation von Pilzzellen geeignet sind. Insbesondere bevorzugt führt die Transformation von Pilzzellen oder Pilzen mit Hilfe erfindungsgemäßer rekombinanter Nucleinsäuremoleküle zur stabilen Integration einer Hyaluronansynthase codierenden Nucleinsäuresequenz in das Genom der Pilzzelle bzw. des Pilzes.

Erfindungsgemäße rekombinante Nucleinsäuremoleküle können auch so genannte Selektionsmarker enthalten, mit deren Hilfe auf Pilzzellen oder Pilze siektiert werden kann, die das rekombinante Nucleinsäuremolekül aufweisen. Selektionsmarker für die Transformation von Pilzzellen sind dem Fachmann bekannt. Es kann sich dabei um so genannte auxotrophe Selektionsmarker, die das Wachstum der transformierten Pilzzelle oder des Pilzes auf einem definierten Medium gewährleisten (Burns et al. (2005, Fungal Gentics and Biology, in Press, prebubished online at www.sciencedirect.com, 05.01.2005, doi:10.1016/j.fgb.2004.11.005; Ogawa et al., 1998, Appl Microbiol Biotechnol 49, 285-289; Noel und Labarère, 1994, Curr Genet 25, 432-437), um Selektionsmarker, die Resistenz gegenüber einem Antibiotikum vermitten (Schuren und Wessels, 1994, Curr Genet 26, 179-183; Hanif et al., 2002, Curr Genet 41, 183.188; Chen et al., 2000, Applied and Environmental Microbiology 66(10), 4510-4513; Sato et al., 1998, Biosci. Biotechnol. Biochem. 62(12), 2346-2350), um Selektionsmarker, die Resistenz gegen ein Herbizid vermitteln (Yanai et al., 1996, Biosci. Biotechnol. Biochem. 60(3), 472-475; Sun et al., Plant Molecular Biology Reporter 19, 383a-383j) oder um Selektionsmarker, die Resistenz gegen ein Fungizid vermitteln (Honda et al., 2000, Curr Genet 37, 209-212), handeln. Weiterhin ist auch die Verwendung von so genannten Marker Proteinen, wie z.B. von fluoreszierenden Proteinen (Sun et al., Plant Molecular Biology Reporter 19, 383a-383j; Burns et al. (2005, Fungal Gentics and Biology, in Press, prebubished online at www.sciencedirect.com, 05.01.2005, doi:10.1016/j.fgb.2004.11.005; MA et al., 2001, Applied and Environmental Microbiology 67(2), 948-955Lugones et al., Molecular Biology 32(4), 681-689) oder der beta-Glucoronidase (Sun et al., 2002, Molecular Biotechnology 20, 239-244; Sun et al., Plant Molecular Biology Reporter 19, 383a-383j; Yanai et al., 1996, Biosci. Biotechnol. Biochem. 60(3), 472-475).
Die Sequenz codierend den Selektionsmarker kann dabei auch Modifikationen enthalten, so dass eine verbesserte Expression (Transkription und/oder Translation) in der betreffenden Pilzzelle erreicht wird (Scholtmeijer et al., Applied and Environmental Microbiology 67(1), 481-483; Burns et al. (2005, Fungal Gentics and Biology, in Press, prebubished online at www.sciencedirect.com, 05.01.2005, doi:10.1016/j.fgb.2004.11.005; MA et al., 2001, Applied and Environmental Microbiology 67(2), 948-955Lugones et al., Molecular Biology 32(4), 681-689).

In weiteren Ausführungsformen betrifft die voriiegende Erfindung erfindungsgemäße rekombinante Nucleinsäuremoleküle, wobei die Nucleinsäuresequenz, die eine Hyaluronansynthase codiert, ausgesucht ist aus der Gruppe bestehend aus:
a) Nucleinsäuremoleküle, dadurch gekennzeichnet, dass sie eine Hyaluronansynthase Klasse I codieren,
b) Nucleinsäuremoleküle, dadurch gekennzeichnet, dass sie eine vertebraten Hyaluronansynthase oder eine virale Hyaluronansynthase codieren,
c) Nucleinsäuremoleküle, dadurch gekennzeichnet, dass sie eine Hyaluronansynthase aus Amphibien oder eine Hyaluronansynthase eines Virus, der Algen infiziert codieren,
d) Nucleinsäuremoleküle, dadurch gekennzeichnet, dass sie eine Hyaluronansynthase eines Chlorella infizierenden Virus oder eine Hyaluronansynthase aus einem Frosch codieren,
e) Nucleinsäuremoleküle, dadurch gekennzeichnet, dass sie eine Hyaluronansynthase eines *Paramecium bursaria Chlorella* Virus 1 oder eine Hyaluronansynthase aus *Xenopus laevis* codieren,
f) Nucleinsäuremoleküle, dadurch gekennzeichnet, dass die Codons des Nucleinsäuremoleküls codierend eine Hyaluronansynthase verändert sind, im Vergleich zu den Codons des Nucleinsäuremoleküls, welches die Hyaluronansynthase des Ursprungsorganismus der Hyaluronansynthase codieren,
g) Nucleinsäuremoleküle, dadurch gekennzeichnet, dass die Codons der Hyaluronansynthase dahingehend verändert sind, dass sie an die Häufigkeit der Verwendung der Codons der Pilzzelle, in dessen Genom sie integriert werden oder sind, angepasst sind,
h) Nucleinsäuremoleküle, dadurch gekennzeichnet, dass sie eine Hyaluronansynthase mit der unter SEQ ID NO 2, SEQ ID NO 4, SEQ ID NO 6, SEQ ID NO 8, SEQ ID NO 10, SEQ ID NO 12, SEQ ID NO 14, SEQ ID NO 16, SEQ ID NO 18, SEQ ID NO 20, SEQ ID NO 22, SEQ ID NO 24, SEQ ID NO 26, SEQ ID NO 28, SEQ ID NO 30, SEQ ID NO 32, SEQ ID NO 34, SEQ ID NO 36, SEQ ID NO 38, SEQ ID NO 40, SEQ ID NO 42, SEQ ID NO 44, SEQ ID NO 46, SEQ ID NO 48, SEQ ID NO 50, SEQ ID NO 52, SEQ ID NO 54, SEQ ID NO 56, SEQ ID NO 58, SEQ ID NO 60, SEQ ID NO 62 oder SEQ ID NO 64 dargestellten Aminosäuresequenz codieren,
i) Nucleinsäuremoleküle, dadurch gekennzeichnet , dass sie ein Protein codieren, dessen Aminosäuresequenz von der codierenden Region der im Plasmid DSM 16664 inserierten Nucleinsäuresequenz abgeleitet werden kann,
j) Nucleinsäuremoleküle, die eine unter SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 53 SEQ ID NO 55, SEQ ID NO 57, SEQ ID NO 59, SEQ ID NO 61 oder SEQ ID NO 63 dargestellte Nucleinsäuresequenz umfassen, oder
k) Nucleinsäuremoleküle, die die in Plasmid DSM16664 inserierte Nucleinsäuresequenz umfassen.

Pilzzellen oder Pilze, die erfindungsgemäße rekombinante Nucleinsäuremoleküle enthalten, sind ebenfalls Gegenstand der vorliegenden Erfindung.

Die Verwendung von erfindungsgemäßen rekombinanten Nucleinsäuren zur Herstellung einer erfindungsgemäßen Pilzzelle oder eines erfindungsgemäßen Pilzes sind ebenfalls Gegenstand der vorliegenden Erfindung.

Weiterhin ist die Verwendung erfindungsgemäßer rekombinanter Nucleinsäuren für die Durchführung erfindungsgemäßer Verfahren zur Herstellung eines Pilzes Gegenstand der vorliegenden Erfindung.

### Beschreibung der Sequenzen

SEQ ID NO 1: Nucleinsäuresequenz, codierend eine Hyaluronansynthase des *Paramecium bursaria Chlorella* Virus 1.
SEQ ID NO 2: Aminosäuresequenz einer Hyaluronansynthase des *Paramecium bursaria Chlorella* Virus 1. Die dargestellte Aminosäuresequenz kann von SEQ ID NO 1 abgeleitet werden.
SEQ ID NO 3: Synthetische Nucleinsäuresequenz, codierend eine Hyaluronansynthase des *Paramecium bursaria Chlorella* Virus 1.
SEQ ID NO 4: Aminosäuresequenz einer Hyaluronansynthase des *Paramecium bursaria Chlorella* Virus 1. Die dargestellte Aminosäuresequenz kann von SEQ ID NO 3 abgeleitet werden.
SEQ ID NO 5: Nucleinsäuresequenz, codierend eine Hyaluronansynthase 3 aus *Homo sapiens.*
SEQ ID NO 6: Aminosäuresequenz einer Hyaluronansynthase 3 aus *Homo sapiens.* Die dargestellte Aminosäuresequenz kann von SEQ ID NO 5 abgeleitet werden.
SEQ ID NO 7: Synthetische Nucleinsäuresequenz, codierend eine Hyaluronansynthase 3 aus *Homo sapiens.*
SEQ ID NO 8: Aminosäuresequenz einer Hyaluronansynthase 3 aus *Homo sapiens.* Die dargestellte Aminosäuresequenz kann von SEQ ID NO 7 abgeleitet werden.
SEQ ID NO 9: Nucleinsäuresequenz, codierend eine Hyaluronansynthase 1 aus *Homo sapiens.*
SEQ ID NO 10: Aminosäuresequenz einer Hyaluronansynthase 1 aus *Homo sapiens.* Die dargestellte Aminosäuresequenz kann von SEQ ID NO 9 abgeleitet werden.
SEQ ID NO 11: Nucleinsäuresequenz, codierend eine Hyaluronansynthase 2 aus *Homo sapiens.*
SEQ ID NO 12: Aminosäuresequenz einer Hyaluronansynthase 2 aus *Homo sapiens.* Die dargestellte Aminosäuresequenz kann von SEQ ID NO 11 abgeleitet werden.
SEQ ID NO 13: Nucleinsäuresequenz, codierend eine Hyaluronansynthase 1 aus *Papio anubis.*
SEQ ID NO 14: Aminosäuresequenz einer Hyaluronansynthase 1 aus *Papio anubis.* Die dargestellte Aminosäuresequenz kann von SEQ ID NO 13 abgeleitet werden.
SEQ ID NO 15: Nucleinsäuresequenz, codierend eine Hyaluronansynthase 1 aus *Mus musculus.*
SEQ ID NO 16: Aminosäuresequenz einer Hyaluronansynthase 1 aus *Mus musculus.* Die dargestellte Aminosäuresequenz kann von SEQ ID NO 13 abgeleitet werden.
SEQ ID NO 17: Nucleinsäuresequenz, codierend eine Hyaluronansynthase 2 aus *Mus musculus.*
SEQ ID NO 18: Aminosäuresequenz einer Hyaluronansynthase 2 aus *Mus musculus.* Die dargestellte Aminosäuresequenz kann von SEQ ID NO 17 abgeleitet werden.
SEQ ID NO 19: Nucleinsäuresequenz, codierend eine Hyaluronansynthase 3 aus *Mus musculus.*
SEQ ID NO 20: Aminosäuresequenz einer Hyaluronansynthase 3 aus *Mus musculus.* Die dargestellte Aminosäuresequenz kann von SEQ ID NO 19 abgeleitet werden.
SEQ ID NO 21: Nucleinsäuresequenz, codierend eine Hyaluronansynthase 1 aus *Rattus norvegicus.*
SEQ ID NO 22: Aminosäuresequenz einer Hyaluronansynthase 1 aus *Rattus norvegicus.* Die dargestellte Aminosäuresequenz kann von SEQ ID NO 21 abgeleitet werden.
SEQ ID NO 23: Nucleinsäuresequenz, codierend eine Hyaluronansynthase 2 aus *Rattus norvegicus.*
SEQ ID NO 24: Aminosäuresequenz einer Hyaluronansynthase 2 aus *Rattus norvegicus.* Die dargestellte Aminosäuresequenz kann von SEQ ID NO 23 abgeleitet werden.
SEQ ID NO 25: Nucleinsäuresequenz, codierend eine Hyaluronansynthase 3 aus *Rattus norvegicus.*
SEQ ID NO 26: Aminosäuresequenz einer Hyaluronansynthase 3 aus *Rattus norvegicus.* Die dargestellte Aminosäuresequenz kann von SEQ ID NO 25 abgeleitet werden.
SEQ ID NO 27: Nucleinsäuresequenz, codierend eine Hyaluronansynthase 2 aus *Oryctolagus cuniculus.*
SEQ ID NO 28: Aminosäuresequenz einer Hyaluronansynthase 2 aus *Oryctolagus cuniculus.* Die dargestellte Aminosäuresequenz kann von SEQ ID NO 27 abgeleitet werden.
SEQ ID NO 29: Nucleinsäuresequenz, codierend eine Hyaluronansynthase 3 aus *Oryctolagus cuniculus.*
SEQ ID NO 30: Aminosäuresequenz einer Hyaluronansynthase 3 aus *Oryctolagus cuniculus.* Die dargestellte Aminosäuresequenz kann von SEQ ID NO 29 abgeleitet werden.
SEQ ID NO 31: Nucleinsäuresequenz, codierend eine Hyaluronansynthase 2 aus *Equus caballus.*
SEQ ID NO 32: Aminosäuresequenz einer Hyaluronansynthase 2 aus *Equus caballus.* Die dargestellte Aminosäuresequenz kann von SEQ ID NO 31 abgeleitet werden.
SEQ ID NO 33: Nucleinsäuresequenz, codierend eine Hyaluronansynthase 2 aus *Sus scrofa.*
SEQ ID NO 34: Aminosäuresequenz einer Hyaluronansynthase 2 aus *Sus scrofa.* Die dargestellte Aminosäuresequenz kann von SEQ ID NO 33 abgeleitet werden.
SEQ ID NO 35: Nucleinsäuresequenz, codierend eine Hyaluronansynthase 3 aus *Sus scrofa.*
SEQ ID NO 36: Aminosäuresequenz einer Hyaluronansynthase 3 aus *Sus scrofa.* Die dargestellte Aminosäuresequenz kann von SEQ ID NO 35 abgeleitet werden.
SEQ ID NO 37: Nucleinsäuresequenz, codierend eine Hyaluronansynthase 2 aus *Bos taurus.*
SEQ ID NO 38: Aminosäuresequenz einer Hyaluronansynthase 2 aus *Bos taurus.* Die dargestellte Aminosäuresequenz kann von SEQ ID NO 37 abgeleitet werden.
SEQ ID NO 39: Nucleinsäuresequenz, codierend eine Hyaluronansynthase 2 aus *Gallus gallus.*
SEQ ID NO 40: Aminosäuresequenz einer Hyaluronansynthase 2 aus *Gallus gallus.* Die dargestellte Aminosäuresequenz kann von SEQ ID NO 39 abgeleitet werden.
SEQ ID NO 41: Nucleinsäuresequenz, codierend eine Hyaluronansynthase 1 aus *Xenopus laevis.*
SEQ ID NO 42: Aminosäuresequenz einer Hyaluronansynthase 1 aus *Xenopus laevis.* Die dargestellte Aminosäuresequenz kann von SEQ ID NO 41 abgeleitet werden.
SEQ ID NO 43: Nucleinsäuresequenz, codierend eine Hyaluronansynthase 2 aus *Xenopus laevis.*
SEQ ID NO 44: Aminosäuresequenz einer Hyaluronansynthase 2 aus *Xenopus laevis.* Die dargestellte Aminosäuresequenz kann von SEQ ID NO 43 abgeleitet werden.
SEQ ID NO 45: Nucleinsäuresequenz, codierend eine Hyaluronansynthase 3 aus *Xenopus laevis.*
SEQ ID NO 46: Aminosäuresequenz einer Hyaluronansynthase 3 aus *Xenopus laevis.* Die dargestellte Aminosäuresequenz kann von SEQ ID NO 45 abgeleitet werden.
SEQ ID NO 47: Nucleinsäuresequenz, codierend eine Hyaluronansynthase 2 aus *Danio rerio.*
SEQ ID NO 48: Aminosäuresequenz einer Hyaluronansynthase 2 aus *Danio rerio.* Die dargestellte Aminosäuresequenz kann von SEQ ID NO 47 abgeleitet werden.
SEQ ID NO 49: Genomische Nucleinsäuresequenz, codierend eine Hyaluronansynthase 3 aus *Danio rerio.*
SEQ ID NO 50: Aminosäuresequenz einer Hyaluronansynthase 3 aus *Danio rerio.* Die dargestellte Aminosäuresequenz kann von SEQ ID NO 49 abgeleitet werden.
SEQ ID NO 51: Nucleinsäuresequenz, codierend eine Hyaluronansynthase aus *Pasteurella multocida.*
SEQ ID NO 52: Aminosäuresequenz einer Hyaluronansynthase aus *Pasteurella multocida.* Die dargestellte Aminosäuresequenz kann von SEQ ID NO 51 abgeleitet werden.
SEQ ID NO 53: Nucleinsäuresequenz, codierend eine Hyaluronansynthase aus *Streptococcus pyogenes.*
SEQ ID NO 54: Aminosäuresequenz einer Hyaluronansynthase aus *Streptococcus pyogenes.* Die dargestellte Aminosäuresequenz kann von SEQ ID NO 53 abgeleitet werden.
SEQ ID NO 55: Nucleinsäuresequenz, codierend eine Hyaluronansynthase aus *Streptococcus equi.*
SEQ ID NO 56: Aminosäuresequenz einer Hyaluronansynthase aus *Streptococcus equi.* Die dargestellte Aminosäuresequenz kann von SEQ ID NO 55 abgeleitet werden.
SEQ ID NO 57: Nucleinsäuresequenz, codierend eine Hyaluronansynthase aus *Streptococcus uberis.*
SEQ ID NO 58: Aminosäuresequenz einer Hyaluronansynthase aus *Streptococcus uberis.* Die dargestellte Aminosäuresequenz kann von SEQ ID NO 57 abgeleitet werden.
SEQ ID NO 59: Nucleinsäuresequenz, codierend eine Hyaluronansynthase aus *Streptococcus equisimilis.*
SEQ ID NO 60: Aminosäuresequenz einer Hyaluronansynthase aus *Streptococcus equisimilis.* Die dargestellte Aminosäuresequenz kann von SEQ ID NO 59 abgeleitet werden.
SEQ ID NO 61: Nucleinsäuresequenz, codierend eine Hyaluronansynthase aus *Sulfolobus tokodaii* Strain 7.
SEQ ID NO 62: Aminosäuresequenz einer Hyaluronansynthase aus *Sulfolobus tokodaii* Strain 7. Die dargestellte Aminosäuresequenz kann von SEQ ID NO 61 abgeleitet werden.
SEQ ID NO 63: Synthetische Nucleinsäuresequenz, codierend eine Hyaluronansynthase 1 aus *Xenopus laevis.*
SEQ ID NO 64: Aminosäuresequenz einer Hyaluronansynthase 1 aus *Xenopus laevis.* Die dargestellte Aminosäuresequenz kann von SEQ ID NO 3 abgeleitet werden.
SEQ ID NO 65: Synthetisch hergestellte Nucleinsäuresequenz, umfassend die in Beispiel 2 beschriebenen funktionellen Elemente gpd-promotor (bp 16-269, Polylinker *(Pac I, Kpn I, Spe I, BamH 1;* bp 271-296), Intron (bp 298-352), mnp- Polyadenylierungssignal I (bp356-491), Poly-Adenylierungssignal II (bp 492-540), Transkriptionsblocker (Pause, bp 541-632).

### Beschreibung der Abbildungen

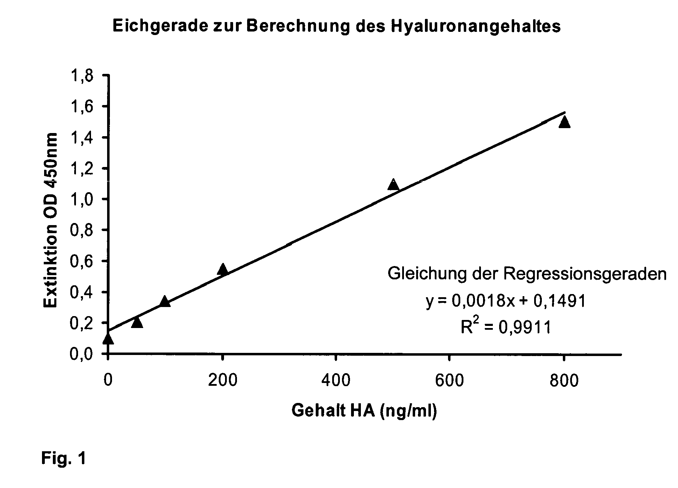

- Fig.1:: Stellt eine Eichgerade und die dazugehörige Gleichung der Regressionsgeraden dar, welche zur Berechnung des Hyaluronangehaltes in Pilzgewebe verwendet wurde. Die Eichgerade wurde mit Hilfe des käuflich erwerbbaren Testkits (Hyaluronic Acid (HA) Test Kit der Firma Corgenix, Inc., Colorado, USA, Prod. Nr. 029-001) und den darin mitgelieferten Standardlösungen erstellt.

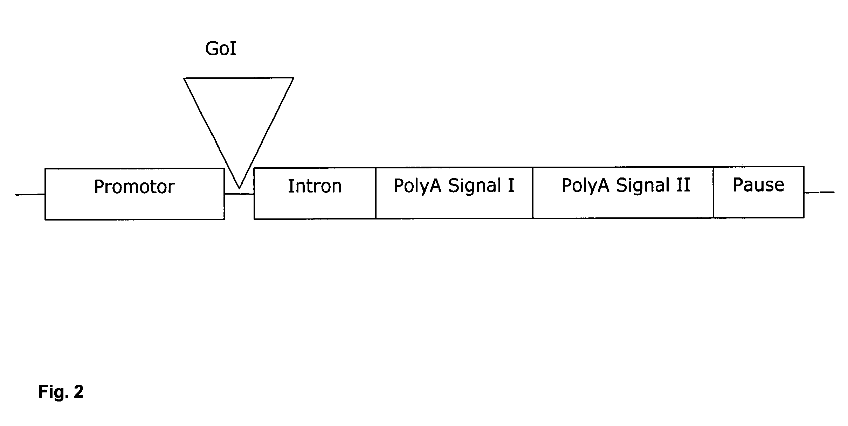

- Fig. 2:: Stellt schematisch die Anordnung der funktionellen Elemente der synthetisch hergestellten Sequenz enthaltend einen Promotor (Promotor), einen Polyinker mit unterschiedlichen Restriktionsendonuclease Schnittstellen, ein Intron (Intron) sowie eine Terminationssequenz dar. In den Polylinker kann eine gewünschte Nucleinsäuresequenz (Gol), codierend ein zu exprimierendes Protein eingefügt werden. Betreffend die vorliegende Erfindung wurden in die mit Gol gekennzeichnete Region Nucleinsäuresequenzen codierend Hyaluronansynthasen eingefügt.

### Allgemeine Methoden

Im Folgenden werden Methoden beschrieben, die im Zusammenhang mit der vorliegenden Erfindung verwendet werden können. Diese Methoden stellen konkrete Ausführungsformen dar, beschränken die vorliegende Erfindung jedoch nicht auf diese Methoden. Dem Fachmann ist bekannt, dass er durch Modifikation der beschriebenen Methoden und/oder durch Ersetzen einzelner Methoden bzw.

Methodenteile durch alternative Methoden bzw. alternative Methodenteile die Erfindung in gleicher Weise ausführen kann.

### 1. Transformation von Pilzen

Pilze *(Agaricus bisporus)* wurden mit Hilfe von *Agrobacterium,* wie bei Chen et al. (2000, Applied and Environmental Microbiology 66(10), 4510-4513) beschrieben, transformiert.

### 2. Kultivierung von Pilzen

Vegetatives Mycel von *Agaricus bisporus* wurde auf sterilem PDY Agar bei 24°C kultiviert (Romaine und Schlagnhaufer, 1992, Appl. Environ. Microbiol. 58(9), 3060-3066).
*Agaricus bisporus* Fruchtkörper wurden auf sterilem Kompost nach der bei Romaine und Schlagnhaufer (1992, Appl. Environ. Microbiol. 58(9), 3060-3066) beschriebenen Methode produziert.

### 3. Isolierung von Hyaluronan aus Pilzewebe

Pilzmaterial wurde zum Nachweis des Vorliegens von Hyaluronan und zur Bestimmung des Hyaluronangehaltes in Pilzgewebe folgendermaßen aufgearbeitet: Zu ca. 0,3 g Gewebe von Mycel oder Fruchtkörper wurden 200 µl Wasser (demineralisiert, Leitfähigkeit ≥18 MΩ) zugegeben und mit einer Labor-Schwingkugelmühle (MM200, Firma Retsch, Germany) zerkleinert (30 sec. bei 30 HZ). Anschließend erfolgte eine weitere Zugabe von 800 µl Wasser (demineralisiert, Leitfähigkeit ≥18 MΩ) und eine gute Durchmischung (z.B. mit Hilfe eines Vortex). Die Zelltrümmer und unlösliche Bestandteile wurden vom Überstand durch 5 minütiges Zentrifugieren bei 16000xg abgetrennt.

### 4. Nachweis von Hyaluronan und Bestimmung des Hyaluronangehaltes

Der Nachweis von Hyaluronan erfolgt mit Hilfe eines käuflich erwerbbaren Tests (Hyaluronic Acid (HA) Test Kit der Firma Corgenix, Inc., Colorado, USA, Prod. Nr. 029-001) nach den Angaben des Herstellers, die durch Bezugnahme hiermit als Gegenstand in die Beschreibung aufgenommen sind. Das Testprinzip beruht auf der Verfügbarkeit eines spezifisch an Hyaluronan bindenden Proteins (HABP) und erfolgt ähnlich einem ELISA, wobei eine Farbreaktion den Hyaluronangehalt in der untersuchten Probe anzeigt. Die zu vermessenden Proben sollten daher für die quantitative Bestimmung von Hyaluronan in solch einer Konzentration eingesetzt werden (z.B: verdünnen der jeweiligen Probe oder Verwendung von weniger Wasser für die Extraktion von Hyaluronan aus dem Pilzgewebe, je nachdem ob ein Grenzwert über- oder unterschritten wurde), dass sie innerhalb der angegebenen Grenzen liegen.
In parallelen Ansätzen werden Aliquots der zu bestimmenden Proben zunächst einem Hyaluronidaseverdau unterzogen, bevor sie mit dem käuflich erwerbbaren Test (Hyaluronic Acid (HA) Test Kit der Firma Corgenix, Inc., Colorado, USA, Prod. Nr. 029-001) vermessen werden. Der Hyaluronidaseverdau erfolgt mit 400 µl Pilzextrakt in Hyaluronidase Puffer (0,1 M Kalium Phosphatpuffer, pH 5,3; 150 mm NaCl) durch Zugabe von 5 µg (~3 units) Hyaluronidase (Hyaluronidase Typ III from Sigma, Prd. Nr. H 2251) bei einer Inkubation von 30 Minuten bei 37 °C.
Anschließend werden alle Proben jeweils für die Bestimmung des Hyaluronangehaltes eingesetzt.

### 5. Nachweis von Hyaluronan mit Hilfe von NMR-Spektroskopie

Die NMR spektroskopische Analyse kann mit einem DRX 700 Spektrometer bei 700MHz durchgeführt (Bruker Biospin GMBH D-76287 Rheinstetten/Karlsruhe) werden. Das Spektrometer war mit einem TXI-Prüfkopf ausgestattet und einer SGI "workstation", wobei für die Auswertung die Bruker Biospin Software XWIN-NMR version 3.5 genutzt wurde. Etwa 0,5 mg bis 2 mg der Probe wurde in 550 ul D₂O gelöst. The ¹H-NMR Spectren werden mit 1024 bis 12000 "Scans" aufgenommen und einer Relaxationszeit von 1 sec. Die ¹H NMR Spektren werden auf das Wasser Signal bei 4,7 ppm bezogen.

### 6. Molekuargewichtsanalysen von Hyaluronan

a) Agarosegelelektrophorese
   Zur Größencharakterisierung des Hyaluronans, isoliert aus Pilzen, wird ein auf Agarosegelelektrophorese basierendes System genutzt das bei Lee und Cowman (1994, Anal.Biochem. 219, 278-287) oder Armstrong und Bell (2002, Anal. Biochem. 308, 255-264) beschrieben ist. Hierfür werden Hyaluronan enthaltende Proben auf ein 0,7% TAE (40mM Tris, 5mM Natrium Acetat, 0.8mM EDTA, pH 7.9) Agarosegel aufgetragen und in 1x TAE Puffer über 3 Stunden bei 50 V aufgetrennt. Anschließend erfolgt eine Färbung des Agarosegels über Nacht mit 0,005% Stainsall (3,3'-Diethyl-9-methyl-4,5,4',5'-dibenzothiacarbocyanine, Fluka, Prod. Nr. 85663) in 50% Ethanol und 50% 1xTAE Puffer, bevor das Gel in Wasser entfärbt und eingescannt wird.
b) Gel Permeations Chromatographie (GPC)
   Die Proben werden in einer Konzentration von 1 mg/ml⁻¹ im GPC-Laufmittel (0,2 M NaNO₃) gelöst. Dazu wird zunächst 1 Stunde auf einem Magnetrührwerk gerührt und anschließend 20 Stunden zur Gleichgewichtseinstellung bei Raumtemperatur stehen gelassen. Vor der Messung wird die Proben durch einen 5 µm Membranfilter filtriert. Anschließend werden die Proben mittels GPC analysiert, wobei der Refraktionsindex, die Lichtstreuung und die Viscosität des Eluates bestimmt werden. Es können folgende Geräte und Materialien benutzt werden:

### GPC-Bedingungen:

Geräte: Gelchromatograph PL120 der Firma Polymer Laboratories, Midas Autosampler von Spark,
   DAWN-EOS-Lichtstreudetektor von Wyatt Technology Santa Barbara mit λ₀= 690 nm und 16 Detektoren im Winkelbereich von 14,9° bis 162,9°,
   K5-Durchflusszelle,
   Viskositäts-Brechungsindex-Kombinationsdetektor η-1002 (WEG Dr. Bures GmbH & Co KG).
Säulen: SUPREMA-Gel von PSS, Mainz
   Vorsäule sowie drei Säulen mit den Trennbereichen 300 bis 10⁴; 5·10⁴ bis 2·10⁶ und 10⁶ bis 10⁸ wurden in Reihe geschaltet.
Elution: Laufmittel 0,2 M NaNO₃, Flussrate 0,8 ml/Minute, Temperatur 30°C, Injektionsvolumen 500µl
Auswertung:
   Aus den erhaltenen Daten werden die in den Beispielen angegebenen Werte errechnet. Die Lichtstreudaten können mit der Software ASTRA Software 4.90.08 ausgewertet werden. Die Viskositätsmessungen können über die PSS Win GPC 6 ausgewertet werden.

### Beispiele

### 1. Angaben zu Vektoren und Plasmiden

### Herstellung des Expressionsvektors IC 400-271

Das Plasmid IC 400-271 ist ein Derivat des binären Vektorplasmids pBHg (Chen et al, 2000, Appl. Environ. Microbiol. 66, 4510-4513), das folgendermaßen konstruiert wurde:
Das Plasmid pBGh wurde mit den Restriktionsendonuclease *BamH* / geschnitten, die Enden mit Klenow-Polymerase geglättet, und das erhaltene Fragment wurde anschließend erneut mit der Restriktionsendonuclease *Sma I* geschnitten. Anschließend wurde der Vektor wieder ligiert. Mittels dieses Vorgehens wurde ein Teil des Polylinkers des Plasmids pBGh deletiert, wobei der für Pilze geeignete Expressionsvektor IC 400-271 entstand.

### 2. Synthese der Sequenzen, codierend für einen Promotor, einen Polylinker, ein Intron und eine Terminatinssequenz,

Die Nucleinsäuresequenz, umfassend den Promotor des Proteins Glyceraldehyd-3-Phosphat Dehydrogenase *(gpd)* aus *Agaricus bisporus* (Harmsen at al., 1992, Curr. Genet. 22, 447-454), einen Polyinker mit unterschiedlichen Restriktionsendonuclease Schittstellen *((Pac I, Kpn I, Spe I, BamH I),* ein Intron mit entsprechender Donor und Akzeptor Erkennungssequenz (Ma et al., 2001, Appl. Environ. Microbiol. 67, 948-955) sowie eine Terminationssequenz enthaltend ein erstes Polyadenylierungssignal des Mangan Peroxidase isoenzyms 1 *(mnp1,* GenBank Acc: J04621) aus *Phanerochaete chrysosporium* (Pribnow et al., 1989, J. Biol. Chem. 264, 5036-5040), ein zweites Polyadenylierungssignal (Levitt et al.,Genes & Dev.,1989, 3, 1019 - 1025) und einen Transkriptionsblocker (Pause Signal, Enriquez-Harris et al., EMBO Journal, 1991, 10, 1833-1842) wurde von der Firma Entelechon GmbH synthetisiert und in den Vektor pCR4Topo der Firma Invitrogen (Prod. Nr. K4510-20) kloniert. Der Aufbau ist schematisch in Fig. 2 dargestellt. Das erhaltene Plasmid wurde mit IC 401-271 bezeichnet. Die synthetische Nucleinsäuresequenz für die beschriebenen Elemente, ist unter SEQ ID NO 65 dargestellt.

### 3. Synthese der Nucleinsäuresequenzen, codierend ein HAS Protein des Paramecium bursaria Chlorella Virus 1

Die Nucleinsäuresequenz, codierend ein HAS (Hyaluronansynthase) Protein aus *Paramecium bursaria Chlorella* Virus 1, wurde von der Firma Medigenomix GmbH (München, Deutschland) synthetisiert und in den Vektor pCR2.1 der Firma Invitrogen (Prod. Nr. K2000-01) kloniert. Das erhaltene Plasmid wurde mit IC 323-215 bezeichnet. Die synthetische Nucleinsäuresequenz codierend das HAS Protein aus *Paramecium bursaria Chlorella* Virus 1, ist unter SEQ ID NO 3 dargestellt. Die korrespondierende, ursprünglich aus dem *Paramecium bursaria Chlorella* Virus 1 isolierte Nucleinsäuresequenz, ist unter SEQ ID NO 1 dargestellt.

### 4. Herstellung eines Expressionsvektors, mit der codierenden Sequenz für ein HAS Proteins aus Paramecium bursaria Chlorella virus 1

Aus dem Plasmid IC 323-215 wird mittels der Restriktionsendonucleasen *BamHl* und *Pacl* die codierende Sequenz des HAS Proteins isoliert und in die *BamHl* und *Pa*cl Schnittstellen von IC 401-271 kloniert. Dabei entstand der Vektor IC 402-271. Aus IC 402-271 wurde nun mit *Hpal* und *Sbfl* die Kassette gpd Promotor - HAS Protein - Intron - Terminationssequnez in die *Pvull* und *Sbfl* Restriktionsschnittstellem des Vektor IC 400-271 kloniert. Somit entstand der Expressionsvektor IC 403-271.

### 5. Synthese der Sequenzen, codierend für ein HAS1 (DG42) Protein aus Xenopus laevis

Die Nucleinsäuresequenz, codierend ein xlHAS (Hyaluronansynthase 1) Protein aus Xenopus laevis, wurde von der Firma Entelechon GmbH synthetisiert und den Vektor pCR4Topo der Firma Invitrogen (Prod. Nr. K4510-20) kloniert. Das erhaltene Plasmid wurde mit IC 406-271 bezeichnet. Die synthetische Nucleinsäuresequenz codierend das synthetische HAS Protein aus Xenopus laevis, ist unter SEQ ID NO 63 dargetsellt. Die korrespondierende, ursprünglich aus Xenopus laevis isolierte Nucleinsäuresequenz (GenBank M22249), ist unter SEQ ID NO 41 dargestellt.

### 6. Transformation von Pilzen, mit Expressionsvektoren, die Nucleinsäuremoleküle enthalten, die HAS Proteine codieren

Lamellengewebe von Fruchtkörpern von *Agaricus bisporus* wurden in unabhängigen Transformationen mit dem Expressionsvektor IC 403-271, enthaltend eine codierende Nucleinsäuresequenz für ein HAS Protein aus *Paramecium bursaria Chlorella* Virus 1 bzw. mit dem Expressionsvektor IC 406-271, enthaltend eine codierende Nucleinsäuresequenz für ein HAS-1 Protein aus *Xenopus laevis* nach der unter Allgemeine Methoden Punkt 1 angegebnen Methode transformiert. Die erhaltenen transgenen Pilzle, die mit dem Plasmid IC 403-271 transformiert waren, wurden mit A.b.-cvHAS bezeichnet. Die erhaltenen transgenen Pilze, die mit dem Plasmid IC 406-271 transformiert waren, wurden mit A.b.-xIHAS1 bezeichnet.

### 7. Analyse der transgenen Pilze

Einzelne Pilz mit der Bezeichnung A.b.-cvHAS und mit der Bezeichnung A.b.-xIHAS1 wurden als Mycel nach dem unter Punkt 2, Allgemeine Methoden beschriebenen Verfahren auf PDY Agar kultiviert. Ca. jeweils 0,3 g Material des Mycels der einzelnen Pilze wurden nach der unter Allgemeine Methoden Punkt 3 beschriebenen Methode aufgearbeitet.
Fruchtkörper von Pilzen mit der Bezeichnung A.b.-cvHAS und von Pilzen mit der Bezeichnung A.b.-xIHAS1 wurden nach dem unter Allgemeine Methoden, Punkt 2 beschriebenen Verfahren auf sterilem Kompost hergestellt. Ca. jeweils 0,3 g Material von Fruchtkörpern der einzelnen Pilze wurde nach der unter Allgemeine Methoden Punkt 3 beschriebenen Methode aufgearbeitet.
Hyaluronan in den jeweiligen Pilzextrakten wurde mit der unter Allgemeine Methoden Punkt 4. beschriebenen Methode bestimmt. Es konnten Pilzlinien identifiziert werden, die Hyaluronan produzierten.

## Patentansprüche

1. Pilzzelle, **dadurch gekennzeichnet, dass** sie ein Nucleinsäuremolekül, codierend eine Hyaluronansynthase, aufweist und dass sie Hyaluronan synthetisiert.

2. Pilzzelle, die von einer Pilzzelle nach Anspruch 1 abstammt, **dadurch gekennzeichnet, dass** sie besagtes Nucleinsäuremolekül, codierend eine Hyaluronansynthase, aufweist.

3. Pilz enthaltend Pilzzellen nach einem der Ansprüche 1 oder 2.

4. Vermehrungsmaterial eines Pilzes nach Anspruch 3, **dadurch gekennzichnet, dass** es besagtes Nucleinsäuremolekül, codierend eine Hyaluronansynthase, aufweist.

5. Verfahren zur Herstellung eines Pilzes, der Hyaluronan synthetisiert, worin
a) ein Nucleinsäuremolekül, codierend eine Hyaluronansynthase in das Genom einer Pilzzelle integriert wird
b) aus der Pilzzelle von Schritt a) ein Pilz regeneriert wird ; und
c) gegebenenfalls weitere Pilze mit Hilfe des Pilzes nach Schritt b) erzeugt werden.

6. Verfahren zur Herstellung von Hyaluronan, umfassend den Schritt der Extraktion von Hyaluronan aus Pilzzellen nach einem der Ansprüche 1 oder 2, aus Pilzen nach Anspruch 3, oder aus Pilzen, erhältlich nach einem Verfahren nach Anspruch 5.

7. Verwendung einer Pilzzelle nach einem der Ansprüch 1 oder 2, eines Pilzes nach Anspruch 3, von Vermehrungsmaterial nach Anspruch 4 oder eines Pilzes, erhältlich nach einem Verfahren nach Anspruch 5 zur Herstellung von Hyaluronan.

8. Zusammensetzung enthaltend Bestandteile von Pilzzellen nach einem der Ansprüche 1 oder 2, eines Pilzes nach Anspruch 3, oder eines Pilzes, erhältlich nach einem Verfahren nach Anspruch 5.

9. Verfahren zur Herstellung einer Zusammensetzung nach Anspruch 8, worin Pilzzellen nach einem der Ansprucüche 1 oder 2, Pilze nach Anspruch 3 oder ein Pilz, erhältlich nach einem Verfahren nach Anspruch 5 verwendet werden.

10. Verwendung von Pilzzellen nach einem der ansprüche 1 oder2, von Pilzen nach Anspruch 3, von Pilzen, erhältlich nach einem Verfahren nach Anspruch 5, zur Herstellung einer Zusammensetzung nach Anspruch 8.

11. Rekombinantes Nucleinsäuremolekül, umfassend eine Nucleinsäuresequenz, die die Initiation des Transkription in einer Pilzzelle der systematischen Abteilung Basidiomycota bewirkt und ein Nucleinsäuremolekül, welches eine Hyaluronansynthase codiert.
